# EUROPEAN PATENT APPLICATION

(11) **EP 2 671 584 A2**
(43) Date of publication of application: **11.12.2013**
(21) Application number: 13171506.2
(22) Date of filing: 23.04.2008
(51) Int. Cl.: A61K 31/4706, A61K 31/675, A61K 38/04, A61P 9/00

(54) **Compositions and methods for treating disorders associated with salt or fluid retention**

(30) Priority: 04.05.2007 US 916257 P; 17.10.2007 US 980573 P
(62) Divisional of application: 08746595.1
(71) Applicant: Ironwood Pharmaceuticals, Inc., Cambridge, MA 02141 (US)
(72) Inventor: Currie, Mark G., Sterling, MA Massachusetts 01564 (US); Zimmer, Daniel P., Somerville, MA Massachusetts 02145 (US)
(74) Representative: Simpson, Tobias Rutger

(57) **Abstract**

Methods for reducing the risk of or treating a disorder associated with fluid and/or salt retention in a patient are described. The methods include administering to the patient an agent selected from: a) an agent that reduces sodium absorption in the intestine; b) an agent that increases anion secretion in the intestine; or c) an agent that both reduces sodium absorption in the intestine and increases anion secretion in the intestine.

SEQUENCE LISTING

<110>  Ironwood Pharmaceuticals, Inc.

<120>  Compositions and Methods for Treating Disorders Associated with
       Salt or Fluid Retention

<130>  P34400EP-D1-PCT

<140>  EP13171506.2
<141>  2008-04-23

<150>  US 60/916,257
<151>  2007-05-04

<150>  US 60/980,573
<151>  2007-10-17

<160>  69

<170>  PatentIn version 3.5

<210>  1
<211>  14
<212>  PRT
<213>  Artificial Sequence

<220>
<223>  Synthetically generated peptide

<400>  1

Cys Cys Glu Leu Cys Cys Asn Pro Ala Cys Thr Gly Cys Tyr
1               5                   10

<210>  2
<211>  13
<212>  PRT
<213>  Artificial Sequence

<220>
<223>  Synthetically generated peptide

<220>
<221>  VARIANT
<222>  (4)..(4)
<223>  Xaa = Phe, Trp and Tyr

<400>  2

Cys Cys Glu Xaa Cys Cys Asn Pro Ala Cys Thr Gly Cys
1               5                   10

<210>  3
<211>  14
<212>  PRT
<213>  Artificial Sequence

<220>
<223>  Synthetically generated peptide

<220>
<221>  VARIANT
<222>  (4)..(4)
<223>  Xaa = Phe, Trp and Tyr

<400>  3

Cys Cys Glu Xaa Cys Cys Asn Pro Ala Cys Thr Gly Cys Tyr
1               5                   10

<210>  4
<211>  19
<212>  PRT
<213>  Escherichia coli

<400>  4

Asn Ser Ser Asn Tyr Cys Cys Glu Leu Cys Cys Asn Pro Ala Cys Thr
1               5                   10                  15

Gly Cys Tyr

<210>  5
<211>  18
<212>  PRT
<213>  Escherichia coli

<400>  5

Asn Thr Phe Tyr Cys Cys Glu Leu Cys Cys Tyr Pro Ala Cys Ala Gly
1               5                   10                  15

Cys Asn

<210>  6
<211>  18
<212>  PRT
<213>  Citrobacter freundii

<400>  6

Asn Thr Phe Tyr Cys Cys Glu Leu Cys Cys Asn Pro Ala Cys Ala Gly
1               5                   10                  15

Cys Tyr

<210>  7
<211>  18
<212>  PRT
<213>  Escherichia coli

<400>  7

Asn Thr Phe Tyr Cys Cys Glu Leu Cys Cys Asn Pro Ala Cys Ala Gly
1               5                   10                  15

Cys Tyr

<210>  8
<211>  30
<212>  PRT
<213>  Yersinia enterocolitica

<400>  8

Gln Ala Cys Asp Pro Pro Ser Pro Pro Ala Glu Val Ser Ser Asp Trp
1               5                   10                  15

Asp Cys Cys Asp Val Cys Cys Asn Pro Ala Cys Ala Gly Cys
            20                  25                  30

<210>  9
<211>  29
<212>  PRT
<213>  Yersinia enterocolitica

<400>  9

Gln Ala Cys Asp Pro Pro Leu Pro Ala Glu Val Ser Ser Asp Trp Asp
1               5                   10                  15

Cys Cys Asp Val Cys Cys Asn Pro Ala Cys Ala Gly Cys
            20                  25

<210>  10
<211>  30
<212>  PRT
<213>  Yersinia enterocolitica

<400>  10

Lys Ala Cys Asp Thr Gln Thr Pro Ser Pro Ser Glu Glu Asn Asp Asp
1               5                   10                  15

Trp Cys Cys Glu Val Cys Cys Asn Pro Ala Cys Ala Gly Cys
            20                  25                  30

<210>  11
<211>  53
<212>  PRT
<213>  Yersinia enterocolitica

<400>  11

Gln Glu Thr Ala Ser Gly Gln Val Gly Asp Val Ser Ser Ser Thr Ile

1                   5                   10                  15

Ala Thr Glu Val Ser Glu Ala Glu Cys Gly Thr Gln Ser Ala Thr Thr
            20                  25                  30

Gln Gly Glu Asn Asp Trp Asp Trp Cys Cys Glu Leu Cys Cys Asn Pro
        35                  40                  45

Ala Cys Phe Gly Cys
    50

<210>  12
<211>  16
<212>  PRT
<213>  Yersinia kristensenii

<400>  12

Ser Asp Trp Cys Cys Glu Val Cys Cys Asn Pro Ala Cys Ala Gly Cys
1                   5                   10                  15

<210>  13
<211>  17
<212>  PRT
<213>  Vibrio cholerae

<400>  13

Ile Asp Cys Cys Glu Ile Cys Cys Asn Pro Ala Cys Phe Gly Cys Leu
1                   5                   10                  15

Asn

<210>  14
<211>  17
<212>  PRT
<213>  Vibrio mimicus

<400>  14

Ile Asp Cys Cys Glu Ile Cys Cys Asn Pro Ala Cys Phe Gly Cys Leu
1                   5                   10                  15

Asn

<210>  15
<211>  72
<212>  PRT
<213>  Escherichia coli

<400>  15

Met Lys Lys Leu Met Leu Ala Ile Phe Ile Ser Val Leu Ser Phe Pro
1               5                   10                  15

Ser Phe Ser Gln Ser Thr Glu Ser Leu Asp Ser Ser Lys Glu Lys Ile
            20                  25                  30

Thr Leu Glu Thr Lys Lys Cys Asp Val Val Lys Asn Asn Ser Glu Lys
        35                  40                  45

Lys Ser Glu Asn Met Asn Asn Thr Phe Tyr Cys Cys Glu Leu Cys Cys
    50                  55                  60

Asn Pro Ala Cys Ala Gly Cys Tyr
65                  70

<210>  16
<211>  72
<212>  PRT
<213>  Escherichia coli

<400>  16

Met Lys Lys Ser Ile Leu Phe Ile Phe Leu Ser Val Leu Ser Phe Ser
1               5                   10                  15

Pro Phe Ala Gln Asp Ala Lys Pro Val Glu Ser Ser Lys Glu Lys Ile
            20                  25                  30

Thr Leu Glu Ser Lys Lys Cys Asn Ile Ala Lys Lys Ser Asn Lys Ser
        35                  40                  45

Gly Pro Glu Ser Met Asn Ser Ser Asn Tyr Cys Cys Glu Leu Cys Cys
    50                  55                  60

Asn Pro Ala Cys Thr Gly Cys Tyr
65                  70

<210>  17
<211>  71
<212>  PRT
<213>  Yersinia enterocolitica

<400>  17

Met Lys Lys Ile Val Phe Val Leu Val Leu Met Leu Ser Ser Phe Gly
1               5                   10                  15

Ala Phe Gly Gln Glu Thr Val Ser Gly Gln Phe Ser Asp Ala Leu Ser
            20                  25                  30

Thr Pro Ile Thr Ala Glu Val Tyr Lys Gln Ala Cys Asp Pro Pro Leu

35                  40                  45

Pro Pro Ala Glu Val Ser Ser Asp Trp Asp Cys Cys Asp Val Cys Cys
    50                  55                  60

Asn Pro Ala Cys Ala Gly Cys
65                  70

<210>  18
<211>  14
<212>  PRT
<213>  Artificial Sequence

<220>
<223>  Synthetically generated peptide

<400>  18

Cys Cys Glu Tyr Cys Cys Asn Pro Ala Cys Thr Gly Cys Tyr
1               5                   10

<210>  19
<211>  14
<212>  PRT
<213>  Artificial Sequence

<220>
<223>  Synthetically generated peptide

<400>  19

Cys Cys Glu Leu Cys Cys Asn Pro Ala Cys Thr Gly Cys Tyr
1               5                   10

<210>  20
<211>  14
<212>  PRT
<213>  Artificial Sequence

<220>
<223>  Synthetically generated peptide

<400>  20

Cys Cys Glu Phe Cys Cys Asn Pro Ala Cys Thr Gly Cys Tyr
1               5                   10

<210>  21
<211>  14
<212>  PRT
<213>  Artificial Sequence

<220>
<223>  Synthetically generated peptide

<400>  21

Cys Cys Glu Trp Cys Cys Asn Pro Ala Cys Thr Gly Cys Tyr
1               5                   10

<210>  22
<211>  13
<212>  PRT
<213>  Artificial Sequence

<220>
<223>  Synthetically generated peptide

<400>  22

Cys Cys Glu Tyr Cys Cys Asn Pro Ala Cys Thr Gly Cys
1               5                   10

<210>  23
<211>  13
<212>  PRT
<213>  Artificial Sequence

<220>
<223>  Synthetically generated peptide

<400>  23

Cys Cys Glu Leu Cys Cys Asn Pro Ala Cys Thr Gly Cys
1               5                   10

<210>  24
<211>  13
<212>  PRT
<213>  Artificial Sequence

<220>
<223>  Synthetically generated peptide

<400>  24

Cys Cys Glu Phe Cys Cys Asn Pro Ala Cys Thr Gly Cys
1               5                   10

<210>  25
<211>  13
<212>  PRT
<213>  Artificial Sequence

<220>
<223>  Synthetically generated peptide

<400>  25

Cys Cys Glu Trp Cys Cys Asn Pro Ala Cys Thr Gly Cys
1               5                   10

<210>  26
<211>  14
<212>  PRT

<213>  Artificial Sequence

<220>
<223>  Synthetically generated peptide


<220>
<221>  VARIANT
<222>  (1)..(1)
<223>  Xaa = D-Cys

<400>  26

Xaa Cys Glu Tyr Cys Cys Asn Pro Ala Cys Thr Gly Cys Tyr
1               5                   10


<210>  27
<211>  14
<212>  PRT
<213>  Artificial Sequence

<220>
<223>  Synthetically generated peptide


<220>
<221>  VARIANT
<222>  (1)..(1)
<223>  Xaa = D-Cys

<400>  27

Xaa Cys Glu Leu Cys Cys Asn Pro Ala Cys Thr Gly Cys Tyr
1               5                   10


<210>  28
<211>  14
<212>  PRT
<213>  Artificial Sequence

<220>
<223>  Synthetically generated peptide


<220>
<221>  VARIANT
<222>  (1)..(1)
<223>  Xaa = D-Cys

<400>  28

Xaa Cys Glu Phe Cys Cys Asn Pro Ala Cys Thr Gly Cys Tyr
1               5                   10


<210>  29
<211>  14
<212>  PRT
<213>  Artificial Sequence

<220>

<223>  Synthetically generated peptide


<220>
<221>  VARIANT
<222>  (1)..(1)
<223>  Xaa = D-Cys

<400>  29

Xaa Cys Glu Trp Cys Cys Asn Pro Ala Cys Thr Gly Cys Tyr
1               5                   10


<210>  30
<211>  13
<212>  PRT
<213>  Artificial Sequence

<220>
<223>  Synthetically generated peptide


<220>
<221>  VARIANT
<222>  (1)..(1)
<223>  Xaa = D-Cys

<400>  30

Xaa Cys Glu Tyr Cys Cys Asn Pro Ala Cys Thr Gly Cys
1               5                   10


<210>  31
<211>  13
<212>  PRT
<213>  Artificial Sequence

<220>
<223>  Synthetically generated peptide


<220>
<221>  VARIANT
<222>  (1)..(1)
<223>  Xaa = D-Cys

<400>  31

Xaa Cys Glu Leu Cys Cys Asn Pro Ala Cys Thr Gly Cys
1               5                   10


<210>  32
<211>  13
<212>  PRT
<213>  Artificial Sequence

<220>
<223>  Synthetically generated peptide

<220>
<221>  VARIANT
<222>  (1)..(1)
<223>  Xaa = D-Cys

<400>  32

Xaa Cys Glu Phe Cys Cys Asn Pro Ala Cys Thr Gly Cys
1               5                   10


<210>  33
<211>  13
<212>  PRT
<213>  Artificial Sequence

<220>
<223>  Synthetically generated peptide


<220>
<221>  VARIANT
<222>  (1)..(1)
<223>  Xaa = D-Cys

<400>  33

Xaa Cys Glu Trp Cys Cys Asn Pro Ala Cys Thr Gly Cys
1               5                   10


<210>  34
<211>  16
<212>  PRT
<213>  Artificial Sequence

<220>
<223>  Synthetically generated peptide

<400>  34

Asn Asp Asp Cys Glu Leu Cys Val Asn Val Ala Cys Thr Gly Cys Leu
1               5                   10                  15


<210>  35
<211>  16
<212>  PRT
<213>  Artificial Sequence

<220>
<223>  Synthetically generated peptide

<400>  35

Asn Asp Glu Cys Glu Leu Cys Val Asn Val Ala Cys Thr Gly Cys Leu
1               5                   10                  15


<210>  36
<211>  24
<212>  PRT
<213>  Artificial Sequence

<220>
<223>  Synthetically generated peptide

<400>  36

Phe Lys Thr Leu Arg Thr Ile Ala Asn Asp Asp Cys Glu Leu Cys Val
1               5                   10                  15

Asn Val Ala Cys Thr Gly Cys Leu
            20

<210>  37
<211>  24
<212>  PRT
<213>  Artificial Sequence

<220>
<223>  Synthetically generated peptide

<400>  37

Phe Lys Thr Leu Arg Thr Ile Ala Asn Asp Glu Cys Glu Leu Cys Val
1               5                   10                  15

Asn Val Ala Cys Thr Gly Cys Leu
            20

<210>  38
<211>  27
<212>  PRT
<213>  Artificial Sequence

<220>
<223>  Synthetically generated peptide

<400>  38

Val Tyr Ile Gln Tyr Gln Gly Phe Arg Val Gln Leu Glu Ser Met Lys
1               5                   10                  15

Lys Leu Ser Asp Leu Glu Ala Gln Trp Ala Pro
            20                  25

<210>  39
<211>  27
<212>  PRT
<213>  Artificial Sequence

<220>
<223>  Synthetically generated peptide

<400>  39

Ser Pro Arg Leu Gln Ala Gln Ser Leu Leu Pro Ala Val Cys His His
1               5                   10                  15

Pro Ala Leu Pro Gln Asp Leu Gln Pro Val Cys
            20                  25

<210>  40
<211>  32
<212>  PRT
<213>  Artificial Sequence

<220>
<223>  Synthetically generated peptide

<400>  40

Ala Ser Gln Glu Ala Ser Ser Ile Phe Lys Thr Leu Arg Thr Ile Ala
1               5                   10                  15

Asn Asp Asp Cys Glu Leu Cys Val Asn Val Ala Cys Thr Gly Cys Leu
            20                  25                  30

<210>  41
<211>  27
<212>  PRT
<213>  Artificial Sequence

<220>
<223>  Synthetically generated peptide

<400>  41

Val Tyr Ile Gln Tyr Gln Gly Phe Arg Val Gln Leu Glu Ser Met Lys
1               5                   10                  15

Lys Leu Ser Asp Leu Glu Ala Gln Trp Ala Pro
            20                  25

<210>  42
<211>  27
<212>  PRT
<213>  Artificial Sequence

<220>
<223>  Synthetically generated peptide

<400>  42

Ser Pro Arg Leu Gln Ala Gln Ser Leu Leu Pro Ala Val Cys His His
1               5                   10                  15

Pro Ala Leu Pro Gln Asp Leu Gln Pro Val Cys
            20                  25

<210>  43
<211>  32
<212>  PRT
<213>  Artificial Sequence

<220>
<223>  Synthetically generated peptide

<400>  43

Ala Ser Gln Glu Ala Ser Ser Ile Phe Lys Thr Leu Arg Thr Ile Ala
1               5                   10                  15

Asn Asp Glu Cys Glu Leu Cys Val Asn Val Ala Cys Thr Gly Cys Leu
            20                  25                  30

<210>  44
<211>  15
<212>  PRT
<213>  Artificial Sequence

<220>
<223>  Synthetically generated peptide

<400>  44

Pro Gly Thr Cys Glu Ile Cys Ala Tyr Ala Ala Cys Thr Gly Cys
1               5                   10                  15

<210>  45
<211>  27
<212>  PRT
<213>  Artificial Sequence

<220>
<223>  Synthetically generated peptide

<400>  45

Val Thr Val Gln Asp Gly Asn Phe Ser Phe Ser Leu Glu Ser Val Lys
1               5                   10                  15

Lys Leu Lys Asp Leu Gln Glu Pro Gln Glu Pro
            20                  25

<210>  46
<211>  27
<212>  PRT
<213>  Artificial Sequence

<220>
<223>  Synthetically generated peptide

<400>  46

Arg Val Gly Lys Leu Arg Asn Phe Ala Pro Ile Pro Gly Glu Pro Val
1               5                   10                  15

Val Pro Ile Leu Cys Ser Asn Pro Asn Phe Pro
            20                  25

<210>  47
<211>  40
<212>  PRT
<213>  Artificial Sequence

<220>
<223>  Synthetically generated peptide

<400>  47

Glu Glu Leu Lys Pro Leu Cys Lys Glu Pro Asn Ala Gln Glu Ile Leu
1               5                   10                  15

Gln Arg Leu Glu Glu Ile Ala Glu Asp Pro Gly Thr Cys Glu Ile Cys
            20                  25                  30

Ala Tyr Ala Ala Cys Thr Gly Cys
        35                  40

<210>  48
<211>  38
<212>  PRT
<213>  Artificial Sequence

<220>
<223>  Synthetically generated peptide

<400>  48

Met Pro Ser Thr Gln Tyr Ile Arg Arg Pro Ala Ser Ser Tyr Ala Ser
1               5                   10                  15

Cys Ile Trp Cys Thr Thr Ala Cys Ala Ser Cys His Gly Arg Thr Thr
            20                  25                  30

Lys Pro Ser Leu Ala Thr
        35

<210>  49
<211>  16
<212>  PRT
<213>  Artificial Sequence

<220>
<223>  Synthetically generated peptide

<400>  49

Ala Asp Leu Cys Glu Ile Cys Ala Phe Ala Ala Cys Thr Gly Cys Leu
1               5                   10                  15

<210>  50
<211>  26
<212>  PRT
<213>  Artificial Sequence

<220>
<223>  Synthetically generated peptide

<400>  50

Val Gln Val Glu Glu Gly Gly Phe Ser Phe Pro Leu Asp Ala Val Lys
1               5                   10                  15

Lys Leu Glu Glu Leu Met Gly Val Asp Met
            20                  25

<210>  51
<211>  27
<212>  PRT
<213>  Artificial Sequence

<220>
<223>  Synthetically generated peptide

<400>  51

Thr Val Lys Gln Ser Pro Arg Leu Ala Lys Thr Ser Thr Thr Ala Val
1               5                   10                  15

Cys Thr Asn Pro Asp Leu Pro Ala Val Phe Leu
            20                  25

<210>  52
<211>  37
<212>  PRT
<213>  Artificial Sequence

<220>
<223>  Synthetically generated peptide

<400>  52

Pro Leu Cys Lys Ser Lys Gly Ala Ala Asn Ser Phe Phe Arg Leu Gly
1               5                   10                  15

Phe Val Ala Ala Arg Ala Asp Leu Cys Glu Ile Cys Ala Phe Ala Ala
            20                  25                  30

Cys Thr Gly Cys Leu
        35

<210>  53
<211>  15
<212>  PRT
<213>  Artificial Sequence

<220>
<223>  Synthetically generated peptide

<400>  53

Gln Glu Glu Cys Glu Leu Cys Ile Asn Met Ala Cys Thr Gly Tyr
1               5                   10                  15

<210>  54
<211>  27
<212>  PRT
<213>  Artificial Sequence

<220>
<223>  Synthetically generated peptide

<400>  54

Val Tyr Ile Gln Tyr Glu Gly Phe Gln Val Asn Leu Asp Ser Val Lys
1               5                   10                  15

Lys Leu Asp Lys Leu Leu Glu Gln Leu Arg Gly
            20                  25

<210>  55
<211>  27
<212>  PRT
<213>  Artificial Sequence

<220>
<223>  Synthetically generated peptide

<400>  55

Phe His His Gln Met Gly Asp Gln Arg Asp Pro Ser Ile Leu Cys Ser
1               5                   10                  15

Asp Pro Ala Leu Pro Ser Asp Leu Gln Pro Val
            20                  25

<210>  56
<211>  32
<212>  PRT
<213>  Artificial Sequence

<220>
<223>  Synthetically generated peptide

<400>  56

Cys Glu Asn Ser Gln Ala Val Asn Ile Phe Arg Ala Leu Arg Tyr Ile
1               5                   10                  15

Asn Gln Glu Glu Cys Glu Leu Cys Ile Asn Met Ala Cys Thr Gly Tyr
            20                  25                  30

<210>  57
<211>  4
<212>  PRT
<213>  Artificial Sequence

<220>
<223>  Synthetically generated peptide

<400>  57

Phe Ala Leu Ala
1



<210>  58
<211>  5
<212>  PRT
<213>  Artificial Sequence

<220>
<223>  Synthetically generated peptide

<400>  58

Val Leu Ala Leu Ala
1               5



<210>  59
<211>  4
<212>  PRT
<213>  Artificial Sequence

<220>
<223>  Synthetically generated peptide

<400>  59

Ala Leu Ala Leu
1



<210>  60
<211>  5
<212>  PRT
<213>  Artificial Sequence

<220>
<223>  Synthetically generated peptide

<400>  60

Ala Leu Ala Leu Ala
1               5



<210>  61
<211>  11
<212>  PRT
<213>  Artificial Sequence

<220>
<223>  HIV  tat peptide residues

<400>  61

Tyr Gly Arg Lys Lys Arg Arg Gln Arg Arg Arg

1                   5                   10


<210>  62
<211>  11
<212>  PRT
<213>  Homo sapiens

<400>  62

Tyr Ala Arg Lys Ala Arg Arg Gln Ala Arg Arg
1               5                   10


<210>  63
<211>  11
<212>  PRT
<213>  Homo sapiens

<400>  63

Tyr Ala Arg Ala Ala Ala Arg Gln Ala Arg Ala
1               5                   10


<210>  64
<211>  11
<212>  PRT
<213>  Homo sapiens

<400>  64

Tyr Ala Arg Ala Ala Arg Arg Ala Ala Arg Arg
1               5                   10


<210>  65
<211>  11
<212>  PRT
<213>  Homo sapiens

<400>  65

Tyr Ala Arg Ala Ala Arg Arg Ala Ala Arg Ala
1               5                   10


<210>  66
<211>  10
<212>  PRT
<213>  Homo sapiens

<400>  66

Ala Arg Arg Arg Arg Arg Arg Arg Arg Arg
1               5                   10


<210>  67
<211>  11
<212>  PRT
<213>  Homo sapiens

<400>  67

Tyr Ala Ala Ala Arg Arg Arg Arg Arg Arg Arg
1               5                   10

<210>  68
<211>  34
<212>  PRT
<213>  Artificial Sequence

<220>
<223>  Synthetically generated peptide

<400>  68

Ile Lys Pro Glu Ala Pro Gly Glu Asp Ala Ser Pro Glu Glu Leu Asn
1               5                   10                  15

Arg Tyr Tyr Ala Ser Leu Arg His Tyr Leu Asn Leu Val Thr Arg Gln
            20                  25                  30

Arg Tyr

<210>  69
<211>  27
<212>  PRT
<213>  Artificial Sequence

<220>
<223>  Synthetically generated peptide

<400>  69

His Ala Glu Gly Thr Phe Thr Ser Asp Val Ser Tyr Leu Glu Gly Gln
1               5                   10                  15

Ala Ala Lys Glu Phe Ile Ala Trp Leu Val Lys
            20                  25

## Description

### BACKGROUND

Excessive retention of fluid or salt is associated with various disorders, including cardiovascular disorders such as congestive heart failure, hypertension, cardiac hypertrophy and stroke. Excessive retention of fluid or salt can also cause or be associated with renal disorders and can lead to acites build up in the abdomen, for example, in the case of liver disease.

### SUMMARY

Described herein as a method of reducing the risk of or treating a disorder associated with fluid and/or salt retention in a patient, the method comprising administering to the patient an agent selected from: a) an agent that reduces sodium absorption in the intestine; b) an agent that increases anion secretion in the intestine; or c) an agent that both reduces sodium absorption in the intestine and increases anion secretion in the intestine. In certain cases the method comprises administering two or more agents selected from: a) an agent that reduces sodium absorption in the intestine; b) an agent that increases anion secretion in the intestine; or c) an agent that both reduces sodium absorption in the intestine and increases anion secretion in the intestine. In various embodiments: the agent reduces sodium absorption in the intestine; the agent increases anion secretion in the intestine; and the agent both reduces sodium absorption in the intestine and increases anion secretion in the intestine.

In certain embodiments of the method: the agent is selected from: a) a guanylate cyclase receptor C agonist, b) a soluble guanylate cyclase modulator (preferably an agonist of soluble guanylate cyclase), c) a prostanoid, d) a chloride channel activator, e) a 5HT4 agonist, f) a cyclic nucleotide, g) a sodium transport inhibitor, h) a laxative, i) a cystic fibrosis transmembrane conductance regulator (CTFR) modulator, j) an agent that affects cAMP levels (preferably an agent the increases cAMP levels in the body or a tissue), k) a phosphodiesterase inhibitor, 1) a renin inhibitor, m) an aldosterone antagonist, n) potassium, and o) a polymer resin. In sertain embodiments the method comprises administering two or more agents selected from: a) a guanylate cyclase receptor C agonist, b) a soluble guanylate cyclase modulator (preferably an agonist of soluble guanylate cyclase), c) a prostanoid, d) a chloride channel activator, e) a 5HT4 agonist, f) a cyclic nucleotide, g) a sodium transport inhibitor, h) a laxative, i) a cystic fibrosis transmembrane conductance regulator (CTFR) modulator, j) an agent that affects cAMP levels (preferably an agent the increases cAMP levels in the body or a tissue), k) a phosphodiesterase inhibitor, l) a renin inhibitor, m) an aldosterone antagonist, n) potassium, and o) a polymer resin.

In various embodiments: the chloride channel activator is lubiprostone; the sodium transport inhibitor is amiloride; the sodium transport inhibitor is an NHE3 inhibitor; the 5HT4 agonist is Zelnorm; the prostanoid is selected from: the compound represented by CAS Registry No. 333963-40-9, the compound represented by CAS Registry No. 136790-76-6, (-)-7-[(2R,4aR,5R,7aR)-2-(1,1-difluoropentyl)-2-hydroxy-6-oxooctahydrocyclopenta[b]pyran-5-yl]heptanoic acid; and the 13, 14-dihydro-15-keto prostaglandins E disclosed in US5284858 including 13,14-dihydro-15-keto-PGE₂ alkyl ester, 13,14-dihydro-15-keto-PGE₂ cycloalkyl ester; 13,14-dihydro-15-keto-PGE₂ hydroxy alkyl ester, 13,14-dihydro-15-keto-PGE₂ benzyl ester, 13,14-dihydro-15-keto-PGE₁ alkyl ester, 13,14-dihydro-6,15-diketo-PGE₁ alkyl ester, 13,14-dihydro-15-keto-18-methoxy-19, 20-dinor-PGE₂ or an alkyl ester thereof, 13,14-dihydro-15-keto-18-methoxy-PGE₂ or an alkyl ester thereof, 13,14-dihydro-15-keto-Δ²-PGE₂ or an alkyl ester thereof, 13,14-dihydro-15-keto-20-methoxy-Δ²-PGE₂ or an alkyl ester thereof, 13,14-dihydro-15-keto-3R,S-methyl-PGE₂ or an alkyl ester thereof, 13,14-dihydro-15-keto-3R,S-methyl-20-methoxy-PGE₂ or an alkyl ester thereof, 13, 14-dihydro-15-keto-11-dehydroxy-11R-methyl-PGE₂ or an alkyl ester thereof, 13,14-dihydro-15-keto-16R,S-fluoro-11-dehydroxy-11R-methyl-PGE₂ or an alkyl ester thereof, 13,14-dihydro-15-keto-16R,S-hydroxy-PGE₂ or an alkyl ester thereof, 13,14-dihydro-15-keto-16R,S-fluoro-PGE₂ or an alkyl ester thereof, 13,14-dihydro-15-keto-16R,S-methyl-PGE₂ or an alkyl ester thereof, 13,14-dihydro-15-keto-16,16-dimethyl-PGE₂ or an alkyl ester thereof, 13,14-dihydro-15-keto-16,16-dimethyl-20-methoxy-PGE₂ or an alkyl ester thereof, 13,14-dihydro-15-keto-17S-methyl-PGE₂ or an alkyl ester thereof, 13,14-dihydro-15-keto-19-methy-PGE₂ or an alkyl ester thereof, 13,14-dihydro-15-keto-20-isopropropylidene PGE₂ or an alkyl ester thereof, 13,14-dihydro-15-keto-20-ethyl-PGE₂ or an alkyl ester thereof, 13,14-dihydro-15-keto-20-ethyl-11-dehydroxy-11R-methyl-PGE₂ or an alkyl ester thereof, 13,14-dihydro-15 -keto-20-n-propyl-PGE₂ or an alkyl ester thereof, 13,14-dihydro-15-keto-20-ethyl-PGE₁ or an alkyl ester thereof, 13,14-dihydro-6,15-diketo-16R,S-fluoro-PGE₁ or an alkyl ester thereof, 13,14-dihydro-6,15-diketo-16R,S-fluoro-11-dehydroxy-11R-methyl-PGE₁ or an alkyl ester thereof, 13,14-dihydro-6,15-diketo-16R,S-methyl-PGE₁ or an alkyl ester thereof, 13,14-dihydro-6,15-diketo-16,16-dimethyl-PGE₁ or an alkyl ester thereof, 13,14-dihydro-6,15-diketo-19-methyl-PGE₁ or an alkyl ester thereof, 13,14-dihydro-6,15-diketo-20-methyl-PGE₁ or an alkyl ester thereof, 13,14-dihydro-6,15-diketo-11-dehydroxy-11R-methyl-PGE₁ or an alkyl ester thereof, 13, 14-dihydro-6,15-diketo-11-dehydroxy-11R-hydroxymethyl PGE₁ alkyl ester, 13,14-dihydro-15-keto-20-methyl-PGE₁ or an alkyl ester thereof, 13,14-dihydro-15-keto-Δ²-PGE₁ or an alkyl ester thereof, 13,14-dihydro-15-keto-16R,S-fluoro-20-methyl-PGE₂ or an alkyl ester thereof, 13,14-dihydro-15-keto-16,16-difluoro-PGE₂ or an alkyl ester thereof, 13,14-dihydro-15-keto-5,6-dehydro-20-methoxy-PGE₂ or an alkyl ester thereof, and 13,14-dihydro-6,15-diketo-16R,S-fluoro-PGE₁ or an alkyl ester thereof; the prostanoid is misoprostol; the prostanoid is the free acid of the compound associated with CAS registry NO. 59122-49-5; the prostanoid comprises a mixture of steroisomers; only a single isomer of a prostanoid is administered; the laxative is selected from: a CCK-1 antagonist, a stimulant, a bulk-producing agent and a stool softener; the laxative is selected from dexloxiglumide, psyllium husk, docusate sodium, bisacodyl, and phenolphthalein; the polymer resin is selected from psyllium, lipid lowering polymers, nonabsorbed polymer resins, and sodium binding polymers; the lipid lowering polymer is selected from: Colesevelam, Sevalmer, Cholestyramine; the nonabsorbed polymer resin is selected from: hyaluronic acid, polycarbophil calcium, polyvinyl acetate, polyvinyl pyrrolidone, polystyrene sulfate; the sodium-binding polymer is selected from: crosslinked polyvinylsulfamate polymer, N-(bis-phosphonic-ethyl) polyvinylamine polymer, poly-α-acrylic acid polymer, poly-α-fluoroacrylic acid polymer, polyvinylphosphoramidic polymer, polyvinylsulfamate polymer, polyvinylsulfamate/vinylsulfate copolymer, polyvinylsulfate polymer, polyvinylsulfonate polymer, polyvinylsulfonate polymer, vinylphosphonate/α-fluoroacrylic acid copolymer, vinylphosphonate/α-fluoroacrylic acid copolymer, or vinylphosphonate/acrylic acid copolymer; the sodium-binding polymer is administered as core-shell composition which further comprises a semi-permeable shell; the semi-permeable shell comprises at least one of a poly-11 trimethylammonioundecylmethacrylate polymer, a styrene-vinylpyridine polymer, 11-dimethyl-aminodecylmethacrylate/laurylmethacrylate copolymer, or a polyallylamine/polystyrene sulfonate polymer.

In certain embodiments the method includes: administering an anti-diabetic agent; administering an anti-obesity agent; administering potassium or a salt thereof; administering a PDE inhibitor (e.g., PDE5-specific PDE inhibitor, a cGMP-specific PDE inhibitor or a cAMP-specific PDE inhibitor); administering a polymer resin (e.g., psyllium, a nonabsorbed polymer resin, a nonabsorbed polymer resin is selected from hyaluronic acid, polycarbophil calcium, polyvinyl acetate, and polyvinyl pyrrolidine; a polymer resin that is a lipid lowering polymer, a lipid lowering polymer is selected from: colesevelam or sevalmer); administering an anti-hypertensive agent (e.g., an antihypertensive agent is selected from: a diuretic, an inhibitor of angiotensin converting enzyme, an angiotensin II receptor antagonist, a calcium channel blocker, a beta-adrenergic antagonist, alpha-adrenergic antagonist, a renin inhibitor, and an aldosterone antagonist); administering two or more anti-hypertensive agents wherein the two or more antihypertensive agents are independently selected from: a diuretic, an inhibitor of angiotensin converting enzyme, an angiotensin II receptor antagonist, a calcium channel blocker, a beta-adrenergic antagonist, alpha-adrenergic antagonist, a renin inhibitor, and an aldosterone antagonist. In some cases: the anti-hypertensive agent is a diuretic; the diuretic is selected from the group consisting of: a loop diuretic, a thiazide, a potassium sparing agent, and an osmotic diuretic; the diuretic is a loop diuretic; the diuretic is furosemide, bumetanide, ethacrynic or torsemide; the diuretic is a thiazide; the thiazide is bendroflumethiazide, hydrochlorothiazide, indapamide, chlortalidone or metolazone; the diuretic is a potassium sparing agent; the potassium sparing agent is amiloride or triamterene; the diuretic is an osmotic diuretic; the osmotic diuretic is glucose or mannitol; the antihypertensive agent is an angiotensin converting enzyme inhibitor; the angiotensin converting enzyme inhibitor is selected from: Benazepril (Lotensin), Captopril (Capoten), Enalapril/Enalaprilat (Vasotec), Fosinopril (Monopril), Lisinopril (Zestril and Prinivil), Moexipril (Univasc), Perindopril (Aceon), Quinapril (Accupril), Ramipril (Altace), and Trandolapril (Mavik); the antihypertensive agent is an angiotensin II receptor antagonist; the angiotensin II receptor antagonist is selected from: Candesartan, Eprosartan, Irbesartan, Losartan, Olmesartan, Telmisartan, Valsartan; the antihypertensive agent is a calcium channel blocker; the calcium channel blocker is selected from: Amlodipine (Norvasc), Felodipine (Plendil), Nicardipine (Cardene), Nifedipine (Procardia, Adalat), Nimodipine (Nimotop), Nisoldipine (Sular), Nitrendipine (Cardif, Nitrepin), and Lacidipine (Motens), Lercanidipine (Zanidip), Verapamil (Calan, Isoptin), Gallopamil (D600), Diltiazem (Cardizem), and Menthol (mint oil); the calcium channel blocker is Amlodipine; the antihypertensive agent is a beta-adrenergic antagonist; the beta-adrenergic antagonist is selected from: Dichloroisoprenaline, Practolol, Pronethaolol, Alprenolol, Carteolol, Levobunolol, Mepindolol, Metipranolol, Nadolol, Oxprenolol, Penbutolol, Pindolol, Propranolol, Sotalol, Timolol, Acebutolol, Atenolol, Betaxolol, Bisoprolol, Esmolol Metoprolol, Nebivolol, Carvedilol, Celiprolol, Labetalol, and Butoxamine; the antihypertensive agent is an alpha-adrenergic antagonist; the alpha-adrenergic antagonist is selected from: Doxazosin (Cardura), Prazosin (Minipress), Phenoxybenzamine, Phentolamine (Regitine), Tamsulosin (Flomaxtra/Flomax), Alfuzosin (Uroxatral), and Terazosin (Hytrin); the antihypertensive agent is a renin inhibitor; the renin inhibitor is selected from: Tekturna® (Rasilez and Aliskiren) and SPP635; the antihypertensive agent is an aldosterone antagonist; and the aldosterone antagonist is Spironolactone, Canrenone, or Eplerenone.

In some case the method includes: administering a lipid altering agent. In certain cases: the lipid altering agent is a cholesterol lowering agent; the agent lowers low density cholesterol; the lipid altering agent is selected from the group consisting of: a statin; a fibrate; niacin; a CETP inhibitor; a MTP inhibitor; a cholesterol absorption inhibitor; a squalene synthesis inhibitor; and a bile acid sequestrant; the lipid altering agent is a statin; the statin is chosen from simvastatin, rosuvastatin and atorvastatin; the lipid altering agent is a cholesterol absorption inhibitor; the cholesterol absorption inhibitor is ezetimibe; the lipid altering agent is a bile acid sequestrant; the bile acid sequestrant is chosen from cholestyramine, colesevelam and colestipol; the lipid altering agent is a fibrate; and the fibrate is fenofibrate.

In some cases the method includes: administering misoprostol and psyllium; administering methyl 7-[(1R,2R,3R)-3-hydroxy-2-[(E,4S)-4-hydroxy-4-methyloct-1-enyl]-5-oxocyclopentyl]heptanoate; administering psyllium and a peptide that activates the guanylate cyclase C receptor. In some cases the peptides is selected from:
Cys Cys Glu Tyr Cys Cys Asn Pro Ala Cys Thr Gly Cys Tyr;
Cys Cys Glu Leu Cys Cys Asn Pro Ala Cys Thr Gly Cys Tyr;
Cys Cys Glu Phe Cys Cys Asn Pro Ala Cys Thr Gly Cys Tyr;
Cys Cys Glu Trp Cys Cys Asn Pro Ala Cys Thr Gly Cys Tyr;
Cys Cys Glu Tyr Cys Cys Asn Pro Ala Cys Thr Gly Cys;
Cys Cys Glu Leu Cys Cys Asn Pro Ala Cys Thr Gly Cys;
Cys Cys Glu Phe Cys Cys Asn Pro Ala Cys Thr Gly Cys;
Cys Cys Glu Trp Cys Cys Asn Pro Ala Cys Thr Gly Cys;
d-Cys Cys Glu Tyr Cys Cys Asn Pro Ala Cys Thr Gly Cys Tyr;
d-Cys Cys Glu Leu Cys Cys Asn Pro Ala Cys Thr Gly Cys Tyr;
d-Cys Cys Glu Phe Cys Cys Asn Pro Ala Cys Thr Gly Cys Tyr;
d-Cys Cys Glu Trp Cys Cys Asn Pro Ala Cys Thr Gly Cys Tyr;
d-Cys Cys Glu Tyr Cys Cys Asn Pro Ala Cys Thr Gly Cys;
d-Cys Cys Glu Leu Cys Cys Asn Pro Ala Cys Thr Gly Cys;
d-Cys Cys Glu Phe Cys Cys Asn Pro Ala Cys Thr Gly Cys;
d-Cys Cys Glu Trp Cys Cys Asn Pro Ala Cys Thr Gly Cys;
Asn Asp Asp Cys Glu Leu Cys Val Asn Val Ala Cys Thr Gly Cys Leu;
Asn Asp Glu Cys Glu Leu Cys Val Asn Val Ala Cys Thr Gly Cys Leu;
Phe Lys Thr Leu Arg Thr Ile Ala Asn Asp Asp Cys Glu Leu Cys Val Asn Val Ala Cys Thr Gly Cys Leu;
Phe Lys Thr Leu Arg Thr Ile Ala Asn Asp Glu Cys Glu Leu Cys Val Asn Val Ala Cys Thr Gly Cys Leu;
Val Tyr Ile Gln Tyr Gln Gly Phe Arg Val Gln Leu Glu Ser Met Lys Lys Leu Ser Asp Leu Glu Ala Gln Trp Ala Pro;
Ser Pro Arg Leu Gln Ala Gln Ser Leu Leu Pro Ala Val Cys His His Pro Ala Leu Pro Gln Asp Leu Gln Pro Val Cys;

Ala Ser Gln Glu Ala Ser Ser Ile Phe Lys Thr Leu Arg Thr Ile Ala Asn Asp Asp Cys Glu Leu Cys Val Asn Val Ala
Cys Thr Gly Cys Leu;

Val Tyr Ile Gln Tyr Gln Gly Phe Arg Val Gln Leu Glu Ser Met Lys Lys Leu Ser Asp Leu Glu Ala Gln Trp Ala Pro;
Ser Pro Arg Leu Gln Ala Gln Ser Leu Leu Pro Ala Val Cys His His Pro Ala Leu Pro Gln Asp Leu Gln Pro Val Cys;

Ala Ser Gln Glu Ala Ser Ser Ile Phe Lys Thr Leu Arg Thr Ile Ala Asn Asp Glu Cys Glu Leu Cys Val Asn Val Ala
Cys Thr Gly Cys Leu;

Pro Gly Thr Cys Glu Ile Cys Ala Tyr Ala Ala Cys Thr Gly Cys; 
Val Thr Val Gln Asp Gly Asn Phe Ser Phe Ser Leu Glu Ser Val Lys Lys Leu Lys Asp Leu Gln Glu Pro Gln Glu
Pro;
Arg Val Gly Lys Leu Arg Asn Phe Ala Pro Ile Pro Gly Glu Pro Val Val Pro Ile Leu Cys Ser Asn Pro Asn Phe Pro;

Glu Glu Leu Lys Pro Leu Cys Lys Glu Pro Asn Ala Gln Glu Ile Leu Gln Arg Leu Glu Glu Ile Ala Glu Asp Pro Gly
Thr Cys Glu Ile Cys Ala Tyr Ala Ala Cys Thr Gly Cys;

Met Pro Ser Thr Gln Tyr Ile Arg Arg Pro Ala Ser Ser Tyr Ala Ser Cys Ile Trp Cys Thr Thr Ala Cys Ala Ser Cys His
Gly Arg Thr Thr Lys Pro Ser Leu Ala Thr;

Ala Asp Leu Cys Glu Ile Cys Ala Phe Ala Ala Cys Thr Gly Cys Leu;
Val Gln Val Glu Glu Gly Gly Phe Ser Phe Pro Leu Asp Ala Val Lys Lys Leu Glu Glu Leu Met Gly Val Asp Met;
Thr Val Lys Gln Ser Pro Arg Leu Ala Lys Thr Ser Thr Thr Ala Val Cys Thr Asn Pro Asp Leu Pro Ala Val Phe Leu;

Pro Leu Cys Lys Ser Lys Gly Ala Ala Asn Ser Phe Phe Arg Leu Gly Phe Val Ala Ala Arg Ala Asp Leu Cys Glu Ile
Cys Ala Phe Ala Ala Cys Thr Gly Cys Leu;

Gln Glu Glu Cys Glu Leu Cys Ile Asn Met Ala Cys Thr Gly Tyr;

Val Tyr Ile Gln Tyr Glu Gly Phe Gln Val Asn Leu Asp Ser Val Lys Lys Leu Asp Lys Leu Leu Glu Gln Leu Arg
Gly;

Phe His His Gln Met Gly Asp Gln Arg Asp Pro Ser Ile Leu Cys Ser Asp Pro Ala Leu Pro Ser Asp Leu Gln Pro Val; and

Cys Glu Asn Ser Gln Ala Val Asn Ile Phe Arg Ala Leu Arg Tyr Ile Asn Gln Glu Glu Cys Glu Leu Cys Ile Asn Met
Ala Cys Thr Gly Tyr

In some cases: the disorder is associated with fluid retention; the disorder is associated with salt retention; the disorder is a cardiovascular disorder; the cardiovascular disorder is cardiomyopathy; the cardiomyopathy is associated with Chagas disease; the cardiovascular disorder is hypertension; the hypertension is salt-sensitive hypertension; the cardiovascular disorder is congestive heart failure; the cardiovascular disorder is cardiac hypertrophy; the cardiovascular disorder is a heart attack; the cardiovascular disorder is stroke; the patient is suffering from salt-sensitive hypertension; the patient is suffering from congestive heart failure; the patient is suffering from cardiac hypertrophy; the patient has suffered a heart attack; the patient has suffered a stroke; the patient is salt sensitive; the patient is suffering from hypertension; the disorder is a renal disorder; the renal disorder is selected from chronic renal failure or acute renal failure; the disorder is associated with ascites; the disorder is cirrhosis; the patient is suffering from alcoholism; the disorder hepatitis; the hepatitis is chronic hepatitis; the hepatitis is severe alcoholic hepatitis without cirrhosis; the disorder is Budd-Chiari syndrome; and the disorder is constrictive pericarditis.

Also described herein is a pharmaceutical composition comprising: a first agent that is an anti-hypertensive agent and a second agent selected from: a) an agent that reduces sodium absorption in the intestine; b) an agent that increases anion secretion in the intestine; or c) an agent that both reduces sodium absorption in the intestine and increases anion secretion in the intestine.

In various embodiments: the second agent reduces sodium absorption in the intestine; the second agent increases anion secretion in the intestine; the second agent both reduces sodium absorption in the intestine and increases anion secretion in the intestine; the second agent is selected from:
a) a guanylate cyclase receptor C agonist, b) a soluble guanylate cyclase modulator, c) a prostanoid, d) a chloride channel activator, e) a 5HT4 agonist, f) a cyclic nucleotide, g) a sodium transport inhibitor, h) a laxative, i) a cystic fibrosis transmembrane conductance regulator (CTFR) modulator, j) an agent that affects cAMP levels, k) a phosphodiesterase inhibitor, l) a renin inhibitor, m) an aldosterone antagonist, n) potassium, and o) a polymer resin;

Also described herein is a pharmaceutical composition comprising: a first agent that is an anti-diabetic agent and a second agent selected from: a) an agent that reduces sodium absorption in the intestine; b) an agent that increases anion secretion in the intestine; or c) an agent that both reduces sodium absorption in the intestine and increases anion secretion in the intestine.

Also described herein is a pharmaceutical composition comprising: an agent that reduces sodium absorption in the intestine; b) an agent that increases anion secretion in the intestine; or c) an agent that both reduces sodium absorption in the intestine and increases anion secretion in the intestine. In various embodiments: the second agent reduces sodium absorption in the intestine; the second agent increases anion secretion in the intestine; the second agent both reduces sodium absorption in the intestine and increases anion secretion in the intestine; the second agent is selected from: a) a guanylate cyclase receptor C agonist, b) a soluble guanylate cyclase modulator, c) a prostanoid, d) a chloride channel activator, e) a 5HT4 agonist, f) a cyclic nucleotide, g) a sodium transport inhibitor, h) a laxative, i) a cystic fibrosis transmembrane conductance regulator (CTFR) modulator, j) an agent that affects cAMP levels, k) a phosphodiesterase inhibitor,l) a renin inhibitor, m) an aldosterone antagonist, n) potassium, and o) a polymer resin.

Also disclosed is a pharmaceutical composition comprising: potassium or a salt thereof and a second agent selected from: a) an agent that reduces sodium absorption in the intestine; b) an agent that increases anion secretion in the intestine; or c) an agent that both reduces sodium absorption in the intestine and increases anion secretion in the intestine. In various embodiments: the second agent reduces sodium absorption in the intestine; the second agent increases anion secretion in the intestine; the second agent both reduces sodium absorption in the intestine and increases anion secretion in the intestine; the second agent is selected from: a) a guanylate cyclase receptor C agonist, b) a soluble guanylate cyclase modulator, c) a prostanoid, d) a chloride channel activator, e) a 5HT4 agonist, f) a cyclic nucleotide, g) a sodium transport inhibitor, h) a laxative, i) a cystic fibrosis transmembrane conductance regulator (CTFR) modulator, j) an agent that affects cAMP levels, k) a phosphodiesterase inhibitor, l) a renin inhibitor, m) an aldosterone antagonist, n) potassium, and o) a polymer resin.

Also disclosed is a pharmaceutical composition comprising: a first agent that is a PDE inhibitor and a second agent selected from: a) an agent that reduces sodium absorption in the intestine; b) an agent that increases anion secretion in the intestine; or c) an agent that both reduces sodium absorption in the intestine and increases anion secretion in the intestine. In various embodiments: the second agent reduces sodium absorption in the intestine; the second agent increases anion secretion in the intestine; the second agent both reduces sodium absorption in the intestine and increases anion secretion in the intestine; the second agent is selected from: a) a guanylate cyclase receptor C agonist, b) a soluble guanylate cyclase modulator, c) a prostanoid, d) a chloride channel activator, e) a 5HT4 agonist, f) a cyclic nucleotide, g) a sodium transport inhibitor, h) a laxative, i) a cystic fibrosis transmembrane conductance regulator (CTFR) modulator, j) an agent that affects cAMP levels, k) a phosphodiesterase inhibitor, l) a renin inhibitor, m) an aldosterone antagonist, n) potassium, and o) a polymer resin.

Some salt sensitive individuals may have a greater than 5-mm Hg decrease in mean blood pressure when daily salt intake is reduced from 260 mmol to 20 mmol. There are various tests for assessing salt sensitivity (see, for example, de la Sierra et al. 2002 Journal of Human Hypertension 16:256).

In various embodiments: the method comprises administering to the patient an agent that reduces sodium absorption in the intestine or increases anion secretion in the intestine; the agent comprises at least one GCC receptor agonist; the GCC receptor agonist is a peptide (e.g., the peptide comprises guanylin or uroguanylin; the peptide comprises ST peptide; the peptide comprises the amino acid sequence Cys Cys Glu Xaa Cys Cys Asn Pro Ala Cys Thr Gly Cys wherein Xaa is selected from Phe, Trp and Tyr (SEQ ID NO:A); the peptide comprises the amino acid sequence Cys Cys Glu Xaa Cys Cys Asn Pro Ala Cys Thr Gly Cys Tyr wherein Xaa is selected from Phe, Trp and Tyr (SEQ ID NO:B)); the agent comprises a prostanoid, prostaglandin E1 or a prostaglandin E1 derivative; the prostaglandin E1 derivative is misoprostol; the prostaglandin E1 derivative is lubiprostone; the agent comprises at least one 5HT4 receptor agonist; the 5HT4 receptor agonist is Zelnorm; the agent comprises at least one cyclic nucleotide; the cyclic nucleotide is cGMP; the cyclic nucleotide is cAMP.

In other embodiments: the disorder is a cardiovascular disorder; the cardiovascular disorder is cardiomyopathy; the cardiomyopathy is associated with Chagas disease; the cardiovascular disorder is hypertension; the hypertension is salt-sensitive hypertension; the cardiovascular disorder is congestive heart failure; the cardiovascular disorder is cardiac hypertrophy; the cardiovascular disorder is a heart attack; the cardiovascular disorder is stroke; the patient has been diagnosed with a cardiovascular disorder; the cardiovascular disorder is cardiomyopathy; the cardiomyopathy is associated with Chagas disease; the patient is suffering from a cardiovascular disorder; the patient is suffering from salt-sensitive hypertension; the patient is suffering from congestive heart failure; the patient is suffering from cardiac hypertrophy; the patient has suffered a heart attack; the patient has suffered a stroke; the patient is salt sensitive and/or the patient is suffering from hypertension; the disorder is a renal disorder (e.g., chronic renal failure or acute renal failure); the patient has been diagnosed with a renal disorder; the disorder is associated with ascites; the disorder is cirrhosis; the patient is suffering from alcoholism; the disorder hepatitis; the hepatitis is chronic hepatitis; the hepatitis is severe alcoholic hepatitis without cirrhosis; the disorder is congestive heart failure; the disorder is Budd-Chiari syndrome; the disorder is constrictive pericarditis; and the patient is suffering from congestive heart failure.

In various embodiments the method further comprises administering an agent that reduces sodium absorption in the intestine and/or increases anion secretion and an agent useful in the treatment of congestive heart failure. For example, the agent useful in the treatment of congestive heart failure is selected from: Nesiritide, Dobutamine, Milrinone, Levosimendan (Simdax®), Adenosine, an adenosine analog, an agent which increases cellular availability of adenosine, an adenosine A₂ receptor agonist, an adenosine transport inhibitor, and an adenosine deaminase inhibitor; and the adenosine analog is selected from: N⁶ -[(1,2-dihydro-1-acenaphthylenyl)methyl]adenosine, Dipyridamole and iodotulercidin

In various embodiments the method further comprises administering an anti-hypertensive agent. For example: the antihypertensive agent is selected from: a diuretic, an inhibitor of angiotensin converting enzyme, an angiotensin II receptor antagonist; a calcium channel blocker; a beta-adrenergic antagonist; alpha-adrenergic antagonist; a renin inhibitor; and an aldosterone antagonist; the method comprises administering two or more anti-hypertensive agents wherein the two or more antihypertensive agents are independently selected from: a diuretic, an inhibitor of angiotension converting enzyme, an angiotension II receptor antagonist; a calcium channel blocker; a beta-adrenergic antagonist; alpha-adrenergic antagonist; a renin inhibitor; and an aldosterone antagonist; the agent that reduces sodium absorption in the intestine or increases anion secretion is co-administered with the antihypertensive agent; the co-administered agents are combined in a unit dosage form; the diuretic is selected from the group consisting of: a loop diuretic, a thiazide, a potassium sparing agent, and an osmotic diuretic; the loop diuretic is furosemide, bumetanide, ethacrynic or torsemide; the diuretic is a thiazide (e.g., bendroflumethiazide, hydrochlorothiazide, indapamide, chlortalidone or metolazone); the diuretic is a potassium sparing agent (e.g., amiloride or triamterene); the diuretic is an osmotic diuretic; the osmotic diuretic is glucose or mannitol; the antihypertensive agent is an angiotensin converting enzyme inhibitor; angiotensin converting enzyme inhibitor is selected from: Benazepril (Lotensin), Captopril (Capoten), Enalapril/Enalaprilat (Vasotec), Fosinopril (Monopril), Lisinopril (Zestril and Prinivil), Moexipril (Univasc), Perindopril (Aceon), Quinapril (Accupril), Ramipril (Altace), and Trandolapril (Mavik); the antihypertensive agent is an angiotensin II receptor antagonist; the angiotensin II receptor antagonist is selected from: Candesartan, Eprosartan, Irbesartan, Losartan, Olmesartan, Telmisartan, Valsartan; the antihypertensive agent is a calcium channel blocker; the calcium channel blocker is selected from: Amlodipine (Norvasc), Felodipine (Plendil), Nicardipine (Cardene), Nifedipine (Procardia, Adalat), Nimodipine (Nimotop), Nisoldipine (Sular), Nitrendipine (Cardif, Nitrepin), and Lacidipine (Motens), Lercanidipine (Zanidip), Verapamil (Calan, Isoptin), Gallopamil (D600), Diltiazem (Cardizem), and Menthol (mint oil); the calcium channel blocker is Amlodipine; the antihypertensive agent is a beta-adrenergic antagonist; the beta-adrenergic antagonist is selected from: Dichloroisoprenaline, Practolol, Pronethaolol, Alprenolol, Carteolol, Levobunolol, Mepindolol, Metipranolol, Nadolol, Oxprenolol, Penbutolol, Pindolol, Propranolol, Sotalol, Timolol, Acebutolol, Atenolol, Betaxolol, Bisoprolol, Esmolol Metoprolol, Nebivolol, Carvedilol, Celiprolol, Labetalol, and Butoxamine; the antihypertensive agent is an alpha-adrenergic antagonist; the alpha-adrenergic antagonist is selected from: Doxazosin (Cardura), Prazosin (Minipress), Phenoxybenzamine, Phentolamine (Regitine), Tamsulosin (Flomaxtra/Flomax), Alfuzosin (Uroxatral), and Terazosin (Hytrin); the antihypertensive agent is a renin inhibitor; the renin inhibitor is selected from: Tekturna® (Rasilez and Aliskiren) and SPP635; the antihypertensive agent is an aldosterone antagonist; and the aldosterone antagonist is Spironolactone, Canrenone, or Eplerenone.

In some embodiments the method further comprises administering a lipid altering agent. For example: the lipid altering agent is a cholesterol lowering agent, the agent lowers low density cholesterol; the lipid altering agent is selected from the group consisting of: a statin; a fibrate; niacin; a CETP inhibitor; a MTP inhibitor; a cholesterol absorption inhibitor; a squalene synthesis inhibitor; and a bile acid sequestrant; the lipid altering agent is a statin; the statin is chosen from simvastatin, rosuvastatin and atorvastatin; the lipid altering agent is a cholesterol absorption inhibitor; the cholesterol absorption inhibitor is ezetimibe; the lipid altering agent is a bile acid sequestrant; the bile acid sequestrant is chosen from cholestyramine, colesevelam and colestipol; the lipid altering agent is a fibrate; and the fibrate is fenofibrate.

Also featured is a pharmaceutical composition comprising an agent that reduces sodium absorption in the intestine and/or increases anion secretion and an anti-hypertensive agent. In various embodiments: the antihypertensive agent is selected from: a diuretic, an inhibitor of angiotensin converting enzyme, an angiotensin II receptor antagonist; a calcium channel blocker; a beta-adrenergic antagonist; alpha-adrenergic antagonist; a renin inhibitor; and an aldosterone antagonist; the pharmaceutical composition comprises administering two or more anti-hypertensive agents wherein the two or more antihypertensive agents are independently selected from: a diuretic, an inhibitor of angiotension converting enzyme, an angiotension II receptor antagonist; a calcium channel blocker; a beta-adrenergic antagonist; alpha-adrenergic antagonist; a renin inhibitor; and an aldosterone antagonist; the diuretic is selected from the group consisting of: a loop diuretic, a thiazide, a potassium sparing agent, and an osmotic diuretic; the diuretic is a loop diuretic; the loop diuretic is furosemide, bumetanide, ethacrynic or torsemide; the diuretic is a thiazide (e.g., the thiazide is bendroflumethiazide, hydrochlorothiazide, indapamide, chlortalidone or metolazone); the diuretic is a potassium sparing agent; the potassium sparing agents is amiloride or triamterene; the diuretic is an osmotic diuretic; osmotic diuretic is glucose or mannitol; the antihypertensive agent is an angiotensin converting enzyme inhibitor; the angiotensin converting enzyme inhibitor is selected from: Benazepril (Lotensin), Captopril (Capoten), Enalapril/Enalaprilat (Vasotec), Fosinopril (Monopril), Lisinopril (Zestril and Prinivil), Moexipril (Univasc), Perindopril (Aceon), Quinapril (Accupril), Ramipril (Altace), and Trandolapril (Mavik); the antihypertensive agent is an angiotensin II receptor antagonist; the angiotensin II receptor antagonist is selected from: Candesartan, Eprosartan, Irbesartan, Losartan, Olmesartan, Telmisartan, Valsartan; the antihypertensive agent is a calcium channel blocker.; the calcium channel blocker is selected from: Amlodipine (Norvasc), Felodipine (Plendil), Nicardipine (Cardene), Nifedipine (Procardia, Adalat), Nimodipine (Nimotop), Nisoldipine (Sular), Nitrendipine (Cardif, Nitrepin), and Lacidipine (Motens), Lercanidipine (Zanidip), Verapamil (Calan, Isoptin), Gallopamil (D600), Diltiazem (Cardizem), and Menthol (mint oil); the calcium channel blocker is Amlodipine; the antihypertensive agent is a beta-adrenergic antagonist; the beta-adrenergic antagonist is selected from: Dichloroisoprenaline, Practolol, Pronethaolol, Alprenolol, Carteolol, Levobunolol, Mepindolol, Metipranolol, Nadolol, Oxprenolol, Penbutolol, Pindolol, Propranolol, Sotalol, Timolol, Acebutolol, Atenolol, Betaxolol, Bisoprolol, Esmolol Metoprolol, Nebivolol, Carvedilol, Celiprolol, Labetalol, and Butoxamine; the antihypertensive agent is an alpha-adrenergic antagonist; the alpha-adrenergic antagonist is selected from: Doxazosin (Cardura), Prazosin (Minipress), Phenoxybenzamine, Phentolamine (Regitine), Tamsulosin (Flomaxtra/Flomax), Alfuzosin (Uroxatral), and Terazosin (Hytrin); the antihypertensive agent is a renin inhibitor; the renin inhibitor is selected from: Tekturna® (Rasilez and Aliskiren) and SPP635; the antihypertensive agent is an aldosterone antagonist; and the aldosterone antagonist is Spironolactone, Canrenone, or Eplerenone.

Also featured is a pharmaceutical composition comprising an agent that reduces sodium absorption in the intestine and/or increases anion secretion and a lipid altering agent. In various embodiments: the lipid altering agent is a cholesterol lowering agent; the agent lowers low density cholesterol; the lipid altering agent is selected from the group consisting of: a statin; a fibrate; niacin; a CETP inhibitor; a MTP inhibitor; a cholesterol absorption inhibitor; a squalene synthesis inhibitor; and a bile acid sequestrant; the lipid altering agent is a statin; the statin is chosen from simvastatin, rosuvastatin and atorvastatin; the lipid altering agent is a cholesterol absorption inhibitor; the cholesterol absorption inhibitor is ezetimibe; the lipid altering agent is a bile acid sequestrant; the bile acid sequestrant is chosen from cholestyramine, colesevelam and colestipol; the lipid altering agents is a fibrate; and the fibrate is fenofibrate.

Also featured is a pharmaceutical composition comprising an agent that reduces sodium absorption in the intestine and/or increases anion secretion and an agent useful in the treatment of congestive heart failure. In various embodiments: the agent useful in the treatment of congestive heart failure is selected from: Nesiritide, Dobutamine, Milrinone, Levosimendan (Simdax®), Adenosine, an adenosine analog, an agent which increases cellular availability of adenosine, an adenosine A₂ receptor agonist, an adenosine transport inhibitor, and an adenosine deaminase inhibitor; the adenosine analog is selected from: N⁶-[(1,2-dihydro-1-acenaphthylenyl)methyl]adenosine, Dipyridamole and iodotulercidin

In the various pharmaceutical compositions: the agent that reduces sodium absorption in the intestine or increases anion secretion comprises at least one GCC receptor agonist; the GCC receptor agonist is a peptide; the peptide comprises guanylin or uroguanylin; the peptide comprises ST peptide; the peptide comprises the amino acid sequence Cys Cys Glu Xaa Cys Cys Asn Pro Ala Cys Thr Gly Cys wherein Xaa is selected from Phe, Trp and Tyr (SEQ ID NO:A); the peptide comprises the amino acid sequence Cys Cys Glu Xaa Cys Cys Asn Pro Ala Cys Thr Gly Cys Tyr wherein Xaa is selected from Phe, Trp and Tyr (SEQ ID NO:B); the peptide comprises an amino acid sequence selected from:
Cys Cys Glu Tyr Cys Cys Asn Pro Ala Cys Thr Gly Cys Tyr;
Cys Cys Glu Leu Cys Cys Asn Pro Ala Cys Thr Gly Cys Tyr;
Cys Cys Glu Phe Cys Cys Asn Pro Ala Cys Thr Gly Cys Tyr;
Cys Cys Glu Trp Cys Cys Asn Pro Ala Cys Thr Gly Cys Tyr;
Cys Cys Glu Tyr Cys Cys Asn Pro Ala Cys Thr Gly Cys;
Cys Cys Glu Leu Cys Cys Asn Pro Ala Cys Thr Gly Cys;
Cys Cys Glu Phe Cys Cys Asn Pro Ala Cys Thr Gly Cys;
Cys Cys Glu Trp Cys Cys Asn Pro Ala Cys Thr Gly Cys;
d-Cys Cys Glu Tyr Cys Cys Asn Pro Ala Cys Thr Gly Cys Tyr;
d-Cys Cys Glu Leu Cys Cys Asn Pro Ala Cys Thr Gly Cys Tyr;
d-Cys Cys Glu Phe Cys Cys Asn Pro Ala Cys Thr Gly Cys Tyr;
d-Cys Cys Glu Trp Cys Cys Asn Pro Ala Cys Thr Gly Cys Tyr;
d-Cys Cys Glu Tyr Cys Cys Asn Pro Ala Cys Thr Gly Cys;
d-Cys Cys Glu Leu Cys Cys Asn Pro Ala Cys Thr Gly Cys;
d-Cys Cys Glu Phe Cys Cys Asn Pro Ala Cys Thr Gly Cys;
d-Cys Cys Glu Trp Cys Cys Asn Pro Ala Cys Thr Gly Cys;
Asn Asp Asp Cys Glu Leu Cys Val Asn Val Ala Cys Thr Gly Cys Leu;
Asn Asp Glu Cys Glu Leu Cys Val Asn Val Ala Cys Thr Gly Cys Leu;
Phe Lys Thr Leu Arg Thr Ile Ala Asn Asp Asp Cys Glu Leu Cys Val Asn Val Ala Cys Thr Gly Cys Leu;
Phe Lys Thr Leu Arg Thr Ile Ala Asn Asp Glu Cys Glu Leu Cys Val Asn Val Ala Cys Thr Gly Cys Leu;
Val Tyr Ile Gln Tyr Gln Gly Phe Arg Val Gln Leu Glu Ser Met Lys Lys Leu Ser Asp Leu Glu Ala Gln Trp Ala Pro;
Ser Pro Arg Leu Gln Ala Gln Ser Leu Leu Pro Ala Val Cys His His Pro Ala Leu Pro Gln Asp Leu Gln Pro Val Cys;

Ala Ser Gln Glu Ala Ser Ser Ile Phe Lys Thr Leu Arg Thr Ile Ala Asn Asp Asp Cys Glu Leu Cys Val Asn Val Ala
Cys Thr Gly Cys Leu;

Val Tyr Ile Gln Tyr Gln Gly Phe Arg Val Gln Leu Glu Ser Met Lys Lys Leu Ser Asp Leu Glu Ala Gln Trp Ala Pro;
Ser Pro Arg Leu Gln Ala Gln Ser Leu Leu Pro Ala Val Cys His His Pro Ala Leu Pro Gln Asp Leu Gln Pro Val Cys;

Ala Ser Gln Glu Ala Ser Ser Ile Phe Lys Thr Leu Arg Thr Ile Ala Asn Asp Glu Cys Glu Leu Cys Val Asn Val Ala
Cys Thr Gly Cys Leu;

Pro Gly Thr Cys Glu Ile Cys Ala Tyr Ala Ala Cys Thr Gly Cys;

Val Thr Val Gln Asp Gly Asn Phe Ser Phe Ser Leu Glu Ser Val Lys Lys Leu Lys Asp Leu Gln Glu Pro Gln Glu
Pro;

Arg Val Gly Lys Leu Arg Asn Phe Ala Pro Ile Pro Gly Glu Pro Val Val Pro Ile Leu Cys Ser Asn Pro Asn Phe Pro;
Glu Glu Leu Lys Pro Leu Cys Lys Glu Pro Asn Ala Gln Glu Ile Leu Gln Arg Leu Glu Glu Ile Ala Glu Asp Pro Gly Thr Cys Glu Ile Cys Ala Tyr Ala Ala Cys Thr Gly Cys;

Met Pro Ser Thr Gln Tyr Ile Arg Arg Pro Ala Ser Ser Tyr Ala Ser Cys Ile Trp Cys Thr Thr Ala Cys Ala Ser Cys His
Gly Arg Thr Thr Lys Pro Ser Leu Ala Thr;

Ala Asp Leu Cys Glu Ile Cys Ala Phe Ala Ala Cys Thr Gly Cys Leu;
Val Gln Val Glu Glu Gly Gly Phe Ser Phe Pro Leu Asp Ala Val Lys Lys Leu Glu Glu Leu Met Gly Val Asp Met;
Thr Val Lys Gln Ser Pro Arg Leu Ala Lys Thr Ser Thr Thr Ala Val Cys Thr Asn Pro Asp Leu Pro Ala Val Phe Leu;

Pro Leu Cys Lys Ser Lys Gly Ala Ala Asn Ser Phe Phe Arg Leu Gly Phe Val Ala Ala Arg Ala Asp Leu Cys Glu Ile
Cys Ala Phe Ala Ala Cys Thr Gly Cys Leu;

Gln Glu Glu Cys Glu Leu Cys Ile Asn Met Ala Cys Thr Gly Tyr;

Val Tyr Ile Gln Tyr Glu Gly Phe Gln Val Asn Leu Asp Ser Val Lys Lys Leu Asp Lys Leu Leu Glu Gln Leu Arg
Gly;

Phe His His Gln Met Gly Asp Gln Arg Asp Pro Ser Ile Leu Cys Ser Asp Pro Ala Leu Pro Ser Asp Leu Gln Pro Val; and

Cys Glu Asn Ser Gln Ala Val Asn Ile Phe Arg Ala Leu Arg Tyr Ile Asn Gln Glu Glu Cys Glu Leu Cys Ile Asn Met
Ala Cys Thr Gly Tyr.

In the various pharmaceutical compositions: the agent that reduces sodium absorption in the intestine and/or increases anion secretion comprises a prostanoid, prostaglandin E1 or a prostaglandin E1 derivative; the prostaglandin E1 derivative is misoprostol; the prostaglandin E1 derivative is lubiprostone; the agent that reduces sodium absorption in the intestine and/or increases anion secretion comprises at least one 5HT4 receptor agonist; the 5HT4 receptor agonist is Zelnorm; the agent that reduces sodium absorption in the intestine and/or increases anion secretion comprises at least one cyclic nucleotide; the cyclic nucleotide is cGMP; and the cyclic nucleotide is cAMP.

In various cases the method comprises administering an agent that reduces sodium absorption in the intestine and/or increases anion secretion and an agent useful in the treatment of congestive heart failure. For example, the agent useful in the treatment of congestive heart failure is selected from: Nesiritide, Dobutamine, Milrinone, Levosimendan (Simdax®), Adenosine, an adenosine analog, an agent which increases cellular availability of adenosine, an adenosine A₂ receptor agonist, an adenosine transport inhibitor, and an adenosine deaminase inhibitor; and the adenosine analog is selected from: N⁶ -[(1,2-dihydro-1-acenaphthylenyl)methyl]adenosine, Dipyridamole and iodotulercidin

### DESCRIPTION OF DRAWINGS

Figure 1 depicts the results of a study of the effect of lubiprostone of urinary sodium and urine volume in rats.
Figure 2 depicts the results of a study on the effect of demonstrate ST peptide (SEQ ID NO: 1: CCELCCNPACTGCY) at 300 Tg/kg on urine sodium and urine volume in rats.
Figure 3 depicts the results of a study on the effect of demonstrate ST peptide (SEQ ID NO: 1: CCELCCNPACTGCY) at 1000 Tg/kg on urine sodium and urine volume in rats.

### DETAILED DESCRIPTION

A major function of salt is to regulate blood volume and pressure including the flexibility of the blood vessels. When blood volume increases or the blood vessel walls don't expand enough, blood pressure increases. Blood pressure is an indicator of the incidence of cardiovascular events like heart attacks and strokes. Salt sensitivity is a measure of how blood pressure responds to a decrease in salt intake. Salt-sensitive individuals experience an exaggerated blood pressure elevation when they are given a salt load. Among those who already have hypertension, salt sensitivity increases the risk of developing cardiovascular conditions such as heart attack, left ventricular hypertrophy and kidney problems. Recent studies by the National Institutes of Health suggest that salt sensitivity increases the risk of death even for those with normal blood pressure. In the absence of hypertension, salt may increase the reactivity of platelets, thus causing abnormal blood clot formation which in turn leads to cardiovascular events like stroke, heart attack, and kidney disease directly. Although global initiatives have been undertaken to reduce dietary salt intake voluntary compliance is thus far, unachievable. Therefore, agents that reduce sodium absorption (e.g. dietary sodium) or increase anion secretion in the intestine are useful in the treatment of cardiovascular disease and other disorders associated with fluid and/or sodium retention.

The agents described herein can be used alone or in combination therapy to prevent and/or treat cardiovascular disorders, including: hypertension (for example, including salt sensitive hypertension), cardiomyopathy (for example, associated with Chagas disease), cardiac hypertrophy, heart attack, stroke, congestive heart failure (including heart failure at any of stages I-IV according to New York Heart Association (NYHA) Functional Classification), and bone wasting (e.g. bone wasting associated with cardiovascular disorders). Congestive heart failure is a hemodynamic disorder resulting from impairment of the ability of the ventricle to fill with and/or eject blood. The disorder is commonly characterized by shortness of breath, fatigue, limited exercise tolerance, and fluid retention (both pulmonary congestion and peripheral edema). Congestive heart failure is generally progressive and can result in Class IV heart failure (NYHA Heart Failure Classification) in which any physical activity brings on symptoms such as shortness of breath, and symptoms can occur even when the patient is at rest. Patients with symptoms of advanced heart failure are treated by tightly controlling fluid status and are often administered intravenous peripheral vasodilators and/or positive inotropic agents. Patients suffering Class IV heart failure should be at complete rest (confined to a bed or chair).

The agents described herein can be used alone or in combination therapy to prevent and/or treat renal disorders associated with fluid or salt retention. Renal disorders includes renal failure (including acute and chronic renal failure), renal insufficiency, nephrotic edema, glomerulonephritis, pyelonephritis, kidney failure, chronic renal failure, nephritis, nephrosis, uremia, immune renal disease, acute nephritic syndrome, rapidly progressive nephritic syndrome, nephrotic syndrome, chronic nephritic/proteinuric syndrome, tubulointerstital disease, nephrotoxic disorders, renal cortical necrosis, polycystic kidney disease, and hereditary nephritis, along with any disease or disorder that relates to the renal system and related disorders, as well as symptoms indicative of, or related to, renal or kidney disease and related disorders.

The agents described herein can be used alone or in combination therapy to prevent and/or treat disorders associated with ascites. Ascites (also known as peritoneal cavity fluid, peritoneal fluid excess, hydroperitoneum or abdominal dropsy) is an accumulation of fluid in the peritoneal cavity. Currently, salt restriction is a baseline step in therapy for ascites related disorders including liver disorders such as cirrhosis (e.g. cirrhosis associated with alcoholism, viral hepatitis, or cryptogenic cirrhosis), chronic hepatitis, severe alcoholic hepatitis without cirrhosis, Budd-Chiari syndrome (a veno-occlusive disease involving obstruction of the hepatic vein), and cardiovascular disorders such as heart failure and constrictive pericarditis.

Without being bound by any particular theory, in the case of disorders associated with fluid or salt retention, the methods may elicit one or more of decreased sodium absorption and/or increased anion absorption.

### Useful Agents

Agents that reduce sodium absorption in the intestine or increases anion secretion in the intestine include, but are not limited to, guanylate cyclase receptor C agonists, soluble guanylate cyclase modulators, prostanoids including prostaglandin E and derivatives thereof, chloride channel activators (e.g. Amitiza® (lubiprostone)), 5HT4 agonists, cyclic nucleotides, laxatives, CFTR

(cystic fibrosis transmembrane conductance regulator) modulators, agents which affect cAMP levels, sodium transport inhibitors (e.g. sodium channel inhibitors such as amiloride), phosphodiesterase inhibitors, renin inhibitors and aldosterone antagonists, potassium, polymer resins, combinations thereof.

### Guanylate cyclase-C agonists

The guanylate cyclase-C (GC-C) receptor (reviewed by Lucas et al. 2000 Pharmacol. Rev 52:375-414 and Vaandrager et al. 2002 Molecular and Cellular Biochemistry 230:73-83) is a key regulator in mammals of intestinal function (although low levels of GC-C have been detected in other tissues). GC-C responds to the endogenous hormones, guanylin and uroguanylin, and to enteric bacterial peptides from the heat stable enterotoxin family (ST peptides). When agonists bind to GC-C, there is an elevation of the second messenger, cyclic GMP, and an increase in chloride and bicarbonate secretion, resulting in an increase in intestinal fluid secretion.

In addition to being expressed in the intestine by gastrointestinal epithelial cells, GC-C is expressed in extra-intestinal tissues including kidney, lung, pancreas, pituitary, adrenal, developing liver and gall bladder (reviewed in Vaandrager 2002 Mol Cell Biochem 230:73-83, Kulaksiz et al. 2004, Gastroenterology 126:732-740) and male and female reproductive tissues (reviewed in Vaandrager 2002 Mol Cell Biochem 230:73-83). This suggests that the GC-C receptor agonists can be used in the treatment of disorders outside the GI tract, for example, congestive heart failure and benign prostatic hyperplasia.

In humans, the GC-C receptor is activated by guanylin (Gn) (U.S. 5,969,097), uroguanylin (Ugn) (U.S. 5,140,102) and lymphoguanylin (Forte et al. 1999 Endocrinology 140:1800-1806). Interestingly, these agents are 10-100 fold less potent than a class of bacterially derived peptides, termed ST (reviewed in Gianella 1995 J Lab Clin Med 125:173-181). ST peptides are considered super agonists of GC-C and are very resistant to proteolytic degradation. In bacteria, ST peptides are derived from a preproprotein that generally has at least 70 amino acids. The pre and pro regions are cleaved as part of the secretion process, and the resulting mature protein, which generally includes fewer than 20 amino acids, is biologically active.

Among the known bacterial ST peptides are: *E. coli* ST Ib (Moseley et al. 1983 Infect. Immun. 39:1167) having the mature amino acid sequence Asn Ser Ser Asn Tyr Cys Cys Glu Leu Cys Cys Asn Pro Ala Cys Thr Gly Cys Tyr (SEQ ID NO:_); *E. coli* ST Ia (So and McCarthy 1980 Proc. Natl. Acad. Sci. USA 77:4011) having the mature amino acid sequence Asn Thr Phe Tyr Cys Cys Glu Leu Cys Cys Asn Pro Ala Cys Ala Gly Cys Tyr (SEQ ID NO:7). *E. coli* ST I* (Chan and Giannella 1981 J. Biol. Chem. 256:7744) having the mature amino acid sequence Asn Thr Phe Tyr Cys Cys Glu Leu Cys Cys Tyr Pro Ala Cys Ala Gly Cys Asn (SEQ ID NO:__); *C.freundii* ST peptide (Guarino et al. 1989b *Infect. Immun.* 57:649) having the mature amino acid sequence Asn Thr Phe Tyr Cys Cys Glu Leu Cys Cys Asn Pro Ala Cys Ala Gly Cys Tyr (SEQ ID NO:__);*Y. enterocolitica* ST peptides, Y-ST(Y-STa), Y-STb, and Y-STc (reviewed in Huang et al. 1997 Microb. Pathog. 22:89) having the following pro-form amino acid sequences: Gln Ala Cys Asp Pro Pro Ser Pro Pro Ala Glu Val Ser Ser Asp Trp Asp Cys Cys Asp Val Cys Cys Asn Pro Ala Cys Ala Gly Cys (SEQ ID NO:__) (as well as a Ser-7 to Leu-7 variant of Y-STa (SEQ ID NO:__), (Takao et al. 1985 Eur. J. Biochem. 152:199)); Lys Ala Cys Asp Thr Gln Thr Pro Ser Pro Ser Glu Glu Asn Asp Asp Trp Cys Cys Glu Val Cys Cys Asn Pro Ala Cys Ala Gly Cys (SEQ ID NO:__);Gln Glu Thr Ala Ser Gly Gln Val Gly Asp Val Ser Ser Ser Thr Ile Ala Thr Glu Val Ser Glu Ala Glu Cys Gly Thr Gln Ser Ala Thr Thr Gln Gly Glu Asn Asp Trp Asp Trp Cys Cys Glu Leu Cys Cys Asn Pro Ala Cys Phe Gly Cys (SEQ ID NO:__), respectively; *Y. kristensenii* ST peptide having the mature amino acid sequence Ser Asp Trp Cys Cys Glu Val Cys Cys Asn Pro Ala Cys Ala Gly Cys (SEQ ID NO:__);V. *cholerae* non-01 ST peptide (Takao et al. (1985) *FEBS lett.* 193:250) having the mature amino acid sequence Ile Asp Cys Cys Glu Ile Cys Cys Asn Pro Ala Cys Phe Gly Cys Leu Asn (SEQ ID NO:_); and *V. mimicus* ST peptide (Arita et al. 1991 FEMS Microbiol. Lett. 79:105) having the mature amino acid sequence Ile Asp Cys Cys Glu Ile Cys Cys Asn Pro Ala Cys Phe Gly Cys Leu Asn (SEQ ID NO:__). The immature (including pre and pro regions) form of *E. coli* ST-1A (ST-P) protein has the sequence: mkklmlaifisvlsfpsfsqstesldsskekitletkkcdvvknnsekksenmnntfyccelccnpacagcy (SEQ ID NO:__; see GenBank^{®} Accession No. P01559 (gi:123711). The pre sequence extends from aa 1-19. The pro sequence extends from aa 20-54. The mature protein extends from 55-72. The immature (including pre and pro regions) form of *E. coli* ST-1B (ST-H) protein has the sequence: mkksilfiflsvlsfspfaqdakpvesskekitleskkcniakksnksgpesmnssnyccelccnpactgcy (SEQ ID NO:__; see GenBank^{®} Accession No. P07965 (gi:3915589)). The immature (including pre and pro regions) form of *Y. enterocolitica* ST protein has the sequence:
mkkivfvlvlmlssfgafgqetvsgqfsdalstpitaevykqacdpplppaevssdwdccdvccnpacagc (SEQ ID NO:__; see GenBank^{®} Accession No. S25659 (gi:282047)).

GC-C agonist peptides of particular interest include peptides consisting, consisting essentially of or comprising:
Cys Cys Glu Tyr Cys Cys Asn Pro Ala Cys Thr Gly Cys Tyr;
Cys Cys Glu Leu Cys Cys Asn Pro Ala Cys Thr Gly Cys Tyr;
Cys Cys Glu Phe Cys Cys Asn Pro Ala Cys Thr Gly Cys Tyr;
Cys Cys Glu Trp Cys Cys Asn Pro Ala Cys Thr Gly Cys Tyr;
Cys Cys Glu Tyr Cys Cys Asn Pro Ala Cys Thr Gly Cys;
Cys Cys Glu Leu Cys Cys Asn Pro Ala Cys Thr Gly Cys;
Cys Cys Glu Phe Cys Cys Asn Pro Ala Cys Thr Gly Cys;
Cys Cys Glu Trp Cys Cys Asn Pro Ala Cys Thr Gly Cys;
d-Cys Cys Glu Tyr Cys Cys Asn Pro Ala Cys Thr Gly Cys Tyr;
d-Cys Cys Glu Leu Cys Cys Asn Pro Ala Cys Thr Gly Cys Tyr;
d-Cys Cys Glu Phe Cys Cys Asn Pro Ala Cys Thr Gly Cys Tyr;
d-Cys Cys Glu Trp Cys Cys Asn Pro Ala Cys Thr Gly Cys Tyr;
d-Cys Cys Glu Tyr Cys Cys Asn Pro Ala Cys Thr Gly Cys;
d-Cys Cys Glu Leu Cys Cys Asn Pro Ala Cys Thr Gly Cys;
d-Cys Cys Glu Phe Cys Cys Asn Pro Ala Cys Thr Gly Cys;
d-Cys Cys Glu Trp Cys Cys Asn Pro Ala Cys Thr Gly Cys;
Asn Asp Asp Cys Glu Leu Cys Val Asn Val Ala Cys Thr Gly Cys Leu;
Asn Asp Glu Cys Glu Leu Cys Val Asn Val Ala Cys Thr Gly Cys Leu;
Phe Lys Thr Leu Arg Thr Ile Ala Asn Asp Asp Cys Glu Leu Cys Val Asn Val Ala Cys Thr Gly Cys Leu;
Phe Lys Thr Leu Arg Thr Ile Ala Asn Asp Glu Cys Glu Leu Cys Val Asn Val Ala Cys Thr Gly Cys Leu;
Val Tyr Ile Gln Tyr Gln Gly Phe Arg Val Gln Leu Glu Ser Met Lys Lys Leu Ser Asp Leu Glu Ala Gln Trp Ala Pro;
Ser Pro Arg Leu Gln Ala Gln Ser Leu Leu Pro Ala Val Cys His His Pro Ala Leu Pro Gln Asp Leu Gln Pro Val Cys;

Ala Ser Gln Glu Ala Ser Ser Ile Phe Lys Thr Leu Arg Thr Ile Ala Asn Asp Asp Cys Glu Leu Cys Val Asn Val Ala
Cys Thr Gly Cys Leu;

Val Tyr Ile Gln Tyr Gln Gly Phe Arg Val Gln Leu Glu Ser Met Lys Lys Leu Ser Asp Leu Glu Ala Gln Trp Ala Pro;
Ser Pro Arg Leu Gln Ala Gln Ser Leu Leu Pro Ala Val Cys His His Pro Ala Leu Pro Gln Asp Leu Gln Pro Val Cys;

Ala Ser Gln Glu Ala Ser Ser Ile Phe Lys Thr Leu Arg Thr Ile Ala Asn Asp Glu Cys Glu Leu Cys Val Asn Val Ala
Cys Thr Gly Cys Leu;

Pro Gly Thr Cys Glu Ile Cys Ala Tyr Ala Ala Cys Thr Gly Cys;

Val Thr Val Gln Asp Gly Asn Phe Ser Phe Ser Leu Glu Ser Val Lys Lys Leu Lys Asp Leu Gln Glu Pro Gln Glu
Pro;

Arg Val Gly Lys Leu Arg Asn Phe Ala Pro Ile Pro Gly Glu Pro Val Val Pro Ile Leu Cys Ser Asn Pro Asn Phe Pro;

Glu Glu Leu Lys Pro Leu Cys Lys Glu Pro Asn Ala Gln Glu Ile Leu Gln Arg Leu Glu Glu Ile Ala Glu Asp Pro Gly
Thr Cys Glu Ile Cys Ala Tyr Ala Ala Cys Thr Gly Cys;

Met Pro Ser Thr Gln Tyr Ile Arg Arg Pro Ala Ser Ser Tyr Ala Ser Cys Ile Trp Cys Thr Thr Ala Cys Ala Ser Cys His
Gly Arg Thr Thr Lys Pro Ser Leu Ala Thr;

Ala Asp Leu Cys Glu Ile Cys Ala Phe Ala Ala Cys Thr Gly Cys Leu;
Val Gln Val Glu Glu Gly Gly Phe Ser Phe Pro Leu Asp Ala Val Lys Lys Leu Glu Glu Leu Met Gly Val Asp Met;
Thr Val Lys Gln Ser Pro Arg Leu Ala Lys Thr Ser Thr Thr Ala Val Cys Thr Asn Pro Asp Leu Pro Ala Val Phe Leu;

Pro Leu Cys Lys Ser Lys Gly Ala Ala Asn Ser Phe Phe Arg Leu Gly Phe Val Ala Ala Arg Ala Asp Leu Cys Glu Ile
Cys Ala Phe Ala Ala Cys Thr Gly Cys Leu;

Gln Glu Glu Cys Glu Leu Cys Ile Asn Met Ala Cys Thr Gly Tyr;

Val Tyr Ile Gln Tyr Glu Gly Phe Gln Val Asn Leu Asp Ser Val Lys Lys Leu Asp Lys Leu Leu Glu Gln Leu Arg
Gly;

Phe His His Gln Met Gly Asp Gln Arg Asp Pro Ser Ile Leu Cys Ser Asp Pro Ala Leu Pro Ser Asp Leu Gln Pro Val; and

Cys Glu Asn Ser Gln Ala Val Asn Ile Phe Arg Ala Leu Arg Tyr Ile Asn Gln Glu Glu Cys Glu Leu Cys Ile Asn Met
Ala Cys Thr Gly Tyr.

The peptides which act as GC-C receptor agonists include peptides related to guanlyin, uroguanylin, ST peptide and other peptides described in US 2004/0266989; US 2005/0020811; US 2006/0094658; US 2006/0258593; US 2006/0281682; and US 2007/0010450, all of which are hereby incorporated by reference in their entirety.

Described herein are pharmaceutical compositions comprising certain peptides that are capable of activating the guanylate-cyclase C (GC-C) receptor and one or more additional therapeutic agents including, without limitation, the agents described herein. The other agents can be administered with the peptides described herein (simultaneously or sequentially). They can also be linked to a peptide described herein to create therapeutic conjugates.

### Variant GC-C agonist Peptides

The disclosure includes variant peptides which can include one, two, three, four, five, six, seven, eight, nine, or ten (in some embodiments fewer than 5 or fewer than 3 or 2 or fewer) amino acid substitutions and/or deletions compared to the sequence of a peptided described herein. In certain embodiments, the cysteine resides are neither substituted nor deleted. The substitution(s) can be conservative or non-conservative. The naturally-occurring amino acids can be substituted by D-isomers of any amino acid, non-natural amino acids, natural and natural amino acid analogs and other groups. A conservative amino acid substitution results in the alteration of an amino acid for a similar acting amino acid, or amino acid of like charge, polarity, or hydrophobicity. At some positions, even conservative amino acid substitutions can alter the activity of the peptide. A conservative substitution can substitute a naturally-occurring amino acid for a non-naturally-occurring amino acid. The amino acid substitutions among naturally-occurring amino acids are listed in Table I.

**Table I**

| For Amino Acid | Code | Replace with any of |
|---|---|---|
| Alanine | Ala | Gly, Cys, Ser |
| Arginine | Arg | Lys, His |
| Asparagine | Asn | Asp, Glu, Gln, |
| Aspartic Acid | Asp | Asn, Glu, Gln |
| Cysteine | Cys | Met, Thr, Ser |
| Glutamine | Gln | Asn, Glu, Asp |
| Glutamic Acid | Glu | Asp, Asn, Gln |
| Glycine | Gly | Ala |
| Histidine | His | Lys, Arg |
| Isoleucine | Ile | Val, Leu, Met |
| Leucine | Leu | Val, Ile, Met |
| Lysine | Lys | Arg, His |
| Methionine | Met | Ile, Leu, Val |
| Phenylalanine | Phe | Tyr, His, Trp |
| Proline | Pro | |
| Serine | Ser | Thr, Cys, Ala |
| Threonine | Thr | Ser, Met, Val |
| Tryptophan | Trp | Phe, Tyr |
| Tyrosine | Tyr | Phe, His |
| Valine | Val | Leu, Ile, Met |

In some circumstances it can be desirable to treat patients with a variant peptide that binds to and activates intestinal GC-C receptor, but is less active than the non-variant form the peptide. This reduced activity can arise from reduced affinity for the receptor or a reduced ability to activate the receptor once bound or reduced stability of the peptide.

In certain embodiments, one or more amino acids can be replaced by a non-naturally occurring amino acid or a naturally or non-naturally occurring amino acid analog. In certain embodiments, one or more L-amino acids can be substituted with a D-amino acid. There are many amino acids beyond the standard 20 amino acids (Ala, Arg, Asn, Asp, Cys, Gln, Glu, Gly, His, Ile, Leu, Lys, Met, Phe, Pro, Ser, Thr, Trp, Tyr, and Val). Some are naturally-occurring others are not (see, for example, Hunt, The Non-Protein Amino Acids: In Chemistry and Biochemistry of the Amino Acids, Barrett, Chapman and Hall, 1985). For example, an aromatic amino acid can be replaced by 3,4-dihydroxy-L-phenylalanine, 3-iodo-L-tyrosine, triiodothyronine, L-thyroxine, phenylglycine (Phg) or nor-tyrosine (norTyr). Phg and norTyr and other amino acids including Phe and Tyr can be substituted by, e.g., a halogen, -CH₃, -OH, -CH₂NH₃, -C(O)H, -CH₂CH₃,-CN, -CH₂CH₂CH₃, -SH, or another group. Any amino acid can be substituted by the D-form of the amino acid. Thus, for example, a cysteine residue can be substituted by a D-cysteine residue.

Glu can be replaced by *gamma*-Hydroxy-Glu or *gamma*-Carboxy-Glu.

Ala can be replaced by an alpha substituted amino acid such as L-*alpha*-methylphenylalanine or by analogues such as: 3-Amino-Tyr; Tyr(CH₃); Tyr(PO₃(CH₃)₂); Tyr(SO₃H); *beta*-Cyclohexyl-Ala; *beta*-(1-Cyclopentenyl)-Ala; *beta*-Cyclopentyl-Ala; *beta*-Cyclopropyl-Ala; *beta*-Quinolyl-Ala; *beta*-(2-Thiazolyl)-Ala; *beta*-(Triazole-1-yl)-Ala; *beta*-(2-Pyridyl)-Ala; *beta*-(3-Pyridyl)-Ala; Amino-Phe; Fluoro-Phe; Cyclohexyl-Gly; tBu-Gly; *beta*-(3-benzothienyl)-Ala; *beta*-(2-thienyl)-Ala; 5-Methyl-Trp; and 4-Methyl-Trp.

Pro can be an N(alpha)-C(*alpha*) cyclized amino acid analogues with the structure:

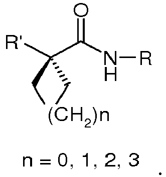

Pro can also be homopro (L-pipecolic acid); hydroxy-Pro; 3,4-Dehydro-Pro; 4-fluoro-Pro; or *alpha*-methyl-Pro.

Val or Leu can also be an alpha-substitued or N-methylated amino acid such as *alpha-*amino isobutyric acid (*aib*)*,* L/D-*alpha*-ethylalanine (L/D-isovaline), L/D-methylvaline, or L/D-*alpha-*methylleucine or a non-natural amino acid such as *beta*-fluoro-Ala.

Gly can be *alpha*-amino isobutyric acid (*aib*) or L/D-*alpha*-ethylalanine (L/D-isovaline).

Further examples of unnatural amino acids include: an unnatural analogue of tyrosine; an unnatural analogue of glutamine; an unnatural analogue of phenylalanine; an unnatural analogue of serine; an unnatural analogue of threonine; an alkyl, aryl, acyl, azido, cyano, halo, hydrazine, hydrazide, hydroxyl, alkenyl, alkynl, ether, thiol, sulfonyl, seleno, ester, thioacid, borate, boronate, phospho, phosphono, phosphine, heterocyclic, enone, imine, aldehyde, hydroxylamine, keto, or amino substituted amino acid, or any combination thereof; an amino acid with a photoactivatable cross-linker; a spin-labeled amino acid; a fluorescent amino acid; an amino acid with a novel functional group; an amino acid that covalently or noncovalently interacts with another molecule; a metal binding amino acid; an amino acid that is amidated at a site that is not naturally amidated, a metal-containing amino acid; a radioactive amino acid; a photocaged and/or photoisomerizable amino acid; a biotin or biotin-analogue containing amino acid; a glycosylated or carbohydrate modified amino acid; a keto containing amino acid; amino acids comprising polyethylene glycol or polyether; a heavy atom substituted amino acid (e.g., an amino acid containing deuterium, tritium, ¹³C, ¹⁵N, or ¹⁸O); a chemically cleavable or photocleavable amino acid; an amino acid with an elongated side chain; an amino acid containing a toxic group; a sugar substituted amino acid, e.g., a sugar substituted serine or the like; a carbon-linked sugar-containing amino acid; a redox-active amino acid; an α.-hydroxy containing acid; an amino thio acid containing amino acid; an α, α, disubstituted amino acid; a β-amino acid; a cyclic amino acid other than proline; an O-methyl-L-tyrosine; an L-3-(2-naphthyl)alanine; a 3-methyl-phenylalanine; a *p*-acetyl-L-phenylalanine; an 0-4-allyl-L-tyrosine; a 4-propyl-L-tyrosine; a tri-O-acetyl-GlcNAcβ-serine; an L-Dopa; a fluorinated phenylalanine; an isopropyl-L-phenylalanine; a p-azido-L-phenylalanine; a p-acyl-L-phenylalanine; a p-benzoyl-L-phenylalanine; an L-phosphoserine; a phosphonoserine; a phosphonotyrosine; a p-iodo-phenylalanine; a 4-fluorophenylglycine; a p-bromophenylalanine; a p-amino-L-phenylalanine; an isopropyl-L-phenylalanine; L-3-(2-naphthyl)alanine; an amino-, isopropyl-, or O-allyl-containing phenylalanine analogue; a dopa, O-methyl-L-tyrosine; a glycosylated amino acid; a p-(propargyloxy)phenylalanine; dimethyl-Lysine; hydroxy-proline; mercaptopropionic acid; methyl-lysine; 3-nitro-tyrosine; norleucine; pyro-glutamic acid; Z (Carbobenzoxyl); ε-Acetyl-Lysine; β-Alanine; aminobenzoyl derivative; aminobutyric acid (Abu); citrulline; aminohexanoic acid; aminoisobutyric acid; cyclohexylalanine; d-cyclohexylalanine; hydroxyproline; nitro-arginine; nitro-phenylalanine; nitro-tyrosine; norvaline; octahydroindole carboxylate; ornithine; penicillamine; tetrahydroisoquinoline; acetamidomethyl protected amino acids and pegylated amino acids. Further examples of unnatural amino acids and amino acid analogs can be found in U.S. 20030108885, U.S. 20030082575, US20060019347 (paragraphs 410-418) and the references cited therein. The peptides described herein can include further modifications including those described in US20060019347

In some embodiments, an amino acid can be replaced by a naturally-occurring, non-essential amino acid, e.g., taurine.

Methods to manfacture peptides containing unnatural amino acids can be found in, for example, U.S. 20030108885, U.S. 20030082575, US20060019347, Deiters et al., J Am Chem Soc. (2003) 125:11782-3, Chin et al., Science (2003) 301:964-7, and the references cited therein.

Peptides that include non-natural amino acids can also be prepared using the methods described in WO02086075

The peptides described herein can have one or more conventional peptide bonds replaced by an alternative bond. Such replacements can increase the stability of the peptide. For example, replacement of the peptide bond between D-Cys₁₅ or Cys₁₅ and Xaa₁₆ with an alternative bond can reduce cleavage by carboxy peptidases and may increase half-life in the digestive tract. Bonds that can replace peptide bonds include: a retro-inverso bonds (C(O)-NH instead of NH-C(O); a reduced amide bond (NH-CH₂); a thiomethylene bond (S-CH₂ or CH₂-S); an oxomethylene bond (O- CH₂ or CH₂-O); an ethylene bond (CH₂-CH₂); a thioamide bond (C(S)-NH); a trans-olefine bond (CH=CH); an fluoro substituted trans-olefine bond (CF=CH); a ketomethylene bond (C(O)-CHR or CHR-C(O) wherein R is H or CH₃; and a fluoro-ketomethylene bond (C(O)-CFR or CFR-C(O) wherein R is H or F or CH₃.

The peptides described herein can be modified using standard modifications. Modifications may occur at the amino (N-), carboxy (C-) terminus, internally or a combination of any of the preceeding. In one aspect described herein, there may be more than one type of modification of the peptide. Modifications include but are not limited to: acetylation, amidation, biotinylation, cinnamoylation, farnesylation, formylation, myristoylation, palmitoylation, phosphorylation (Ser, Tyr or Thr), stearoylation, succinylation, sulfurylation and cyclisation (via disulfide bridges or amide cyclisation), and modification by Cy3 or Cy5. The peptides described herein may also be modified by 2, 4-dinitrophenyl (DNP), DNP-lysin, modification by 7-Amino-4-methyl-coumarin (AMC), flourescein, NBD (7-Nitrobenz-2-Oxa-1,3-Diazole), p-nitro-anilide, rhodamine B, EDANS (5-((2-aminoethyl)amino)naphthalene-1- sulfonic acid), dabcyl, dabsyl, dansyl, texas red, FMOC, and Tamra (Tetramethylrhodamine). The peptides described herein may also be conjugated to, for example, polyethylene glycol (PEG); alkyl groups (e.g., C1-C20 straight or branched alkyl groups); fatty acid radicals; combinations of PEG, alkyl groups and fatty acid radicals (see U.S. Patent 6,309,633; Soltero et al., 2001 Innovations in Pharmaceutical Technology 106-110); BSA and KLH (Keyhole Limpet Hemocyanin). The addition of PEG and other polymers which can be used to modify peptides described herein is described in US2006019347 section IX.

The peptides and agonists described herein can be chemically modified to increase therapeutic activity by synthetically adding sugar moieties (WO 88/02756; WO 89/09786; DE 3910667 A1, EP 0 374 089 A2; and U.S. 4,861,755), adding cationic anchors (EP0363589), lipid moieties (WO91/09837; U.S. 4,837,303) or the substituents described as compounds I, II, and III in US5552520.

Certain GCC agonist peptides described herein bear some sequence similarity to ST peptides. However, they include amino acid changes and/or additions that improve functionality. These changes can, for example, increase or decrease activity (e.g., increase or decrease the ability of the peptide to stimulate intestinal motility), alter the ability of the peptide to fold correctly, alter the stability of the peptide, alter the ability of the peptide to bind the GC-C receptor and/or decrease toxicity. In some cases the peptides may function more desirably than wild-type ST peptide. For example, they may limit undesirable side effects such as diarrhea and dehydration.

In some embodiments one or both members of one or more pairs of Cys residues (including where a Cys residue has been substituted with a D-cys residue) which normally form a disulfide bond can be replaced by homocysteine, penicillamine, 3-mercaptoproline (Kolodziej et al. 1996 Int J Pept Protein Res 48:274); β, β dimethylcysteine (Hunt et al. 1993 Int J Pept Protein Res 42:249) or diaminopropionic acid (Smith et al. 1978 J Med Chem 21:117) to form alternative internal cross-links at the positions of the normal disulfide bonds.

In addition, one or more disulfide bonds can be replaced by alternative covalent cross-links, e.g., an amide linkage (-CH₂CH(O)NHCH₂- or -CH₂NHCH(O)CH₂-), an ester linkage, a thioester linkage, a lactam bridge, a carbamoyl linkage, a urea linkage, a thiourea linkage, a phosphonate ester linkage, an alkyl linkage (-CH₂CH₂CH₂CH₂-), an alkenyl linkage(-CH₂CH=CHCH₂-), an ether linkage (-CH₂CH₂OCH₂- or -CH₂OCH₂CH₂-), a thioether linkage (-CH₂CH₂SCH₂- or-CH₂SCH₂CH₂-), an amine linkage (-CH₂CH₂NHCH₂- or -CH₂NHCH₂CH₂-) or a thioamide linkage (-CH₂CH(S)HNHCH₂- or -CH₂NHCH(S)CH₂-). For example, Ledu et al. (Proc Nat'l Acad. Sci. 100:11263-78, 2003) describe methods for preparing lactam and amide cross-links. Schafmeister et al. (J. Am. Chem. Soc. 122:5891, 2000) describes stable, hydrocarbon cross-links. Hydrocarbon cross links can be produced via metathesis (or methathesis followed by hydrogenation in the case of saturated hydrocarbons cross-links) using one or another of the Grubbs catalysts (available from Materia, Inc. and Sigma-Aldrich and described, for example, in U.S. Patent No. 5,831,108 and 6,111,121). In some cases, the generation of such alternative cross-links requires replacing the Cys residues with other residues such as Lys or Glu or non-naturally occurring amino acids. In addition the lactam, amide and hydrocarbon cross-links can be used to stabilize the peptide even if they link amino acids at postions other than those occupied by Cys. Such cross-links can occur between two amino acids that are separated by two amino acids or between two amino acids that are separated by six amino acids (see, e.g., Schafmeister et al. (J. Am. Chem. Soc. 122:5891, 2000)).

The peptide may contain additional carboxyterminal or amino terminal amino acids or both. For example, the peptide can include an amino terminal sequence that facilitates recombinant production of the peptide and is cleaved prior to administration of the peptide to a patient. The peptide can also include other amino terminal or carboxyterminal amino acids. In some cases the additional amino acids protect the peptide, stabilize the peptide or alter the activity of the peptide. In some cases some or all of these additional amino acids are removed prior to administration of the peptide to a patient. The peptide can include 1, 2, 3, 4, 5, 10, 15, 20, 25, 30, 40, 50, 60, 70 80, 90, 100 or more amino acids at its amino terminus or carboxy terminus or both. The number of flanking amino acids need not be the same. For example, there can be 10 additional amino acids at the amino terminus of the peptide and none at the carboxy terminus.

The peptides can be co-administered with or linked, e.g., covalently linked to any of a variety of other peptides or compounds including analgesic peptides or analgesic compounds including, without limitation, the agents described herein.

Amino acid, non-amino acid, peptide and non-peptide spacers can be interposed between a peptide that is a GC-C receptor agonist and a peptide that has some other biological function, e.g., an analgesic peptide or a peptide used to treat obesity. The linker can be one that is cleaved from the flanking peptides *in vivo* or one that remains linked to the flanking peptides *in vivo.* For example, glycine, beta-alanine, glycyl-glycine, glycyl-beta-alanine, gamma-aminobutyric acid, 6-aminocaproic acid, L-phenylalanine, L-tryptophan and glycil-L-valil-L-phenylalanine can be used as spacers (Chaltin et al. 2003 Helvetica Chimica Acta 86:533-547; Caliceti et al. 1993 FARMCO 48:919-32) as can polyethylene glycols (Butterworth et al. 1987 J. Med. Chem 30:1295-302) and maleimide derivatives (King et al. 2002 Tetrahedron Lett. 43:1987-1990). Various other linkers are described in the literature (Nestler 1996 Molecular Diversity 2:35-42; Finn et al. 1984 Biochemistry 23:2554-8; Cook et al. 1994 Tetrahedron Lett. 35:6777-80; Brokx et al. 2002 Journal of Controlled Release 78:115-123; Griffin et al. 2003 J. Am. Chem. Soc. 125:6517-6531; Robinson et al. 1998 Proc. Natl. Acad. Sci. USA 95:5929-5934). Linkers are also described in US20050171014, for example, amino acid linkers such as FALA, VLALA, ALAL, ALALA, 2-cyclohexyl-L-alanine-LALA, 2-cyclohexyl-L-alanine-2-cyclohexyl-L-alanine-LAL, 1-naphtyl-alanine-ChaLAL and 1-naphtyl-alanine-LALA. Peptides and agonists described herein can also be conjugated to: an affinity tag (such as (histidine 6) H6), a HIV tat peptide residues 49-57, HIV tat peptide residues 49-56, the tat sequence YGRKKRRQRRR, a polyarginine peptide having from 6 to 20 residues (such as R6) and the following peptide sequences: YARKARRQARR, YARAAARQARA, YARAARRAARR, YARAARRAARA, ARRRRRRRRR, and YAAARRRRRRR, which are disclosed in WO 99/29721 and in US patent No. 6,221,355 (seq. id. nos. 3-8).

The peptides described herein can be attached to one, two or more different moieties each providing the same or different functions. For example, the peptide can be linked to a molecule that is an analgesic and to a peptide that is used to treat obesity. The peptide and various moieties can be ordered in various ways. For example, a peptide described herein can have an analgesic peptide linked to its amino terminus and an anti-obesity peptide linked to its carboxy terminus. The additional moieties can be directly covalently bonded to the peptide or can be bonded via linkers.

The peptides described herein can be a cyclic peptide or a linear peptide. In addition, multiple copies of the same peptide can be incorporated into a single cyclic or linear peptide.

The peptides can include the amino acid sequence of a peptide that occurs naturally in a vertebrate (e.g., mammalian) species or in a bacterial species. In addition, the peptides can be partially or completely non-naturally occurring peptides. Also within the disclosure are peptidomimetics corresponding to the peptides described herein.

### Soluble Guanylate Cyclase modulators

Soluble guanylate cyclase (sGC) is a nitric oxide (NO) sensing haemprotein that has been described in many eukaryotes. In response to various stimuli sGC converts GTP into the cyclic cGMP. sGC is a heterodimeric protein consisting of homologous alpha and beta subunits. Each subunit consists of an N-terminal domain which may bind haem-nitric oxide and/or oxygen, a central domain of unknown function, and a C-terminal consensus nucleotide cyclase domain. sGC can be activated via both nitric oxide (NO) dependent and independent manners. When NO binds to the haem prosthetic group in the beta subunit of sGC, catalysis is accelerated by 2-3 orders of magnitude.

Agents that function as sGC modulators useful alone alone and in combination in the compositions and methods of the present disclosure include but are not limited to: NO donors, eNOS transcriptional enhancers, haem-dependent sGC stimulators, haem-independent sGC activators and NOS substrates.

Nitric oxide (NO) donors are pharmacologically active substances that release NO *in vivo* or *in vitro.* There are different classes of NO donors, which include organic nitrates (e.g., nitroglycerin), isosorbides (e.g. isosorbide dinitrate, isosorbide mononitrate, isosorbide 5-mononitrate, isosorbide 2-mononitrate, CA Registry no. 16051-77-7), S-nitrosothiols, ironnitrosyl complexes (e.g., sodium nitroprusside), sydnonimines, C-nitroso compounds, and secondary amine/NO complex ions. Specific examples of some of the classes of NO donors named above include: amyl nitrite, Isosorbide (Dilatrate®-SR, Imdur®, Ismo®, Isordil®, Isordil® Titradose®, Monoket®), FK 409 (NOR-3); FR 144420 (NOR-4); 3-morpholinosydnonimine; Linsidomine chlorohydrate ("SIN-1"); S-nitroso-N-acetylpenicillamine ("SNAP"), AZD3582 (CINOD lead compound), NCX 4016 (an m-nitroxymethyl phenyl ester of acetyl salicyclic acid), NCX 701, NCX 1022, HCT 1026, NCX 1015, NCX 950, NCX 1000, NCX 1020, AZD 4717, NCX 1510/NCX 1512, NCX 2216, and NCX 4040 (all available from NicOx S.A.), S-nitrosoglutathione (GSNO), S-nitrosoglutathione mono-ethyl-ester (GSNO-ester), diethylenetriamine/NO (DETA/NO, a compound of nitric oxide covalently linked to diethylenetriamine), 6-(2-hydroxy-1-methyl-nitrosohydrazino)-*N*-methyl-1-hexanamine (NOC-9) or diethylamine NONOate, S-nitrosothiol, a nitrite, a sydnonimine, a NONOate, a N-nitrosoamine, a N-hydroxyl nitrosamine, a nitrosimine, a diazetine dioxide, an oxatriazole 5-imine, an oxime, a hydroxylamine, a N-hydroxyguanidine, a hydroxyurea or a furoxan. Nitric oxide donors are also as disclosed in U.S. Pat. Nos. 5,155,137, 5,366,997, 5,405,919, 5,650,442, 5,700,830, 5,632,981, 6,290,981, 5,691,423 5,721,365, 5,714,511, 6,511,911, and 5,814,666, Chrysselis et al. (2002) J Med Chem. 45:5406-9 (such as NO donors 14 and 17), and Nitric Oxide Donors for Pharmaceutical and Biological Research, Eds: Peng George Wang, Tingwei Bill Cai, Naoyuki Taniguchi, Wiley, 2005.

Endothelial NO synthase is subject to physiological and pathophysiological regulation both at the transcriptional and at the post-transcriptional level. Compounds which enhance eNOS transcription are described in WO 02/064146, WO 02/064545, WO 02/064546 and WO 02/064565, and corresponding patent documents such as US2003/0008915, US2003/0022935, US2003/0022939 and US2003/0055093 for example. Other eNOS transcriptional enhancers include those described in US20050101599 (e.g. 2,2-difluorobenzo[1,3]dioxol-5-carboxylic acid indan-2-ylamide, and 4-fluoro-N-(indan-2-yl)-benzamide), and Sanofi-Aventis compounds AVE3085 and AVE9488 (CA Registry NO. 916514-70-0; Schäfer et al., Journal of Thrombosis and Haemostasis 2005; Volume 3, Supplement 1: abstract number P1487).

Evgenov et al. (2006) Nature Reviews-Drug Discovery 5:755-768 review a novel class of haem-dependent sGC-stimulators which share several characteristics including crucial dependency on the presence of the reduced prosthetic haem moiety and strong synergistic enzyme activation when combined with NO. Haem-dependent sGC stimulators include but are not limited to:
YC-1 (see patent publications EP667345 and DE 19744026) 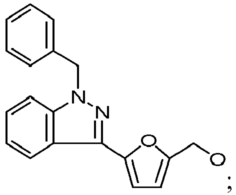
BAY 41-2272 (see patent publications DE19834047 and DE19942809) 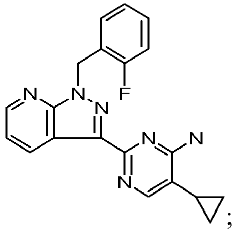
BAY 41-8543 (see patent publication DE19834044) 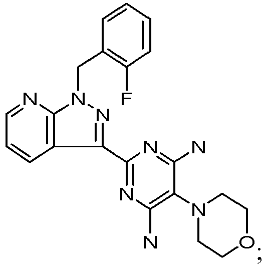
CFM-1571 (see patent publication WO2000027394) 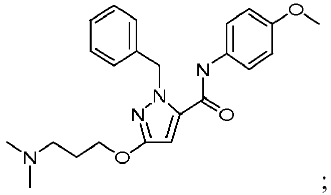
A350-619 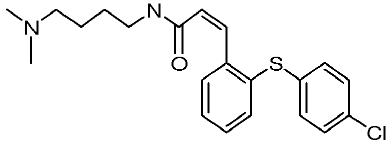 and other compounds disclosed in Tetrahedron Letters (2003), 44(48): 8661-8663.

sGC can also be activated in a NO- and haem-independent manner by haem-independent sGC activators which include but are not limited to:
BAY 58-2667 (see patent publication DE19943635) 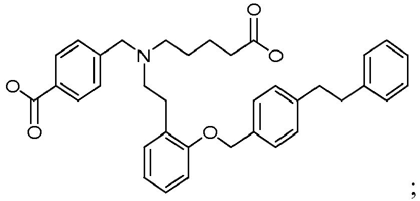
HMR-1766 (ataciguat sodium, see patent publication WO2000002851) 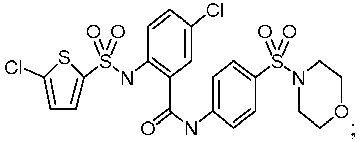
S 3448 (2-(4-chloro-phenylsulfonylamino)-4,5-dimethoxy-N-(4-(thiomorpholine-4-sulfonyl)-phenyl)-benzamide; see patent publications DE19830430 and WO2000002851) 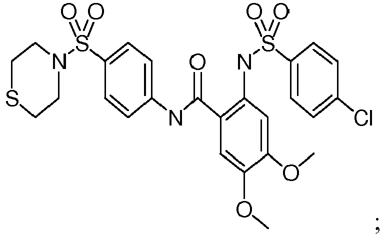 and
HMR-1069 (Sanofi-Aventis).

L-arginine acts as the endogenous substrate of NOS. Other NOS substrates which can be converted to NO may be useful in the methods and compositions described herein. NOS substrates include but are not limited to L-arginine, n-hydroxyguanidine based analogs (such as N[G]-hydroxy-L-arginine (NOHA), (1-(3, 4-dimethoxy-2-chlorobenzylideneamino)-3-hydroxyguanidine), and PR5 (1-(3, 4-dimethoxy-2-chlorobenzylideneamino)-3-hydroxyguanidine); L-arginine derivatives (such as homo-Arg, homo-NOHA, N-tert-butyloxy- and N-(3-methyl-2-butenyl)oxy-L-arginine, canavanine, epsilon guanidine-carpoic acid, agmatine, hydroxyl-agmatine, and L-tyrosyl-L-arginine); N-alkyl-N'-hydroxyguanidines (such as N-cyclopropyl-N'-hydroxyguanidine and N-butyl-N'-hydroxyguanidine), N-aryl-N'-hydroxyguanidines (such as N-phenyl-N'-hydroxyguanidine and its para-substituted derivatives which bear -F, -Cl, -methyl, -OH substituents, respectively); guanidine derivatives such as 3-(trifluormethyl) propylguanidine; and others reviewed in Cali et al. (2005) Current Topics in Medicinal Chemistry 5:721-736) and disclosed in the references cited therein.

### Prostanoids, Prostaglandin E1 (PGE1), Prostaglandin E2 (PGE2), and derivatives thereof

Prostanoids are a class of eicosanoids which include prostaglandins, thromboxanes and prostacyclins. Prostaglandin E1 and its derivatives have been implicated in multiple functions including anion secretion and the regulation of urinary sodium excretion (Lifschitz et al. (1978) Prostaglandins 16:607-19). Agents useful in the methods described herein include dinoprostone, gemeprost, alprostadil, limaprost, butaprost, 11-deoxy PGE1, AH23848, AH13205, or a 19-hydroxy PGE, the prostaglandin E agonists described in patent publications EP 1097922 and EP1114816, the 19-hydroxy prostaglandin analogues described in US4,127,612, Enprostil (a synthetic prostaglandin), PGE2, PGE1 and PGE1 derivatives (such as those described in US5,219,885, the chloride channel activator lubiprostone (Amitiza®) and derivatives thereof, and misoprostol) as well as 6-keto prostaglandin E1 (6-k PGE1) and derivatives thereof as disclosed in patent publications US4,205,178 and DE2840032. Misoprostol, a synthetic prostaglandin E₁ (PGE₁) analogue sold as Cytotec®, is a 1:1:1:1 mixture of 11R, 16S; 11S, 16R; 11R, 16R; and 11S, 16S isomers. In certain embodiments, misoprostol comprises all 4 isomers. In other embodiments, misoprostol comprises, consists essentially of or consists of the single isomer (SC 30249) which is associated with CAS registry NO. 59122-49-5. In other embodiments, misoprostol comprises, consists essentially of or consists of the free acid of the single isomer (SC 30249). Prostaglandin E derivatives also include the compounds represented by CAS Registry Nos. 333963-40-9 and 136790-76-6, (-)-7-[(2R,4aR,5R,7aR)-2-(1,1-difluoropentyl)-2-hydroxy-6-oxooctahydrocyclopenta[b]pyran-5-yl]heptanoic acid; and the 13, 14-dihydro-15-keto prostaglandins E disclosed in US5284858 including 13,14-dihydro-15-keto-PGE₂ alkyl ester, 13,14-dihydro-15-keto-PGE₂ cycloalkyl ester; 13,14-dihydro-15-keto-PGE₂ hydroxy alkyl ester, 13,14-dihydro-15-keto-PGE₂ benzyl ester, 13,14-dihydro-15-keto-PGE₁ alkyl ester, 13,14-dihydro-6,15-diketo-PGE₁ alkyl ester, 13,14-dihydro-15-keto-18-methoxy-19, 20-dinor-PGE₂ or an alkyl ester thereof, 13,14-dihydro-15-keto-18-methoxy-PGE₂ or an alkyl ester thereof, 13,14-dihydro-15-keto-Δ²-PGE₂ or an alkyl ester thereof, 13,14-dihydro-15-keto-20-methoxy-Δ²-PGE₂ or an alkyl ester thereof, 13,14-dihydro-15-keto-3R,S-methyl-PGE₂ or an alkyl ester thereof, 13,14-dihydro-15-keto-3R,S-methyl-20-methoxy-PGE₂ or an alkyl ester thereof, 13, 14-dihydro-15-keto-11-dehydroxy-11R-methyl-PGE₂ or an alkyl ester thereof, 13,14-dihydro-15-keto-16R,S-fluoro-11-dehydroxy-11R-methyl-PGE₂ or an alkyl ester thereof, 13,14-dihydro-15-keto-16R,S-hydroxy-PGE₂ or an alkyl ester thereof, 13,14-dihydro-15-keto-16R,S-fluoro-PGE₂ or an alkyl ester thereof, 13,14-dihydro-15-keto-16R,S-methyl-PGE₂ or an alkyl ester thereof, 13,14-dihydro-15-keto-16,16-dimethyl-PGE₂ or an alkyl ester thereof, 13,14-dihydro-15-keto-16,16-dimethyl-20-methoxy-PGE₂ or an alkyl ester thereof, 13,14-dihydro-15-keto-17S-methyl-PGE₂ or an alkyl ester thereof, 13,14-dihydro-15-keto-19-methy-PGE₂ or an alkyl ester thereof, 13,14-dihydro-15-keto-20-isopropropylidene PGE₂ or an alkyl ester thereof, 13,14-dihydro-15-keto-20-ethyl-PGE₂ or an alkyl ester thereof, 13,14-dihydro-15-keto-20-ethyl-11-dehydroxy-11R-methyl-PGE₂ or an alkyl ester thereof, 13,14-dihydro-15-keto-20-n-propyl-PGE₂ or an alkyl ester thereof, 13,14-dihydro-15-keto-20-ethyl-PGE₁ or an alkyl ester thereof, 13,14-dihydro-6,15-diketo-16R,S-fluoro-PGE₁ or an alkyl ester thereof, 13,14-dihydro-6,15-diketo-16R,S-fluoro-11-dehydroxy-11R-methyl-PGE₁ or an alkyl ester thereof, 13,14-dihydro-6,15-diketo-16R,S-methyl-PGE₁ or an alkyl ester thereof, 13,14-dihydro-6,15-diketo-16,16-dimethyl-PGE₁ or an alkyl ester thereof, 13,14-dihydro-6,15-diketo-19-methyl-PGE₁ or an alkyl ester thereof, 13,14-dihydro-6,15-diketo-20-methyl-PGE₁ or an alkyl ester thereof, 13,14-dihydro-6,15-diketo-11-dehydroxy-11R-methyl-PGE₁ or an alkyl ester thereof, 13, 14-dihydro-6,15-diketo-11-dehydroxy-11R-hydroxymethyl PGE₁ alkyl ester, 13,14-dihydro-15-keto-20-methyl-PGE₁ or an alkyl ester thereof, 13,14-dihydro-15-keto-Δ²-PGE₁ or an alkyl ester thereof, 13,14-dihydro-15-keto-16R,S-fluoro-20-methyl-PGE₂ or an alkyl ester thereof, 13,14-dihydro-15-keto-16,16-difluoro-PGE₂ or an alkyl ester thereof, 13,14-dihydro-15-keto-5,6-dehydro-20-methoxy-PGE₂ or an alkyl ester thereof, 13,14-dihydro-6,15-diketo-16R,S-fluoro-PGE₁ or an alkyl ester thereof, the compounds disclosed in examples 1-60, and the prostaglandin E's as described in claims 1-6.

### 5HT4 receptor agonists

The 5HT4 receptor (i.e. 5-hydroxy-tryptamine receptor or serotonin-4 receptor) is a seven transmembrane-spanning G-protein which mediates many cellular functions both in the central nervous system and at the periphery. 5HT4 receptor agonists including Cisapride (and analogs thereof) and Zelnorm® (Tegaserod) which is known to increase intestinal fluid secretion, and the compounds and pharmaceutically acceptable salts thereof disclosed in patent publications WO2005080389, US2005228014, US2006100426, US2006100236, US 2006135764, US2006183901, WO2006183901, WO2006094063, WO2006094063, WO2006108127, WO2006127815, US2006276482 and US200711796 including TD-5108, CAS Registry No. 866933-46-2 (disclosed in WO2006108127) and CAS Registry No. 863248-59-3 (disclosed in US20060183901) are useful in the methods and compositions described herein.

### Cyclic nucleotides

Cyclic nucleotides and variants thereof useful in the methods described herein include but are not limited to cyclic AMP (cAMP) and cyclic GMP (cGMP) and analogues and variants thereof. Cyclic nucleotides include those discussed in F. Schwede et al. (2000) Pharmacology & Therapeutics 87:199-226, including, for example, 2-Cl-cAMP, 6-MBC-cAMP (N⁶-mono-tert-butylcarbamoyl-cyclic AMP), 7-deaza-cAMP, 8-aminocAMP, 8-Br-cAMP (8-bromo-cyclic AMP), 8-Br-cGMP (8-bromo-cyclic GMP), 8-Br-PET-cGMP, 8-Cl-cAMP (8-chloro-cAMP), 8-methylamino-cAMP, 8-pCPT-cAMP (8-para-chlorophenylthio-cyclic AMP), 8-pCPT-cGMP (8-para-chlorophenylthio-cyclic GMP), 8-piperidino-cAMP, 8-thioisopropyl-cAMP, 8-thiomethyl-cAMP, dibutyryl-cyclic AMP, Bt₂-cAMP (N⁶, O²'-dibutyryl-cyclic AMP), Bt₂-cAMP/AM (Bt₂-cAMP acetoxymethyl ester), Bt₂-cGMP (N², O²'-dibutyryl-cyclic GMP), cAMP, cGMP, cGMP/AM, N²-Bt-cGMP (N²-monobutyryl-cyclic GMP), N⁶-benzoyl-cAMP, N⁶-benzyl-cAMP, N⁶-Bt-cAMP (N⁶-monobutyryl-cyclic AMP), N⁶-carbamoylphenyl-cAMP, N⁶-phenyl-8-pCPT-cAMP, N⁶-phenyl-cAMP, PET-cGMP (β-phenyl-1 (N²-etheno-cyclic GMP), Rp-8-Br-cAMPS (Rp-8-bromo-adenosine 3',5'-cyclic monophosphorothioate), Rp-8-Br-cGMPS, Rp-8-Br-PET-cGMPS (Rp-8-bromo-β-phenyl-1 (N²-etheno-guanosine 3', 5'- cyclic monophosphorothioate), Rp-8-Cl-cAMPS, Rp-8-Cl-cGMPS, Rp-8-pCPT-cAMPS, Rp-8-pCPT-cGMPS, Rp-cAMPS (Rp-diastereomer of adenosine 3', 5'-cyclic monophosphorothioate), Rp-cGMPS (Rp-diastereomer of guanosine 3', 5'-cyclic monophosphorothioate), Sp-5,6-Cl ₂-cBIMPS (Sp-5,6-dichloro-1-β-D-ribofuranosyl-benzimidazole-3', 5'-cyclic monophosphorothioate), Sp-8-Br-PET-cGMPS (Sp-8-bromo-β-phenyl-1 (N²-etheno-guanosine 3', 5'-cyclic monophosphorothioate), Sp-8-Cl-cGMPS, Sp-8-pCPT-cGMPS, Sp-cAMPS (Sp-diastereomer of adenosine 3', 5'- cyclic monophosphorothioate), and Sp-cGMPS (Sp-diastereomer of guanosine 3', 5'-cyclic monophosphorothioate).

cGMP can include a product created by exposure of cGMP to one or more enzymes present in the digestive tract, e.g., the dephosphorylated cGMP (e.g., riboguanosine or guanosine or deoxyriboguanosine or deoxyguanosine), phosphorylated forms of GMP and GMP derivatives (e.g., guanylate monophosphate or riboguanylate monophosphate or ribodeoxyguanylate monophosphate or deoxyriboguanylate monophosphate whether as the 5'-monophosphate, the 2'-monophosphate, the 3'-monophosphate or the 2', 3'-monophosphate intermediate form); the hydroxylated or deoxy forms of the ribose sugar of the nucleotide (e.g., ribose, deoxyribose, ribose monophosphate, or deoxyribose monophosphate); guanine; and methylated guanine and cGMP such N2-methylguanine, N7-methylguanine, cGMP methylated at N2 or cGMP methylated at N7. Thus analogues of cGMP include, but are not limited to, those that have modifications to the purine ring system, to the ribose, or to the phosphate group. In some cases the metabolic end products of cGMP such as xanthine and uric acid might be useful in the methods and compositions described herein. The analogue of cGMP may be cell membrane permeable.

cGMP and analogs thereof useful in the methods and compositions described herein also include, but are not limited to: 8-(4-chlorophenylthio)guanosine 3',5'-cyclic monophosphate (Menshikov et al. 1993 Eur J Pharmacol. 245:281-4), dibutyryl guanosine 3',5'-cyclic monophosphate (db cGMP), 8-bromo-guanosine 3',5'-cyclic monophosphate (8-bromo cGMP), 8-(4-chlorophenylthio)-guanosine 3',5'-cyclic monophosphate (8-(4, chlorophenylthio) cGMP, Rp-guanosine 3',5'-cyclic monophosphate (Rp-cGMP) and Sp-guanosine 3',5'-cyclic monophosphate (Sp-cGMPS) (the S isomer of cGMP), cyclic guanosine-3',5'-triphosphate, cyclic guanosine-3',5'-diphosphate, cyclic guanosine-3',5'-triphosphate, cyclic deoxyguanosine-3',5'-monophosphate, cyclic deoxyguanosine-3',5'diphosphate, cyclic deoxyguanosine-3',5'-triphosphate, cyclic guanosine-2',3'-monophosphate, cyclic guanosine-2,3'-diphosphate, cyclic guanosine-2',3'-triphosphate, cyclic 2-(N-methyl)-guanosine-3',5'-monophosphate, cyclic 2-(N-methyl)-guanosine-3',5'-diphosphate, cyclic 2-(N-methyl)-guanosine-3',5'-triphosphate, cyclic 2-(N-methyl)-deoxyguanosine-3',5'-monophosphate, cyclic 2-(N-methyl)-doxyguanosine-3',5'-diphosphate, cyclic 2-(N-methyl)-deoxyguanosine-3',5'-triphosphate, cyclic 2-(N-methyl)-guanosine-2',3'-monophosphate, cyclic 2-(N-methyl)-guanosine-2',3'-diphosphate, cyclic 2-(N-methyl)-guanosine-2',3'-triphosphate, cyclic 7-(N-methyl)-guanosine-3',5'-monophosphate, cyclic 7-(N-methyl)-guanosine-3',5'-diphosphate, cyclic 7-(N-methyl)-guanosine-3',5'-triphosphate, cyclic 7-(N-methyl)-deoxyguanosine-3',5'-monophosphate, cyclic 7-(N-methyl)-deoxyguanosine-3',5'-diphosphate, cyclic 7-(N-methyl)-deoxyguanosine-3',5'-triphosphate, cyclic 7-(N-methyl)-guanosine-2',3'-monophosphate, cyclic 7-(N-methyl)-guanosine-2',3'-diphosphate, cyclic 7-(N-methyl)-guanosine-2',3'-triphosphate, cyclic 2,7-(N,N'-dimethyl)-guanosine-3',5'-monophosphate, cyclic 2,7-(N,N'-dimethyl)-guanosine-3',5'-diphosphate, cyclic 2,7-(N,N'-dimethyl)-guanosine-3',5'-triphosphate, cyclic 2,7-(N,N'-dimethyl)-deoxyguanosine-3',5'-monophosphate, cyclic 2,7-(N,N'-dimethyl)-deoxyguanosine-3',5'-diphosphate, cyclic 2,7-(N,N'-dimethyl)-deoxyguanosine-3',5'-triphosphate, cyclic 2,7-(N,N'-dimethyl)-guanosine-2',3'-monophosphate, cyclic 2,7-(N,N'-dimethyl)-guanosine-2',3'-diphosphate, and cyclic 2,7-(N,N'-dimethyl)-guanosine-2',3'-triphosphate; cGMP analogs available from TWC Biosearch International (Hong Kong, China) including but not limited to: Rp-8-pCPT-cGMPS (CN-206), Sp-8-pCPT-cGMPS (CN-207), 8-Bromoguanosine-3',5'-cyclic monophosphate, sodium salt (CN-205), Rp-8-Bromoguanosine-3',5'-cyclic monophosphorothioate, sodium salt (CN-216), Sp-8-Bromoguanosine-3',5'-cyclic monophosphorothioate, sodium salt (CN-217), and N2,2'-O-Dibutyrylguanosine-3',5'-cyclic monophosphate, sodium salt (CN-215); cGMP analogs disclosed in Corbin et al. 1986 Journ. Biol. Chem. 261:1208 including but not limited to: 5'-NH-cGMP, 3'-NH-cGMP, cGMPS(Rₚ), cGMPS(Sₚ), cGMP-N(CH₃)₂(Sₚ), cGMP-N(CH₃)₂(Rₚ), 8-BR-cGMP, β-H₅C₆-1-N²-etheno-cGMP, 8-S(4-Cl)-C₆H₄-cGMP, 7-Deaza-cGMP, 8-H₅C₆H₂CS-cGMP, 6-HS-cGMP, 1-H₃C-cGMP, 8-HS-cGMP, N²-nH₁₃C₆-cGMP, 8-H₅C₆(O)C-_{c}GMP, 8-HO-cGMP, N²-ₙH₇C₃(O)C-_{c}GMP, 8-H(2-HO-iH₇C₃)_{c}GMP, 8-H₃C(O)C-_{c}GMP, 8-(H₅C₂)₂N-_{c}GMP, N²-[2,4-(O₂N)₂-H₃C₆]_{C}GMP, 8-H₂N-_{c}IMP, and 2'-Deoxy-_{c}GMP; cGMP analogs available from Biolog Life Science Institute (Hayward, CA) including but not limited to : N²-MB-cGMP (cIMP), 3-deaza cGMP, 2- Aminopurine riboside- 3', 5'- cyclic monophosphate (2-NH2-cPuMP), 2'- Deoxyguanosine- 3', 5'- cyclic monophosphate (2'-cdGMP), 2'- O- (N-Methylanthraniloyl)guanosine- 3', 5'- cyclic monophosphate (MANT-cGMP), 2'-O-Me-cGMP, and cGMP-AM; 8-bromoadenosine-cGMP, N2,2'-O-Dibutyrylguanosine-3',5'-cyclic monophosphate, sodium salt (available from Biomol; Plymouth, PA), and cGMP analogs available from other commercial supplier including Sigma Aldrich and Boehringer Mannheim).

### Laxatives

Laxatives useful alone and in combination in the methods described herein include but are not limited to bulk-producing agents, stool softeners, hydrating agents, stimulants, and other laxatives including dexloxiglumide/ CR 2017 (a CCK-1 antagonist, Forest Laboratories and Rottapharm - Rotta Research Laboratorium SpA) and CR 3700 (Rottapharm (Rotta Research Laboratorium SpA).

Bulk-producing agents (i.e., bulk-forming or bulking agents) cause the stool to be bulkier and to retain more water, as well as forming an emollient gel. Bulk-producing agents have the gentlest of effects among laxatives and can also be taken just for maintaining regular bowel movements. Bulk-producing agents include but are not limited to dietary fiber, bran, psyllium husk (e.g., Metamucil), methylcellulose (e.g., Citrucel), and polycarbophil including calcium polycarbophil (e.g., Fibercon).

Stool softeners (i.e. surfactants) cause water & fats to penetrate the stool, making it easier to move along. Stool softeners include anionic surfactants such as docusate and salts and derivatives thereof. Perfusion studies suggest that docusates may inhibit fluid absorption or stimulate secretion in jejunum. Docusates include docusate sodium (e.g., Colace, Diocto) and Docusate calcium (e.g., Surfak).

Hydrating agents (i.e., osmotics) cause the intestines to concentrate more water within, softening the stool. There are two principal types of hydrating agents, saline and hyperosmotic. Hydrating agents include but are not limited to magnesium hydroxide (e.g., milk of magnesia), magnesium citrate, magnesium sulfate, and epsom salt.

Stimulants (i.e., irritants) alter water and electrolyte secretion and can stimulate peristaltic action. Stimulants include but are not limited to the diphenylmethane derivative Bisacodyl (e.g., Dulcolax, Fleet, Alophen, Correctol, Carter's Little Pills), phenolphthalein (e.g., yellow or white phenolphthalein) which inhibits re-absorption of digestive secretions, and naturally derived laxatives including senna (derived from the senna plant, may be sold as Senokot or Sennoside), aloe vera, and Cascara sagrada which comprises as active ingredients, anthraquinones, ricinoleic acid, and castor oil. Castor oil acts directly on intestinal mucosa or nerve plexus and alters water and electrolyte secretion. It is converted into ricinoleic acid (the active component) in the gut.

### CFTR (cystic fibrosis transmembrane conductance regulator) modulators

The cystic fibrosis transmembrane conductance regulator protein (CFTR) is a cAMP- activated chloride (Cl) channel expressed in epithelial cells in mammalian airways, intestine, pancreas and testis. CFTR is the chloride-channel responsible for cAMP-mediated Cl secretion. Hormones, such as a β -adrenergic agonist, or toxins, such as cholera toxin, lead to an increase in cAMP, activation of cAMP-dependent protein kinase, and phosphorylation of the CFTR Cl channel, which causes the channel to open. CFTR is predominantly located in epithelia where it provides a pathway for the movement of Cl ions across the apical membrane and a key point at which to regulate the rate of transepithelial salt and water transport.

CFTR modulators may function for example, by increasing protein kinase regulated chloride channel gating of wild-type or mutant CFTR ("direct CFTR regulators"), by correcting folding or cellular processing of a mutant CFTR proteins ("correctors) or by activating molecules such as G-protein coupled P2Y and P2X purinergic receptors which regulate CFTR ("P2Y and P2X receptor agonists").

Direct CFTR regulators useful alone and in combination in the methods and compositions described herein include but are not limited to the pyrrolo[2,3-b]pyrazines derivatives described in Noel et. al. (2006) J Pharmacol Exp Ther 319:349-359 (e.g. compounds represented by CAS. Nos. 913748-30-8, 655239-23-9, 655239-22-8, 655239-21-7, 496864-17-6, 496864-16-5, 496864-15-4, 496864-06-3, 496864-05-2, 479551-48-9, 913748-29-5, 496864-10-9, 496864-09-6, 139962-78-0, and 139962-77-9, specifically those including the compounds described as RP107 [7-n-butyl-6-(4-hydroxyphenyl)[5H]-pyrrolo[2,3-b]pyrazine], RP108 [7-n-butyl-6-(4-chlorophenyl)[5H]pyrrolo[2,3-b]pyrazine], and those compounds comprising 4-hydroxyphenyl and 7-n-butyl moieties); isoflavones such as genistein; 5,6-dichloro-1-ethyl-1,3-dihydro-2H-benzimidazol-2-one(DCEBIO); 1-ethyl-2-benzimidazolone (1-EBIO); chlorzoxazone; zoxazolamine (2-amino-5-chlorzoxazole); 4-Chlorobenzo[F]isoquinoline (CBIQ); the phenylglycine and sulfonamide derivatives including 2-[(2-1H-indol-3-yl-acetyl)-methylamino]-N-(4-isopropylphenyl)-2-phenylacetamide and 6-(ethylphenylsulfamoyl)-4-oxo-1,4-dihydroquinoline-3-carboxylic acid cycloheptylamide which are decribed in Pedemonte et al. (2005) Molecular Pharmacology 67: 1797-1807; the compounds disclosed in Yang et al. (2003) Journal of Biological chemistry 278:35079-85 which have tetrahydrobenzothiophene, benzofuran, pyramidinetrione, dihydropyridine, or anthraquinone core structures and which activate defective Delta F508-CFTR chloride channel gating; the benzoquinolizinium compounds disclosed in Mariving-Mounir et al. (2004) J Med Chem 47:962-972 including 5-butyl-7-chloro-6 hydroxybenzo[c]quinolizinium chloride (8u, MPB-104); the thiazolidinone compounds disclosed in Ma et al. (2002) Clin Invest 110:1651-1658 including CFTR(inh)-172, compound Vrt-532 (CAS Registry No. 38214-71-0) and the xanthine derivatives disclosed in Chappe et al. (1998) Br J Pharmacol. 123:683-93 including 3,7-dimethyl-1-propyl xanthine and 3,7-dimethyl-1-isobutyl xanthine.

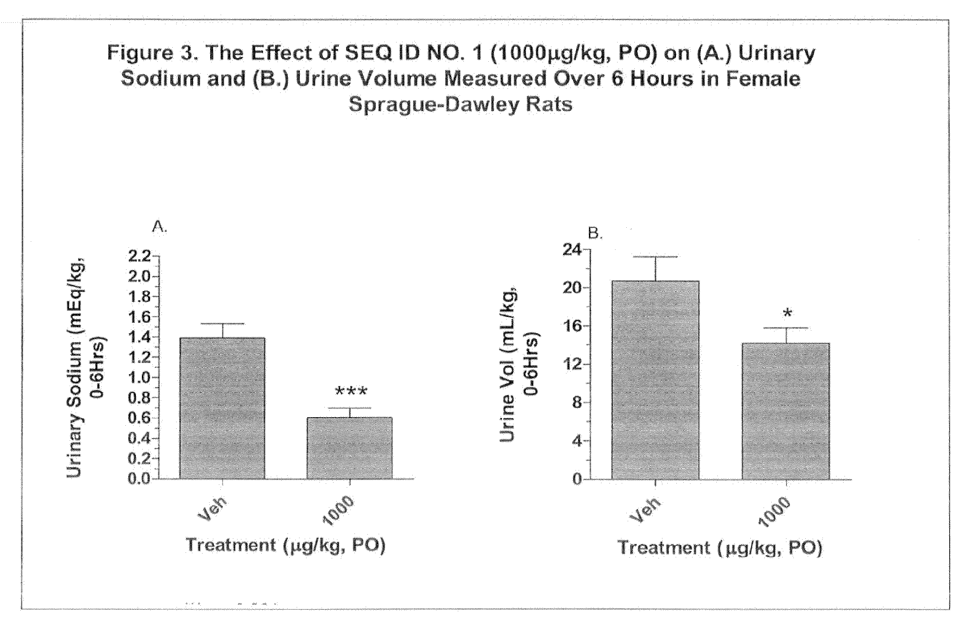

Correctors useful alone and in combination in the methods described herein include but are not limited to 4-phenylbutyrate (4-PBA); the 1,2,3,4-tetrahydroisoquinoline-3-carboxylic acid diamides described in Hirth et al. (2005) Bioorg Med Chem 15:2087-2091 including compound 1 and the compounds with reasonable activity disclosed in tables 1, 2, and 3; curcumin; the "corrector" compounds disclosed in patent publication W02006101740 including the compounds disclosed in paragraph 2, figure 3a (compounds corr-1a, corr-3a, corr-2a, and corr-4a (CAS Registry No. 421580-53-2)), claim 1 (compounds of formula I), claim 4 (compounds having formula Ia), claim 7 (compounds having formula Ib), claim 12 (compounds having formula II), claim 19 (compounds having formula III), claim 30 (compounds having formula IV), claim 36 (compounds having formula V), and the individual compounds disclosed in claims 11, 18, 29, 35, and 44; the 1,4-dihydropyridines compounds which are CFTR correctors disclosed in Pedemonte et al. (2005) Molecular Pharmacology 68:1736-1746 including methyl-1,4-dihydro-2,6-dimethyl-3-nitro-4-2(trifluoromethylphenyl)pyridine-5-carboxylate (BayK-8644); the phenylglycine and sulfonamide comprising "corrector" compounds disclosed in patent publication WO2005120497 including 2-[(2-1H-Indol-3-yl-acetyl)-methyl-amino]-N-(4-isopropyl-phenyl)-2-phenyl-acetamide, 2-[(2-IH-Indol-3-yl-acetyl)-methyl-amino]-N-(4-isopropyl-phenyl)-2-(4-methoxy-phenyl)-acetamide, 2-[(2-1H-Indol-3-yl-acetyl)-methyl-amino]-N-(4-methoxy-phenyl)-2-phenyl-acetamide, 2-((2-1H-Indol-3-yl-acetyl)-methyl-amino]-2,N-bis-(4-methoxy-phenyl)-acetamide, N-(2,3-Dihydro-benzo[1,4]dioxin-6-yl)-2-(2-1H-indol-2-yl-acetylamino)-2-p-tolyl-acetamide, N-(2,3-Dihydro-benzo[1,4]dioxin-6-yl)-2-[(2-1H-indol-3-yl-acetyl)-methyl-amino]-2-(4-methoxy-phenyl)-acetamide, 2-(2-1H-Indol-3-yl-acetylamino)-N-(4-isopropyl-phenyl) -2-phenyl-acetamide, N-Benzo[1,3]dioxol-5-yl-2-[(2-1H-indol-3-yl-acetyl)-methyl-amino] -2-p-tolyl-acetamide, 2-[(2-Acetylamino-acetyl)-methyl-amino]-N-(2,3-dihydro-benzo [1,4]dioxin-6-yl)-2-phenyl-acetamide, 6-[(2-Ethoxy-phenyl)-methyl-sulfamoyl]-4-oxo-1,4-dihydro-quinoline-3-carboxylic acid allylamide,6-(Ethyl-phenyl-sulfamoyl)-4-oxo-1,4-dihydro-quinoline-3-carboxylic acid (3-methoxy-propyl)-amide, 6-(Methyl-m-tolyl-sulfamoyl)-4-oxo-1,4-dihydro-quinoline-3-carboxylic acid (pyridin-2-ylmethyl)-amide,6-(Methyl-m-tolyl-sulfamoyl)-4-oxo-1,4-dihydro-quinoline-3-carboxylic acid(2-cyclohex-1-enyl-ethyl)-amide, 6-(1,4-Dioxa-8-aza-spiro[4.5]decane-8-sulfonyl)-4-oxo-1,4-dihydro-quinoline-3-carboxylic acid (3-methyl-butyl)-amide,6-[Ethyl-(4-fluoro-phenyl)-sulfamoyl]-4-oxo-1,4-dihydro-quinoline-3-carboxylic acid cyclopentylamide,6-(Methyl-o-tolyl-sulfamoyl)-4-oxo-1,4-dihydro-quinoline-3-carboxylic acid (3-methyl-butyl)-amide, 6-[(2,6-Dimethyl-phenyl)-methyl-sulfamoyl]-4-oxo-1,4-dihydro-quinoline-3-carboxylic acid butylamide, 6-(Allyl-phenyl-sulfamoyl)-4-oxo-1,4-dihydro-quinoline-3-carboxylic acid (furan-2-ylmethyl)-amide, 6-[Ethyl-(4-fluoro-phenyl)-sulfamoyl]-4-oxo-1,4-dihydro-quinoline-3-carboxylic acid(tetrahydro-furan-2-ylmethyl)-amide, 6-(Methyl-m-tolyl-sulfamoyl)-4-oxo-1,4-dihydro-quinoline-3-carboxylic acid sec-butylamide, and 6-(2,3-Dihydro-indole-1-sulfonyl)-4-oxo-1,4-dihydro-quinoline-3-carboxylic acid cyclohexylamide; the aminoarylthiazoles, quinazolinylaminopyrimidinones, and bisaminomethylbithiazoles compounds disclosed as correctors of defective DeltaF508-CFTR cellular processing disclosed in Pedemonte et al. (2005) Journal of Clinical Investigation 115:2564-2571; the compounds disclosed in Van Goor et al. (2006) Am J Physiol Lung Cell Mol Physiol 290:L1117-30 including VRT-325 (CAS Registry No. 815592-21-3) and VRT-422(CAS Registry No. 815589-62-9); and the pyrimidine derivatives disclosed as modulators of ATP-binding cassette transporters in W02004111014.

P2Y and P2X receptor agonists useful alone and in combination in the methods and compositions described herein include but are not limited to Denufosol, Diquafosol, the dinucleotide compounds disclosed as selective agonists of P2Y receptors in Shaver et al. (2005) Purinergic Signalling 1:183-191.

### Agents which affect cAMP levels

Agents that modulate (e.g. increase cAMP levels) are useful in the methods and compositions described herein. Agents that modulate cAMP levels useful alone and in combination in the methods described herein include but are not limited to vanadate, cholera toxin, heat labile toxin, and adenylate cyclase agonists. Adenylate cyclase is a transmembrane protein that catalyzes the conversion of ATP to cAMP and pyrophosphate. Thus, adenylate cyclase agonists may increase cAMP levels. Adenylate cyclase agonists include but are not limited to forskolin and isoproterenol (e.g. isoprenaline).

### Sodium Transport Inhibitors

Agents that inhibit the transport of sodium are useful in the methods and combinations described herein. These agents may function, for example, by inhibiting the activity of molecules involved in Na⁺-H⁺ exchange (NHE inhibitors) or sodium glucose transport (SGLT1 inhibitors).

NHE inhibitors useful alone and in combination in the methods described herein include but are not limited to Amiloride and NHE3 inhibitors such as Aventis compounds AVE-0657, S1611 (disclosed in Wiemann M et al. (1999). Pflugers Arch 438:255-262.), S8218, and S3226 (CAS Registry No. 215183-03-2); Cariporide (HOE642, CAS Registry No. 159138-81-5), Zoniporide (CAS Registry No. 241800-98-6), T-162559 (CAS Registry No. 339532-12-6), HOE 694 (3-methanesulfonyl-4-piperidino-benzoyl)guanidine methanesulfonate, CAS Registry No. 149725-40-6), and the compounds disclosed in patent publications DE102005044817, W02006074813, W02005117977, WO2005026173, DE10312963, DE10304294, W02003101984, WO2003053434, DE10163992, WO2003051866, WO2003048129, WO2002024637, WO2002020496, WO2002046169, and WO2001079186.
Taurine is an example of a sodium glucose transport inhibitor.

### Phosphodiesterase Inhibitors

Phosphodiesterase inhibitors, which block one or more of the five subtypes of the enzyme phosphodiesterase (PDE) and therefore prevent the inactivation of cAMP and cGMP, are useful alone and in combination in the methods and compositions described herein. Certain PDEs exhibit cyclic nucleotide specificity, for example, PDE5 is cGMP specific; thus PDE5 inhibitors specifically prevent the inactivation of cGMP.

PDE inhibitors include PDE3 inhibitors, PDE4 inhibitors, PDE5 inhibitors, and inhibitors with multiple specificity including PDE3/4 and PDE3/4/5 inhibitors. Specific PDE inhibitors include but are not limited to those disclosed in patent publications DE1470341, DE2108438, DE2123328, DE2305339, DE2305575, DE2315801, DE2402908, DE2413935, DE2451417, DE2459090, DE2646469, DE2727481, DE2825048, DE2837161, DE2845220, DE2847621, DE2934747, DE3021792, DE3038166, DE3044568, EP000718, EP0008408, EP0010759, EP0059948, EP0075436, EP0096517, EP0112987, EP0116948, EP0150937, EP0158380, EP0161632, EP0161918, EP0167121, EP0199127, EP0220044, EP0247725, EP0258191, EP0272910, EP0272914, EP0294647, EP0300726, EP0335386, EP0357788, EP0389282, EP0406958, EP0426180, EP0428302, EP0435811, EP0470805, EP0482208, EP0490823, EP0506194, EP0511865, EP0527117, EP0626939, EP0664289, EP0671389, EP0685474, EP0685475, EP0685479, JP92234389, JP94329652, JP95010875, US4963561, US5141931, W09117991, WO9200968, WO9212961, WO9307146, WO9315044, WO9315045, WO9318024, WO9319068, WO9319720, WO9319747, WO9319749, WO9319751, WO9325517, WO9402465, WO9406423, WO9412461, WO9420455, WO9422852, WO9425437, WO9427947, WO9500516, WO9501980, WO9503794, WO9504045, WO9504046, WO9505386, WO9508534, WO9509623, WO9509624, WO9509627, WO9509836, WO9514667, WO9514680, WO9514681, WO9517392, WO9517399, WO9519362, WO9522520, WO9524381, WO9527692, WO9528926, WO9535281, WO9535282, WO9600218, WO9601825, WO9602541, W09611917, DE3142982, DE1116676, DE2162096, EP0293063, EP0463756, EP0482208, EP0579496, EP0667345 US6331543, US20050004222 (including those disclosed in formulas I-XIII and paragraphs 37-39, 85-0545 and 557-577), WO9307124, EP0163965, EP0393500, EP0510562, EP0553174, WO9501338 and WO9603399, as well as PDE5 inhibitors (such as RX-RA-69, SCH-51866, KT-734, vesnarinone, zaprinast, SKF-96231, ER-21355, BF/GP-385, NM-702, vardenafil (LEVITRA®); and tadalafil (CIALIS® and sildenafil (Viagra™)), PDE4 inhibitors (such as etazolate, ICI63197, RP73401, imazolidinone (RO-20-1724), MEM 1414 (R1533/R1500; Pharmacia Roche), denbufylline, rolipram, oxagrelate, nitraquazone, Y-590, DH-6471, SKF-94120, motapizone, lixazinone, indolidan, olprinone, atizoram, KS-506-G, dipamfylline, BMY-43351, atizoram, arofylline, filaminast, PDB-093, UCB-29646, CDP-840, SKF-107806, piclamilast, RS-17597, RS-25344-000, SB-207499, TIBENELAST, SB-210667, SB-211572, SB-211600, SB-212066, SB-212179, GW-3600, CDP-840, mopidamol, anagrelide, ibudilast, amrinone, pimobendan, cilostazol, quazinone and N-(3,5-dichloropyrid-4-yl)-3-cyclopropylmethoxy4-difluoromethoxybenzamide, PDE3 inhibitors (such as ICI153, 100, bemorandane (RWJ 22867), MCI-154, UD-CG 212, sulmazole, ampizone, cilostamide, carbazeran, piroximone, imazodan, CI-930, siguazodan, adibendan, saterinone, SKF-95654, SDZ-MKS-492, 349-U-85, emoradan, EMD-53998, EMD-57033, NSP-306, NSP-307, revizinone, NM-702, WIN-62582 and WIN-63291, enoximone and milrinone, PDE3/4 inhibitors (such as benafentrine, trequinsin, ORG-30029, zardaverine, L-686398, SDZ-ISQ-844, ORG-20241, EMD-54622, and tolafentrine) and other PDE inhibitors (such as vinpocetin, papaverine, enprofylline, cilomilast, fenoximone, pentoxifylline, roflumilast, and theophylline.

### Renin inhibitors and aldosterone antagonists

Renin functions in the Renin Angiotensin System (RAS) and is a key regulator of blood pressure. Suppression of the RAS has been shown to treat hypertension and reduce cardiovascular events. Renin inhibitors useful alone and in combination in the methods and compositions described herein include but are not limited to Tekturna® (Rasilez and Aliskiren) and SPP635. Aldosterone is a steroid hormone which functions in the regulation of sodium and potassium balance in the blood. Aldosterone antagonists useful alone and in combination in the methods and compositions described herein include but are not limited to Spironolactone, Canrenone, and Eplerenone.

### Polymer Resins

Polymer resins useful alone and in combination in the methods and compositions described herein include but are not limited to the dietary fiber psyllium seed husk (psyllium), lipid lowering polymers (e.g. Colesevelam, Sevalmer, Cholestyramine), nonabsorbed polymer resins (e.g. hyaluronic acid, polycarbophil calcium, polyvinyl acetate, polyvinyl pyrrolidone, polystyrene sulfate (alone and in combination with yellow phenolphthalein and including potassium salt derivatives thereof)), and sodium binding polymers.

Sodium-binding polymers (also called cation exchange cores) include but are not limited to those disclosed in US20050220750 and US20060024265 including crosslinked polyvinylsulfamate polymer, N-(bis-phosphonic-ethyl) polyvinylamine polymer, poly-α-acrylic acid polymer, poly-α-fluoroacrylic acid polymer, polyvinylphosphoramidic polymer, polyvinylsulfamate polymer, polyvinylsulfamate/vinylsulfate copolymer, polyvinylsulfate polymer, polyvinylsulfonate polymer, polyvinylsulfonate polymer, vinylphosphonate/α-fluoroacrylic acid copolymer, vinylphosphonate/α-fluoroacrylic acid copolymer, or vinylphosphonate/acrylic acid copolymer and combinations thereof. In certain embodiments, the sodium-binding polymer is administered as core-shell composition which further comprises a semi-permeable shell, said semi-permeable shell comprising at least one of a poly-11 trimethylammonioundecylmethacrylate polymer, a styrene-vinylpyridine polymer, 11-dimethyl-aminodecylmethacrylate/laurylmethacrylate copolymer, or a polyallylamine/polystyrene sulfonate polymer. An example of a core-shell composition useful alone and the combination in the methods and compositions described herein is ILY101 (Ilypsa).

### Example 1: Preparation of peptides

Peptides can be recombinantly produced in bacteria as follows. T7 expression vectors, pET26b(+) (Novagen) expressing the peptide of interest are constructed using standard molecular biology techniques and are transformed into E. *coli* bacterial host BL21 λ DE3 (Invitrogen). A single colony is innoculated and grown shaking overnight at 30°C in L broth + 25 mg/l kanamycin. The overnight culture is added to 3.2 L of batch medium (Glucose 25 g/l, Casamino Acids 5 g/l,Yeast Extract 5 g/l,KH₂PO₄ 13.3 g/l, (NH₄)₂HPO₄ 4 g/l, MgSO₄-7H₂0 1.2 g/l, Citric Acid 1.7 g/l, EDTA 8.4 mg/l, CoCl₂-6H₂O 2.5 mg/l, MnCl₂-4H₂O 15 mg/l, CuCl₂-4H₂O 1.5 mg/l, H₃BO₃ 3 mg/l, Na₂MoO₄-2H₂0 2.5 mg/l, Zn Acetate-2H₂0 13 mg/l, Ferric Citrate 100 mg/l, Kanamycin 25 mg/l, Antifoam DF₂0₄ 1 ml/l) and fermented using the following process parameters : pH 6.7 - control with base only (28% NH₄OH), 30°C, aeration : 5 liters per minute. After the initial consumption of batch glucose (based on monitoring dissolved oxygen (DO) levels), 1.5 L of feed medium (Glucose 700 g/l, Casamino Acids 10 g/l, Yeast Extract 10 g/l, MgSO₄-7H₂0 4 g/l, EDTA 13 mg/l, CoCl₂-6H₂O 4 mg/l, MnCl₂-4H₂O 23.5 mg/l, CuCl₂-4H₂0 2.5 mg/l, H₃BO₃ 5 mg/l, Na₂MoO₄-2H₂0 4 mg/l, Zn Acetate-2H₂0 16 mg/l, Ferric Citrate 40 mg/l, Antifoam DF₂0₄ 1 ml/l) is added at a feed rate controlled to maintain 20% DO. IPTG is added to 0.2 mM 2 hours post feed start. The total run time is approximately 40-45 hours (until feed exhaustion).

Cells are collected by centrifugation at 5,000 g for 10 minutes. The cell pellet is discarded and the supernatant is passed through a 50 Kd ultrafiltration unit. The 50 Kd filtrate (0.6 liters) is loaded onto a 110 ml Q-Sepharose fast Flow column (Amersham Pharmacia, equilibrated with 20 mM Tris-HCl pH 7.5) at a flow rate of 400 ml/hour. The column is washed with six volumes of 20 mM Tris-HCl pH 7.5 and proteins are eluted with 50 mM acetic acid collecting 50 ml fractions. Fractions containing peptide are pooled and the solvent is removed by rotary evaporation. The dried proteins are resuspended in 10 ml of 8% acetic acid, 0.1% trifluoroacetic acid (TFA) and loaded onto a Varian Polaris C18-A column (250 X 21.2 mm 10 Tm, equilibrated in the same buffer) at a flow rate of 20 ml/min. The column is washed with 100 ml of 8% methanol, 0.1% TFA and developed with a gradient (300 ml) of 24 to 48% methanol, 0.1% TFA, collecting 5-ml fractions. Fractions containing peptide are pooled and the solvent is removed by rotary evaporation. The peptides are dissolved in 0.1%TFA and lyophilized.

Peptide fractions are analyzed by standard LCMS and HPLC. Peptides can also be chemically synthesized by a commercial peptide synthesis company.

### Example 2: Activation of the intestinal GC-C receptor by peptides

The ability of peptides to activate the intestinal GC-C receptor can be assessed in an assay employing the T84 human colon carcinoma cell line (American Type Culture Collection (Bethesda, Md)). For the assays cells are grown to confluency in 24-well culture plates with a 1:1 mixture of Ham's F12 medium and Dulbecco's modified Eagle's medium (DMEM), supplemented with 5% fetal calf serum and were used at between passages 54 and 60.

Briefly, monolayers of T84 cells in 24-well plates are washed twice with 1 ml/well DMEM, then incubated at 37°C for 10 min with 0.45 ml DMEM containing 1 mM isobutylmethylxanthine (IBMX), a cyclic nucleotide phosphodiesterase inhibitor. Test peptides (50µl) are then added and incubated for 30 minutes at 37°C. The media is aspirated and the reaction was then terminated by the addition of ice cold 0.5 ml of 0.1N HCl. The samples are held on ice for 20 minutes and then evaporated to dryness using a heat gun or vacuum centrifugation. The dried samples are resuspended in 0.5ml of phosphate buffer provided in the Cayman Chemical Cyclic GMP EIA kit (Cayman Chemical, Ann Arbor, MI). Cyclic GMP is measured by EIA according to procedures outlined in the Cayman Chemical Cyclic GMP EIA kit. EC₅₀ is defined as the concentration by which 50% of the maximal activity is seen. Maximum cGMP level in assay is determined as the activity of a positive ST control, Cys-Cys-Glu-Leu-Cys-Cys-Asn-Pro-Ala-Cys-Thr-Gly-Cys-Tyr and set to 100%.

### Example 3: Anion Secretion in T84 cells

The ability of agents to increase chloride anion secretion can be examined using the T84 human colon carcinoma cell line (American Type Culture Collection (Bethesda, Md)). Briefly, cells are grown to confluency in 24-well culture plates with a 1:1 mixture of Ham's F12 medium and Dulbecco's modified Eagle's medium (DMEM), supplemented with 5% fetal calf serum and were used at between passages 54 and 60. Chloride ion secretion is measured in the presence of vehicle only or test article similar the methods described in Dharmasthaphorn et al. (1985) J Clin Invest 75:462-471 and Barrett and Bigby (1993) Am J Physiol 264:C446-52. Briefly, an Ussing chamber is modified to allow maintenance of the integrity of the cell monolayers during the study. The modified chamber is designed to minimize turbulence created by the air lift system and to avoid edge damage to the monolayers. 10⁶ T84 cells are plated on a permeable support (1.98 cm² surface area) and maintained for 5-6 day before use. The supports are suspended over the bottom of a 100-mm culture dish to permit "bottom feeding" by laying them on top of a layer of glass beads as described in Barret and Bigby supra. After cell growth, the entire ring assembly is inserted into the Ussing chamber. No pressure is exerted directly on the monolayers and hence edge damage is avoided. Mucosal and serosal reservoirs contain identical volumes of oxygenated Ringer's solution (pH 7.4, at 37°C) that contained (in millimolar): Na, 140; K, 5.2; Ca, 1.2; Mg, 1.2; Cl, 119.8; HCO₃, 25; H₂PO₄, 2.4; HPO₄, 0.4; and glucose, 10. Potential difference (PD) across the cell monolayer is measured by calomel electrodes in 3 M KCl and monitored with a potentiometer. Throughout the experiment, except for 5-10 seconds every 5 min while the PD is being recorded, spontaneous tissue PD is short circuited and nullified by an automatic voltage clamp (WPI, New Haven, CT) with Ag:AgCl₂ electrodes. Tissue conductance (G) is calculated from the PD and the imposed current according to Ohm's law. The magnitude of changes in the short circuit current (Isc) is used as an index of chloride secretion.

### Example 4: Effect on fluid secretion and sodium excretion in ligated loops rodent models

The effect of agents described herein on fluid and sodium secretion can be assessed by injecting vehicle or a test agent (e.g., one or more agents described herein) directly into an isolated loop. This is done by surgically ligating a loop in the small intestine of a mouse. The methodology for ligated loop formation is described in London et al. 1997 Am J Physiol p.G93-105. The loop is roughly centered and is approximately 1-3 cm. The loops are injected with a test agent or vehicle. Following a recovery time of 90 minutes the loops are excised. Weights are recorded for each loop before and after removal of the fluid contained therein. The length of each loop is also recorded. A weight to length ratio (W/L) for each loop is calculated to determine the effects of test agent as compared to vehicle. To determine the effect of a test agent on sodium excretion, fluid from the loops is collected and profiled for electrolyte levels. Similar assays can be performed using rats instead of mice.

### Example 5: Animal Models of Hypertension

Various animal models of hypertension can be used to screen the agents described herein for anti-hypertensive activity. In general, hypertension can be induced in rats in at least four ways, including: genetically-induced, environmentally-induced, pharmacologically-induced, and renal-induced. A variety of rodent hypertension models are described in Pinto et al. (1998 Cardiovascular Research 39:77-88), Badyal et al. (2003 Indian Journal of Pharmacology 35: 349-362) and the references cited therein. One of the most widely used rodent models of hypertension is the Spontaneously Hypertensive Rat (SHR). Other models include: (1) the two-kidney one-clip, (2) transgenic rats overexpressing the murine Ren2 gene, (3) DOCA (deoxycorticosterone acetate)-salt model and (4) the Dahl salt sensitive rat. Thus, for example, agents described herein, can be administered to Dahl salt sensitive rats (Rapp and Dene 1985 Hypertension 7:340-9) to determine effects on blood pressure, urine volume and urinary sodium excretion and left ventricular wall thickness (for example as described in examples 4 and 5 herein).

### Example 6: Measurement of the Effects of Lubiprostone and ST peptide on Urinary Sodium Excretion and Urine Volume.

All experimental subjects were female Sprague-Dawley rats which weighed between 200-230 g at the time of experimentation. Following arrival at the animal facility, rats were housed in solid bottom cages in groups of three, where they had unlimited access to food and water. Temperature was maintained at 21 ± 2°C, and lights were on a 12:12 hr cycle (with lights on at 6:00AM).

Following at least 3 days of acclimation to the facility prior to experimentation, rats were dosed orally (PO) with either vehicle (phosphate buffered saline) or test article, and transferred to individual metabolism cages where they had access to food and water. The volume of urine excreted was recorded from 0-3 hours, and 4-6 hours post dose. In addition, 0.5-1.0mL urine samples were taken at each of the above time points and frozen for later analysis. Urine samples were analyzed for sodium concentration using ISE crown-ether membrane methodology on an Olympus AU5400 chemistry immuno analyzer (Olympus America Inc).

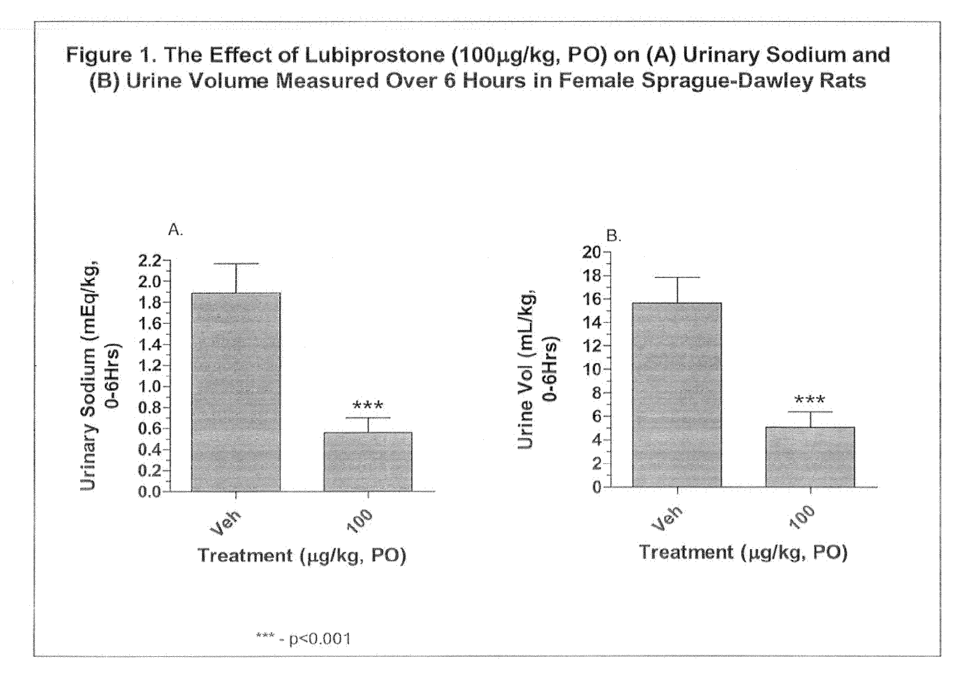

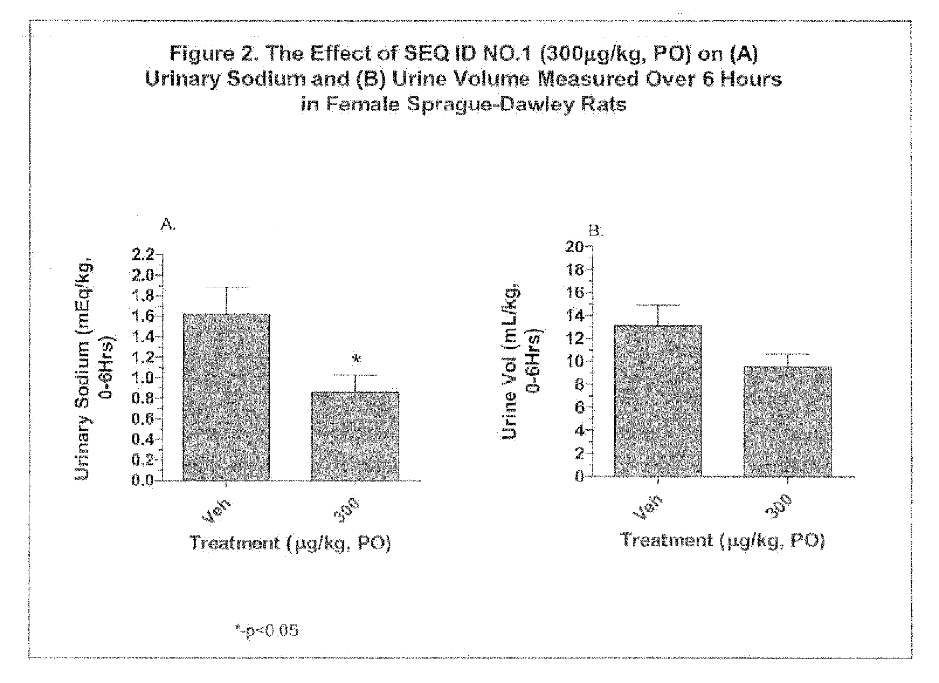

Figures 1, 2, and 3 demonstrate the effects of Lubiprostone and ST peptide (SEQ ID NO: 1: CCELCCNPACTGCY) on urine sodium and urine volume in this assay.

### Example 7. Effects of a test agent of left ventricular wall thickness in salt-sensitive and salt-resistant rats.

Salt-sensitive and salt-resistant, 4-5 week-old male Dahl rats (Brookhaven National Laboratory, Upton, New York, USA) are fed with Purina rat chow with 0.4% NaCl for the first 3-4 weeks. Thereafter salt-sensitive and salt-resistant rats are randomized into two populations receiving either a high-salt (8% NaCl) or a low-salt (0.4% NaCl) diet for a further 3 weeks. Following this, each population is separated into two groups, one receiving test agent in tap water and the other vehicle only. Test agent is given in incremental doses until systolic blood pressure (tail-cuff measurement) is < 140 mmHg.

At the end of the study rats are anaesthetized with intraperitoneal sodium pentobarbital (45 mg/kg), and systolic and diastolic blood pressures are measured directly through catheterization of the right femoral artery, using a Beckman R611 recorder. Blood (8-10 ml) for determination of plasma rennin activity (New England Nuclear Corporation, Boston, Massachusetts, USA) and aldosterone concentration (Diagnostic Products Corporation, Los Angeles, USA) is obtained by decapitation. Hearts are removed and placed in a Petri dish, and blood and blood clots are flushed out with cold saline. Superficial water is removed by blotting. The whole heart is weighed, thereafter the atria and the right ventricular free wall are dissected from the interventricular septum. The remaining interventricular septum and the left ventricle represented left ventricular weight, and the left ventricular weight: body weight ratio is taken as a measure for left ventricular mass or left ventricular hypertrophy.

### Methods of Treatment

A number of disorders associated with fluid or salt retention may be prevented or treated with agents that reduce sodium absorption in the intestine and/or increase anion secretion (e.g., in the instestine). Useful agents include: guanylate cyclase receptor C agonists, soluble guanylate cyclase modulators, prostanoids including prostaglandin E and derivatives thereof, chloride channel activators (e.g. Amitiza® (lubiprostone)), 5HT4 agonists, cyclic nucleotides, laxatives, CFTR (cystic fibrosis transmembrane conductance regulator) modulators, agents which affect cAMP levels, sodium transport inhibitors (e.g. sodium channel inhibitors such as amiloride), phosphodiesterase inhibitors, renin inhibitors and aldosterone antagonists, potassium, polymer resins, and combinations thereof described herein. The agents that reduce sodium absorption in the intestine and/or increase anion secretion can be used alone or in combination with one or more agents useful in the treatment of congestive heart failure, and/or one or more lipid lowering agent and/or one or more anti-hypertensive agents.

### Agents useful in the treatment of congestive heart failure

The agents described herein can be administered together with one or more agents useful in the treatment of congestive heart failure including, for example, nesiritide, dobutamine (beta receptor antagonist), milrinone (phosphodiesterase inhibitor), Levosimendan (Simdax®), adenosine, an adenosine analog (e.g. N⁶ -[(1,2-dihydro-1-acenaphthylenyl)methyl]adenosine, dipyridamole or iodotulercidin), an agent which increases the cellular availability of adenosine, an adenosine A₂ receptor agonist, an adenosine transport inhibitor, or an adenosine deaminase inhibitor.

### Lipid Lowering Agents

Lipid lowering agents or dilipidemia agents are those agents that act directly or indirectly to reduce serum cholesterol. Such agents include, but are not limited to, bile acid sequestrants such as cholestyramine (a styrene-divinylbenzene copolymer containing quaternary ammonium cationic groups capable of binding bile acids, such as QUESTRAN® or QUESTRAN LIGHT® cholestyramine which are available from Bristol-Myers Squibb), colesevelam hydrochloride (such as WELCHOL® Tablets (polyallylamine hydrochloride) cross-linked with epichlorohydrin and alkylated with 1-bromodecane and (6-bromohexyl)-trimethylammonium bromide) which are available from Sankyo), colestipol (a copolymer of diethylenetriamine and 1-chloro-2,3-epoxypropane, such as COLESTID® tablets which are available from Pharmacia), dialkylaminoalkyl derivatives of a cross-linked dextran, LOCHOLEST®, DEAE-Sephadex (SECHOLEX®, POLICEXIDE®), water soluble derivatives such as 3,3-ioene, N-(cycloalkyl)alkylamines and poliglusam, insoluble quaternized polystyrenes, saponins and mixtures thereof and those bile acid sequestrants disclosed in WO97/11345, WO98/57652, US3692895, and US5703188. Suitable inorganic cholesterol sequestrants include bismuth salicylate plus montmorillonite clay, aluminum hydroxide and calcium carbonate antacids.

HMG-CoA reductase inhibitors are dyslipidemic agents that can be used in therapeutic combination with GC-C receptor agonist. s with compounds described herein. Suitable HMG-CoA reductase inhibitors for use in therapeutic combination with a compounds described herein include: atorvastatin (LIPITOR®; disclosed in US4681893, US5385929 and US5686104), atorvastatin calcium (disclosed in US5273995), dihydrocompactin, (disclosed in US4450171), bervastatin (disclosed in US5082859), carvastatin, cerivastatin (BAYCOL®; disclosed in US5006530, US5502199, and US5177080), crilvastatin, dalvastatin (disclosed in EP738510A2), fluvastatin (LESCOL®; disclosed in US4739073 and US534772), glenvastatin, fluindostatin (disclosed in EP363934A1), velostatin (visinolin; disclosed in US4448784 and US4450171), lovastatin (mevinolin; MEVACOR® (Merck and Co.) and related compounds disclosed in US4231938), mevastatin (and related compound disclosed in US3983140), compactin (and related compounds disclosed in US4804770), pitavastatin ( also known as NK-104, itavastatin, nisvastatin, nisbastatin disclosed in US5102888), pravastatin (PRAVACHOL® (Bristol Myers Squibb) and related compounds disclosed in US4346227), rivastatin (sodium 7-(4-fluorophenyl)-2,6-diisopropyl-5-methoxymethylpyridin-3-yl )-3,5-dihydroxy-6-heptanoate), rosuvastatin (CRESTOR®; also known as ZD-4522 disclosed in US5260440), atavastatin, visastatin, simvastatin (ZOCOR® (Merck and Co.) and related compounds as disclosed in US4448784 and US4450171), sirrivastatin, CI-981, compounds disclosed in WO03/033481, US4231938, US4444784, US4647576, US4686237, US4499289, US4346227, US5753675, US4613610, EP0221025, and EP491226, and optical or geometric isomers thereof; and nontoxic pharmaceutically acceptable salts, N-oxides, esters, quaternary ammonium salts, and prodrugs thereof. In HMG-CoA reductase inhibitors where an open-acid form can exist, salt and ester forms may preferably be formed from the open-acid, and all such forms are included within the meaning of the term "HMG-CoA reductase inhibitor" as used herein. Pharmaceutically acceptable salts with respect to the HMG-CoA reductase inhibitor includes non-toxic salts of the compounds which are generally prepared by reacting the free acid with a suitable organic or inorganic base, particularly those formed from cations such as sodium, potassium, aluminum, calcium, lithium, magnesium, zinc and tetramethylammonium, as well as those salts formed from amines such as ammonia, ethylenediamine, N-methylglucamine, lysine, arginine, ornithine, choline, N,N'-dibenzylethylenediamine, chloroprocaine, diethanolamine, procaine, N-benzylphenethylamine, 1-p-chlorobenzyl-2-pyrrolidine-1'-yl-methylbenzim- idazole, diethylamine, piperazine, and tris(hydroxymethyl) aminomethane. Further examples of salt forms of HMG-CoA reductase inhibitors may include, but are not limited to, acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynapthoate, iodide, isothionate, lactate, lactobionate, laurate, malate, maleate, mandelate, mesylate, methylsulfate, mucate, napsylate, nitrate, oleate, oxalate, pamaote, palmitate, panthothenate, phosphate/diphosphate, polygalacturonate, salicylate, stearate, subacetate, succinate, tannate, tartrate, teoclate, tosylate, triethiodide, and valerate.

Other dyslipidemic agents which can be used in therapeutic combination with a GC-C receptor agonist described herein include:
HMG-CoA synthase inhibitors such as L-659,699 ((E,E)-11-[3'R-(hydroxy-methyl)-4'-oxo-2'R-oxetanyl]-3,5,7R-trimethyl-2,4-undecadienoic acid) and those disclosed in US5120729, US5064856, and US4847271;
cholesterol absorption inhibitors such as plant sterols, plant stanols and/or fatty acid estesrs of plant stanols such as sitostanol ester used in BENECOL® margarine, stanol esters, beta-sitosterol, and sterol glycosides such as tiqueside. Other cholesterol absorption inhibitors include 1,4-Diphenylazetidin-2-ones; 4-biarylyl-1-phenylazetidin-2-ones; 4-(hydroxyphenyl)azetidin-2-ones; 1,4-diphenyl-3-hydroxyalkyl-2-azetidinones; 4-biphenyl-1-phenylazetidin-2-ones; 4-biarylyl-1-phenylazetidin-2-ones; and 4-biphenylylazetidinones.
acyl coenzyme A -cholesterol acyl transferase (ACAT) inhibitors such as avasimibe (Current Opinion in Investigational Drugs. 3(9):291-297 (2003)), eflucimibe, HL-004, lecimibe, DuP-128, KY505, SMP 797, CL-277,082 (Clin Pharmacol Ther. 48(2):189-94 (1990)) and the like; and those disclosed in US5510379, WO96/26948 and WO96/10559;
CETP inhibitors such as JTT 705 identified as in Nature 406, (6792):203-7 (2000), torcetrapib (CP-529,414 described in US20030186952 and WO00/017164), CP 532,632, BAY63-2149, SC 591, SC 795, and the like including those described in Current Opinion in Investigational Drugs. 4(3):291-297 (2003) and those disclosed in J. Antibiot., 49(8): 815-816 (1996), and Bioorg. Med. Chem. Lett., 6:1951-1954 (1996) and patent publications US5512548, US6147090, WO99/20302, WO99/14204, WO99/41237, WO95/04755, WO96/15141, WO96/05227, WO038721, EP796846, EP818197, EP818448, DE19704244, DE19741051, DE19741399, DE197042437, DE19709125, DE19627430, DE19832159, DE19741400, JP 11049743, and JP 09059155;
squalene synthetase inhibitors such as squalestatin-1, TAK-475, and those disclosed in US4871721, US4924024, US5712396 (α-phosphono-sulfonates), Biller et al (1988) J. Med. Chem., 31:1869 (e.g. isoprenoid (phosphinyl-methyl)phosphonates), Biller et al (1996) Current Pharmaceutical Design, 2:1, P. Ortiz de Montellano et al (1977) J. Med. Chem. 20:243 (terpenoid pyrophosphates), Corey and Volante (1976) J. Am. Chem. Soc., 98:1291 (farnesyl diphosphate analog A and presqualene pyrophosphate (PSQ-PP) analogs), McClard et al (1987) J.A.C.S., 109:5544 (phosphinylphosphonates), Capson, T. L., PhD dissertation, June, 1987, Dept. Med. Chem. U of Utah, Abstract, Table of Contents, pp 16, 17, 40-43, 48-51, Summary, (cyclopropanes), Curr. Op. Ther. Patents (1993) 861, and patent publications EP0567026Al, EP0645378Al, EP0645377Al, EP0611749Al, EP0705607A2, EP0701725Al, and WO96/09827;
antioxidants such as probucol (and related compounds disclosed in US3674836), probucol derivatives such as AGI-1067 (and other derivatives disclosed in US6121319 and US6147250), tocopherol, ascorbic acid, β-carotene, selenium and vitamins such as vitamin B6 or vitamin B12 and pharmaceutically acceptable salts and esters thereof;
PPARα agonists such as those disclosed in US6028109 (fluorophenyl compounds), WO00/75103 (substituted phenylpropionic compounds), WO98/43081 and fibric acid derivatives (fibrates) such as beclofibrate, benzafibrate, bezafibrate (C.A.S. Registry No. 41859-67-0, see US3781328), binifibrate (C.A.S. Registry No. 69047-39-8, see BE884722), ciprofibrate (C.A.S. Registry No. 52214-84-3, see US3948973), clinofibrate (C.A.S. Registry No. 30299-08-2, see US3716583), clofibrate (such as ethyl 2-(p-chlorophenoxy)-2-methyl-propionate, e.g. Atromid-S® capsules (Wyeth-Ayerst), etofibrate, fenofibrate (such as Tricor® micronized fenofibrate ((2-[4-(4-chlorobenzoyl)phenoxy]-2-methyl-propanoic acid, 1-methylethyl ester; Abbott Laboratories) or Lipanthyl® micronized fenofibrate (Labortoire Founier, France)), gemcabene, gemfibrozil (such as 5-(2,5-dimethylphenoxy)-2,2-dimethylpentanoic acid, e.g. Lopid® tablets (Parke Davis)), lifibrol, GW 7647, BM 170744, LY518674 and those fibrate and fibrate acid derivatives disclosed in WO03/033456, WO03/033481, WO03/043997, WO03/048116, WO03/053974, WO03/059864, and WO03/05875;
FXR receptor modulators such as GW 4064, SR 103912, and the like;
LXR receptor modulators such as GW 3965, T9013137, and XTC0179628, and those disclosed in US20030125357, WO03/045382, WO03/053352, WO03/059874, and the like;
HM74 and HM74A (human HM74A is Genbank Accession No. AY148884 and rat HM74A is EMM_patAR098624) receptor agonists such as nicotinic acid (niacin) and derivatives thereof (e.g. compounds comprising a pyridine-3-carboxylate structure or a pyrazine-2-carboxylate structure, including acid forms, salts, esters, zwitterions and tautomers, where available) including but not limited to those disclosed in Wise et al (2003) J. Biol. Chem. 278: 9869 (e.g. 5-methylpyrazole-3-carboxylic acid and acifran (4,5-dihydro-5-methyl-4-oxo-5-phenyl-2-furan carboxylic acid pyradine-3-acetic acid)), as well as 5-methyl nicotinic acid, nicotinuric acid, niceritrol, nicofuranose, acipimox (5-methylpyrazine-2-carboxylic acid 4-oxide), Niaspan® (niacin extended-release tablets; Kos) and those which can be easily identified by one skilled in the art which bind to and agonize the HM74A or HM74 receptor (for example using the assays disclosed in Wise et al (2003) J. Biol. Chem 278:9869 (nicotine binding and [35S]-GTPγS binding assays), Soga et al (2003) Biochem. Biophys. Res. Comm. 303:364 (radiolabel binding assay using the HM74 receptor which could be adapted to the HM74A receptor), Tunaru et al (2003) Nature Medicine 9:352 (calcium mobilization assay using the HM74 receptor which could be adapted to the HM74A receptor) and US6420183 (FLIPR assays are described generally in and may be adapted to the HM74A or HM74 receptor);
renin angiotensin system inhibitors;
bile acid reabsorption inhibitors (bile acid reuptake inhibitors), such as BARI 1453, SC435, PHA384640, S8921, AZD7706, and the like;
PPARδ agonists (including partial agonists) such as GW 501516, and GW 590735, and those disclosed in US5859051 (acetophenols), WO03/024395, W097/28149, WO01/79197, WO02/14291, WO02/46154, WO02/46176, WO02/076957, WO03/016291, WO03/033493, WO99/20275 (quinoline phenyl compounds), WO99/38845 (aryl compounds), WO00/63161 (1,4-disubstituted phenyl compounds), WO01/00579 (aryl compounds), WO01/12612 & WO01/12187 (benzoic acid compounds), and WO97/31907 (substituted 4-hydroxy-phenylalconic acid compound);
sterol biosynthesis inhibitors such as DMP-565;
triglyceride synthesis inhibitors;
microsomal triglyceride transport (MTTP) inhibitors, such as inplitapide, LAB687, and CP346086, AEGR 733, implitapide and the like;
HMG-CoA reductase gene expression inhibitors (e.g. compounds that decrease HMG-CoA reductase expression by affecting (e.g. blocking) transcription or translation of HMG-CoA reductase into protein or compounds that may be biotransformed into compounds that have the aforementioned attributes by one or more enzymes in the cholesterol biosynthetic cascade or may lead to the accumulation of an isoprene metabolite that has the aforementioned activities (such regulation is readily determined by those skilled in the art according to standard assays (Methods of Enzymology, 110:9-19 1985))) such as those disclosed in US5041432 (certain 15-substituted lanosterol derivatives) and E. I. Mercer (1993) Prog. Lip. Res. 32:357 (oxygenated sterols that suppress the biosynthesis of HMG-CoA reductase);
squalene epoxidase inhibitors such as NB-598 ((E)-N-ethyl-N-(6,6-dimethyl-2-hepten-4-γ- nyl )-3-[(3,3'-bithiophen-5-yl)methoxy]benzene-methanamine hydrochloride);
low density lipoprotein (LDL) receptor inducers such as HOE-402 (an imidazolidinyl-pyrimidine derivative that directly stimulates LDL receptor activity, see Huettinger et al (1993) Arterioscler. Thromb. 13:1005);
platelet aggregation inhibitors;
5-LO or FLAP inhibitors;
PPAR modulators (including compounds that may have multiple functionality for activating various combinations of PPARα, PPARγ, and PPARδ) such as those disclosed in US6008237, US6248781, US6166049, WO00/12491, WO00/218355, WO00/23415, WO00/23416, WO00/23425, WO00/23442, WO00/23445, WO00/23451, WO00/236331, WO00/236332, WO00/238553, WO00/50392, WO00/53563, WO00/63153, WO00/63190, WO00/63196, WO00/63209, WO00/78312, WO00/78313, WO01/04351, WO01/14349, WO01/14350, WO01/16120, WO01/17994, WO01/21181, WO01/21578, WO01/25181, WO01/25225, WO01/25226, WO01/40192, WO01/79150, WO02/081428, WO02/100403, WO02/102780, WO02/79162, WO03/016265, WO03/033453, WO03/042194, WO03/043997, WO03/066581, WO97/25042, WO99/07357, WO99/11255, WO99/12534, WO99/15520, WO99/46232, and WO98/05331 (including GW2331 or (2-(4-[difluorophenyl]-1 heptylureido)ethyl]phenoxy)-2-methylbutyric));
niacin-bound chromium, as disclosed in WO03/039535;
substituted acid derivatives disclosed in WO03/040114;
apolipoprotein B inhibitors such as those disclosed in WO02/090347, WO02/28835, WO03/045921, WO03/047575;
Factor Xa modulators such as those disclosed in WO03/047517, WO03/047520, WO03/048081;
ileal bile acid transport ("IBAT") inhibitors (or apical sodium co-dependent bile acid transport ("ASBT") inhibitors) such as benzothiepines (including 1,2-benzothiazepines; 1,4-benzothiazepines; 1,5-benzothiazepines; 1,2, 5-benzothiadiazepines);
PPARδ activators such as disclosed in WO01/00603 (thiazole and oxazole derivates (e.g. C.A.S. Registry No. 317318-32-4), WO97/28149 (fluoro, chloro and thio phenoxy phenylacetic), US5093365 (non-1-oxidizable fatty acid analogues), and WO99/04815.

### Anti-hypertensive agents

The agents described herein can be used in therapeutic combination with one or more anti-hypertensive agents, including but not limited to:
diuretics, such as thiazides (e.g., chlorthalidone, cyclothiazide (CAS RN 2259-96-3), chlorothiazide (CAS RN 72956-09-3, which may be prepared as disclosed in US2809194), dichlorophenamide, hydroflumethiazide, indapamide, polythiazide, bendroflumethazide, methyclothazide, polythiazide, trichlormethazide, chlorthalidone, indapamide, metolazone, quinethazone, althiazide (CAS RN 5588-16-9, which may be prepared as disclosed in British Patent No. 902,658), benzthiazide (CAS RN 91-33-8, which may be prepared as disclosed in US3108097), buthiazide (which may be prepared as disclosed in British Patent Nos. 861,367), and hydrochlorothiazide), loop diuretics (e.g., bumetanide, ethacrynic acid, furosemide, and torasemide), potassium sparing agents (e.g., amiloride, and triamterene (CAS Number 396-01-0)), and aldosterone antagonists (e.g., spironolactone (CAS Number 52-01-7 and active metabolites thereof including canrenone), epirenone, and the like);
β-adrenergic blockers such as Amiodarone (Cordarone, Pacerone), bunolol hydrochloride (CAS RN 31969-05-8, Parke-Davis), acebutolol (±N-[3-Acetyl-4-[2-hydroxy-3-[(1 methylethyl)amino]propoxy]phenyl]-butanamide, or (±)-3'-Acetyl-4'-[2-hydroxy -3-(isopropylamino) propoxy] butyranilide), acebutolol hydrochloride (e.g., Sectral®, Wyeth-Ayerst), alprenolol hydrochloride (CAS RN 13707-88-5 see Netherlands Patent Application No. 6,605,692), atenolol (e.g., Tenormin®, AstraZeneca), carteolol hydrochloride (e.g., Cartrol® Filmtab®, Abbott), Celiprolol hydrochloride (CAS RN 57470-78-7, also see in US4034009), cetamolol hydrochloride (CAS RN 77590-95-5, see also US4059622), labetalol hydrochloride (e.g., Normodyne®, Schering), esmolol hydrochloride (e.g., Brevibloc®,Baxter), levobetaxolol hydrochloride (e.g., Betaxon™ Ophthalmic Suspension, Alcon), levobunolol hydrochloride (e.g., Betagan® Liquifilm® with C CAP® Compliance Cap, Allergan), nadolol (e.g., Nadolol, Corgard, Mylan), practolol (CAS RN 6673-35-4, see also US3408387), propranolol hydrochloride (CAS RN 318-98-9), sotalol hydrochloride (e.g., Betapace AF™,Berlex), timolol (2-Propanol,1-[(1,1-dimethylethyl)amino]-3-[[4-4(4-morpholinyl)-1,2,5-thiadiazol-3-yl]oxy]-, hemihydrate, (S)-, CAS RN 91524-16-2), timolol maleate (S)-1-[(1,1-dimethylethyl) amino]-3-[[4- (4-morpholinyl)-1,2,5-thiadiazol -3- yl] oxy]-2-propanol (Z)-2-butenedioate (1:1) salt, CAS RN 26921-17-5), bisoprolol (2-Propanol, 1-[4-[[2-(1-methylethoxy)ethoxy]-methyl]phenoxyl]-3-[(1-meth- ylethyl)amino]-, (±), CAS RN 66722-44-9), bisoprolol fumarate (such as (±)-1-[4-[[2-(1-Methylethoxy) ethoxy]methyl]phenoxy]-3-[(1-methylethyl)amino]-2-propanol (E) -2-butenedioate (2:1) (salt), e.g., Zebeta™, Lederle Consumer), nebivalol (2H-1-Benzopyran-2-methanol, αα'-[iminobis(methylene)]bis[6-fluoro-3,4-dihydro-, CAS RN 99200-09-6 see also U.S. Pat. No. 4,654,362), cicloprolol hydrochloride, such 2-Propanol, 1-[4-[2-(cyclopropylmethoxy)ethoxy]phenoxy]-3-[1-methylethyl)amino]-, hydrochloride, A.A.S. RN 63686-79-3), dexpropranolol hydrochloride (2-Propanol,1-[1-methylethy)-amino]-3-(1-naphthalenyloxy)-hydrochloride (CAS RN 13071-11-9), diacetolol hydrochloride (Acetamide, N-[3-acetyl-4-[2-hydroxy-3-[(1-methyl-ethyl)amino]propoxy][phenyl]-, monohydrochloride CAS RN 69796-04-9), dilevalol hydrochloride (Benzamide, 2-hydroxy-5-[1-hydroxy-2-[1-methyl-3-phenylpropyl)amino]ethyl]-, monohydrochloride, CAS RN 75659-08-4), exaprolol hydrochloride (2-Propanol, 1-(2-cyclohexylphenoxy)-3-[(1-methylethyl)amino]-, hydrochloride CAS RN 59333-90-3), flestolol sulfate (Benzoic acid, 2-fluro-,3-[[2-[aminocarbonyl)amino]- - dimethylethyl]amino]-2-hydroxypropyl ester, (±)- sulfate (1:1) (salt), CAS RN 88844-73-9; metalol hydrochloride (Methanesulfonamide, N-[4-[1-hydroxy-2-(methylamino)propyl]phenyl]-, monohydrochloride CAS RN 7701-65-7), metoprolol 2-Propanol, 1-[4-(2-methoxyethyl)phenoxy]-3-[1-methylethyl)amino]-; CAS RN 37350-58-6), metoprolol tartrate (such as 2-Propanol, 1-[4-(2-methoxyethyl)phenoxy]-3-[(1-methylethyl)amino]-, e.g., Lopressor®, Novartis), pamatolol sulfate (Carbamic acid, [2-[4-[2-hydroxy-3-[(1-methylethyl)amino]propoxyl]phenyl]-ethyl]-, methyl ester, (±) sulfate (salt) (2:1), CAS RN 59954-01-7), penbutolol sulfate (2-Propanol, 1-(2-cyclopentylphenoxy)-3-[1,1-dimethylethyl)amino]1, (S)-, sulfate (2:1) (salt), CAS RN 38363-32-5), practolol (Acetamide, N-[4-[2-hydroxy-3-[(1-methylethyl)amino]-propoxy]phenyl]-, CAS RN 6673-35-4;) tiprenolol hydrochloride (Propanol, 1-[(1-methylethyl)amino]-3-[2-(methylthio)-phenoxy]-, hydrochloride, (±), CAS RN 39832-43-4), tolamolol (Benzamide, 4-[2-[[2-hydroxy-3-(2-methylphenoxy)-propyl]amino]ethoxyl]-, CAS RN 38103-61-6), bopindolol, indenolol, pindolol (e.g., Visken), propanolol (e.g., Inderal, Inderal-LA), tertatolol, Coreg (carvedilol), and tilisolol, and the like;
calcium channel blockers such as besylate salt of amlodipine (such as 3-ethyl-5-methyl-2-(2-aminoethoxymethyl)-4-(2-chlorophenyl)-1,4-dihydro-6-methyl-3,5-pyridinedicarboxylate benzenesulphonate, e.g., Norvasc®, Pfizer), clentiazem maleate (1,5-Benzothiazepin-4(5H)-one, 3-(acetyloxy)-8-chloro-5-[2-(dimethylamino)ethyl]-2,3-dihydro-2-(4-methoxyphenyl)-(2S-cis)-, (Z)-2-butenedioate (1:1), see also US4567195), isradipine (3,5-Pyridinedicarboxylic acid, 4-(4-benzofurazanyl)-1,4-dihydro-2,6-dimethyl-, methyl 1-methylethyl ester, (±)-4(4-benzofurazanyl)-1,4-dihydro-2,6-dimethyl-3,5-pyridinedicarboxylate, see also US4466972); nimodipine (such as is isopropyl (2- methoxyethyl) 1, 4- dihydro -2,6- dimethyl -4- (3-nitrophenyl) -3,5- pyridine - dicarboxylate, e.g., Nimotop®, Bayer), felodipine (such as ethyl methyl 4-(2,3-dichlorophenyl)-1,4-dihydro-2,6-dimethyl-3,5-pyridinedicarboxylate-, e.g., Plendil® Extended-Release, AstraZeneca LP), nilvadipine (3,5-Pyridinedicarboxylic acid, 2-cyano-1,4-dihydro-6-methyl-4-(3-nitrophenyl)-,3-methyl 5-(1-methylethyl) ester, also see US3799934), nifedipine (such as 3,5-pyridinedicarboxylic acid,1,4-dihydro-2,6-dimethyl-4-(2-nitrophenyl)-, dimethyl ester, e.g., Procardia XL® Extended Release Tablets, Pfizer), diltiazem hydrochloride (such as 1,5-Benzothiazepin-4(5H)-one,3-(acetyloxy)-5[2-(dimethylamino)ethyl]-2,-3-dihydro-2(4-methoxyphenyl)-, monohydrochloride, (+)-cis., e.g., Tiazac®, Forest), verapamil hydrochloride (such as benzeneacetronitrile, (alpha)-[[3-[[2-(3,4-dimethoxyphenyl) ethyl]methylamino]propyl]-3,4-dimethoxy-(alpha)-(1-methylethyl) hydrochloride, e.g., Isoptin® SR, Knoll Labs), teludipine hydrochloride (3,5-Pyridinedicarboxylic acid, 2-[(dimethylamino)methyl]4-[2-[(1E)-3-(1,1-dimethylethoxy)-3-oxo-1-propenyl]phenyl]-1,4-dihydro-6-methyl-, diethyl ester, monohydrochloride) CAS RN 108700-03-4), belfosdil (Phosphonic acid, [2-(2-phenoxyethyl)-1,3-propane- diyl]bis-, tetrabutyl ester CAS RN 103486-79-9), fostedil (Phosphonic acid, [[4-(2-benzothiazolyl)phenyl]methyl]-, diethyl ester CAS RN 75889-62-2), aranidipine, azelnidipine, barnidipine, benidipine, bepridil, cinaldipine, clevidipine, efonidipine, gallopamil, lacidipine, lemildipine, lercanidipine, monatepil maleate (1-Piperazinebutanamide, N-(6,11-dihydrodibenzo(b,e)thiepin-11-yl)₄-(4-fluorophenyl)-, (±)-, (Z)-2-butenedioate (1:1) (±)-N-(6,11-Dihydrodibenzo(b,e)thiep-in-11-yl)-4-(p-fluorophenyl)-1-piperazinebutyramide maleate (1:1) CAS RN 132046-06-1), nicardipine, nisoldipine, nitrendipine, manidipine, pranidipine, and the like;
T-channel calcium antagonists such as mibefradil;
angiotensin converting enzyme (ACE) inhibitors such as benazepril, benazepril hydrochloride (such as 3-[[1-(ethoxycarbonyl)-3-phenyl-(1S)-propyl]amino]-2,3,4,5-tetrahydro-2-oxo-1H-1-(3S)-benzazepine-1-acetic acid monohydrochloride, e.g., Lotrel®, Novartis), captopril (such as 1-[(2S)-3-mercapto-2-methylpropionyl]-L-proline, e.g., Captopril, Mylan, CAS RN 62571-86-2 and others disclosed in US4046889), ceranapril (and others disclosed in US4452790), cetapril (alacepril, Dainippon disclosed in Eur. Therap. Res. 39:671 (1986); 40:543 (1986)), cilazapril (Hoffman-LaRoche) disclosed in J. Cardiovasc. Pharmacol. 9:39 (1987), indalapril (delapril hydrochloride (2H-1,2,4-Benzothiadiazine-7-sulfonamide, 3-bicyclo[2.2.1]hept-5-en-2-yl-6-chloro-3,4-dihydro-, 1,1-dioxide CAS RN 2259-96-3); disclosed in US4385051), enalapril (and others disclosed in US4374829), enalopril, enaloprilat, fosinopril, ((such as *trans*-L-proline, 4-cyclohexyl-1-[[[2-methyl-1-(1-oxopropoxy) propoxy](4-phenylbutyl) phosphinyl]acetyl]-, sodium salt, e.g., Monopril, Bristol-Myers Squibb and others disclosed in US4168267), fosinopril sodium (L-Proline, 4-cyclohexyl-1-[[(R)-[(1S)-2-methyl-1-(1-ox-opropoxy)propox), imidapril, indolapril (Schering, disclosed in J. Cardiovasc. Pharmacol. 5:643, 655 (1983)), lisinopril (Merck), losinopril, moexipril, moexipril hydrochloride (3-Isoquinolinecarboxylic acid, 2-[(2S)-2-[[(1S)-1-(ethoxycarbonyl)-3-phenylpropyl]amino]-1-oxopropyl]-1,-2,3,4-tetrahydro-6,7-dimethoxy-, monohydrochloride, (3S)- CAS RN 82586-52-5), quinapril, quinaprilat, ramipril (Hoechsst) disclosed in EP 79022 and Curr. Ther. Res. 40:74 (1986), perindopril erbumine (such as 2S,3aS,7aS-1-[(S)-N-[(S)-1-Carboxybutyl]alanyl]hexahydro-2-indolinecarboxylic acid, 1-ethyl ester, compound with tert-butylamine (1:1), e.g., Aceon®, Solvay), perindopril (Servier, disclosed in Eur. J. clin. Pharmacol. 31:519 (1987)), quanipril (disclosed in US4344949), spirapril (Schering, disclosed in Acta. Pharmacol. Toxicol. 59 (Supp. 5):173 (1986)), tenocapril, trandolapril, zofenopril (and others disclosed in US4316906), rentiapril (fentiapril, disclosed in Clin. Exp. Pharmacol. Physiol. 10:131 (1983)), pivopril, YS980, teprotide (Bradykinin potentiator BPP9a CAS RN 35115-60-7), BRL 36,378 (Smith Kline Beecham, see EP80822 and EP60668), MC-838 (Chugai, see C.A. 102:72588v and Jap. J. Pharmacol. 40:373 (1986), CGS 14824 (Ciba-Geigy, 3-([1-ethoxycarbonyl-3-phenyl-(1S)-propyl]amino)-2,3,4,5-tetrahydro-2-ox-o-1-(3S)-benzazepine-1 acetic acid HCl, see U.K. Patent No. 2103614), CGS 16,617 (Ciba-Geigy, 3(S)-[[(1S)-5-amino-1-carboxypentyl]amino]-2,3,4,-5-tetrahydro-2-oxo-1H-1-benzazepine-1-ethanoic acid, see US4473575), Ru 44570 (Hoechst, see Arzneimittelforschung 34:1254 (1985)), R 31-2201 (Hoffman-LaRoche see FEBS Lett. 165:201 (1984)), CI925 (Pharmacologist 26:243, 266 (1984)), WY-44221 (Wyeth, see J. Med. Chem. 26:394 (1983)), and those disclosed in US2003006922 (paragraph 28), US4337201, US4432971 (phosphonamidates);
neutral endopeptidase inhibitors such as omapatrilat (Vanlev®), CGS 30440, cadoxatril and ecadotril, fasidotril (also known as aladotril or alatriopril), sampatrilat, mixanpril, and gemopatrilat, AVE7688, ER4030, and those disclosed in US5362727, US5366973, US5225401, US4722810, US5223516, US4749688, US5552397, US5504080, US5612359, US5525723, EP0599444,EP0481522,EP0599444,EP0595610,EP0534363,EP534396,EP534492, EP0629627;
endothelin antagonists such as tezosentan, A308165, and YM62899, and the like;
vasodilators such as hydralazine (apresoline), clonidine (clonidine hydrochloride (1H-Imidazol-2-amine, N-(2,6-dichlorophenyl)4,5-dihydro-, monohydrochloride CAS RN 4205-91-8), catapres, minoxidil (loniten), nicotinyl alcohol (roniacol), diltiazem hydrochloride (such as 1,5-Benzothiazepin-4(5H)-one,3-(acetyloxy)-5[2-(dimethylamino)ethyl]-2,-3-dihydro-2(4-methoxyphenyl)-, monohydrochloride, (+)-cis, e.g., Tiazac®, Forest), isosorbide dinitrate (such as 1,4:3,6-dianhydro-D-glucitol 2,5-dinitrate e.g., Isordil® Titradose®, Wyeth-Ayerst), sosorbide mononitrate (such as 1,4:3,6-dianhydro-D-glucito-1,5-nitrate, an organic nitrate, e.g., Ismo®, Wyeth-Ayerst), nitroglycerin (such as 2,3 propanetriol trinitrate, e.g., Nitrostat® Parke-Davis), verapamil hydrochloride (such as benzeneacetonitrile, (±)-(alpha)[3-[[2-(3,4 dimethoxyphenyl)ethyl]methylamino]propyl]-3,4-dimethoxy-(alpha)- (1-methylethyl) hydrochloride, e.g., Covera HS® Extended-Release, Searle), chromonar (which may be prepared as disclosed in US3282938), clonitate (Annalen 1870 155), droprenilamine (which may be prepared as disclosed in DE2521113), lidoflazine (which may be prepared as disclosed in US3267104); prenylamine (which may be prepared as disclosed in US3152173), propatyl nitrate (which may be prepared as disclosed in French Patent No. 1,103,113), mioflazine hydrochloride (1-Piperazineacetamide, 3-(aminocarbonyl)₄-[4,4-bis(4-fluorophenyl)butyl]-N-(2,6-dichlorophenyl)-, dihydrochloride CAS RN 83898-67-3), mixidine (Benzeneethanamine, 3,4-dimethoxy-N-(1-methyl-2-pyrrolidinylidene)- Pyrrolidine, 2-[(3,4-dimethoxyphenethyl)imino]-1-methyl-1-Methyl-2-[(3,4-dimethoxyphenethyl)imino]pyrrolidine CAS RN 27737-38-8), molsidomine (1,2,3-Oxadiazolium, 5-[(ethoxycarbonyl)amino]-3-(4-morpholinyl)-, inner salt CAS RN 25717-80-0), isosorbide mononitrate (D-Glucitol, 1,4:3,6-dianhydro-, 5-nitrate CAS RN 16051-77-7), erythrityl tetranitrate (1,2,3,4-Butanetetrol, tetranitrate, (2R,3S)-rel-CAS RN 7297-25-8), clonitrate(1,2-Propanediol, 3-chloro-, dinitrate (7CI, 8CI, 9CI) CAS RN 2612-33-1), dipyridamole Ethanol, 2,2',2",2"'-[(4,8-di-1-piperidinylpyrimido[5,4-d]pyrimidine-2,6-diyl)dinitrilo]tetrakis- CAS RN 58-32-2), nicorandil (CAS RN 65141-46-0 3-), pyridinecarboxamide (N-[2-(nitrooxy)ethyl]-Nisoldipine3,5-Pyridinedicarboxylic acid, 1,4-dihydro-2,6-dimethyl-4-(2-nitrophenyl)-, methyl 2-methylpropyl ester CAS RN 63675-72-9), nifedipine3,5-Pyridinedicarboxylic acid, 1,4-dihydro-2,6-dimethyl-4-(2-nitrophenyl)-, dimethyl ester CAS RN 21829-25-4), perhexiline maleate (Piperidine, 2-(2,2-dicyclohexylethyl)-, (2Z)-2-butenedioate (1:1) CAS RN 6724-53-4), oxprenolol hydrochloride (2-Propanol, 1-[(1-methylethyl)amino]-3-[2-(2-propenyloxy)phenoxy]-, hydrochloride CAS RN 6452-73-9), pentrinitrol (1,3-Propanediol, 2,2-bis[(nitrooxy)methyl]-, mononitrate (ester) CAS RN 1607-17-6), verapamil (Benzeneacetonitrile, α-[3-[[2-(3,4-dimethoxyphenyl)ethyl]- methylamino]propyl]-3,4-dimethoxy-α-(1-methylethyl)- CAS RN 52-53-9) and the like;
angiotensin II receptor antagonists such as, aprosartan, zolasartan, olmesartan, pratosartan, FI6828K, RNH6270, candesartan (1 H-Benzimidazole-7-carboxylic acid, 2-ethoxy-1-[[2'-(1H-tetrazol-5-yl)[1,1'-biphenyl]4-yl]methyl]- CAS RN 139481-59-7), candesartan cilexetil ((+/-)-1-(cyclohexylcarbonyloxy)ethyl-2-ethoxy-1-[[2'-(1H-tetrazol-5-yl)biphenyl-4-yl]-1H-benzimidazole carboxylate, CAS RN 145040-37-5, US5703110 and US5196444), eprosartan (3-[1-4-carboxyphenylmethyl)-2-n-butyl-imidazol-5-yl]-(2-thienylmethyl) propenoic acid, US5185351 and US5650650), irbesartan (2-n-butyl-3- [[2'-(1h-tetrazol-5-yl)biphenyl-4-yl]methyl]1,3-diazazspiro[4,4]non-1-en-4-one, US5270317 and US5352788), losartan (2-N-butyl-4-chloro-5-hydroxymethyl-1-[(2'-(1H-tetrazol-5-yl)biphenyl-4-yl)-methyl]imidazole, potassium salt, US5138069, US5153197 and US5128355), tasosartan (5,8-dihydro-2,4-dimethyl-8-[(2'-(1H-tetrazol-5-yl)[1,1'-biphenyl]4-yl)methyl]-pyrido[2,3-d]pyrimidin-7(6H)-one, US5149699), telmisartan (4'-[(1,4-dimethyl-2'-propyl-(2,6'-bi-1H-benzimidazol)-1'-yl)]-[1,1'-biphenyl]-2-carboxylic acid, CAS RN 144701-48-4, US5591762), milfasartan, abitesartan, valsartan (Diovan® (Novartis), (S)-N-valeryl-N-[[2'-(1H-tetrazol-5-yl)biphenyl-4-yl)methyl]valine, US5399578), EXP-3137 (2-N-butyl-4-chloro-1-[(2'-(1H-tetrazol-5-yl)biphenyl-4-yl)-methyl]imidazole-5-carboxylic acid, US5138069, US5153197 and US5128355), 3-(2'-(tetrazol-5-yl)-1,1'-biphen-4-yl)methyl-5,7-dimethyl-2-ethyl-3H-imidazo[4,5-b]pyridine, 4'[2-ethyl-4-methyl-6-(5,6,7,8-tetrahydroimidazo[1,2-a]pyridin-2-yl]-benzimidazol-1-yl]-methyl]-1,1'-biphenyl]-2- carboxylic acid, 2-butyl-6-(1-methoxy-1-methylethyl)-2-[2'-)IH-tetrazol-5-yl)biphenyl-4-ylmethyl]guinazolin-4(3H)-one, 3-[2'-carboxybiphenyl-4-yl)methyl]-2-cyclopropyl-7-methyl- 3H-imidazo[4,5-b]pyridine, 2-butyl-4-chloro-1-[(2'-tetrazol-5-yl)biphenyl-4-yl)methyl]imidazole-carboxylic acid, 2-butyl-4-chloro-1-[[2'-(1H-tetrazol-5-yl)[1,1'-biphenyl]-4-yl]methyl]-1H-imidazole-5-carboxylic acid-1-(ethoxycarbonyl-oxy)ethyl ester potassium salt, dipotassium 2-butyl-4-(methylthio)-1-[[2-[[[(propylamino)carbonyl]amino]-sulfonyl](1,1'-biphenyl)-4-yl]methyl]-1H-imidazole-5-carboxylate, methyl-2-[[4-butyl-2-methyl-6-oxo-5-[[2'-(1H-tetrazol-5-yl)-[1,1'-biphenyl]-4-yl]methyl]-1-(6H)-pyrimidinyl]methyl]-3-thiophencarboxylate, 5-[(3,5-dibutyl-1H-1,2,4-triazol-1-yl)methyl]-2-[2-(1H-tetrazol-5-ylphenyl)]pyridine, 6-butyl-2-(2-phenylethyl)-5[[2'-(1H-tetrazol-5-yl)[1,1'-biphenyl]-4-methyl]pyrimidin-4-(3H)-one D,L lysine salt, 5-methyl-7-n-propyl-8-[[2'-(1H-tetrazol-5-yl)biphenyl-4-yl]methyl]-[1,2,4]-triazolo[1,5-c]pyrimidin-2(3H)-one, 2,7-diethyl-5-[[2'-(5-tetrazoly)biphenyl-4-yl]methyl]-5H-pyrazolo[1,5-b][1,2,4]triazole potassium salt, 2-[2-butyl-4,5-dihydro-4-oxo-3-[2'-(1H-tetrazol-5-yl)-4-biphenylmethyl]-3H-imidazol[4,5-c]pyridine-5-ylmethyl]benzoic acid, ethyl ester, potassium salt, 3-methoxy-2,6-dimethyl-4-[[2'(1H-tetrazol-5-yl)-1,1'-biphenyl-4-yl]methoxy]pyridine, 2-ethoxy-1-[[2'-(5-oxo-2,5-dihydro-1,2,4-oxadiazol-3-yl)biphenyl-4-yl]methyl]-1H-benzimidazole-7-carboxylic acid, 1-[N-(2'-(1H-tetrazol-5-yl)biphenyl-4-yl-methyl)-N-valerolylaminomethyl)cyclopentane-1-carboxylic acid, 7-methyl-2n-propyl-3-[[2'1H-tetrazol-5-yl)biphenyl-4-yl]methyl]-3H-imidazo[4,5-6]pyridine, 2-[5-[(2-ethyl-5,7-dimethyl-3H-imidazo[4,5-b]pyridine-3-yl)methyl]-2-quinolinyl]sodium benzoate, 2-butyl-6-chloro-4-hydroxymethyl-5-methyl-3-[[2'-(1H-tetrazol-5-yl)biphenyl-4-yl]methyl]pyridine, 2-[[[2-butyl-1-[(4-carboxyphenyl)methyl]-1H-imidazol-5-yl]methyl]amino]benzoic acid tetrazol-5-yl)biphenyl-4-yl]methyl]pyrimidin-6-one, 4(S)-[4-(carboxymethyl)phenoxy]-N-[2(R)-[4-(2-sulfobenzamido)imidazol-1-yl]octanoyl]-L-proline, 1-(2,6-dimethylphenyl)-4-butyl-1,3-dihydro-3-[[6-[2-(1H-tetrazol-5-yl)phenyl]-3-pyridinyl]methyl]-2H-imidazol-2-one, 5,8-ethano-5,8-dimethyl-2-n-propyl-5,6,7,8-tetrahydro-1-[[2'(1 H-tetrazol-5-yl)biphenyl-4-yl]methyl]-1H,4H-1,3,4a,8a-tetrazacyclopentanaphthalene-9-one, 4-[1-[2'-(1,2,3,4-tetrazol-5-yl)biphen-4-yl)methylamino]-5,6,7,8-tetrahydro-2-trifylquinazoline, 2-(2-chlorobenzoyl)imino-5-ethyl-3-[2'-(1H-tetrazole-5-yl)biphenyl-4-yl)methyl-1,3,4-thiadiazoline, 2-[5-ethyl-3-[2-(1H-tetrazole-5-yl)biphenyl-4-yl]methyl-1,3,4-thiazoline-2-ylidene]aminocarbonyl-1-cyclopentencarboxylic acid dipotassium salt, and 2-butyl-4-[N-methyl-N-(3-methylcrotonoyl)amino]-1-[[2'-(1H-tetrazol-5-yl)biphenyl-4-yl]methyl]-1H-imidzole-5-carboxylic acid 1-ethoxycarbonyloxyethyl ester, those disclosed in patent publications EP475206, EP497150, EP539086, EP539713, EP535463, EP535465, EP542059, EP497121, EP535420, EP407342, EP415886, EP424317, EP435827, EP433983, EP475898, EP490820, EP528762, EP324377, EP323841, EP420237, EP500297, EP426021, EP480204, EP429257, EP430709, EP434249, EP446062, EP505954, EP524217, EP514197, EP514198, EP514193, EP514192, EP450566, EP468372, EP485929, EP503162, EP533058, EP467207 EP399731, EP399732, EP412848, EP453210, EP456442, EP470794, EP470795, EP495626, EP495627, EP499414, EP499416, EP499415, EP511791, EP516392, EP520723, EP520724, EP539066, EP438869, EP505893, EP530702, EP400835, EP400974, EP401030, EP407102, EP411766, EP409332, EP412594, EP419048, EP480659, EP481614, EP490587, EP467715, EP479479, EP502725, EP503838, EP505098, EP505111 EP513,979 EP507594, EP510812, EP511767, EP512675, EP512676, EP512870, EP517357, EP537937, EP534706, EP527534, EP540356, EP461040, EP540039, EP465368, EP498723, EP498722, EP498721, EP515265, EP503785, EP501892, EP519831, EP532410, EP498361, EP432737, EP504888, EP508393, EP508445, EP403159, EP403158, EP425211, EP427463, EP437103, EP481448, EP488532, EP501269, EP500409, EP540400, EP005528, EP028834, EP028833, EP411507, EP425921, EP430300, EP434038, EP442473, EP443568, EP445811, EP459136, EP483683, EP518033, EP520423, EP531876, EP531874, EP392317, EP468470, EP470543, EP502314, EP529253, EP543263, EP540209, EP449699, EP465323, EP521768, EP415594, WO92/14468, WO93/08171, WO93/08169, WO91/00277, WO91/00281, WO91/14367, WO92/00067, WO92/00977, WO92/20342, WO93/04045, WO93/04046, WO91/15206, WO92/14714, WO92/09600, WO92/16552, WO93/05025, WO93/03018, WO91/07404, WO92/02508, WO92/13853, WO91/19697, WO91/11909, WO91/12001, WO91/11999, WO91/15209, WO91/15479, WO92/20687, WO92/20662, WO92/20661, WO93/01177, WO91/14679, WO91/13063, WO92/13564, WO91/17148, WO91/18888, WO91/19715, WO92/02257, WO92/04335, WO92/05161, WO92/07852, WO92/15577, WO93/03033, WO91/16313, WO92/00068, WO92/02510, WO92/09278, WO9210179, WO92/10180, WO92/10186, WO92/10181, WO92/10097, WO92/10183, WO92/10182, WO92/10187, WO92/10184, WO92/10188, WO92/10180, WO92/10185, WO92/20651, WO93/03722, WO93/06828, WO93/03040, WO92/19211 WO92/22533, WO92/06081, WO92/05784, WO93/00341, WO92/04343, WO92/04059, US5104877, US5187168, US5149699, US5185340, US4880804, US5138069, US4916129, US5153197, US5173494, US5137906, US5155126, US5140037, US5137902, US5157026, US5053329, US5132216, US5057522, US5066586, US5089626, US5049565, US5087702, US5124335, US5102880, US5128327, US5151435, US5202322, US5187159, US5198438, US5182288, US5036048, US5140036, US5087634, US5196537, US5153347, US5191086, US5190942, US5177097, US5212177, US5208234, US5208235, US5212195, US5130439, US5045540, US5041152, and US5210204, and pharmaceutically acceptable salts and esters thereof;
α/β adrenergic blockers such as nipradilol, arotinolol, amosulalol, bretylium tosylate (CAS RN: 61-75-6), dihydroergtamine mesylate (such as ergotaman-3', 6',18-trione,9,-10-dihydro-12'-hydroxy-2'-methyl-5'-(phenylmethyl)-,(5'(α))-, monomethanesulfonate, e.g., DHE 45® Injection, Novartis), carvedilol (such as (±)-1-(Carbazol-4-yloxy)-3-[[2-(o-methoxyphenoxy)ethyl]amino]-2-propanol, e.g., Coreg®, SmithKline Beecham), labetalol (such as 5-[1-hydroxy-2-[(1-methyl-3-phenylpropyl) amino] ethyl]salicylamide monohydrochloride, e.g., Normodyne®, Schering), bretylium tosylate (Benzenemethanaminium, 2-bromo-N-ethyl-N,N-dimethyl-, salt with 4-methylbenzenesulfonic acid (1:1) CAS RN 61-75-6), phentolamine mesylate (Phenol, 3-[[(4,5-dihydro-1H-imidazol-2-yl)methyl](4-methylphenyl)amino]-, monomethanesulfonate (salt) CAS RN 65-28-1), solypertine tartrate (5H-1,3-Dioxolo[4,5-f]indole, 7-[2-[4-(2-methoxyphenyl)-1-piperazinyl]ethyl]-, (2R,3R)-2,3-dihydroxybutanedioate (1:1) CAS RN 5591-43-5), zolertine hydrochloride (Piperazine, 1-phenyl4-[2-(1H-tetrazol-5-yl)ethyl]-, monohydrochloride (8Cl, 9Cl) CAS RN 7241-94-3) and the like;
α adrenergic receptor blockers, such as alfuzosin (CAS RN: 81403-68-1), terazosin, urapidil, prazosin (Minipress®), tamsulosin, bunazosin, trimazosin, doxazosin, naftopidil, indoramin, WHP 164, XEN010, fenspiride hydrochloride (which may be prepared as disclosed in US3399192), proroxan (CAS RN 33743-96-3), and labetalol hydrochloride and combinations thereof;
α 2 agonists such as methyldopa, methyldopa HCL, lofexidine, tiamenidine, moxonidine, rilmenidine, guanobenz, and the like;
aldosterone inhibitors, and the like;
angiopoietin-2-binding agents such as those disclosed in WO03/030833;
anti-angina agents such as ranolazine (hydrochloride 1-Piperazineacetamide, N-(2,6-dimethylphenyl)-4-[2-hydroxy-3-(2-methoxyphenoxy)propyl]-, dihydrochloride CAS RN 95635-56-6), betaxolol hydrochloride (2-Propanol, 1-[4-[2 (cyclopropylmethoxy)ethyl]phenoxy]-3-[(1-methylethyl)amino]-, hydrochloride CAS RN 63659-19-8), butoprozine hydrochloride (Methanone, [4-[3(dibutylamino)propoxy]phenyl](2-ethyl-3-indolizinyl)-, monohydrochloride CAS RN 62134-34-3), cinepazetmaleate1-Piperazineacetic acid, 4-[1-oxo-3-(3,4,5-trimethoxyphenyl)-2-propenyl]-, ethyl ester, (2Z)-2-butenedioate (1:1) CAS RN 50679-07-7), tosifen (Benzenesulfonamide, 4-methyl-N-[[[(1S)-1-methyl-2-phenylethyl]amino]carbonyl]-CAS RN 32295-184), verapamilhydrochloride (Benzeneacetonitrile, α-[3-[[2-(3,4-dimethoxyphenyl)ethyl]methylamino]propyl]-3,4-dimethoxy-α-(1-methylethyl)-, monohydrochloride CAS RN 152-114), molsidomine (1,2,3-Oxadiazolium, 5-[(ethoxycarbonyl)amino]-3-(4-morpholinyl)-, inner salt CAS RN 25717-80-0), and ranolazine hydrochloride (1-Piperazineacetamide, N-(2,6-dimethylphenyl)₄-[2-hydroxy-3-(2-meth-oxyphenoxy)propyl]-, dihydrochloride CAS RN 95635-56-6); tosifen (Benzenesulfonamide, 4-methyl-N-[[[(1S)-1-methyl-2-phenylethyl]amino]carbonyl]- CAS RN 32295-184); and
adrenergic stimulants such as guanfacine hydrochloride (such as N-amidino-2-(2,6-dichlorophenyl) acetamide hydrochloride, e.g., Tenex® Tablets available from Robins); methyldopa-hydrochlorothiazide (such as levo-3-(3,4-dihydroxyphenyl)-2-methylalanine) combined with Hydrochlorothiazide (such as 6-chloro-3,4-dihydro-2H-1,2,4-benzothiadiazine-7- sulfonamide 1,1-dioxide, e.g., the combination as, e.g., Aldoril® Tablets available from Merck), methyldopa-chlorothiazide (such as 6-chloro-2H-1, 2,4-benzothiadiazine-7-sulfonamide 1,1-dioxide and methyldopa as described above, e.g., Aldoclor®, Merck), clonidine hydrochloride (such as 2-(2,6-dichlorophenylamino)-2-imidazoline hydrochloride and chlorthalidone (such as 2-chloro-5-(1-hydroxy-3-oxo-1-isoindolinyl) benzenesulfonamide), e.g., Combipres®, Boehringer Ingelheim), clonidine hydrochloride (such as 2-(2,6-dichlorophenylamino)-2-imidazoline hydrochloride, e.g., Catapres®, Boehringer Ingelheim), clonidine (1H-Imidazol-2-amine, N-(2,6-dichlorophenyl)4,5-dihydro-CAS RN 4205-90-7); and those agents disclosed in US20030069221.

### Agents useful in the treatment of obesity

The agents described herein can be administered together with one or more agents useful in the treatment of obesity. Suitable anti-obesity agents include, but are not limited to:
11β HSD-1 (11-beta hydroxy steroid dehydrogenase type 1) inhibitors, such as BVT 3498, BVT 2733, 3-(1-adamantyl)-4-ethyl-5-(ethylthio)- 4H-1,2,4-triazole, 3-(1-adamantyl)-5-(3,4,5-trimethoxyphenyl)-4-methyl-4H-1,2,4-triazole, 3- adamantanyl-4,5,6,7,8,9,10,11,12,3a-decahydro-1,2,4-triazolo[4,3-a][11]annulene, and those compounds disclosed in WO01/90091, WO01/90090, WO01/90092 and WO02/072084;
5HT antagonists such as those in WO03/037871, WO03/037887, and the like;
5HT1a modulators such as carbidopa, benserazide and those disclosed in US6207699, WO03/031439, and the like;
5HT2c (serotonin receptor 2c) agonists, such as BVT933, DPCA37215, IK264, PNU 22394, WAY161503, R-1065, SB 243213 (Glaxo Smith Kline) and YM 348 and those disclosed in US3914250, WO00/77010, WO02/36596, WO02/48124, WO02/10169WO01/66548, WO02/44152, WO02/51844, WO02/40456, and WO02/40457;
5HT6 receptor modulators, such as those in WO03/030901, WO03/035061, WO03/039547, and the like;
acyl-estrogens, such as oleoyl-estrone, disclosed in del Mar-Grasa, M. et al., Obesity Research, 9:202-9 (2001) and Japanese Patent Application No. JP 2000256190;
anorectic bicyclic compounds such as 1426 (Aventis) and 1954 (Aventis), and the compounds disclosed in WO00/18749, WO01/32638, WO01/62746, WO01/62747, and WO03/015769;
CB 1 (cannabinoid-1 receptor) antagonist/inverse agonists such as rimonabant (Acomplia; Sanofi), SR-147778 (Sanofi), SR-141716 (Sanofi), BAY 65-2520 (Bayer), and SLV 319 (Solvay), and those disclosed in patent publications US4973587, US5013837, US5081122, US5112820, US5292736, US5532237, US5624941, US6028084, US6509367, US6509367, WO96/33159, WO97/29079, WO98/31227, WO98/33765, WO98/37061, WO98/41519, WO98/43635, WO98/43636, WO99/02499, WO00/10967, WO00/10968, WO01/09120, WO01/58869, WO01/64632, WO01/64633, WO01/64634, WO01/70700, WO01/96330, WO02/076949, WO03/006007, WO03/007887, WO03/020217, WO03/026647, WO03/026648, WO03/027069, WO03/027076, WO03/027114, WO03/037332, WO03/040107, WO03/086940, WO03/084943 and EP658546;
CCK-A (cholecystokinin-A) agonists, such as AR-R 15849, GI 181771 (GSK), JMV-180, A-71378, A-71623 and SR146131 (Sanofi), and those described in US5739106;
CNTF (Ciliary neurotrophic factors), such as GI-181771 (Glaxo-SmithKline), SR146131 (Sanofi Synthelabo), butabindide, PD170,292, and PD 149164 (Pfizer);
CNTF derivatives, such as Axokine® (Regeneron), and those disclosed in WO94/09134, WO98/22128, and WO99/43813;
dipeptidyl peptidase IV (DP-IV) inhibitors, such as isoleucine thiazolidide, valine pyrrolidide, NVP-DPP728, LAF237, P93/01, P 3298, TSL 225 (tryptophyl-1,2,3,4-tetrahydroisoquinoline-3-carboxylic acid; disclosed by Yamada et al, Bioorg. & Med. Chem. Lett. 8 (1998) 1537-1540), TMC-2A/2B/2C, CD26 inhibtors, FE 999011, P9310/K364, VIP 0177, SDZ 274-444, 2-cyanopyrrolidides and 4-cyanopyrrolidides as disclosed by Ashworth et al, Bioorg. & Med. Chem. Lett., Vol. 6, No. 22, pp 1163-1166 and 2745-2748 (1996) and the compounds disclosed patent publications. WO99/38501, WO99/46272, WO99/67279 (Probiodrug), WO99/67278 (Probiodrug), WO99/61431 (Probiodrug), WO02/083128, WO02/062764, WO03/000180, WO03/00018 WO03/000250, WO03/002530, WO03/002531, WO03/002553, WO03/002593, WO03/004498, WO03/004496,WO03/017936, WO03/024942, WO03/024965, WO03/033524, WO03/037327 and EP1258476;
growth hormone secretagogue receptor agonists/antagonists, such as NN703, hexarelin, MK-0677 (Merck), SM-130686, CP-424391 (Pfizer), LY 444,711 (Eli Lilly), L-692,429 and L-163,255, and such as those disclosed in USSN 09/662448, US provisional application 60/203335, US6358951, US2002049196, US2002/022637, WO01/56592 and WO02/32888;
H3 (histamine H3) antagonist/inverse agonists, such as thioperamide, 3-(1H-imidazol-4-yl)propyl N-(4-pentenyl)carbamate), clobenpropit, iodophenpropit, imoproxifan, GT2394 (Gliatech), and A331440, O-[3-(1H-imidazol-4-yl)propanol]carbamates (Kiec-Kononowicz, K. et al., Pharmazie, 55:349-55 (2000)), piperidine-containing histamine H3-receptor antagonists (Lazewska, D. et al., Pharmazie, 56:927-32 (2001), benzophenone derivatives and related compounds (Sasse, A. et al., Arch. Pharm.(Weinheim) 334:45-52 (2001)), substituted N-phenylcarbamates (Reidemeister, S. et al., Pharmazie, 55:83-6 (2000)), and proxifan derivatives (Sasse, A. et al., J. Med. Chem.. 43:3335-43 (2000)) and histamine H3 receptor modulators such as those disclosed in WO02/15905, WO03/024928 and WO03/024929;
leptin derivatives, such as those disclosed in US5552524, US5552523, US5552522, US5521283, WO96/23513, WO96/23514, WO96/23515, WO96/23516, WO96/23517, WO96/23518, WO96/23519, and WO96/23520;
leptin, including recombinant human leptin (PEG-OB, Hoffman La Roche) and recombinant methionyl human leptin (Amgen);
lipase inhibitors, such as tetrahydrolipstatin (orlistat/Xenical®), Triton WR1339, RHC80267, lipstatin, teasaponin, diethylumbelliferyl phosphate, FL-386, WAY-121898, Bay-N-3176, valilactone, esteracin, ebelactone A, ebelactone B, and RHC 80267, and those disclosed in patent publications WO01/77094, US4598089, US4452813, USUS5512565, US5391571, US5602151, US4405644, US4189438, and US4242453;
lipid metabolism modulators such as maslinic acid, erythrodiol, ursolic acid uvaol, betulinic acid, betulin, and the like and compounds disclosed in WO03/011267;
Mc4r (melanocortin 4 receptor) agonists, such as CHIR86036 (Chiron), ME-10142, ME-10145, and HS-131 (Melacure), and those disclosed in PCT publication Nos. WO99/64002, WO00/74679, WO01/991752, WO01/25192, WO01/52880, WO01/74844, WO01/70708, WO01/70337, WO01/91752, WO02/059095, WO02/059107, WO02/059108, WO02/059117, WO02/06276, WO02/12166, WO02/11715, WO02/12178, WO02/15909, WO02/38544, WO02/068387, WO02/068388, WO02/067869, WO02/081430, WO03/06604, WO03/007949, WO03/009847, WO03/009850, WO03/013509, and WO03/031410;
Mc5r (melanocortin 5 receptor) modulators, such as those disclosed in WO97/19952, WO00/15826, WO00/15790, US20030092041;
melanin-concentrating hormone 1 receptor (MCHR) antagonists, such as T-226296 (Takeda), SB 568849, SNP-7941 (Synaptic), and those disclosed in patent publications WO01/21169, WO01/82925, WO01/87834, WO02/051809, WO02/06245, WO02/076929, WO02/076947, WO02/04433, WO02/51809, WO02/083134, WO02/094799, WO03/004027, WO03/13574, WO03/15769, WO03/028641, WO03/035624, WO03/033476, WO03/033480, JP13226269, and JP1437059;
mGluR5 modulators such as those disclosed in WO03/029210, WO03/047581, WO03/048137, WO03/051315, WO03/051833, WO03/053922, WO03/059904, and the like;
serotoninergic agents, such as fenfluramine (such as Pondimin® (Benzeneethanamine, N-ethyl-alpha-methyl-3-(trifluoromethyl)-, hydrochloride), Robbins), dexfenfluramine (such as Redux® (Benzeneethanamine, N-ethyl-alpha-methyl-3-(trifluoromethyl)-, hydrochloride), Interneuron) and sibutramine ((Meridia®, Knoll/ReductilTM) including racemic mixtures, as optically pure isomers (+) and (-), and pharmaceutically acceptable salts, solvents, hydrates, clathrates and prodrugs thereof including sibutramine hydrochloride monohydrate salts thereof, and those compounds disclosed in US4746680, US4806570, and US5436272, US20020006964, WO01/27068, and WO01/62341;
NE (norepinephrine) transport inhibitors, such as GW 320659, despiramine, talsupram, and nomifensine;
NPY 1 antagonists, such as BIBP3226, J-115814, BIBO 3304, LY-357897, CP-671906, GI-264879A, and those disclosed in US6001836, WO96/14307, WO01/23387, WO99/51600, WO01/85690, WO01/85098, WO01/85173, and WO01/89528;
NPY5 (neuropeptide Y Y5) antagonists, such as 152,804, GW-569180A, GW-594884A, GW-587081X, GW-548118X, FR235208, FR226928, FR240662, FR252384, 1229U91, GI-264879A, CGP71683A, LY-377897, LY-366377, PD-160170, SR- 120562A, SR-120819A, JCF-104, and H409/22 and those compounds disclosed in patent publications US6140354, US6191160, US6218408, US6258837, US6313298, US6326375, US6329395, US6335345, US6337332, US6329395, US6340683, EP01010691, EP-01044970, WO97/19682, WO97/20820, WO97/20821, WO97/20822, WO97/20823, WO98/27063, WO00/107409, WO00/185714, WO00/185730, WO0O/6488 WO00/68197, WO0O/6984 WO/0113917, WO01/09120, WO01/14376, WO01/85714, WO01/85730, WO01/07409, WO01/02379, WO01/23388, WO01/23389, WO01/44201, WO01/62737, WO01/62738, WO01/09120, WO02/20488, WO02/22592, WO02/48152, WO02/49648, WO02/051806, WO02/094789, WO03/009845, WO03/014083, WO03/022849, WO03/028726 and Norman et al., J. Med. Chem. 43:4288-4312 (2000);
opioid antagonists, such as nalmefene (REVEX ®), 3-methoxynaltrexone, methylnaltrexone, naloxone, and naltrexone (e.g. PT901; Pain Therapeutics, Inc.) and those disclosed in US6734188, US20050004155 and WO00/21509;
orexin antagonists, such as SB-334867-A and those disclosed in patent publications WO01/96302, WO01/68609, WO02/44172, WO02/51232, WO02/51838, WO02/089800, WO02/090355, WO03/023561, WO03/032991, and WO03/037847;
Neuropeptide Y2 (NPY2) agonists include but are not limited to: peptide YY and fragments and variants thereof (e.g. YY3-36 (PYY3-36 )(N. Engl. J. Med. 349:941, 2003; IKPEAPGE DASPEELNRY YASLRHYLNL VTRQRY (SEQ ID NO:XXX)) and PYY agonists such as those disclosed in WO02/47712, WO03/026591, WO03/057235, and WO03/027637;
serotonin reuptake inhibitors, such as, paroxetine, fluoxetine (ProzacTM), fluvoxamine, sertraline, citalopram, and imipramine, and those disclosed in US6162805, US6365633, WO03/00663, WO01/27060, and WO01/162341;
thyroid hormone β agonists, such as KB-2611 (KaroBioBMS), and those disclosed in WO02/15845, WO97/21993, WO99/00353, GB98/284425, U.S. Provisional Application No. 60/183,223, and Japanese Patent Application No. JP 2000256190;
UCP-1 (uncoupling protein-1), 2, or 3 activators, such as phytanic acid, 4-[(E)-2-(5,6,7,8-tetrahydro-5,5,8,8-tetramethyl-2-napthalenyl)-1-propenyl]benzoic acid (TTNPB), retinoic acid, and those disclosed in WO99/00123;
β3 (beta adrenergic receptor 3) agonists, such as AJ9677/TAK677 (Dainippon/Takeda), L750355 (Merck), CP331648 (Pfizer), CL-316,243, SB 418790, BRL-37344, L-796568, BMS-196085, BRL-35135A, CGP12177A, BTA-243, GW 427353, Trecadrine, Zeneca D7114, N-5984 (Nisshin Kyorin), LY-377604 (Lilly), SR 59119A, and those disclosed in US5541204, US5770615, US5491134, US5776983, US488064, US5705515, US5451677, WO94/18161, WO95/29159, WO97/46556, WO98/04526 and WO98/32753, WO01/74782, WO02/32897, WO03/014113, WO03/016276, WO03/016307, WO03/024948, WO03/024953 and WO03/037881;
noradrenergic agents including, but not limited to, diethylpropion (such as Tenuate® (1-propanone, 2-(diethylamino)-1-phenyl-, hydrochloride), Merrell), dextroamphetamine (also known as dextroamphetamine sulfate, dexamphetamine, dexedrine, Dexampex, Ferndex, Oxydess II, Robese, Spancap #1), mazindol ((or 5-(p-chlorophenyl)-2,5-dihydro-3H-imidazo[2,1-a]isoindol-5-ol) such as Sanorex®, Novartis or Mazanor®, Wyeth Ayerst), phenylpropanolamine (or Benzenemethanol, alpha-(1-aminoethyl)-, hydrochloride), phentermine ((or Phenol, 3-[[4,5-duhydro-1H-imidazol-2-yl)ethyl](4-methylpheny-1)amino], monohydrochloride) such as Adipex-P®, Lemmon, FASTIN®, Smith-Kline Beecham and Ionamin®, Medeva), phendimetrazine ((or (2S,3S)-3,4-Dimethyl-2phenylmorpholine L-(+)-tartrate (1:1)) such as Metra® (Forest), Plegine® (Wyeth-Ayerst), Prelu-2® (Boehringer Ingelheim), and Statobex® (Lemmon), phendamine tartrate (such as Thephorin® (2,3,4,9-Tetrahydro-2-methyl-9-phenyl-1H-indenol[2,1-c]pyridine L-(+)-tartrate (1:1)), Hoffmann-LaRoche), methamphetamine (such as Desoxyn®, Abbot ((S)--N, (alpha)-dimethylbenzeneethanamine hydrochloride)), and phendimetrazine tartrate (such as Bontril® Slow-Release Capsules, Amarin (-3,4-Dimethyl-2-phenylmorpholine Tartrate);
fatty acid oxidation upregulator/inducers such as Famoxin® (Genset);
monamine oxidase inhibitors including but not limited to befloxatone, moclobemide, brofaromine, phenoxathine, esuprone, befol, toloxatone, pirlindol, amiflamine, sercloremine, bazinaprine, lazabemide, milacemide, caroxazone and other certain compounds as disclosed by WO01/12176; and
other anti-obesity agents such as 5HT-2 agonists, ACC (acetyl-CoA carboxylase) inhibitors such as those described in WO03/072197, alpha-lipoic acid (alpha-LA), AOD9604, appetite suppressants such as those in WO03/40107, ATL-962 (Alizyme PLC), benzocaine, benzphetamine hydrochloride (Didrex), bladderwrack (focus vesiculosus), BRS3 (bombesin receptor subtype 3) agonists, bupropion, caffeine, CCK agonists, chitosan, chromium, conjugated linoleic acid, corticotropin-releasing hormone agonists, dehydroepiandrosterone, DGAT1 (diacylglycerol acyltransferase 1) inhibitors, DGAT2 (diacylglycerol acyltransferase 2) inhibitors, dicarboxylate transporter inhibitors, ephedra, exendin-4 (an inhibitor of glp-1) FAS (fatty acid synthase) inhibitors (such as Cerulenin and C75), fat resorption inhibitors (such as those in WO03/053451, and the like), fatty acid transporter inhibitors, natural water soluble fibers (such as psyllium, plantago, guar, oat, pectin), galanin antagonists, galega (Goat's Rue, French Lilac), garcinia cambogia, germander (teucrium chamaedrys), ghrelin antibodies and ghrelin antagonists (such as those disclosed in WO01/87335, and WO02/08250), peptide hormones and variants thereof which affect the islet cell secretion, such as the hormones of the secretin/gastric inhibitory peptide (GIP)/vasoactive intestinal peptide (VIP)/pituitary adenylate cyclase activating peptide (PACAP)/glucagon-like peptide II (GLP-II)/glicentin/glucagon gene family and/or those of the adrenomedullin/amylin/calcitonin gene related peptide (CGRP) gene family includingGLP-1 (glucagon-like peptide 1) agonists (e.g. (1) exendin-4, (2) those GLP-1 molecules described in US20050130891 including GLP-1(7-34), GLP-1(7-35), GLP-1(7-36) or GLP-1(7-37) in its C-terminally carboxylated or amidated form or as modified GLP-1 peptides and modifications thereof including those described in paragraphs 17-44 of US20050130891,and derivatives derived from GLP-1-(7-34)COOH and the corresponding acid amide are employed which have the following general formula:

   R-NH-HAEGTFTSDVSYLEGQAAKEFIAWLVK-CONH2
wherein R=H or an organic compound having from 1 to 10 carbon atoms. Preferably, R is the residue of a carboxylic acid. Particularly preferred are the following carboxylic acid residues: formyl, acetyl, propionyl, isopropionyl, methyl, ethyl, propyl, isopropyl, n-butyl, sec-butyl, tert-butyl.) and glp-1 (glucagon-like peptide-1), glucocorticoid antagonists, glucose transporter inhibitors, growth hormone secretagogues (such as those disclosed and specifically described in US5536716), interleukin-6 (IL-6) and modulators thereof (as in WO03/057237, and the like), L-carnitine, Mc3r (melanocortin 3 receptor) agonists, MCH2R (melanin concentrating hormone 2R) agonist/antagonists, melanin concentrating hormone antagonists, melanocortin agonists (such as Melanotan II or those described in WO 99/64002 and WO 00/74679), nomame herba, phosphate transporter inhibitors, phytopharm compound 57 (CP 644,673), pyruvate, SCD-1 (stearoyl-CoA desaturase-1) inhibitors, T71 (Tularik, Inc., Boulder CO), Topiramate (Topimax®, indicated as an anti-convulsant which has been shown to increase weight loss), transcription factor modulators (such as those disclosed in WO03/026576), β-hydroxy steroid dehydrogenase-1 inhibitors (β -HSD-1), β-hydroxy-β-methylbutyrate, p57 (Pfizer), Zonisamide (ZonegranTM, indicated as an anti-epileptic which has been shown to lead to weight loss), and the agents disclosed in US20030119428 paragraphs 20-26.

### Anti-diabetic Agents

The agents described herein can be administered together with one or more agents useful in the treatment of diabetes. Suitable anti-diabetic agents include, but are not limited to:
PPARγ agonists such as glitazones (e.g., WAY-120,744, AD 5075, balaglitazone, ciglitazone, darglitazone (CP-86325, Pfizer), englitazone (CP-68722, Pfizer), isaglitazone (MIT/J&J), MCC-555 (Mitsibishi disclosed in US5594016), pioglitazone (such as such as Actos™ pioglitazone; Takeda), rosiglitazone (Avandia™;Smith Kline Beecham), rosiglitazone maleate, troglitazone (Rezulin, disclosed in US4572912), rivoglitazone (CS-011, Sankyo), GL-262570 (Glaxo Welcome), BRL49653 (disclosed in WO98/05331), CLX-0921, 5-BTZD, GW-0207, LG-100641, JJT-501 (JPNT/P&U), L-895645 (Merck), R-119702 (Sankyo/Pfizer), NN-2344 (Dr. Reddy/NN), YM-440 (Yamanouchi), LY-300512, LY-519818, R483 (Roche), T131 (Tularik), and the like and compounds disclosed in US4687777, US5002953, US5741803, US5965584, US6150383, US6150384, US6166042, US6166043, US6172090, US6211205, US6271243, US6288095, US6303640, US6329404, US5994554, W097/10813, WO97/27857,WO97/28115,WO97/28137,WO97/27847, WO00/76488, WO03/000685,WO03/027112,WO03/035602, WO03/048130,WO03/055867, and pharmaceutically acceptable salts thereof;
biguanides such as metformin hydrochloride (N,N-dimethylimidodicarbonimidic diamide hydrochloride, such as Glucophage™, Bristol-Myers Squibb); metformin hydrochloride with glyburide, such as Glucovance™, Bristol-Myers Squibb); buformin (Imidodicarbonimidic diamide, N-butyl-); etoformine (1-Butyl-2-ethylbiguanide, Schering A. G.); other metformin salt forms (including where the salt is chosen from the group of, acetate, benzoate, citrate, ftimarate, embonate, chlorophenoxyacetate, glycolate, palmoate, aspartate, methanesulphonate, maleate, parachlorophenoxyisobutyrate, formate, lactate, succinate, sulphate, tartrate, cyclohexanecarboxylate, hexanoate, octanoate, decanoate, hexadecanoate, octodecanoate, benzenesulphonate, trimethoxybenzoate, paratoluenesulphonate, adamantanecarboxylate, glycoxylate, glutamate, pyrrolidonecarboxylate, naphthalenesulphonate, 1-glucosephosphate, nitrate, sulphite, dithionate and phosphate), and phenformin;
protein tyrosine phosphatase-1B (PTP-1B) inhibitors, such as A-401,674, KR 61639, OC-060062, OC-83839, OC-297962, MC52445, MC52453, ISIS 113715, and those disclosed in WO99/585521, WO99/58518, WO99/58522, WO99/61435, WO03/032916, WO03/032982, WO03/041729, WO03/055883, WO02/26707, WO02/26743, JP2002114768, and pharmaceutically acceptable salts and esters thereof;
sulfonylureas such as acetohexamide (e.g. Dymelor, Eli Lilly), carbutamide, chlorpropamide (e.g. Diabinese, Pfizer), gliamilide (Pfizer), gliclazide (e.g. Diamcron, Servier Canada Inc), glimepiride (e.g. disclosed in US4379785, such as Amaryl™, Aventis), glipentide, glipizide (e.g. Glucotrol or Glucotrol XL Extended Release, Pfizer), gliquidone, glisolamide, glyburide/glibenclamide (e.g. Micronase or Glynase Prestab, Pharmacia & Upjohn and Diabeta, Aventis), tolazamide (e.g. Tolinase), and tolbutamide (e.g. Orinase), and pharmaceutically acceptable salts and esters thereof; meglitinides such as repaglinide (e.g. Pranidin , Novo Nordisk), KAD1229 (PF/Kissei), and nateglinide (e.g. Starlix , Novartis), and pharmaceutically acceptable salts and esters thereof;
α glucoside hydrolase inhibitors (or glucoside inhibitors) such as acarbose (e.g. Precose™, Bayer disclosed in US4904769), miglitol (such as GLYSET™, Pharmacia & Upjohn disclosed in US4639436), camiglibose (Methyl 6-deoxy-6-[(2R,3R,4R,5S)-3,4,5-trihydroxy-2-(hydroxymethyl)piperidino]-alpha-D-glucopyranoside, Marion Merrell Dow), voglibose (Takeda), adiposine, emiglitate, pradimicin-Q, salbostatin, CKD-711, MDL- 25,637, MDL-73,945, and MOR 14, and the compounds disclosed in US4062950, US4174439, US4254256, US4701559, US4639436, US5192772, US4634765, US5157116, US5504078, US5091418, US5217877, US51091 and WO01/47528 (polyamines);
α-amylase inhibitors such as tendamistat, trestatin, and A1-3688, and the compounds disclosed in US4451455, US4623714, and US4273765;
SGLT2 inhibtors including those disclosed in US6414126 and US6515117;
an aP2 inhibitor such as disclosed in US6548529;
insulin secreatagogues such as linogliride, A-4166, forskilin, dibutyrl cAMP, isobutylmethylxanthine (IBMX), and pharmaceutically acceptable salts and esters thereof;
fatty acid oxidation inhibitors, such as clomoxir, and etomoxir, and pharmaceutically acceptable salts and esters thereof;
A2 antagonists, such as midaglizole, isaglidole, deriglidole, idazoxan, earoxan, and fluparoxan, and pharmaceutically acceptable salts and esters thereof;
insulin and related compounds (e.g. insulin mimetics) such as biota, LP-100, novarapid, insulin detemir, insulin lispro, insulin glargine, insulin zinc suspension (lente and ultralente), Lys-Pro insulin, GLP-1 (1-36) amide, GLP-1 (73-7) (insulintropin, disclosed in US5614492), LY-315902 (Lilly), GLP-1 (7-36)-NH2), AL-401 (AutoImmune), certain compositions as disclosed in US4579730, US4849405, US4963526, US5642868, US5763396, US5824638, US5843866, US6153632, US6191105, and WO 85/05029, and primate, rodent, or rabbit insulin including biologically active variants thereof including allelic variants, more preferably human insulin available in recombinant form (sources of human insulin include pharmaceutically acceptable and sterile formulations such as those available from Eli Lilly (Indianapolis, Ind. 46285) as Humulin™ (human insulin rDNA origin), also see the THE PHYSICIAN'S DESK REFERENCE, 55.sup.th Ed. (2001) Medical Economics, Thomson Healthcare (disclosing other suitable human insulins);
non-thiazolidinediones such as JT-501 and farglitazar (GW-2570/GI- 262579), and pharmaceutically acceptable salts and esters thereof;
PPARα/γ dual agonists such as AR-H039242 (Aztrazeneca), GW-409544 (Glaxo-Wellcome), BVT-142, CLX-0940, GW-1536, GW-1929, GW-2433, KRP-297 (Kyorin Merck; 5-[(2,4-Dioxo thiazolidinyl)methyl] methoxy-N-[[4-(trifluoromethyl)phenyl] methyl]benzamide), L-796449, LR-90, MK-0767 (Merck/Kyorin/Banyu), SB 219994, muraglitazar (BMS), tesaglitzar (Astrazeneca), reglitazar (JTT-501) and those disclosed in WO99/16758, WO99/19313, WO99/20614, WO99/38850, WO00/23415, WO00/23417, WO00/23445, WO00/50414, WO01/00579, WO01/79150, WO02/062799, WO03/004458, WO03/016265, WO03/018010, WO03/033481, WO03/033450, WO03/033453, WO03/043985, WO 031053976, U.S. application Ser. No. 09/664,598, filed Sep. 18, 2000, Murakami et al. Diabetes 47, 1841-1847 (1998), and pharmaceutically acceptable salts and esters thereof;
other insulin sensitizing drugs;
VPAC2 receptor agonists;
GLK modulators, such as those disclosed in WO03/015774;
retinoid modulators such as those disclosed in WO03/000249;
GSK 3β/GSK 3 inhibitors such as 4-[2-(2-bromophenyl)-4-(4-fluorophenyl-1H-imidazol-5-yl]pyridine and those compounds disclosed in WO03/024447, WO03/037869, WO03/037877, WO03/037891, WO03/068773, EP1295884, EP1295885, and the like;
glycogen phosphorylase (HGLPa) inhibitors such as CP-368,296, CP-316,819, BAYR3401, and compounds disclosed in WO01/94300, WO02/20530, WO03/037864, and pharmaceutically acceptable salts or esters thereof;
ATP consumption promotors such as those disclosed in WO03/007990;
TRB3 inhibitors;
vanilloid receptor ligands such as those disclosed in WO03/049702;
hypoglycemic agents such as those disclosed in WO03/015781 and WO03/040114;
glycogen synthase kinase 3 inhibitors such as those disclosed in WO03/035663
agents such as those disclosed in WO99/51225, US20030134890, WO01/24786, and WO03/059870;
insulin-responsive DNA binding protein-1 (IRDBP-1) as disclosed in WO03/057827, and the like;
adenosine A2 antagonists such as those disclosed in WO03/035639, WO03/035640, and the like;
PPARδ agonists such as GW 501516, GW 590735, and compounds disclosed in JP10237049 and WO02/14291;
dipeptidyl peptidase IV (DP-IV) inhibitors, such as isoleucine thiazolidide, NVP-DPP728A (1-[[[2-[(5-cyanopyridin-2-yl)amino]ethyl]amino]acetyl]-2-cyano-(S)-pyrrolidine, disclosed by Hughes et al, Biochemistry, 38(36), 11597-11603, 1999), P32/98, NVP-LAF-237, P3298, TSL225 (tryptophyl-1,2,3,4-tetrahydro-isoquinoline-3-carboxylic acid, disclosed by Yamada et al, Bioorg. & Med. Chem. Lett. 8 (1998) 1537-1540), valine pyrrolidide, TMC-2A/2B/2C, CD-26 inhibitors, FE999011, P9310/K364, VIP 0177, DPP4, SDZ 274-444, 2-cyanopyrrolidides and 4-cyanopyrrolidides as disclosed by Ashworth et al, Bioorg. & Med. Chem. Lett., Vol. 6, No. 22, pp 1163-1166 and 2745-2748 (1996),and the compounds disclosed in US6395767, US6573287, US6395767 (compounds disclosed include BMS-477118, BMS-471211 and BMS 538,305), WO99/38501 WO99/46272, WO99/67279, WO99/67278, WO99/61431WO03/004498, WO03/004496, EP1258476, WO02/083128, WO02/062764, WO03/000250, WO03/002530, WO03/002531, WO03/002553, WO03/002593, WO03/000180, and WO03/000181;
GLP-1 agonists such as exendin-3 and exendin-4 (including the 39 aa peptide synthetic exendin-4 called Exenatide), and compounds disclosed in US2003087821 and NZ 504256, and pharmaceutically acceptable salts and esters thereof;
peptides including amlintide and Symlin (pramlintide acetate); and
glycokinase activators such as those disclosed in US2002103199 (fused heteroaromatic compounds) and WO02/48106 (isoindolin-1-one-substituted propionamide compounds).

### Administration of agents

For therapeutic and preventive treatment of disorders described herein, the agents described herein can be administered orally, e.g., as a tablet or cachet containing a predetermined amount of the active ingredient, pellet, gel, paste, syrup, bolus, electuary, slurry, sachet; capsule; powder; lyophilized powder; granules; as a solution or a suspension in an aqueous liquid or a non-aqueous liquid; as an oil-in-water liquid emulsion or a water-in-oil liquid emulsion, via a liposomal formulation (see, e.g., EP 736299) or in some other form. Orally administered compositions can include binders, lubricants, inert diluents, lubricating, surface active or dispersing agents, flavoring agents, and humectants. Orally administered formulations such as tablets may optionally be coated or scored and may be formulated so as to provide sustained, delayed or controlled release of the active ingredient therein. The agents can be co-administered with other agents used to treat gastrointestinal disorders including but not limited to the agents described herein. The agents can also be administered by rectal suppository. For the treatment of disorders outside the gastrointestinal tract such as congestive heart failure and benign prostatic hypertrophy, agents are preferably administered parenterally or orally.

The agent described herein can be administered alone or in combination with other agents. For example, the agents can be administered together with an analgesic agent. The analgesic agent can be covalently attached to an agent described herein or it can be a separate agent that is administered together with or sequentially with an agent described herein in a combination therapy.

Combination therapy can be achieved by administering two or more agents, e.g., an agent described herein and an analgesic agent or compound, each of which is formulated and administered separately, or by administering two or more agents in a single formulation. Other combinations are also encompassed by combination therapy. For example, two agents can be formulated together and administered in conjunction with a separate formulation containing a third agent. While the two or more agents in the combination therapy can be administered simultaneously, they need not be. For example, administration of a first agent (or combination of agents) can precede administration of a second agent (or combination of agents) by minutes, hours, days, or weeks. Thus, the two or more agents can be administered within minutes of each other or within 1, 2, 3, 6, 9, 12, 15, 18, or 24 hours of each other or within 1, 2, 3, 4, 5, 6, 7, 8, 9, 10, 12, 14 days of each other or within 2, 3, 4, 5, 6, 7, 8, 9, or 10 weeks of each other. In some cases even longer intervals are possible. While in many cases it is desirable that the two or more agents used in a combination therapy be present in within the patient's body at the same time, this need not be so.

Combination therapy can also include two or more administrations of one or more of the agents used in the combination. For example, if agent X and agent Y are used in a combination, one could administer them sequentially in any combination one or more times, e.g., in the order X-Y-X, X-X-Y, Y-X-Y, Y-Y-X, X-X-Y-Y, etc.

Combination therapy can also include the administration of two or more agents via different routes or locations. For example, (a) one agent is administered orally and another agent is administered intravenously or (b) one agent is administered orally and another is administered locally. In each case, the agents can either simultaneously or sequentially. Approximated dosages for some of the combination therapy agents described herein are found in the "BNF Recommended Dose" column of tables on pages 11-17 of WO01/76632 (the data in the tables being attributed to the March 2000 British National Formulary) and can also be found in other standard formularies and other drug prescribing directories. For some drugs, the customary presecribed dose for an indication will vary somewhat from country to country.

The agents, alone or in combination, can be combined with any pharmaceutically acceptable carrier or medium. Thus, they can be combined with materials that do not produce an adverse, allergic or otherwise unwanted reaction when administered to a patient. The carriers or mediums used can include solvents, dispersants, coatings, absorption promoting agents, controlled release agents, and one or more inert excipients (which include starches, polyols, granulating agents, microcrystalline cellulose (e.g. celphere, Celphere beads®), diluents, lubricants, binders, disintegrating agents, and the like), etc. If desired, tablet dosages of the disclosed compositions may be coated by standard aqueous or nonaqueous techniques.

Compositions of the present disclosure may also optionally include other therapeutic ingredients, anti-caking agents, preservatives, sweetening agents, colorants, flavors, desiccants, plasticizers, dyes, glidants, anti-adherents, anti-static agents, surfactants (wetting agents), anti-oxidants, film-coating agents, and the like. Any such optional ingredient must be compatible with the compound described herein to insure the stability of the formulation.

The composition may contain other additives as needed, including for exanple lactose, glucose, fructose, galactose, trehalose, sucrose, maltose, raffinose, maltitol, melezitose, stachyose, lactitol, palatinite, starch, xylitol, mannitol, myoinositol, and the like, and hydrates thereof, and amino acids, for example alanine, glycine and betaine, and peptides and proteins, for example albumen.

Examples of excipients for use as the pharmaceutically acceptable carriers and the pharmaceutically acceptable inert carriers and the aforementioned additional ingredients include, but are not limited to binders, fillers, disintegrants, lubricants, anti-microbial agents, and coating agents such as: BINDERS: corn starch, potato starch, other starches, gelatin, natural and synthetic gums such as acacia, xanthan, sodium alginate, alginic acid, other alginates, powdered tragacanth, guar gum, cellulose and its derivatives (*e.g.,* ethyl cellulose, cellulose acetate, carboxymethyl cellulose calcium, sodium carboxymethyl cellulose), polyvinyl pyrrolidone (*e.g.,* povidone, crospovidone, copovidone, etc), methyl cellulose, Methocel, pre-gelatinized starch (*e.g.,* STARCH 1500® and STARCH 1500 LM®, sold by Colorcon, Ltd.), hydroxypropyl methyl cellulose, microcrystalline cellulose (*e.g*. AVICEL™, such as, AVICEL-PH-101™, -103™ and -105™, sold by FMC Corporation, Marcus Hook, PA, USA), or mixtures thereof,

FILLERS: talc, calcium carbonate (*e.g.,* granules or powder), dibasic calcium phosphate, tribasic calcium phosphate, calcium sulfate (*e.g.,* granules or powder), microcrystalline cellulose, powdered cellulose, dextrates, kaolin, mannitol, silicic acid, sorbitol, starch, pre-gelatinized starch, dextrose, fructose, honey, lactose anhydrate, lactose monohydrate, lactose and aspartame, lactose and cellulose, lactose and microcrystalline cellulose, maltodextrin, maltose, mannitol, microcrystalline cellulose & guar gum, molasses, sucrose,or mixtures thereof,

DISINTEGRANTS: agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, clays, other algins, other celluloses, gums (like gellan), low-substituted hydroxypropyl cellulose, or mixtures thereof,

LUBRICANTS: calcium stearate, magnesium stearate, mineral oil, light mineral oil, glycerin, sorbitol, mannitol, polyethylene glycol, other glycols, stearic acid, sodium lauryl sulfate, sodium stearyl fumarate, vegetable based fatty acids lubricant, talc, hydrogenated vegetable oil (*e.g.,* peanut oil, cottonseed oil, sunflower oil, sesame oil, olive oil, corn oil and soybean oil), zinc stearate, ethyl oleate, ethyl laurate, agar, syloid silica gel (AEROSIL 200, W.R. Grace Co., Baltimore, MD USA), a coagulated aerosol of synthetic silica (Deaussa Co., Plano, TX USA), a pyrogenic silicon dioxide (CAB-O-SIL, Cabot Co., Boston, MA USA), or mixtures thereof,

ANTI-CAKING AGENTS: calcium silicate, magnesium silicate, silicon dioxide, colloidal silicon dioxide, talc, or mixtures thereof,
ANTIMICROBIAL AGENTS: benzalkonium chloride, benzethonium chloride, benzoic acid, benzyl alcohol, butyl paraben, cetylpyridinium chloride, cresol, chlorobutanol, dehydroacetic acid, ethylparaben, methylparaben, phenol, phenylethyl alcohol, phenoxyethanol, phenylmercuric acetate, phenylmercuric nitrate, potassium sorbate, propylparaben, sodium benzoate, sodium dehydroacetate, sodium propionate, sorbic acid, thimersol, thymo, or mixtures thereof, and
COATING AGENTS: sodium carboxymethyl cellulose, cellulose acetate phthalate, ethylcellulose, gelatin, pharmaceutical glaze, hydroxypropyl cellulose, hydroxypropyl methylcellulose (hypromellose), hydroxypropyl methyl cellulose phthalate, methylcellulose, polyethylene glycol, polyvinyl acetate phthalate, shellac, sucrose, titanium dioxide, carnauba wax, microcrystalline wax, gellan gum, maltodextrin, methacrylates, microcrystalline cellulose and carrageenan or mixtures thereof.

The formulation can also include other excipients and categories thereof including but not limited to L-histidine, Pluronic®, Poloxamers (such as Lutrol® and Poloxamer 188), ascorbic acid, glutathione, permeability enhancers (e.g. lipids, sodium cholate, acylcarnitine, salicylates, mixed bile salts, fatty acid micelles, chelators, fatty acid, surfactants, medium chain glycerides), protease inhibitors (e.g. soybean trypsin inhibitor, organic acids), pH lowering agents and absorption enhancers effective to promote bioavailability (including but not limited to those described in US6086918 and US5912014), creams and lotions (like maltodextrin and carrageenans); materials for chewable tablets (like dextrose, fructose, lactose monohydrate, lactose and aspartame, lactose and cellulose, maltodextrin, maltose, mannitol, microcrystalline cellulose and guar gum, sorbitol crystalline); parenterals (like mannitol and povidone); plasticizers (like dibutyl sebacate, plasticizers for coatings, polyvinylacetate phthalate); powder lubricants (like glyceryl behenate); soft gelatin capsules (like sorbitol special solution); spheres for coating (like sugar spheres); spheronization agents (like glyceryl behenate and microcrystalline cellulose); suspending/gelling agents (like carrageenan, gellan gum, mannitol, microcrystalline cellulose, povidone, sodium starch glycolate, xanthan gum); sweeteners (like aspartame, aspartame and lactose, dextrose, fructose, honey, maltodextrin, maltose, mannitol, molasses, sorbitol crystalline, sorbitol special solution, sucrose); wet granulation agents (like calcium carbonate, lactose anhydrous, lactose monohydrate, maltodextrin, mannitol, microcrystalline cellulose, povidone, starch), caramel, carboxymethylcellulose sodium, cherry cream flavor and cherry flavor, citric acid anhydrous, citric acid, confectioner's sugar, D&C Red No. 33, D&C Yellow #10 Aluminum Lake, disodium edetate, ethyl alcohol 15%, FD& C Yellow No. 6 aluminum lake, FD&C Blue #1 Aluminum Lake, FD&C Blue No. 1, FD&C blue no. 2 aluminum lake, FD&C Green No.3, FD&C Red No. 40, FD&C Yellow No. 6 Aluminum Lake, FD&C Yellow No. 6, FD&C Yellow No. 10, glycerol palmitostearate, glyceryl monostearate, indigo carmine, lecithin, manitol, methyl and propyl parabens, mono ammonium glycyrrhizinate, natural and artificial orange flavor, pharmaceutical glaze, poloxamer 188, Polydextrose, polysorbate 20, polysorbate 80, polyvidone, pregelatinized corn starch, pregelatinized starch, red iron oxide, saccharin sodium, sodium carboxymethyl ether, sodium chloride, sodium citrate, sodium phosphate, strawberry flavor, synthetic black iron oxide, synthetic red iron oxide, titanium dioxide, and white wax.

Solid oral dosage forms may optionally be treated with coating systems (e.g. Opadry® fx film coating system, for example Opadry® blue (OY-LS-20921), Opadry® white (YS-2-7063), Opadry® white (YS-1-7040), and black ink (S-1-8106).

The agents either in their free form or as a salt can be combined with a polymer such as polylactic-glycoloic acid (PLGA), poly-(I)-lactic-glycolic-tartaric acid (P(I)LGT) (WO 01/12233), polyglycolic acid (U.S. 3,773,919), polylactic acid (U.S. 4,767,628), poly(M-caprolactone) and poly(alkylene oxide) (U.S. 20030068384) to create a sustained release formulation. Such formulations can be used to implants that release a peptide or another agent over a period of a few days, a few weeks or several months depending on the polymer, the particle size of the polymer, and the size of the implant (see, e.g., U.S. 6,620,422). Other sustained release formulations and polymers for use in are described in EP 0 467 389 A2, WO 93/24150, U.S. 5,612,052, WO 97/40085, WO 03/075887, WO 01/01964A2, U.S. 5,922,356, WO 94/155587, WO 02/074247A2, WO 98/25642, U.S. 5,968,895, U.S. 6,180,608, U.S. 20030171296, U.S. 20020176841, U.S. 5,672,659, U.S. 5,893,985, U.S. 5,134,122, U.S. 5,192,741, U.S. 5,192,741, U.S. 4,668,506, U.S. 4,713,244, U.S. 5,445,832 U.S. 4,931,279, U.S. 5,980,945, WO 02/058672, WO 9726015, WO 97/04744, and. US20020019446. In such sustained release formulations microparticles (Delie and Blanco-Prieto 2005 Molecule 10:65-80) of peptide are combined with microparticles of polymer. One or more sustained release implants can be placed in the large intestine, the small intestine or both. U.S. 6,011,011 and WO 94/06452 describe a sustained release formulation providing either polyethylene glycols (i.e. PEG 300 and PEG 400) or triacetin. WO 03/053401 describes a formulation which may both enhance bioavailability and provide controlled releaseof the agent within the GI tract. Additional controlled release formulations are described in U.S. 6,734,188, WO 02/38129, EP 326 151, U.S. 5,236,704, WO 02/30398, WO 98/13029; U.S. 20030064105, U.S. 20030138488A1, U.S. 20030216307A1, U.S. 6,667,060, WO 01/49249, WO 01/49311, WO 01/49249, WO 01/49311, and U.S. 5,877,224.

The agents can be administered, e.g., by intravenous injection, intramuscular injection, subcutaneous injection, intraperitoneal injection, topical, sublingual, intraarticular (in the joints), intradermal, buccal, ophthalmic (including intraocular), intranasaly (including using a cannula), intraspinally, intrathecally, or by other routes. The agents can be administered orally, e.g., as a tablet or cachet containing a predetermined amount of the active ingredient, gel, pellet, paste, syrup, bolus, electuary, slurry, capsule, powder, lyophilized powder, granules, sachet, as a solution or a suspension in an aqueous liquid or a non-aqueous liquid, as an oil-in-water liquid emulsion or a water-in-oil liquid emulsion, via a micellar formulation (see, e.g. WO 97/11682) via a liposomal formulation (see, e.g., EP 736299,WO 99/59550 and WO 97/13500), via formulations described in WO 03/094886, via bilosome (bile-salt based vesicular system), via a dendrimer, or in some other form. Orally administered compositions can include binders, lubricants, inert diluents, lubricating, surface active or dispersing agents, flavoring agents, and humectants. Orally administered formulations such as tablets may optionally be coated or scored and may be formulated so as to provide sustained, delayed or controlled release of the active ingredient therein. The agents can also be administered transdermally (i.e. via reservoir-type or matrix-type patches, microneedles, thermal poration, hypodermic needles, iontophoresis, electroporation, ultrasound or other forms of sonophoresis, jet injection, or a combination of any of the preceding methods (Prausnitz et al. 2004, Nature Reviews Drug Discovery 3:115-124)). The agents can be administered using high-velocity transdermal particle injection techniques using the hydrogel particle formulation described in U.S. 20020061336. Additional particle formulations are described in WO 00/45792, WO 00/53160, and WO 02/19989. An example of a transdermal formulation containing plaster and the absorption promoter dimethylisosorbide can be found in WO 89/04179. WO 96/11705 provides formulations suitable for transdermal adminisitration. The agents can be administered in the form a suppository or by other vaginal or rectal means. The agents can be administered in a transmembrane formulation as described in WO 90/07923. The agents can be administed non-invasively via the dehydrated particicles described in U.S. 6,485,706. The agent can be administered in an enteric-coated drug formulation as described in WO 02/49621. The agents can be administered intranassaly using the formulation described in U.S. 5,179,079. Formulations suitable for parenteral injection are described in WO 00/62759. The agents can be administered using the casein formulation described in U. S. 20030206939 and WO 00/06108. The agents can be administered using the particulate formulations described in U.S. 20020034536.

The agents, alone or in combination with other suitable components, can be administered by pulmonary route utilizing several techniques including but not limited to intratracheal instillation (delivery of solution into the lungs by syringe), intratracheal delivery of liposomes, insufflation (administration of powder formulation by syringe or any other similar device into the lungs) and aerosol inhalation. Aerosols (e.g., jet or ultrasonic nebulizers, metered-dose inhalers (MDIs), and dry-powder inhalers (DPIs)) can also be used in intranasal applications. Aerosol formulations are stable dispersions or suspensions of solid material and liquid droplets in a gaseous medium and can be placed into pressurized acceptable propellants, such as hydrofluroalkanes (HFAs, i.e. HFA-134a and HFA-227, or a mixture thereof), dichlorodifluoromethane (or other chlorofluocarbon propellants such as a mixture of Propellants 11, 12, and/or 114), propane, nitrogen, and the like. Pulmonary formulations may include permeation enhancers such as fatty acids, saccharides, chelating agents, enzyme inhibitors (e.g., protease inhibitors), adjuvants (e.g., glycocholate, surfactin, span 85, and nafamostat), preservatives (e.g., benzalkonium chloride or chlorobutanol), and ethanol (normally up to 5% but possibly up to 20%, by weight). Ethanol is commonly included in aerosol compositions as it can improve the function of the metering valve and in some cases also improve the stability of the dispersion. Pulmonary formulations may also include surfactants which include but are not limited to bile salts and those described in U.S. 6,524,557 and references therein. The surfactants described in U.S. 6,524,557, e.g., a C8-C16 fatty acid salt, a bile salt, a phospholipid, or alkyl saccaride are advantageous in that some of them also reportedly enhance absorption of the peptide in the formulation. Also suitable in the disclosure are dry powder formulations comprising a therapeutically effective amount of active compound blended with an appropriate carrier and adapted for use in connection with a dry-powder inhaler. Absorption enhancers which can be added to dry powder formulations of the present disclosure include those described in U.S. 6,632,456. WO 02/080884 describes new methods for the surface modification of powders. Aerosol formulations may include U.S. 5,230,884, U.S. 5,292,499, WO 017/8694, WO 01/78696, U.S. 2003019437, U. S. 20030165436, and WO 96/40089 (which includes vegetable oil). Sustained release formulations suitable for inhalation are described in U.S. 20010036481A1, 20030232019A1, and U.S. 20040018243A1 as well as in WO 01/13891, WO 02/067902, WO 03/072080, and WO 03/079885. Pulmonary formulations containing microparticles are described in WO 03/015750, U.S. 20030008013, and WO 00/00176. Pulmonary formulations containing stable glassy state powder are described in U.S. 20020141945 and U.S. 6,309,671. Other aerosol formulations are desribed in EP 1338272A1 WO 90/09781, U. S. 5,348,730, U.S. 6,436,367, WO 91/04011, and U.S. 6,294,153 and U.S. 6,290,987 describes a liposomal based formulation that can be administered via aerosol or other means. Powder formulations for inhalation are described in U.S. 20030053960 and WO 01/60341. The agents can be administered intranasally as described in U.S. 20010038824. The agents can be incorporated into microemulsions, which generally are thermodynamically stable, isotropically clear dispersions of two immiscible liquids, such as oil and water, stabilized by an interfacial film of surfactant molecules (Encyclopedia of Pharmaceutical Technology (New York: Marcel Dekker, 1992), volume 9). For the preparation of microemulsions, surfactant (emulsifier), co-surfactant (co-emulsifier), an oil phase and a water phase are necessary. Suitable surfactants include any surfactants that are useful in the preparation of emulsions, e.g., emulsifiers that are typically used in the preparation of creams. The co-surfactant (or "co-emulsifer") is generally selected from the group of polyglycerol derivatives, glycerol derivatives and fatty alcohols. Preferred emulsifier/co-emulsifier combinations are generally although not necessarily selected from the group consisting of: glyceryl monostearate and polyoxyethylene stearate; polyethylene glycol and ethylene glycol palmitostearate; and caprilic and capric triglycerides and oleoyl macrogolglycerides. The water phase includes not only water but also, typically, buffers, glucose, propylene glycol, polyethylene glycols, preferably lower molecular weight polyethylene glycols (e.g., PEG 300 and PEG 400), and/or glycerol, and the like, while the oil phase will generally comprise, for example, fatty acid esters, modified vegetable oils, silicone oils, mixtures of mono- di- and triglycerides, mono- and di-esters of PEG (e.g., oleoyl macrogol glycerides), etc.

The agents described herein can be incorporated into pharmaceutically-acceptable nanoparticle, nanosphere, and nanocapsule formulations (Delie and Blanco-Prieto 2005 Molecule 10:65-80). Nanocapsules can generally entrap compounds in a stable and reproducible way (Henry-Michelland et al., 1987; Quintanar-Guerrero et al., 1998; Douglas et al., 1987). To avoid side effects due to intracellular polymeric overloading, ultrafine particles (sized around 0.1 µm) can be designed using polymers able to be degraded in vivo (e.g. biodegradable polyalkylcyanoacrylate nanoparticles). Such particles are described in the prior art (Couvreur et al, 1980; 1988; zur Muhlen et al., 1998; Zambaux et al. 1998; Pinto-Alphandry et al., 1995 and U.S. Pat. No. 5,145,684).

The agents described herein can be formulated with pH sensitive materials which may include those described in W004041195 (including the seal and enteric coating described therein) and pH-sensitive coatings that achieve delivery in the colon including those described in US4910021 and WO9001329. US4910021 describes using a pH-sensitive material to coat a capsule. WO9001329 describes using pH-sensitive coatings on beads containing acid, where the acid in the bead core prolongs dissolution of the pH-sensitive coating. U. S. Patent No. 5,175, 003 discloses a dual mechanism polymer mixture composed of pH-sensitive enteric materials and film-forming plasticizers capable of conferring permeability to the enteric material, for use in drug-delivery systems; a matrix pellet composed of a dual mechanism polymer mixture permeated with a drug and sometimes covering a pharmaceutically neutral nucleus; a membrane-coated pellet comprising a matrix pellet coated with a dual mechanism polymer mixture envelope of the same or different composition; and a pharmaceutical dosage form containing matrix pellets. The matrix pellet releases acid-soluble drugs by diffusion in acid pH and by disintegration at pH levels of nominally about 5.0 or higher. The agents described herein may be formulated in the pH triggered targeted control release systems described in W004052339. The agents described herein may be formulated according to the methodology described in any of W003105812 (extruded hyrdratable polymers); WO0243767 (enzyme cleavable membrane translocators); WO03007913 and WO03086297 (mucoadhesive systems); WO02072075 (bilayer laminated formulation comprising pH lowering agent and absorption enhancer); WO04064769 (amidated peptides); W005063156 (solid lipid suspension with pseudotropic and/or thixotropic properties upon melting); WO03035029 and WO03035041 (erodible, gastric retentive dosage forms); US5007790 and US5972389 (sustained release dosage forms); WO04112711 (oral extended release compositions); WO05027878, WO02072033, and WO02072034 (delayed release compositions with natural or synthetic gum); W005030182 (controlled release formulations with an ascending rate of release); WO05048998 (microencapsulation system); US Patent 5,952, 314 (biopolymer); US5108758 (glassy amylose matrix delivery); US 5840860 (modified starch based delivery). JP10324642 (delivery system comprising chitosan and gastric resistant material such as wheat gliadin or zein); US5866619 and US6368629 (saccharide containing polymer); US 6531152 (describes a drug delivery system containing a water soluble core (Ca pectinate or other water-insoluble polymers) and outer coat which bursts (eg hydrophobic polymer-Eudragrit)); US 6234464; US 6403130 (coating with polymer containing casein and high methoxy pectin; WO0174175 (Maillard reaction product); WO05063206 (solubility increasing formulation); WO04019872 (transferring fusion proteins). The agents described herein may be formulated using gastrointestinal retention system technology (GIRES; Merrion Pharmaceuticals). GIRES comprises a controlled-release dosage form inside an inflatable pouch, which is placed in a drug capsule for oral administration. Upon dissolution of the capsule, a gas-generating system inflates the pouch in the stomach where it is retained for 16-24 hours, all the time releasing agents described herein.

The agents described herein can be formulated in an osmotic device including the ones disclosed in US4503030, US5609590 and US5358502. US4503030 discloses an osmotic device for dispensing a drug to certain pH regions of the gastrointestinal tract. More particularly, the disclosure relates to an osmotic device comprising a wall formed of a semi-permeable pH sensitive composition that surrounds a compartment containing a drug, with a passageway through the wall connecting the exterior of the device with the compartment. The device delivers the drug at a controlled rate in the region of the gastrointestinal tract having a pH of less than 3.5, and the device self- destructs and releases all its drug in the region of the gastrointestinal tract having a pH greater than 3.5, thereby providing total availability for drug absorption. U. S. Patent Nos. 5,609, 590 and 5, 358,502 disclose an osmotic bursting device for dispensing a beneficial agent to an aqueous environment. The device comprises a beneficial agent and osmagent surrounded at least in part by a semi-permeable membrane. The beneficial agent may also function as the osmagent. The semi-permeable membrane is permeable to water and substantially impermeable to the beneficial agent and osmagent. A trigger means is attached to the semi-permeable membrane (e. g., joins two capsule halves). The trigger means is activated by a pH of from 3 to 9 and triggers the eventual, but sudden, delivery of the beneficial agent. These devices enable the pH-triggered release of the beneficial agent core as a bolus by osmotic bursting.

The agents described herein may be formulated based on the disclosure described in U. S. Patent No. 5,316, 774 which discloses a composition for the controlled release of an active substance comprising a polymeric particle matrix, where each particle defines a network of internal pores. The active substance is entrapped within the pore network together with a blocking agent having physical and chemical characteristics selected to modify the release rate of the active substance from the internal pore network. In one embodiment, drugs may be selectively delivered to the intestines using an enteric material as the blocking agent. The enteric material remains intact in the stomach but degrades under the pH conditions of the intestines. In another embodiment, the sustained release formulation employs a blocking agent, which remains stable under the expected conditions of the environment to which the active substance is to be released. The use of pH-sensitive materials alone to achieve site-specific delivery is difficult because of leaking of the beneficial agent prior to the release site or desired delivery time and it is difficult to achieve long time lags before release of the active ingredient after exposure to high pH (because of rapid dissolution or degradation of the pH-sensitive materials).

The agents may also be formulated in a hybrid system which combines pH-sensitive materials and osmotic delivery systems. These hybrid devices provide delayed initiation of sustained-release of the beneficial agent. In one device a pH-sensitive matrix or coating dissolves releasing osmotic devices that provide sustained release of the beneficial agent see U. S. Patent Nos. 4,578, 075, 4,681, 583, and 4,851, 231. A second device consists of a semipermeable coating made of a polymer blend of an insoluble and a pH-sensitive material. As the pH increases, the permeability of the coating increases, increasing the rate of release of beneficial agent see U. S. Patent Nos. 4,096, 238,4, 503,030, 4, 522, 625, and 4,587, 117.

The agents described herein may be formulated in terpolumers according to U. S. Patent No. 5,484, 610 which discloses terpolymers which are sensitive to pH and temperature which are useful carriers for conducting bioactive agents through the gastric juices of the stomach in a protected form. The terpolymers swell at the higher physiologic pH of the intestinal tract causing release of the bioactive agents into the intestine. The terpolymers are linear and are made up of 35 to 99 wt % of a temperature sensitive component, which imparts to the terpolymer LCST (lower critical solution temperature) properties below body temperatures, 1 to 30 wt % of a pH sensitive component having a pKa in the range of from 2 to 8 which functions through ionization or deionization of carboxylic acid groups to prevent the bioactive agent from being lost at low pH but allows bioactive agent release at physiological pH of about 7.4 and a hydrophobic component which stabilizes the LCST below body temperatures and compensates for bioactive agent effects on the terpolymers. The terpolymers provide for safe bioactive agent loading, a simple procedure for dosage form fabrication and the terpolymer functions as a protective carrier in the acidic environment of the stomach and also protects the bioactive agents from digestive enzymes until the bioactive agent is released in the intestinal tract.

The agents described herein may be formulated in pH sensitive polymers according to those described in U. S. Patent No. 6,103, 865. U. S. Patent No. 6,103, 865 discloses pH-sensitive polymers containing sulfonamide groups, which can be changed in physical properties, such as swellability and solubility, depending on pH and which can be applied for a drug-delivery system, bio-material, sensor, and the like, and a preparation method therefore. The pH-sensitive polymers are prepared by introduction of sulfonamide groups, various in pKa, to hydrophilic groups of polymers either through coupling to the hydrophilic groups of polymers, such as acrylamide, N, N- dimethylacrylamide, acrylic acid, N-isopropylacrylamide and the like or copolymerization with other polymerizable monomers. These pH-sensitive polymers may have a structure of linear polymer, grafted copolymer, hydrogel or interpenetrating network polymer.

The agents described herein may be formulated according U.S. Patent No. 5, 656, 292 which discloses a composition for pH dependent or pH regulated controlled release of active ingredients especially drugs. The composition consists of a compactable mixture of the active ingredient and starch molecules substituted with acetate and dicarboxylate residues. The preferred dicarboxylate acid is succinate. The average substitution degree of the acetate residue is at least 1 and 0. 2-1.2 for the dicarboxylate residue. The starch molecules can have the acetate and dicarboxylate residues attached to the same starch molecule backbone or attached to separate starch molecule backbones. The present disclosure also discloses methods for preparing said starch acetate dicarboxylates by transesterification or mixing of starch acetates and starch dicarboxylates respectively.

The agents described herein may be formulated according to the methods described in U. S. Patent Nos. 5,554, 147,5, 788, 687, and 6,306, 422 which disclose a method for the controlled release of a biologically active agent wherein the agent is released from a hydrophobic, pH-sensitive polymer matrix. The polymer matrix swells when the environment reaches pH 8.5, releasing the active agent. A polymer of hydrophobic and weakly acidic comonomers is disclosed for use in the controlled release system. Also disclosed is a specific embodiment in which the controlled release system may be used. The pH-sensitive polymer is coated onto a latex catheter used in ureteral catheterization. A ureteral catheter coated with a pH-sensitive polymer having an antibiotic or urease inhibitor trapped within its matrix will release the active agent when exposed to high pH urine.

The agents described herein may be formulated in/with bioadhesive polymers according to US Patent No. 6,365, 187. Bioadhesive polymers in the form of, or as a coating on, microcapsules containing drugs or bioactive substances which may serve for therapeutic, or diagnostic purposes in diseases of the gastrointestinal tract, are described in US6365187. The polymeric microspheres all have a bioadhesive force of at least 11 mN/cm² (110 N/m2) Techniques for the fabrication of bioadhesive microspheres, as well as a method for measuring bioadhesive forces between microspheres and selected segments of the gastrointestinal tract in vitro are also described. This quantitative method provides a means to establish a correlation between the chemical nature, the surface morphology and the dimensions of drug-loaded microspheres on one hand and bioadhesive forces on the other, allowing the screening of the most promising materials from a relatively large group of natural and synthetic polymers which, from theoretical consideration, should be used for making bioadhesive microspheres. Solutions of medicament in buffered saline and similar vehicles are commonly employed to generate an aerosol in a nebulizer. Simple nebulizers operate on Bernoulli's principle and employ a stream of air or oxygen to generate the spray particles. More complex nebulizers employ ultrasound to create the spray particles. Both types are well known in the art and are described in standard textbooks of pharmacy such as Sprowls' American Pharmacy and Remington's The Science and Practice of Pharmacy. Other devices for generating aerosols employ compressed gases, usually hydrofluorocarbons and chlorofluorocarbons, which are mixed with the medicament and any necessary excipients in a pressurized container, these devices are likewise described in standard textbooks such as Sprowls and Remington.

The agents can be a free acid or base, or a pharmacologically acceptable salt thereof. Solids can be dissolved or dispersed immediately prior to administration or earlier. In some circumstances the preparations include a preservative to prevent the growth of microorganisms. The pharmaceutical forms suitable for injection can include sterile aqueous or organic solutions or dispersions which include, e.g., water, an alcohol, an organic solvent, an oil or other solvent or dispersant (e.g., glycerol, propylene glycol, polyethylene glycol, and vegetable oils). The formulations may contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. Pharmaceutical agents can be sterilized by filter sterilization or by other suitable means. The agent can be fused to immunoglobulins or albumin, albumin variants or fragments thereof, or incorporated into a liposome to improve half-life. Thus the agents described herein may be fused directly or via a peptide linker, water soluble polymer, or prodrug linker to albumin or an analog, fragment, or derivative thereof. Generally, the albumin proteins that are part of the fusion proteins of the present disclosure may be derived from albumin cloned from any species, including human. Human serum albumin (HSA) consists of a single non-glycosylated peptide chain of 585 amino acids with a formula molecular weight of 66,500. The amino acid sequence of human HSA is known [See Meloun, et al. (1975) FEBS Letters 58:136; Behrens, et al. (1975) Fed. Proc. 34:591; Lawn, et al. (1981) Nucleic Acids Research 9:6102-6114; Minghetti, et al. (1986) J. Biol. Chem. 261:6747, each of which are incorporated by reference herein]. A variety of polymorphic variants as well as analogs and fragments of albumin have been described. [See Weitkamp, et al., (1973) Ann. Hum. Genet. 37:219]. For example, in EP 322,094, various shorter forms of HSA. Some of these fragments of HSA are disclosed, including HSA(1-373), HSA(1-388), HSA(1-389), HSA(1-369), and HSA(1-419) and fragments between 1-369 and 1-419. EP 399,666 discloses albumin fragments that include HSA(1-177) and HSA(1-200) and fragments between HSA(1-177) and HSA(1-200). Methods related to albumin fusion proteins can be found in US 7,056,701, US 6,994,857, US 6,946,134, US6,926,898, and US 6,905,688 and the related priority documents and references cited therein. The agent can also be conjugated to polyethylene glycol (PEG) chains. Methods for pegylation and additional formulations containing PEG-conjugates (i.e. PEG-based hydrogels, PEG modified liposomes) can be found in Harris and Chess, Nature Reviews Drug Discovery 2: 214-221 and the references therein. Agents can also be modified with alkyl groups (e.g., C1-C20 straight or branched alkyl groups); fatty acid radicals; and combinations of PEG, alkyl groups and fatty acid radicals (see U.S. Patent 6,309,633; Soltero et al., 2001 Innovations in Pharmaceutical Technology 106-110). The agent can be administered via a nanocochleate or cochleate delivery vehicle (BioDelivery Sciences International). The agents can be delivered transmucosally (i.e. across a mucosal surface such as the vagina, eye or nose) using formulations such as that described in U.S. 5,204,108. The agents can be formulated in microcapsules as described in WO 88/01165. The agent can be administered intra-orally using the formulations described in U.S. 20020055496, WO 00/47203, and U.S. 6,495,120. The agent can be delivered using nanoemulsion formulations described in WO 01/91728A2.

### Controlled release formulations

In general, one can provide for controlled release of the agents described herein through the use of a wide variety of polymeric carriers and controlled release systems including erodible and non-erodible matrices, osmotic control devices, various reservoir devices, enteric coatings and multiparticulate control devices.

Matrix devices are a common device for controlling the release of various agents. In such devices, the agents described herein are generally present as a dispersion within the polymer matrix, and are typically formed by the compression of a polymer/drug mixture or by dissolution or melting. The dosage release properties of these devices may be dependent upon the solubility of the agent in the polymer matrix or, in the case of porous matrices, the solubility in the sink solution within the pore network, and the tortuosity of the network. In one instance, when utilizing an erodible polymeric matrix, the matrix imbibes water and forms an aqueous-swollen gel that entraps the agent. The matrix then gradually erodes, swells, disintegrates or dissolves in the GI tract, thereby controlling release of one or more of the agents described herein. In non-erodible devices, the agent is released by diffusion through an inert matrix.

Agents described herein can be incorporated into an erodible or non-erodible polymeric matrix controlled release device. By an erodible matrix is meant aqueous-erodible or water-swellable or aqueous-soluble in the sense of being either erodible or swellable or dissolvable in pure water or requiring the presence of an acid or base to ionize the polymeric matrix sufficiently to cause erosion or dissolution. When contacted with the aqueous environment of use, the erodible polymeric matrix imbibes water and forms an aqueous-swollen gel or matrix that entraps the agent described herein. The aqueous-swollen matrix gradually erodes, swells, disintegrates or dissolves in the environment of use, thereby controlling the release of a compound described herein to the environment of use.

The erodible polymeric matrix into which an agent described herein can be incorporated may generally be described as a set of excipients that are mixed with the agent following its formation that, when contacted with the aqueous environment of use imbibes water and forms a water-swollen gel or matrix that entraps the drug form. Drug release may occur by a variety of mechanisms, for example, the matrix may disintegrate or dissolve from around particles or granules of the agent or the agent may dissolve in the imbibed aqueous solution and diffuse from the tablet, beads or granules of the device. One ingredient of this water-swollen matrix is the water-swellable, erodible, or soluble polymer, which may generally be described as an osmopolymer, hydrogel or water-swellable polymer. Such polymers may be linear, branched, or crosslinked. The polymers may be homopolymers or copolymers. In certain embodiments, they may be synthetic polymers derived from vinyl, acrylate, methacrylate, urethane, ester and oxide monomers. In other embodiments, they can be derivatives of naturally occurring polymers such as polysaccharides (e.g. chitin, chitosan, dextran and pullulan; gum agar, gum arabic, gum karaya, locust bean gum, gum tragacanth, carrageenans, gum ghatti, guar gum, xanthan gum and scleroglucan), starches (e.g. dextrin and maltodextrin), hydrophilic colloids (e.g. pectin), phosphatides (e.g. lecithin), alginates (e.g. ammonium alginate, sodium, potassium or calcium alginate, propylene glycol alginate), gelatin, collagen, and cellulosics. Cellulosics are cellulose polymer that has been modified by reaction of at least a portion of the hydroxyl groups on the saccharide repeat units with a compound to form an ester-linked or an ether-linked substituent. For example, the cellulosic ethyl cellulose has an ether linked ethyl substituent attached to the saccharide repeat unit, while the cellulosic cellulose acetate has an ester linked acetate substituent. In certain embodiments, the cellulosics for the erodible matrix comprises aqueous-soluble and aqueous-erodible cellulosics can include, for example, ethyl cellulose (EC), methylethyl cellulose (MEC), carboxymethyl cellulose (CMC), CMEC, hydroxyethyl cellulose (HEC), hydroxypropyl cellulose (HPC), cellulose acetate (CA), cellulose propionate (CP), cellulose butyrate (CB), cellulose acetate butyrate (CAB), CAP, CAT, hydroxypropyl methyl cellulose (HPMC), HPMCP, HPMCAS, hydroxypropyl methyl cellulose acetate trimellitate (HPMCAT), and ethylhydroxy ethylcellulose (EHEC). In certain embodiments, the cellulosics comprises various grades of low viscosity (MW less than or equal to 50,000 daltons, for example, the Dow Methocel™ series E5, E15LV, E50LV and K100LY) and high viscosity (MW greater than 50,000 daltons, for example, E4MCR, E10MCR, K4M, K15M and K100M and the Methocel™ K series) HPMC. Other commercially available types of HPMC include the Shin Etsu Metolose 90SH series.

The choice of matrix material can have a large effect on the maximum drug concentration attained by the device as well as the maintenance of a high drug concentration. The matrix material can be a concentration-enhancing polymer, for example, as described in WO05/011634. Other materials useful as the erodible matrix material include, but are not limited to, pullulan, polyvinyl pyrrolidone, polyvinyl alcohol, polyvinyl acetate, glycerol fatty acid esters, polyacrylamide, polyacrylic acid, copolymers of ethacrylic acid or methacrylic acid (EUDRAGITO, Rohm America, Inc., Piscataway, New Jersey) and other acrylic acid derivatives such as homopolymers and copolymers of butylmethacrylate, methylmethacrylate, ethylmethacrylate, ethylacrylate, (2-dimethylaminoethyl) methacrylate, and (trimethylaminoethyl) methacrylate chloride.

The erodible matrix polymer may contain a wide variety of the same types of additives and excipients known in the pharmaceutical arts, including osmopolymers, osmagens, solubility-enhancing or-retarding agents and excipients that promote stability or processing of the device.

Alternatively, the agents of the present disclosure may be administered by or incorporated into a non-erodible matrix device. In such devices, an agent described herein is distributed in an inert matrix. The agent is released by diffusion through the inert matrix. Examples of materials suitable for the inert matrix include insoluble plastics (e.g methyl acrylate-methyl methacrylate copolymers, polyvinyl chloride, polyethylene), hydrophilic polymers (e.g. ethyl cellulose, cellulose acetate, crosslinked polyvinylpyrrolidone (also known as crospovidone)), and fatty compounds (e.g. carnauba wax, microcrystalline wax, and triglycerides). Such devices are described further in Remington: The Science and Practice of Pharmacy, 20th edition (2000). Matrix controlled release devices may be prepared by blending an agent described herein and other excipients together, and then forming the blend into a tablet, caplet, pill, or other device formed by compressive forces. Such compressed devices may be formed using any of a wide variety of presses used in the fabrication of pharmaceutical devices. Examples include single-punch presses, rotary tablet presses, and multilayer rotary tablet presses, all well known in the art. See for example, Remington: The Science and Practice of Pharmacy, 20th Edition, 2000. The compressed device may be of any shape, including round, oval, oblong, cylindrical, or triangular. The upper and lower surfaces of the compressed device may be flat, round, concave, or convex.

In certain embodiments, when formed by compression, the device has a strength of at least 5 Kiloponds (Kp)/cm² (for example, at least 7 Kp/cm²). Strength is the fracture force, also known as the tablet hardness required to fracture a tablet formed from the materials, divided by the maximum cross-sectional area of the tablet normal to that force. The fracture force may be measured using a Schleuniger Tablet Hardness Tester, Model 6D. The compression force required to achieve this strength will depend on the size of the tablet, but generally will be greater than about 5 kP/cm². Friability is a well-know measure of a device's resistance to surface abrasion that measures weight loss in percentage after subjecting the device to a standardized agitation procedure. Friability values of from 0.8 to 1.0% are regarded as constituting the upper limit of acceptability. Devices having a strength of greater than 5 kP/cm² generally are very robust, having a friability of less than 0.5%. Other methods for forming matrix controlled-release devices are well known in the pharmaceutical arts. See for example, Remington: The Science and Practice of Pharmacy, 20th Edition, 2000.

As noted above, the agents described herein may also be incorporated into an osmotic control device. Such devices generally include a core containing one or more agents as described herein and a water permeable, non-dissolving and non-eroding coating surrounding the core which controls the influx of water into the core from an aqueous environment of use so as to cause drug release by extrusion of some or all of the core to the environment of use. In certain embodiments, the coating is polymeric, aqueous-permeable, and has at least one delivery port. The core of the osmotic device optionally includes an osmotic agent which acts to imbibe water from the surrounding environment via such a semi-permeable membrane. The osmotic agent contained in the core of this device may be an aqueous-swellable hydrophilic polymer or it may be an osmogen, also known as an osmagent. Pressure is generated within the device which forces the agent(s) out of the device via an orifice (of a size designed to minimize solute diffusion while preventing the build-up of a hydrostatic pressure head). Osmotic agents create a driving force for transport of water from the environment of use into the core of the device. Osmotic agents include but are not limited to water- swellable hydrophilic polymers, and osmogens (or osmagens). Thus, the core may include water-swellable hydrophilic polymers, both ionic and nonionic, often referred to as osmopolymers and hydrogels. The amount of water-swellable hydrophilic polymers present in the core may range from about 5 to about 80 wt% (including for example, 10 to 50 wt%). Nonlimiting examples of core materials include hydrophilic vinyl and acrylic polymers, polysaccharides such as calcium alginate, polyethylene oxide (PEO), polyethylene glycol (PEG), polypropylene glycol (PPG), poly (2-hydroxyethyl methacrylate), poly (acrylic) acid, poly (methacrylic) acid, polyvinylpyrrolidone (PVP) and crosslinked PVP, polyvinyl alcohol (PVA), PVA/PVP copolymers and PVA/PVP copolymers with hydrophobic monomers such as methyl methacrylate, vinyl acetate, and the like, hydrophilic polyurethanes containing large PEO blocks, sodium croscarmellose, carrageenan, hydroxyethyl cellulose (HEC), hydroxypropyl cellulose (HPC), hydroxypropyl methyl cellulose (HPMC), carboxymethyl cellulose (CMC) and carboxyethyl cellulose (CEC), sodium alginate, polycarbophil, gelatin, xanthan gum, and sodium starch glycolat. Other materials include hydrogels comprising interpenetrating networks of polymers that may be formed by addition or by condensation polymerization, the components of which may comprise hydrophilic and hydrophobic monomers such as those just mentioned. Water-swellable hydrophilic polymers include but are not limited to PEO, PEG, PVP, sodium croscarmellose, HPMC, sodium starch glycolate, polyacrylic acid and crosslinked versions or mixtures thereof.

The core may also include an osmogen (or osmagent). The amount of osmogen present in the core may range from about 2 to about 70 wt% (including, for example, from 10 to 50 wt%). Typical classes of suitable osmogens are water-soluble organic acids, salts and sugars that are capable of imbibing water to thereby effect an osmotic pressure gradient across the barrier of the surrounding coating. Typical useful osmogens include but are not limited to magnesium sulfate, magnesium chloride, calcium chloride, sodium chloride, lithium chloride, potassium sulfate, sodium carbonate, sodium sulfite, lithium sulfate, potassium chloride, sodium sulfate, mannitol, xylitol, urea, sorbitol, inositol, raffinose, sucrose, glucose, fructose, lactose, citric acid, succinic acid, tartaric acid, and mixtures thereof. In certain embodiments, the osmogen is glucose, lactose, sucrose, mannitol, xylitol, sodium chloride, including combinations thereof.

The core may include a wide variety of additives and excipients that enhance the performance of the dosage form or that promote stability, tableting or processing. Such additives and excipients include tableting aids, surfactants, water- soluble polymers, pH modifiers, fillers, binders, pigments, disintegrants, antioxidants, lubricants and flavorants. Nonlimiting examples of additives and excipients include but are not limited to those described elsewhere herein as well as microcrystalline cellulose, metallic salts of acids (e.g. aluminum stearate, calcium stearate, magnesium stearate, sodium stearate, zinc stearate), pH control agents (e.g. buffers, organic acids, organic acid salts, organic and inorganic bases), fatty acids, hydrocarbons and fatty alcohols (e.g. stearic acid, palmitic acid, liquid paraffin, stearyl alcohol, and palmitol), fatty acid esters (e.g. glyceryl (mono-and di-) stearates, triglycerides, glyceryl (palmiticstearic) ester, sorbitan esters (e.g. sorbitan monostearate, saccharose monostearate, saccharose monopalmitate, sodium stearyl fumarate), polyoxyethylene sorbitan esters), surfactants (e.g. alkyl sulfates (e.g. sodium lauryl sulfate, magnesium lauryl sulfate), polymers (e.g. polyethylene glycols, polyoxyethylene glycols, polyoxyethylene, polyoxypropylene ethers, including copolymers thereof), polytetrafluoroethylene), and inorganic materials (e.g. talc, calcium phosphate), cyclodextrins, sugars (e.g. lactose, xylitol), sodium starch glycolate). Nonlimiting examples of disintegrants are sodium starch glycolate (e.g., Explotab™ CLV, (microcrystalline cellulose (e. g., Avicel™), microcrystalline silicified cellulose (e.g., ProSolv™), croscarmellose sodium (e. g., Ac-Di-Sol™). When the agent described herein is a solid amorphous dispersion formed by a solvent process, such additives may be added directly to the spray-drying solution when forming an agent described herein/concentration-enhancing polymer dispersion such that the additive is dissolved or suspended in the solution as a slurry, Alternatively, such additives may be added following the spray-drying process to aid in forming the final controlled release device.

A nonlimiting example of an osmotic device consists of one or more drug layers containing an agent described herein, such as a solid amorphous drug/polymer dispersion, and a sweller layer that comprises a water-swellable polymer, with a coating surrounding the drug layer and sweller layer. Each layer may contain other excipients such as tableting aids, osmagents, surfactants, water-soluble polymers and water-swellable polymers.

Such osmotic delivery devices may be fabricated in various geometries including bilayer (wherein the core comprises a drug layer and a sweller layer adjacent to each other), trilayer (wherein the core comprises a sweller layer sandwiched between two drug layers) and concentric (wherein the core comprises a central sweller agent surrounded by the drug layer). The coating of such a tablet comprises a membrane permeable to water but substantially impermeable to drug and excipients contained within. The coating contains one or more exit passageways or ports in communication with the drug-containing layer(s) for delivering the drug agent. The drug-containing layer(s) of the core contains the drug agent (including optional osmagents and hydrophilic water-soluble polymers), while the sweller layer consists of an expandable hydrogel, with or without additional osmotic agents.

When placed in an aqueous medium, the tablet imbibes water through the membrane, causing the agent to form a dispensable aqueous agent, and causing the hydrogel layer to expand and push against the drug-containing agent, forcing the agent out of the exit passageway. The agent can swell, aiding in forcing the drug out of the passageway. Drug can be delivered from this type of delivery system either dissolved or dispersed in the agent that is expelled from the exit passageway.

The rate of drug delivery is controlled by such factors as the permeability and thickness of the coating, the osmotic pressure of the drug-containing layer, the degree of hydrophilicity of the hydrogel layer, and the surface area of the device. Those skilled in the art will appreciate that increasing the thickness of the coating will reduce the release rate, while any of the following will increase the release rate: increasing the permeability of the coating; increasing the hydrophilicity of the hydrogel layer; increasing the osmotic pressure of the drug-containing layer; or increasing the device's surface area.

Other materials useful in forming the drug-containing agent, in addition to the agent described herein itself, include HPMC, PEO and PVP and other pharmaceutically acceptable carriers. In addition, osmagents such as sugars or salts, including but not limited to sucrose, lactose, xylitol, mannitol, or sodium chloride, may be added. Materials which are useful for forming the hydrogel layer include sodium CMC, PEO (e.g. polymers having an average molecular weight from about 5,000,000 to about 7,500,000 daltons), poly (acrylic acid), sodium (polyacrylate), sodium croscarmellose, sodium starch glycolat, PVP, crosslinked PVP, and other high molecular weight hydrophilic materials.

In the case of a bilayer geometry, the delivery port(s) or exit passageway(s) may be located on the side of the tablet containing the drug agent or may be on both sides of the tablet or even on the edge of the tablet so as to connect both the drug layer and the sweller layer with the exterior of the device. The exit passageway(s) may be produced by mechanical means or by laser drilling, or by creating a difficult-to-coat region on the tablet by use of special tooling during tablet compression or by other means.

The osmotic device can also be made with a homogeneous core surrounded by a semipermeable membrane coating, as in US3845770. The agent described herein can be incorporated into a tablet core and a semipermeable membrane coating can be applied via conventional tablet-coating techniques such as using a pan coater. A drug delivery passageway can then be formed in this coating by drilling a hole in the coating, either by use of a laser or mechanical means. Alternatively, the passageway may be formed by rupturing a portion of the coating or by creating a region on the tablet that is difficult to coat, as described above. In one embodiment, an osmotic device comprises: (a) a single-layer compressed core comprising: (i) an agent described herein, (ii) a hydroxyethylcellulose, and (iii) an osmagent, wherein the hydroxyethylcellulose is present in the core from about 2.0% to about 35% by weight and the osmagent is present from about 15% to about 70% by weight; (b) a water-permeable layer surrounding the core; and (c) at least one passageway within the water-permeable layer (b) for delivering the drug to a fluid environment surrounding the tablet. In certain embodiments, the device is shaped such that the surface area to volume ratio (of a water-swollen tablet) is greater than 0.6 mm⁻¹ (including, for example, greater than 1.0 mm⁻¹). The passageway connecting the core with the fluid environment can be situated along the tablet band area. In certain embodiments, the shape is an oblong shape where the ratio of the tablet tooling axes, i.e., the major and minor axes which define the shape of the tablet, are between 1.3 and 3 (including, for example, between 1.5 and 2.5). In one embodiment, the combination of the agent described herein and the osmagent have an average ductility from about 100 to about 200 Mpa, an average tensile strength from about 0.8 to about 2.0 Mpa, and an average brittle fracture index less than about 0.2. The single-layer core may optionally include a disintegrant, a bioavailability enhancing additive, and/or a pharmaceutically acceptable excipient, carrier or diluent.

In certain embodiments, entrainment of particles of agents described herein in the extruding fluid during operation of such osmotic device is desirable. For the particles to be well entrained, the agent drug form is dispersed in the fluid before the particles have an opportunity to settle in the tablet core. One means of accomplishing this is by adding a disintegrant that serves to break up the compressed core into its particulate components. Nonlimiting examples of standard disintegrants include materials such as sodium starch glycolate (e.g., Explotab™ CLV), microcrystalline cellulose (e. g., Avicel™), microcrystalline silicified cellulose (e. g., ProSoIv™) and croscarmellose sodium (e. g., Ac-Di-Sol™), and other disintegrants known to those skilled in the art. Depending upon the particular formulation, some disintegrants work better than others. Several disintegrants tend to form gels as they swell with water, thus hindering drug delivery from the device. Non-gelling, non-swelling disintegrants provide a more rapid dispersion of the drug particles within the core as water enters the core. In certain embodiments, non-gelling, non-swelling disintegrants are resins, for example, ion-exchange resins. In one embodiment, the resin is Amberlite™ IRP 88 (available from Rohm and Haas, Philadelphia, PA). When used, the disintegrant is present in amounts ranging from about 50-74% of the core agent.

Water-soluble polymers are added to keep particles of the agent suspended inside the device before they can be delivered through the passageway(s) (e.g., an orifice). High viscosity polymers are useful in preventing settling. However, the polymer in combination with the agent is extruded through the passageway(s) under relatively low pressures. At a given extrusion pressure, the extrusion rate typically slows with increased viscosity. Certain polymers in combination with particles of the agent described herein form high viscosity solutions with water but are still capable of being extruded from the tablets with a relatively low force. In contrast, polymers having a low weight-average, molecular weight (< about 300,000) do not form sufficiently viscous solutions inside the tablet core to allow complete delivery due to particle settling. Settling of the particles is a problem when such devices are prepared with no polymer added, which leads to poor drug delivery unless the tablet is constantly agitated to keep the particles from settling inside the core. Settling is also problematic when the particles are large and/or of high density such that the rate of settling increases.

In certain embodiments, the water-soluble polymers for such osmotic devices do not interact with the drug. In certain embodiments the water-soluble polymer is a non-ionic polymer. A nonlimiting example of a non-ionic polymer forming solutions having a high viscosity yet still extrudable at low pressures is Natrosol™ 250H (high molecular weight hydroxyethylcellulose, available from Hercules Incorporated, Aqualon Division, Wilmington, DE; MW equal to about 1 million daltons and a degree of polymerization equal to about 3,700). Natrosol 250H™ provides effective drug delivery at concentrations as low as about 3% by weight of the core when combined with an osmagent. Natrosol 250H™ NF is a high-viscosity grade nonionic cellulose ether that is soluble in hot or cold water. The viscosity of a 1% solution of Natrosol 250H using a Brookfield LVT (30 rpm) at 25°C is between about 1, 500 and about 2,500 cps. In certain embodiments, hydroxyethylcellulose polymers for use in these monolayer osmotic tablets have a weight-average, molecular weight from about 300,000 to about 1.5 million. The hydroxyethylcellulose polymer is typically present in the core in an amount from about 2.0% to about 35% by weight.

Another example of an osmotic device is an osmotic capsule. The capsule shell or portion of the capsule shell can be semipermeable. The capsule can be filled either by a powder or liquid consisting of an agent described herein, excipients that imbibe water to provide osmotic potential, and/or a water-swellable polymer, or optionally solubilizing excipients. The capsule core can also be made such that it has a bilayer or multilayer agent analogous to the bilayer, trilayer or concentric geometries described above.

Another class of osmotic device useful in this disclosure comprises coated swellable tablets, for example, as described in EP378404. Coated swellable tablets comprise a tablet core comprising an agent described herein and a swelling material, preferably a hydrophilic polymer, coated with a membrane, which contains holes, or pores through which, in the aqueous use environment, the hydrophilic polymer can extrude and carry out the agent. Alternatively, the membrane may contain polymeric or low molecular weight water-soluble porosigens. Porosigens dissolve in the aqueous use environment, providing pores through which the hydrophilic polymer and agent may extrude. Examples of porosigens are water-soluble polymers such as HPMC, PEG, and low molecular weight compounds such as glycerol, sucrose, glucose, and sodium chloride. In addition, pores may be formed in the coating by drilling holes in the coating using a laser or other mechanical means. In this class of osmotic devices, the membrane material may comprise any film-forming polymer, including polymers which are water permeable or impermeable, providing that the membrane deposited on the tablet core is porous or contains water-soluble porosigens or possesses a macroscopic hole for water ingress and drug release. Embodiments of this class of sustained release devices may also be multilayered, as described, for example, in EP378404.

When an agent described herein is a liquid or oil, such as a lipid vehicle formulation, for example as described in WO05/011634, the osmotic controlled-release device may comprise a soft-gel or gelatin capsule formed with a composite wall and comprising the liquid formulation where the wall comprises a barrier layer formed over the external surface of the capsule, an expandable layer formed over the barrier layer, and a semipermeable layer formed over the expandable layer. A delivery port connects the liquid formulation with the aqueous use environment. Such devices are described, for example, in US6419952, US6342249, US5324280, US4672850, US4627850, US4203440, and US3995631.

The osmotic controlled release devices of the present disclosure can also comprise a coating. In certain embodiments, the osmotic controlled release device coating exhibits one or more of the following features: is water-permeable, has at least one port for the delivery of drug, and is non-dissolving and non-eroding during release of the drug formulation, such that drug is substantially entirely delivered through the delivery port(s) or pores as opposed to delivery primarily via permeation through the coating material itself. Delivery ports include any passageway, opening or pore whether made mechanically, by laser drilling, by pore formation either during the coating process or *in situ* during use or by rupture during use. In certain embodiments, the coating is present in an amount ranging from about 5 to 30 wt% (including, for example, 10 to 20 wt%) relative to the core weight.

One form of coating is a semipermeable polymeric membrane that has the port(s) formed therein either prior to or during use. Thickness of such a polymeric membrane may vary between about 20 and 800 µm (including, for example, between about 100 to 500 µm). The diameter of the delivery port (s) may generally range in size from 0.1 to 3000 µm or greater (including, for example, from about 50 to 3000 µm in diameter). Such port(s) may be formed post-coating by mechanical or laser drilling or may be formed *in situ* by rupture of the coatings; such rupture may be controlled by intentionally incorporating a relatively small weak portion into the coating. Delivery ports may also be formed *in situ* by erosion of a plug of water-soluble material or by rupture of a thinner portion of the coating over an indentation in the core. In addition, delivery ports may be formed during coating, as in the case of asymmetric membrane coatings of the type disclosed in US5612059 and US5698220. The delivery port may be formed *in situ* by rupture of the coating, for example, when a collection of beads that may be of essentially identical or of a variable agent are used. Drug is primarily released from such beads following rupture of the coating and, following rupture, such release may be gradual or relatively sudden. When the collection of beads has a variable agent, the agent may be chosen such that the beads rupture at various times following administration, resulting in the overall release of drug being sustained for a desired duration.

Coatings may be dense, microporous or asymmetric, having a denser region supported by a thick porous region such as those disclosed in US5612059 and US5698220. When the coating is dense the coating can be composed of a water-permeable material. When the coating is porous, it may be composed of either a water-permeable or a water-impermeable material. When the coating is composed of a porous water-impermeable material, water permeates through the pores of the coating as either a liquid or a vapor. Nonlimiting examples of osmotic devices that utilize dense coatings include US3995631 and US3845770. Such dense coatings are permeable to the external fluid such as water and may be composed of any of the materials mentioned in these patents as well as other water-permeable polymers known in the art.

The membranes may also be porous as disclosed, for example, in US5654005 and US5458887 or even be formed from water-resistant polymers. US5120548 describes another suitable process for forming coatings from a mixture of a water-insoluble polymer and a leachable water-soluble additive. The porous membranes may also be formed by the addition of pore-formers as disclosed in US4612008. In addition, vapor-permeable coatings may even be formed from extremely hydrophobic materials such as polyethylene or polyvinylidene difluorid that, when dense, are essentially water-impermeable, as long as such coatings are porous. Materials useful in forming the coating include but are not limited to various grades of acrylic, vinyls, ethers, polyamides, polyesters and cellulosic derivatives that are water-permeable and water-insoluble at physiologically relevant pHs, or are susceptible to being rendered water-insoluble by chemical alteration such as by crosslinking. Nonlimiting examples of suitable polymers (or crosslinked versions) useful in forming the coating include plasticized, unplasticized and reinforced cellulose acetate (CA), cellulose diacetate, cellulose triacetate, CA propionate, cellulose nitrate, cellulose acetate butyrate (CAB), CA ethyl carbamate, CAP, CA methyl carbamate, CA succinate, cellulose acetate trimellitate (CAT), CA dimethylaminoacetate, CA ethyl carbonate, CA chloroacetate, CA ethyl oxalate, CA methyl sulfonate, CA butyl sulfonate, CA p-toluene sulfonate, agar acetate, amylose triacetate, beta glucan acetate, beta glucan triacetate, acetaldehyde dimethyl acetate, triacetate of locust bean gum, hydroxiated ethylene-vinylacetate, EC, PEG, PPG, PEG/PPG copolymers, PVP, HEC, HPC, CMC, CMEC, HPMC, HPMCP, HPMCAS, HPMCAT, poly (acrylic) acids and esters and poly- (methacrylic) acids and esters and copolymers thereof, starch, dextran, dextrin, chitosan, collagen, gelatin, polyalkenes, polyethers, polysulfones, polyethersulfones, polystyrenes, polyvinyl halides, polyvinyl esters and ethers, natural waxes and synthetic waxes. In various embodiments, the coating agent comprises a cellulosic polymer, in particular cellulose ethers, cellulose esters and cellulose ester-ethers, i.e., cellulosic derivatives having a mixture of ester and ether substituents, the coating materials are made or derived from poly (acrylic) acids and esters, poly (methacrylic) acids and esters, and copolymers thereof, the coating agent comprises cellulose acetate, the coating comprises a cellulosic polymer and PEG, the coating comprises cellulose acetate and PEG.

Coating is conducted in conventional fashion, typically by dissolving or suspending the coating material in a solvent and then coating by dipping, spray coating or by pan-coating. In certain embodiments, the coating solution contains 5 to 15 wt% polymer. Typical solvents useful with the cellulosic polymers mentioned above include but are not limited to acetone, methyl acetate, ethyl acetate, isopropyl acetate, n-butyl acetate, methyl isobutyl ketone, methyl propyl ketone, ethylene glycol monoethyl ether, ethylene glycol monoethyl acetate, methylene dichloride, ethylene dichloride, propylene dichloride, nitroethane, nitropropane, tetrachloroethane, 1,4-dioxane, tetrahydrofuran, diglyme, water, and mixtures thereof. Pore-formers and non- solvents (such as water, glycerol and ethanol) or plasticizers (such as diethyl phthalate) may also be added in any amount as long as the polymer remains soluble at the spray temperature. Pore-formers and their use in fabricating coatings are described, for example, in US5612059. Coatings may also be hydrophobic microporous layers wherein the pores are substantially filled with a gas and are not wetted by the aqueous medium but are permeable to water vapor, as disclosed, for example, in US5798119. Such hydrophobic but water-vapor permeable coatings are typically composed of hydrophobic polymers such as polyalkenes, polyacrylic acid derivatives, polyethers, polysulfones, polyethersulfones, polystyrenes, polyvinyl halides, polyvinyl esters and ethers, natural waxes and synthetic waxes. Hydrophobic microporous coating materials include but are not limited to polystyrene, polysulfones, polyethersulfones, polyethylene, polypropylene, polyvinyl chloride, polyvinylidene fluoride and polytetrafluoroethylene. Such hydrophobic coatings can be made by known phase inversion methods using any of vapor-quench, liquid quench, thermal processes, leaching soluble material from the coating or by sintering coating particles. In thermal processes, a solution of polymer in a latent solvent is brought to liquid-liquid phase separation in a cooling step. When evaporation of the solvent is not prevented, the resulting membrane will typically be porous. Such coating processes may be conducted by the processes disclosed, for example, in US4247498, US4490431 and US4744906. Osmotic controlled-release devices may be prepared using procedures known in the pharmaceutical arts. See for example, Remington: The Science and Practice of Pharmacy, 20th Edition, 2000.

As further noted above, the agents described herein may be provided in the form of microparticulates, generally ranging in size from about 10µm to about 2mm (including, for example, from about 100µm to 1mm in diameter). Such multiparticulates may be packaged, for example, in a capsule such as a gelatin capsule or a capsule formed from an aqueous-soluble polymer such as HPMCAS, HPMC or starch; dosed as a suspension or slurry in a liquid ; or they may be formed into a tablet, caplet, or pill by compression or other processes known in the art. Such multiparticulates may be made by any known process, such as wet- and dry-granulation processes, extrusion/spheronization, roller-compaction, melt-congealing, or by spray-coating seed cores. For example, in wet-and dry- granulation processes, the agent described herein and optional excipients may be granulated to form multiparticulates of the desired size. Other excipients, such as a binder (e. g., microcrystalline cellulose), may be blended with the agent to aid in processing and forming the multiparticulates. In the case of wet granulation, a binder such as microcrystalline cellulose may be included in the granulation fluid to aid in forming a suitable multiparticulate. See, for example, Remington : The Science and Practice of Pharmacy, 20 Edition, 2000. In any case, the resulting particles may themselves constitute the therapeutic composition or they may be coated by various film-forming materials such as enteric polymers or water-swellable or water-soluble polymers, or they may be combined with other excipients or vehicles to aid in dosing to patients.

Suitable pharmaceutical compositions in accordance with the disclosure will generally include an amount of the active compound(s) with an acceptable pharmaceutical diluent or excipient, such as a sterile aqueous solution, to give a range of final concentrations, depending on the intended use. The techniques of preparation are generally well known in the art, as exemplified by Remington's Pharmaceutical Sciences (18th Edition, Mack Publishing Company, 1995).

### Kits

The agents described herein and combination therapy agents can be packaged as a kit that includes single or multiple doses of two or more agents, each packaged or formulated individually, or single or multiple doses of two or more agents packaged or formulated in combination. Thus, one or more agents can be present in first container, and the kit can optionally include one or more agents in a second container. The container or containers are placed within a package, and the package can optionally include administration or dosage instructions. A kit can include additional components such as syringes or other means for administering the agents as well as diluents or other means for formulation.

Thus, the kits can comprise: a) a pharmaceutical composition comprising an agent described herein and a pharmaceutically acceptable carrier, vehicle or diluent; and b) a container or packaging. The kits may optionally comprise instructions describing a method of using the pharmaceutical compositions in one or more of the methods described herein (e.g. disorders associated with fluid and sodium retention (such as diseases of the electrolyte-water/electrolyte transport system within the kidney, gut and urogenital system, heart failure (e.g. congestive heart failure including heart failure at any of stages I-IV according to New York Heart Association (NYHA) Functional Classification), hypertension, salt dependent forms of high blood pressure, hepatic edema, liver cirrhosis, kidney disease, polycystic kidney disease) and gastrointestinal disorders (e.g. gastrointestinal motility disorders, chronic intestinal pseudo-obstruction, colonic pseudo-obstruction, Crohn's disease, duodenogastric reflux, dyspepsia, functional dyspepsia, nonulcer dyspepsia, a functional gastrointestinal disorder, functional heartburn, gastroesophageal reflux disease (GERD), gastroparesis, irritable bowel syndrome, post-operative ileus, ulcerative colitis, chronic constipation, and disorders and conditions associated with constipation (e.g. constipation associated with use of opiate pain killers, post-surgical constipation, and constipation associated with neuropathic disorders as well as other conditions and disorders described herein)). The kit may optionally comprise a second pharmaceutical composition comprising one or more additional agents including but not limited to those including analgesic agents, an agent used to treat heart failure (Diuretics (e.g. furesomide (Lasix), bumetanide (Bumex), ethacrynic acid (Edecrin), torsemide (Demadex), amiloride (Midamor), spironolactone (Aldactone), canrenone, chorthiazide (Diuril), metolazone (Zaroxylyn)), Angiotension-Converting Enzyme (ACE) inhibitors (e.g. captopril (Capoten), enalopril (Vasotec), lisinopril (Prinivil, Zestril), ramipril (Altace)), Beta blockers (e.g. carvedilol (Coreg) and Inotropes (e.g. digoxin, dobutaimine, dopamine Milrinone)), a phosphodiesterase inhibitor, an agent used to treat gastrointestinal and other disorders (including those described herein), an agent used to treat constipation, an antidiarrheal agent, an insulin or related compound (including those described herein), an anti-hypertensive agent, an agent useful in the treatment of respiratory and other disorders, an anti-obesity agent, an anti-diabetic agents, an agent that activates soluble guanylate cyclase and a pharmaceutically acceptable carrier, vehicle or diluent. The pharmaceutical composition comprising the compound described herein and the second pharmaceutical composition contained in the kit may be optionally combined in the same pharmaceutical composition.

A kit includes a container or packaging for containing the pharmaceutical compositions and may also include divided containers such as a divided bottle or a divided foil packet. The container can be, for example a paper or cardboard box, a glass or plastic bottle or jar, a re-sealable bag (for example, to hold a "refill" of tablets for placement into a different container), or a blister pack with individual doses for pressing out of the pack according to a therapeutic schedule. It is feasible that more than one container can be used together in a single package to market a single dosage form. For example, tablets may be contained in a bottle which is in turn contained within a box.

An example of a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process, recesses are formed in the plastic foil. The recesses have the size and shape of individual tablets or capsules to be packed or may have the size and shape to accommodate multiple tablets and/or capsules to be packed. Next, the tablets or capsules are placed in the recesses accordingly and the sheet of relatively stiff material is sealed against the plastic foil at the face of the foil which is opposite from the direction in which the recesses were formed. As a result, the tablets or capsules are individually sealed or collectively sealed, as desired, in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

It maybe desirable to provide a written memory aid containing information and/or instructions for the physician, pharmacist or subject regarding when the medication is to be taken. A "daily dose" can be a single tablet or capsule or several tablets or capsules to be taken on a given day. When the kit contains separate compositions, a daily dose of one or more compositions of the kit can consist of one tablet or capsule while a daily dose of another one or more compositions of the kit can consist of several tablets or capsules. A kit can take the form of a dispenser designed to dispense the daily doses one at a time in the order of their intended use. The dispenser can be equipped with a memory-aid, so as to further facilitate compliance with the regimen. An example of such a memory-aid is a mechanical counter which indicates the number of daily doses that have been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

Methods to increase chemical and/or physical stability of the agents the described herein are found in U.S. 6,541,606, U.S. 6,068,850, U.S. 6,124,261, U.S. 5,904,935, and WO 00/15224, U.S. 20030069182 (via the additon of nicotinamide), U.S. 20030175230A1, U.S. 20030175230A1, U.S. 20030175239A1, U.S. 20020045582, U.S. 20010031726, WO 02/26248, WO 03/014304, WO 98/00152A1, WO 98/00157A1, WO 90/12029, WO 00/04880, and WO 91/04743, WO 97/04796 and the references cited therein.

Methods to increase bioavailability of the agents described herein are found in U.S. 6,008,187, U.S. 5,424,289, U.S. 20030198619, WO 90/01329, WO 01/49268, WO 00/32172, and WO 02/064166. Glycyrrhizinate can also be used as an absorption enhancer (see, e.g., EP397447). WO 03/004062 discusses Ulex europaeus I (UEA1) and UEAI mimetics which may be used to target the agents described herein to the GI tract. The bioavailability of the agents described herein can also be incrased by addition of oral bioavailability-enhancing agents such as those described in U.S. 6,818,615 including but not limited to: cyclosporins (including cyclosporins A through Z as defined in Table 1 of U.S. 6,818,615), for example, cyclosporin A (cyclosporin), cyclosporin F, cyclosporin D, dihydro cyclosporin A, dihydro cyclosporin C, acetyl cyclosporin A, PSC-833, (Me-Ile-4)-cyclosporin (SDZ-NIM 811) (both from Sandoz Pharmaceutical Corp.), and related oligopeptides produced by species in the genus Topycladium); antifungals including but not limited to ketoconazole; cardiovascular drug including but not limited to MS-209 (BASF), amiodarone, nifedipine, reserpine, quinidine, nicardipine, ethacrynic acid, propafenone, reserpine, amiloride; anti-migraine natural products including but not limited to ergot alkaloids; antibiotics including but not limited to cefoperazone, tetracycline, chloroquine, fosfomycin; antiparasitics including but not limited to ivermectin; multi-drug resistance reversers including but not limited to VX-710 and VX-853 (Vertex Pharmaceutical Incorporated); tyrosine kinase inhibitors including but not limited to genistein and related isoflavonoids, quercetin; protein kinase C inhibitors including but not limited to calphostin; apoptosis inducers including but not limited to ceramides; and agents active against endorphin receptors including but not limited to morphine, morphine congeners, other opioids and opioid antagonists including (but not limited to) naloxone, naltrexone and nalmefene).

The agents described herein can be fused to a modified version of the blood serum protein transferrin. U.S. 20030221201, U.S. 20040023334, U.S. 20030226155, WO 04/020454, and WO 04/019872 discuss the manufacture and use of transferrin fusion proteins. Transferrin fusion proteins may improve circulatory half life and efficacy, decrease undesirable side effects and allow reduced dosage.

The GCC agonist peptides described herein can be recombinantly expressed in bacteria. Bacteria expressing the peptide or agonists can be administered orally, rectally, mucosally or in via some other mode of administration including but not limited to those described herein. Bacterial hosts suitable for such administration include but are not limited to certain *Lactobacteria (e.g. Lactococcus lactis, Lactobacillus plantarum, Lact. rhamnosus and Lact. paracasei ssp. Paracasie* and other species found in normal human flora (Ahrne et al. Journal of Applied Microbiology 1998 85:88)), certain *Streptococcus sp. (e.g. S. gordonii),* and certain *B. subtilis* strains (including pSM539 described in Porzio et al. BMC Biotechnology 2004 4:27). The peptides and agonists described herein can be administered using the Heliobacter based preparation methods described in WO06/015445.

### Dosage

The dose range for adult humans for various drugs is generally from 0.005 mg to 10 g/day orally. Tablets or other forms of presentation provided in discrete units may conveniently contain an amount of compound described herein which is effective at such dosage or as a multiple of the same, for instance, units containing 5 mg to 500 mg, usually around 10 mg to 200 mg. The precise amount of compound administered to a patient will be the responsibility of the attendant physician. However, the dose employed will depend on a number of factors, including the age and sex of the patient, the precise disorder being treated, and its severity.

A dosage unit (e.g. an oral dosage unit) can include from, for example, 1 to 30 µg, 1 to 40 µg, 1 to 50 µg, 1 to 100 µg, 1 to 200 µg, 1 to 300 µg, 1 to 400 µg, 1 to 500 µg, 1 to 600 µg, 1 to 700 µg, 1 to 800 µg, 1 to 900 µg, 1 to 1000 µg, 10 to 30 µg, 10 to 40 µg, 10 to 50 µg, 10 to 100 µg, 10 to 200 µg, 10 to 300 µg, 10 to 400 µg, 10 to 500 µg, 10 to 600 µg, 10 to 700 µg, 10 to 800 µg, 10 to 900 µg, 10 to 1000 µg, 100 to 200 µg, 100 to 300 µg, 100 to 400 µg, 100 to 500 µg, 100 to 600 µg, 100 to 700 µg, 100 to 800 µg, 100 to 900 µg, 100 to 1000 µg, 100 to 1250 µg, 100 to 1500 µg, 100 to 1750 µg, 100 to 2000 µg, 100 to 2250 µg, 100 to 2500 µg, 100 to 2750 µg, 100 to 3000 µg, 200 to 300 µg, 200 to 400 µg, 200 to 500 µg, 200 to 600 µg, 200 to 700 µg, 200 to 800 µg, 200 to 900 µg, 200 to 1000 µg, 200 to 1250 µg, 200 to 1500 µg, 200 to 1750 µg, 200 to 2000 µg, 200 to 2250 µg, 200 to 2500 µg, 200 to 2750 µg, 200 to 3000 µg, 300 to 400 µg, 300 to 500 µg, 300 to 600 µg, 300 to 700 µg, 300 to 800 µg, 300 to 900 µg, 300 to 1000 µg, 300 to 1250 µg, 300 to 1500 µg, 300 to 1750 µg, 300 to 2000 µg, 300 to 2250 µg, 300 to 2500 µg, 300 to 2750 µg, 300 to 3000 µg, 400 to 500 µg, 400 to 600 µg, 400 to 700 µg, 400 to 800 µg, 400 to 900 µg, 400 to 1000 µg, 400 to 1250 µg, 400 to 1500 µg, 400 to 1750 µg, 400 to 2000 µg, 400 to 2250 µg, 400 to 2500 µg, 400 to 2750 µg, 400 to 3000 µg, 500 to 600 µg, 500 to 700 µg, 500 to 800 µg, 500 to 900 µg, 500 to 1000 µg, 500 to 1250 µg, 500 to 1500 µg, 500 to 1750 µg, 500 to 2000 µg, 500 to 2250 µg, 500 to 2500 µg, 500 to 2750 µg, 500 to 3000 µg, 600 to 700 µg, 600 to 800 µg, 600 to 900 µg, 600 to 1000 µg, 600 to 1250 µg, 600 to 1500 µg, 600 to 1750 µg, 600 to 2000 µg, 600 to 2250 µg, 600 to 2500 µg, 600 to 2750 µg, 600 to 3000 µg, 700 to 800 µg, 700 to 900 µg, 700 to 1000 µg, 700 to 1250 µg, 700 to 1500 µg, 700 to 1750 µg, 700 to 2000 µg, 700 to 2250 µg, 700 to 2500 µg, 700 to 2750 µg, 700 to 3000 µg, 800 to 900 µg, 800 to 1000 µg, 800 to 1250 µg, 800 to 1500 µg, 800 to 1750 µg, 800 to 2000 µg, 800 to 2250 µg, 800 to 2500 µg, 800 to 2750 µg, 800 to 3000 µg, 900 to 1000 µg, 900 to 1250 µg, 900 to 1500 µg, 900 to 1750 µg, 900 to 2000 µg, 900 to 2250 µg, 900 to 2500 µg, 900 to 2750 µg, 900 to 3000 µg, 1000 to 1250 µg, 1000 to 1500 µg, 1000 to 1750 µg, 1000 to 2000 µg, 1000 to 2250 µg, 1000 to 2500 µg, 1000 to 2750 µg, 1000 to 3000 µg, 2 to 500 µg, 50 to 500 µg, 3 to 100 µg, 5 to 20 µg, 5 to 100 µg, 10 µg, 20 µg, 30 µg, 40 µg, 50 µg, 60 µg, 70 µg, 75 µg, 80 µg, 90 µg, 100 µg, 150 µg, 200 µg, 250 µg, 300 µg, 350 µg, 400 µg, 450 µg, 500 µg, 550 µg, 600 µg, 650 µg, 700 µg, 750 µg, 800 µg, 850 µg, 900 µg, 950 µg, 1000 µg, 1050 µg, 1100 µg, 1150 µg, 1200 µg, 1250 µg, 1300 µg, 1350 µg, 1400 µg, 1450 µg, 1500 µg, 1550 µg, 1600 µg, 1650 µg, 1700 µg, 1750 µg, 1800 µg, 1850 µg, 1900 µg, 1950 µg, 2000 µg, 2050 µg, 2100 µg, 2150 µg, 2200 µg, 2250 µg, 2300 µg, 2350 µg, 2400 µg, 2450 µg, 2500 µg, 2550 µg, 2600 µg, 2650 µg, 2700 µg, 2750 µg, 2800 µg, 2850 µg, 2900 µg, 2950 µg, 3000 µg, 3250 µg, 3500 µg, 3750 µg, 4000 µg, 4250 µg, 4500 µg, 4750 µg, 5000 µg of a GCC peptide or agonist described herein. In various embodiments, the dosage unit is administered with food at anytime of the day, without food at anytime of the day, with food after an overnight fast (e.g. with breakfast), at bedtime after a low fat snack. In various embodiments, the dosage unit is administered once a day, twice a day, three times a day, four times a day, five times a day, six timesa day. The dosage unit can optionally comprise other agents.

A dosage unit (e.g. an oral dosage unit) can include, for example, from 1 to 30 µg, 1 to 40 µg, 1 to 50 µg, 1 to 100 µg, 1 to 200 µg, 1 to 300 µg, 1 to 400 µg, 1 to 500 µg, 1 to 600 µg, 1 to 700 µg, 1 to 800 µg, 1 to 900 µg, 1 to 1000 µg, 10 to 30 µg, 10 to 40 µg, 10 to 50 µg, 10 to 100 µg, 10 to 200 µg, 10 to 300 µg, 10 to 400 µg, 10 to 500 µg, 10 to 600 µg, 10 to 700 µg, 10 to 800 µg, 10 to 900 µg, 10 to 1000 µg, 100 to 200 µg, 100 to 300 µg, 100 to 400 µg, 100 to 500 µg, 100 to 600 µg, 100 to 700 µg, 100 to 800 µg, 100 to 900 µg, 100 to 1000 µg, 100 to 1250 µg, 100 to 1500 µg, 100 to 1750 µg, 100 to 2000 µg, 100 to 2250 µg, 100 to 2500 µg, 100 to 2750 µg, 100 to 3000 µg, 200 to 300 µg, 200 to 400 µg, 200 to 500 µg, 200 to 600 µg, 200 to 700 µg, 200 to 800 µg, 200 to 900 µg, 200 to 1000 µg, 200 to 1250 µg, 200 to 1500 µg, 200 to 1750 µg, 200 to 2000 µg, 200 to 2250 µg, 200 to 2500 µg, 200 to 2750 µg, 200 to 3000 µg, 300 to 400 µg, 300 to 500 µg, 300 to 600 µg, 300 to 700 µg, 300 to 800 µg, 300 to 900 µg, 300 to 1000 µg, 300 to 1250 µg, 300 to 1500 µg, 300 to 1750 µg, 300 to 2000 µg, 300 to 2250 µg, 300 to 2500 µg, 300 to 2750 µg, 300 to 3000 µg, 400 to 500 µg, 400 to 600 µg, 400 to 700 µg, 400 to 800 µg, 400 to 900 µg, 400 to 1000 µg, 400 to 1250 µg, 400 to 1500 µg, 400 to 1750 µg, 400 to 2000 µg, 400 to 2250 µg, 400 to 2500 µg, 400 to 2750 µg, 400 to 3000 µg, 500 to 600 µg, 500 to 700 µg, 500 to 800 µg, 500 to 900 µg, 500 to 1000 µg, 500 to 1250 µg, 500 to 1500 µg, 500 to 1750 µg, 500 to 2000 µg, 500 to 2250 µg, 500 to 2500 µg, 500 to 2750 µg, 500 to 3000 µg, 600 to 700 µg, 600 to 800 µg, 600 to 900 µg, 600 to 1000 µg, 600 to 1250 µg, 600 to 1500 µg, 600 to 1750 µg, 600 to 2000 µg, 600 to 2250 µg, 600 to 2500 µg, 600 to 2750 µg, 600 to 3000 µg, 700 to 800 µg, 700 to 900 µg, 700 to 1000 µg, 700 to 1250 µg, 700 to 1500 µg, 700 to 1750 µg, 700 to 2000 µg, 700 to 2250 µg, 700 to 2500 µg, 700 to 2750 µg, 700 to 3000 µg, 800 to 900 µg, 800 to 1000 µg, 800 to 1250 µg, 800 to 1500 µg, 800 to 1750 µg, 800 to 2000 µg, 800 to 2250 µg, 800 to 2500 µg, 800 to 2750 µg, 800 to 3000 µg, 900 to 1000 µg, 900 to 1250 µg, 900 to 1500 µg, 900 to 1750 µg, 900 to 2000 µg, 900 to 2250 µg, 900 to 2500 µg, 900 to 2750 µg, 900 to 3000 µg, 1000 to 1250 µg, 1000 to 1500 µg, 1000 to 1750 µg, 1000 to 2000 µg, 1000 to 2250 µg, 1000 to 2500 µg, 1000 to 2750 µg, 1000 to 3000 µg, 2 to 500 µg, 50 to 500 µg, 3 to 100 µg, 5 to 20 µg, 5 to 100 µg, 10 µg, 20 µg, 30 µg, 40 µg, 50 µg, 60 µg, 70 µg, 75 µg, 80 µg, 90 µg, 100 µg, 150 µg, 200 µg, 250 µg, 300 µg, 350 µg, 400 µg, 450 µg, 500 µg, 550 µg, 600 µg, 650 µg, 700 µg, 750 µg, 800 µg, 850 µg, 900 µg, 950 µg, 1000 µg, 1050 µg, 1100 µg, 1150 µg, 1200 µg, 1250 µg, 1300 µg, 1350 µg, 1400 µg, 1450 µg, 1500 µg, 1550 µg, 1600 µg, 1650 µg, 1700 µg, 1750 µg, 1800 µg, 1850 µg, 1900 µg, 1950 µg, 2000 µg, 2050 µg, 2100 µg, 2150 µg, 2200 µg, 2250 µg, 2300 µg, 2350 µg, 2400 µg, 2450 µg, 2500 µg, 2550 µg, 2600 µg, 2650 µg, 2700 µg, 2750 µg, 2800 µg, 2850 µg, 2900 µg, 2950 µg, 3000 µg, 3250 µg, 3500 µg, 3750 µg, 4000 µg, 4250 µg, 4500 µg, 4750 µg, 5000 µg of an agent that reduces sodium absorption in the intestine or increases anion secretion..

The precise amount of each of the two or more active ingredients in a dosage unit will depend on the desired dosage of each component. Thus, it can be useful to create a dosage unit that will, when administered according to a particular dosage schedule (e.g., a dosage schedule specifying a certain number of units and a particular timing for administration), deliver the same dosage of each component as would be administered if the patient was being treated with only a single component. In other circumstances, it might be desirable to create a dosage unit that will deliver a dosage of one or more components that is less than that which would be administered if the patient was being treated only with a single component. Finally, it might be desirable to create a dosage unit that will deliver a dosage of one or more components that is greater than that which would be administered if the patient was being treated only with a single component. The pharmaceutical composition can include additional ingredients including but not limited to the excipients described herein. In certain embodiments, one or more therapeutic agents of the dosage unit may exist in an extended or control release formulation and additional therapeutic agents may not exist in extended release formulation. For example, an agent described herein may exist in a controlled release formulation or extended release formulation in the same dosage unit with another agent that may or may not be in either a controlled release or extended release formulation. Thus, in certain embodiments, it may be desirable to provide for the immediate release of one or more of the agents described herein, and the controlled release of one or more other agents.

In certain embodiments the dosage unit and daily dose are equivalent. In certain embodiments the dosage unit and the daily dose are not equivalent. In various embodiments, the dosage unit is administered twenty minutes prior to food consumption, twenty minutes after food consumption, with food at anytime of the day, without food at anytime of the day, with food after an overnight fast (e.g. with breakfast), at bedtime after a low fat snack. In various embodiments, the dosage unit is administered once a day, twice a day, three times a day, four times a day, five times a day, six times a day.

When two or more active ingredients are combined in single dosage form, chemical interactions between the active ingredients may occur. For example, acidic and basic active ingredients can react with each other and acidic active ingredients can facilitate the degradation of acid labile substances. Thus, in certain dosage forms, acidic and basic substances can be physically separated as two distinct or isolated layers in a compressed tablet, or in the core and shell of a press-coated tablet. Additional agents that are compatible with acidic as well as basic substances, have the flexibility of being placed in either layer. In certain multiple layer compositions at least one active ingredient can be enteric-coated. In certain embodiments thereof at least one active ingredient can be presented in a controlled release form. In certain embodiments where a combination of three or more active substances are used, they can be presented as physically isolated segments of a compressed mutlilayer tablet, which can be optionally film coated.

The therapeutic combinations described herein can be formulated as a tablet or capsule comprising a plurality of beads, granules, or pellets. All active ingredients including the vitamins of the combination are formulated into granules or beads or pellets that are further coated with a protective coat, an enteric coat, or a film coat to avoid the possible chemical interactions. Granulation and coating of granules or beads is done using techniques well known to a person skilled in the art. At least one active ingredient can present in a controlled release form. Finally these coated granules or beads are filled into hard gelatin capsules or compressed to form tablets.

The therapeutic combinations described herein can be formulated as a capsule comprising microtablets or minitablets of all active ingredients. Microtablets of the individual agents can be prepared using well known pharmaceutical procedures of tablet making like direct compression, dry granulation or wet granulation. Individual microtablets can be filled into hard gelatin capsules. A final dosage form may comprise one or more microtablets of each individual component. The microtablets may be film coated or enteric coated.

The therapeutic combinations described herein can be formulated as a capsule comprising one or more microtablets and powder, or one or more microtablets and granules or beads. In order to avoid interactions between drugs, some active ingredients of a said combination can be formulated as microtablets and the others filled into capsules as a powder, granules, or beads. The microtablets may be film coated or enteric coated. At least one active ingredient can be presented in controlled release form.

The therapeutic combinations described herein can be formulated wherein the active ingredients are distributed in the inner and outer phase of tablets. In an attempt to divide chemically incompatible components of proposed combination, few interacting components are converted in granules or beads using well known pharmaceutical procedures in prior art. The prepared granules or beads (inner phase) are then mixed with outer phase comprising the remaining active ingredients and at least one pharmaceutically acceptable excipient. The mixture thus comprising inner and outer phase is compressed into tablets or molded into tablets. The granules or beads can be controlled release or immediate release beads or granules, and can further be coated using an enteric polymer in an aqueous or non-aqueous system, using methods and materials that are known in the art.

The therapeutic combinations described herein can be formulated as single dosage unit comprising suitable buffering agent. All powdered ingredients of said combination are mixed and a suitable quantity of one or more buffering agents is added to the blend to minimize possible interactions.

The agents described herein, alone or in combination, can be combined with any pharmaceutically acceptable carrier or medium. Thus, they can be combined with materials that do not produce an adverse, allergic or otherwise unwanted reaction when administered to a patient. The carriers or mediums used can include solvents, dispersants, coatings, absorption promoting agents, controlled release agents, and one or more inert excipients (which include starches, polyols, granulating agents, microcrystalline cellulose, diluents, lubricants, binders, disintegrating agents, and the like), etc. If desired, tablet dosages of the disclosed compositions may be coated by standard aqueous or nonaqueous techniques.

When provided as a single dosage form, the potential exists for a chemical interaction between the combined active ingredients (for example, an lipid lowering agents and a GC-C agonist). For this reason, the preferred dosage forms of the combination products of this disclosure are formulated such that although the active ingredients are combined in a single dosage form, the physical contact between the active ingredients is minimized (that is, reduced).

In order to minimize contact, one embodiment of this disclosure where the product is orally administered provides for a combination product wherein one active ingredient is enteric coated. By enteric coating one or more of the active ingredients, it is possible not only to minimize the contact between the combined active ingredients, but also, it is possible to control the release of one of these components in the gastrointestinal tract such that one of these components is not released in the stomach but rather is released in the intestines. Another embodiment of this disclosure where oral administration is desired provides for a combination product wherein one of the active ingredients is coated with a sustained-release material which effects a sustained-release throughout the gastrointestinal tract and also serves to minimize physical contact between the combined active ingredients. Furthermore, the sustained-released component can be additionally enteric coated such that the release of this component occurs only in the intestine. Still another approach would involve the formulation of a combination product in which the one component is coated with a sustained and/or enteric release polymer, and the other component is also coated with a polymer such as a low-viscosity grade of hydroxypropyl methylcellulose (HPMC) or other appropriate materials as known in the art, in order to further separate the active components. The polymer coating serves to form an additional barrier to interaction with the other component.

Dosage forms of the combination products include those wherein one active ingredient is enteric coated can be in the form of tablets such that the enteric coated component and the other active ingredient are blended together and then compressed into a tablet or such that the enteric coated component is compressed into one tablet layer and the other active ingredient is compressed into an additional layer. Optionally, in order to further separate the two layers, one or more placebo layers may be present such that the placebo layer is between the layers of active ingredients. In addition, dosage forms of the present disclosure can be in the form of capsules wherein one active ingredient is compressed into a tablet or in the form of a plurality of microtablets, particles, granules or non-perils, which are then enteric coated. These enteric coated microtablets, particles, granules or non-perils are then placed into a capsule or compressed into a capsule along with a granulation of the other active ingredient.

These as well as other ways of minimizing contact between the components of combination products of the present disclosure, whether administered in a single dosage form or administered in separate forms but at the same time by the same manner, will be readily apparent to those skilled in the art in light of the present disclosure.

Preferred embodiments of the present invention are described below and are referred to as embodiments E1 to E355.
E1. A method of reducing the risk of or treating a disorder associated with fluid and/or salt retention in a patient, the method comprising administering to the patient an agent selected from: a) an agent that reduces sodium absorption in the intestine; b) an agent that increases anion secretion in the intestine; or c) an agent that both reduces sodium absorption in the intestine and increases anion secretion in the intestine.
E2. The method of E1 wherein the agent reduces sodium absorption in the intestine.
E3. The method of E1 wherein the agent increases anion secretion in the intestine.
E4. The method of E1 wherein the agent both reduces sodium absorption in the intestine and increases anion secretion in the intestine.
E5. The method of E1 wherein the agent is selected from: a) a guanylate cyclase receptor C agonist, b) a soluble guanylate cyclase modulator, c) a prostanoid, d) a chloride channel activator, e) a 5HT4 agonist, f) a cyclic nucleotide, g) a sodium transport inhibitor, h) a laxative, i) a cystic fibrosis transmembrane conductance regulator (CTFR) modulator, j) an agent that affects cAMP levels, k) a phosphodiesterase inhibitor, 1) a renin inhibitor, m) an aldosterone antagonist, n) potassium, and o) a polymer resin.
E6. The method of E5 wherein the agent is a guanylate cyclase receptor C agonist.
E7. The method of E5 wherein the agent is a soluble guanylate cyclase modulator.
E8. The method of E5 wherein the agent is a prostanoid.
E9. The method of E5 wherein the agent is a chloride channel activator.
E10. The method of E9 wherein the chloride channel activator is lubiprostone.
E11. The method of E5 wherein the agent is a 5HT4 agonist.
E12. The method of E5 wherein the agent is a cyclic nucleotide.
E13. The method of E5 wherein the agent is a sodium transport inhibitor.
E14. The method of E13 wherein the sodium transport inhibitor is amiloride.
E15. The method of E13 wherein the sodium transport inhibitor is an NHE3 inhibitor.
E16. The method of E5 wherein the agent is a laxative.
E17. The method of E5 wherein the agent is a cystic fibrosis transmembrane conductance regulator (CTFR) modulator.
E18. The method of E5 wherein the agent is an agent that affects cAMP level.
E19. The method of E5 wherein the agent is a phosphodiesterase inhibitor.
E20. The method of E5 wherein the agent is a renin inhibitor.
E21. The method of E5 wherein the agent is an aldosterone antagonist.
E22. The method of E5 wherein the agent is potassium.
E23. The method of E5 wherein the agent is a polymer resin.
E24. The method of E11 wherein the 5HT4 agonist is Zelnorm.
E25. The method of E8 wherein the prostanoid is selected from: the compound represented by CAS Registry No. 333963-40-9, the compound represented by CAS Registry No. 136790-76-6, (-)-7-[(2R,4aR,5R,7aR)-2-(1,1-difluoropentyl)-2-hydroxy-6-oxooctahydrocyclopenta[b]pyran-5-yl]heptanoic acid; and the 13, 14-dihydro-15-keto prostaglandins E disclosed in US5284858 including 13,14-dihydro-15-keto-PGE₂ alkyl ester, 13,14-dihydro-15-keto-PGE₂ cycloalkyl ester; 13,14-dihydro-15-keto-PGE₂ hydroxy alkyl ester, 13,14-dihydro-15-keto-PGE₂ benzyl ester, 13,14-dihydro-15-keto-PGE₁ alkyl ester, 13,14-dihydro-6,15-diketo-PGE₁ alkyl ester, 13,14-dihydro-15-keto-18-methoxy-19, 20-dinor-PGE₂ or an alkyl ester thereof, 13,14-dihydro-15-keto-18-methoxy-PGE₂ or an alkyl ester thereof, 13,14-dihydro-15-keto-Δ²-PGE₂ or an alkyl ester thereof, 13,14-dihydro-15-keto-20-methoxy-Δ²-PGE₂ or an alkyl ester thereof, 13,14-dihydro-15-keto-3R,S-methyl-PGE₂ or an alkyl ester thereof, 13,14-dihydro-15-keto-3R,S-methyl-20-methoxy-PGE₂ or an alkyl ester thereof, 13, 14-dihydro-15-keto-11-dehydroxy-11R-methyl-PGE₂ or an alkyl ester thereof, 13,14-dihydro-15-keto-16R,S-fluoro-11-dehydroxy-11R-methyl-PGE₂ or an alkyl ester thereof, 13,14-dihydro-15-keto-16R,S-hydroxy-PGE₂ or an alkyl ester thereof, 13,14-dihydro-15-keto-16R,S-fluoro-PGE₂ or an alkyl ester thereof, 13,14-dihydro-15-keto-16R,S-methyl-PGE₂ or an alkyl ester thereof, 13,14-dihydro-15-keto-16,16-dimethyl-PGE₂ or an alkyl ester thereof, 13,14-dihydro-15-keto-16,16-dimethyl-20-methoxy-PGE₂ or an alkyl ester thereof, 13,14-dihydro-15-keto-17S-methyl-PGE₂ or an alkyl ester thereof, 13,14-dihydro-15-keto-19-methy-PGE₂ or an alkyl ester thereof, 13,14-dihydro-15-keto-20-isopropropylidene PGE₂ or an alkyl ester thereof, 13,14-dihydro-15-keto-20-ethyl-PGE₂ or an alkyl ester thereof, 13,14-dihydro-15-keto-20-ethyl-11-dehydroxy-11R-methyl-PGE₂ or an alkyl ester thereof, 13,14-dihydro-15-keto-20-n-propyl-PGE₂ or an alkyl ester thereof, 13,14-dihydro-15-keto-20-ethyl-PGE₁ or an alkyl ester thereof, 13,14-dihydro-6,15-diketo-16R,S-fluoro-PGE₁ or an alkyl ester thereof, 13,14-dihydro-6,15-diketo-16R,S-fluoro-11-dehydroxy-11R-methyl-PGE₁ or an alkyl ester thereof, 13,14-dihydro-6,15-diketo-16R,S-methyl-PGE₁ or an alkyl ester thereof, 13,14-dihydro-6,15-diketo-16,16-dimethyl-PGE₁ or an alkyl ester thereof, 13,14-dihydro-6,15-diketo-19-methyl-PGE₁ or an alkyl ester thereof, 13,14-dihydro-6,15-diketo-20-methyl-PGE₁ or an alkyl ester thereof, 13,14-dihydro-6,15-diketo-11-dehydroxy-11R-methyl-PGE₁ or an alkyl ester thereof, 13, 14-dihydro-6,15-diketo-11-dehydroxy-11R-hydroxymethyl PGE₁ alkyl ester, 13,14-dihydro-15-keto-20-methyl-PGE₁ or an alkyl ester thereof, 13,14-dihydro-15-keto-Δ²-PGE or an alkyl ester thereof, 13,14-dihydro-15-keto-16R,S-fluoro-20-methyl-PGE₂ or an alkyl ester thereof, 13,14-dihydro-15-keto-16,16-difluoro-PGE₂ or an alkyl ester thereof, 13,14-dihydro-15-keto-5,6-dehydro-20-methoxy-PGE₂ or an alkyl ester thereof, and 13,14-dihydro-6,15-diketo-16R,S-fluoro-PGE₁ or an alkyl ester thereof.
E26. The method of E8 wherein the prostanoid is misoprostol.
E27. The method of E8 wherein the prostanoid is the free acid of the compound associated with CAS registry NO. 59122-49-5
E28. The method of E26 wherein the prostanoid comprises a mixture of steroisomers of misoprostol.
E29. The method of E8 or 26 wherein only a single isomer of a prostanoid is administered.
E30. The method of E16 wherein the laxative is selected from: a CCK-1 antagonist, a stimulant, a bulk-producing agent and a stool softener.
E31. The method of E30 wherein the laxative is selected from dexloxiglumide, psyllium husk, docusate sodium, bisacodyl, and phenolphthalein.
E32. The method of E23 wherein the polymer resin is selected from psyllium, lipid lowering polymers, nonabsorbed polymer resins, and sodium binding polymers.
E33. The method of E32 wherein the lipid lowering polymer is selected from: Colesevelam, Sevalmer, or Cholestyramine.
E34. The method of E32 wherein the nonabsorbed polymer resin is selected from: hyaluronic acid, polycarbophil calcium, polyvinyl acetate, polyvinyl pyrrolidone, polystyrene sulfate.
E35. The method of E32 wherein the sodium-binding polymer is selected from the group consisting of: crosslinked polyvinylsulfamate polymer, N-(bis-phosphonic-ethyl) polyvinylamine polymer, poly-α-acrylic acid polymer, poly-α-fluoroacrylic acid polymer, polyvinylphosphoramidic polymer, polyvinylsulfamate polymer, polyvinylsulfamate/vinylsulfate copolymer, polyvinylsulfate polymer, polyvinylsulfonate polymer, polyvinylsulfonate polymer, vinylphosphonate/α-fluoroacrylic acid copolymer, vinylphosphonate/α-fluoroacrylic acid copolymer, and vinylphosphonate/acrylic acid copolymer.
E36. The method of E32 wherein the sodium-binding polymer is administered as a core-shell composition which further comprises a semi-permeable shell.
E37. The method of E36 wherein the semi-permeable shell comprises at least one of a poly-11 trimethylammonioundecylmethacrylate polymer, a styrene-vinylpyridine polymer, 11-dimethyl-aminodecylmethacrylate/laurylmethacrylate copolymer, or a polyallylamine/polystyrene sulfonate polymer.
E38. The method of any of E5-37 further comprising administering an anti-diabetic agent.
E39. The method of any of E5-37 further comprising administering an anti-obesity agent.
E40. The method of any of E5-37 further comprising administering potassium or a salt thereof.
E41. The method of any of E5-37 further comprising administering a PDE inhibitor.
E42. The method of E41 wherein the PDE inhibitor is a PDE5-specific PDE inhibitor.
E43. The method of E41 wherein the PDE inhibitor is a cGMP-specific PDE inhibitor.
E44. The method of E41 wherein the PDE inhibitor is a cAMP-specific PDE inhibitor.
E45. The method of any of E5-37 further comprising administering polymer resin.
E46. The method of E45 wherein the polymer resin is psyllium.
E47. The method of E45 wherein the polymer resin is a nonabsorbed polymer resin.
E48. The method of E47 wherein the nonabsorbed polymer resin is selected from hyaluronic acid, polycarbophil calcium, polyvinyl acetate, and polyvinyl pyrrolidine.
E49. The method of E45 wherein the polymer resin is a lipid lowering polymer.
E50. The method of E49 wherein the lipid lowering polymer is selected from: cholestyramine, colesevelam and sevalmer.
E51. The method of any of E5-37 further comprising administering an anti-hypertensive agent.
E52. The method of E51 wherein the antihypertensive agent is selected from: a diuretic, an inhibitor of angiotensin converting enzyme, an angiotensin II receptor antagonist, a calcium channel blocker, a beta-adrenergic antagonist, alpha-adrenergic antagonist, a renin inhibitor, and an aldosterone antagonist.
E53. The method of E51 wherein the method comprises administering two or more anti-hypertensive agents wherein the two or more antihypertensive agents are independently selected from: a diuretic, an inhibitor of angiotensin converting enzyme, an angiotensin II receptor antagonist, a calcium channel blocker, a beta-adrenergic antagonist, alpha-adrenergic antagonist, a renin inhibitor, and an aldosterone antagonist.
E54. The method of E51 wherein the anti-hypertensive agent is a diuretic.
E55. The method of E54 wherein the diuretic is selected from the group consisting of: a loop diuretic, a thiazide, a potassium sparing agent, and an osmotic diuretic.
E56. The method of E54 wherein the diuretic is a loop diuretic.
E57. The method of E54 wherein the diuretic is furosemide, bumetanide, ethacrynic or torsemide.
E58. The method of E54 wherein the diuretic is a thiazide.
E59. The method of E58 wherein the thiazide is bendroflumethiazide, hydrochlorothiazide, indapamide, chlortalidone or metolazone.
E60. The method of E54 wherein the diuretic is a potassium sparing agent.
E61. The method of E60 wherein the potassium sparing agent is amiloride or triamterene.
E62. The method of E54 wherein the diuretic is an osmotic diuretic.
E63. The method of E62 wherein the osmotic diuretic is glucose or mannitol.
E64. The method of E51 wherein the antihypertensive agent is an angiotensin converting enzyme inhibitor.
E65. The method of E64 wherein the angiotensin converting enzyme inhibitor is selected from: Benazepril (Lotensin), Captopril (Capoten), Enalapril/Enalaprilat (Vasotec), Fosinopril (Monopril), Lisinopril (Zestril and Prinivil), Moexipril (Univasc), Perindopril (Aceon), Quinapril (Accupril), Ramipril (Altace), and Trandolapril (Mavik).
E66. The method of E51 wherein the antihypertensive agent is an angiotensin II receptor antagonist.
E67. The method of E66 wherein the angiotensin II receptor antagonist is selected from: Candesartan, Eprosartan, Irbesartan, Losartan, Olmesartan, Telmisartan, and Valsartan.
E68. The method of E51 wherein the antihypertensive agent is a calcium channel blocker.
E69. The method of E68 wherein the calcium channel blocker is selected from: Amlodipine (Norvasc), Felodipine (Plendil), Nicardipine (Cardene), Nifedipine (Procardia, Adalat), Nimodipine (Nimotop), Nisoldipine (Sular), Nitrendipine (Cardif, Nitrepin), and Lacidipine (Motens), Lercanidipine (Zanidip), Verapamil (Calan, Isoptin), Gallopamil (D600), Diltiazem (Cardizem), and Menthol (mint oil).
E70. The method of E69 wherein the calcium channel blocker is Amlodipine.
E71. The method of E51 wherein the antihypertensive agent is a beta-adrenergic antagonist.
E72. The method of E71 wherein the beta-adrenergic antagonist is selected from: Dichloroisoprenaline, Practolol, Pronethaolol, Alprenolol, Carteolol, Levobunolol, Mepindolol, Metipranolol, Nadolol, Oxprenolol, Penbutolol, Pindolol, Propranolol, Sotalol, Timolol, Acebutolol, Atenolol, Betaxolol, Bisoprolol, Esmolol Metoprolol, Nebivolol, Carvedilol, Celiprolol, Labetalol, and Butoxamine.
E73. The method of E51 wherein the antihypertensive agent is an alpha-adrenergic antagonist.
E74. The method of E73 wherein the alpha-adrenergic antagonist is selected from: Doxazosin (Cardura), Prazosin (Minipress), Phenoxybenzamine, Phentolamine (Regitine), Tamsulosin (Flomaxtra/Flomax), Alfuzosin (Uroxatral), and Terazosin (Hytrin).
E75. The method of E51 wherein the antihypertensive agent is a renin inhibitor.
E76. The method of E75 wherein the renin inhibitor is selected from: Tekturna® (Rasilez and Aliskiren) and SPP635.
E77. The method of E51 wherein the antihypertensive agent is an aldosterone antagonist.
E78. The method of E77 wherein the aldosterone antagonist is Spironolactone, Canrenone, or Eplerenone.
E79. The method of any of E5-37 further comprising administering a lipid altering agent.
E80. The method of E79 wherein the lipid altering agent is a cholesterol lowering agent.
E81. The method of E80 wherein the agent lowers low density cholesterol.
E82. The method of E79 wherein the lipid altering agent is selected from the group consisting of: a statin; a fibrate; niacin; a CETP inhibitor; a MTP inhibitor; a cholesterol absorption inhibitor; a squalene synthesis inhibitor; and a bile acid sequestrant.
E83. The method of E82 wherein the lipid altering agent is a statin.
E84. The method of E83 wherein the statin is chosen from simvastatin, rosuvastatin and atorvastatin.
E85. The method of E79 wherein the lipid altering agent is a cholesterol absorption inhibitor.
E86. The method of E85 wherein the cholesterol absorption inhibitor is ezetimibe.
E87. The method of E79 wherein the lipid altering agent is a bile acid sequestrant.
E88. The method of E87 wherein the bile acid sequestrant is chosen from cholestyramine, colesevelam and colestipol.
E89. The method of E79 wherein the lipid altering agent is a fibrate.
E90. The method of E89 wherein the fibrate is fenofibrate.
E91. The method of E1 comprising administering misoprostol and psyllium.
E92. The method of E1 comprising administering methyl 7-[(1R,2R,3R)-3-hydroxy-2-[(E,4S)-4-hydroxy-4-methyloct-1-enyl]-5-oxocyclopentyl]heptanoate.
E93 The method of E1 comprising administering psyllium and a peptide that activates the guanylate cyclase C receptor.
E94. The method of E93 wherein the peptide is selected from:
   Cys Cys Glu Tyr Cys Cys Asn Pro Ala Cys Thr Gly Cys Tyr;
   Cys Cys Glu Leu Cys Cys Asn Pro Ala Cys Thr Gly Cys Tyr;
   Cys Cys Glu Phe Cys Cys Asn Pro Ala Cys Thr Gly Cys Tyr;
   Cys Cys Glu Trp Cys Cys Asn Pro Ala Cys Thr Gly Cys Tyr;
   Cys Cys Glu Tyr Cys Cys Asn Pro Ala Cys Thr Gly Cys;
   Cys Cys Glu Leu Cys Cys Asn Pro Ala Cys Thr Gly Cys;
   Cys Cys Glu Phe Cys Cys Asn Pro Ala Cys Thr Gly Cys;
   Cys Cys Glu Trp Cys Cys Asn Pro Ala Cys Thr Gly Cys;
   d-Cys Cys Glu Tyr Cys Cys Asn Pro Ala Cys Thr Gly Cys Tyr;
   d-Cys Cys Glu Leu Cys Cys Asn Pro Ala Cys Thr Gly Cys Tyr;
   d-Cys Cys Glu Phe Cys Cys Asn Pro Ala Cys Thr Gly Cys Tyr;
   d-Cys Cys Glu Trp Cys Cys Asn Pro Ala Cys Thr Gly Cys Tyr;
   d-Cys Cys Glu Tyr Cys Cys Asn Pro Ala Cys Thr Gly Cys;
   d-Cys Cys Glu Leu Cys Cys Asn Pro Ala Cys Thr Gly Cys;
   d-Cys Cys Glu Phe Cys Cys Asn Pro Ala Cys Thr Gly Cys;
   d-Cys Cys Glu Trp Cys Cys Asn Pro Ala Cys Thr Gly Cys;
   Asn Asp Asp Cys Glu Leu Cys Val Asn Val Ala Cys Thr Gly Cys Leu;
   Asn Asp Glu Cys Glu Leu Cys Val Asn Val Ala Cys Thr Gly Cys Leu;

Phe Lys Thr Leu Arg Thr Ile Ala Asn Asp Asp Cys Glu Leu Cys Val Asn Val Ala Cys Thr Gly
Cys Leu;

Phe Lys Thr Leu Arg Thr Ile Ala Asn Asp Glu Cys Glu Leu Cys Val Asn Val Ala Cys Thr Gly
Cys Leu;

   Val Tyr Ile Gln Tyr Gln Gly Phe Arg Val Gln Leu Glu Ser Met Lys Lys Leu Ser Asp Leu Glu

Ala Gln Trp Ala Pro; Ser Pro Arg Leu Gln Ala Gln Ser Leu Leu Pro Ala Val Cys His His Pro
Ala Leu Pro Gln Asp Leu Gln Pro Val Cys;

Ala Ser Gln Glu Ala Ser Ser Ile Phe Lys Thr Leu Arg Thr Ile Ala Asn Asp Asp Cys Glu Leu Cys
Val Asn Val Ala Cys Thr Gly Cys Leu;

Val Tyr Ile Gln Tyr Gln Gly Phe Arg Val Gln Leu Glu Ser Met Lys Lys Leu Ser Asp Leu Glu
Ala Gln Trp Ala Pro; Ser Pro Arg Leu Gln Ala Gln Ser Leu Leu Pro Ala Val Cys His His Pro
Ala Leu Pro Gln Asp Leu Gln Pro Val Cys; Ala Ser Gln Glu Ala Ser Ser Ile Phe Lys Thr Leu
Arg Thr Ile Ala Asn Asp Glu Cys Glu Leu Cys Val Asn Val Ala Cys Thr Gly Cys Leu;

   Pro Gly Thr Cys Glu Ile Cys Ala Tyr Ala Ala Cys Thr Gly Cys;

Val Thr Val Gln Asp Gly Asn Phe Ser Phe Ser Leu Glu Ser Val Lys Lys Leu Lys Asp Leu Gln
Glu Pro Gln Glu Pro;

Arg Val Gly Lys Leu Arg Asn Phe Ala Pro Ile Pro Gly Glu Pro Val Val Pro Ile Leu Cys Ser
Asn Pro Asn Phe Pro; Glu Glu Leu Lys Pro Leu Cys Lys Glu Pro Asn Ala Gln Glu Ile Leu Gln
Arg Leu Glu Glu Ile Ala Glu Asp Pro Gly Thr Cys Glu Ile Cys Ala Tyr Ala Ala Cys Thr Gly
Cys;

Met Pro Ser Thr Gln Tyr Ile Arg Arg Pro Ala Ser Ser Tyr Ala Ser Cys Ile Trp Cys Thr Thr Ala
Cys Ala Ser Cys His Gly Arg Thr Thr Lys Pro Ser Leu Ala Thr;

   Ala Asp Leu Cys Glu Ile Cys Ala Phe Ala Ala Cys Thr Gly Cys Leu;

Val Gln Val Glu Glu Gly Gly Phe Ser Phe Pro Leu Asp Ala Val Lys Lys Leu Glu Glu Leu Met
Gly Val Asp Met; Thr Val Lys Gln Ser Pro Arg Leu Ala Lys Thr Ser Thr Thr Ala Val Cys Thr
Asn Pro Asp Leu Pro Ala Val Phe Leu; Pro Leu Cys Lys Ser Lys Gly Ala Ala Asn Ser Phe Phe
Arg Leu Gly Phe Val Ala Ala Arg Ala Asp Leu Cys Glu Ile Cys Ala Phe Ala Ala Cys Thr Gly
Cys Leu;

   Gln Glu Glu Cys Glu Leu Cys Ile Asn Met Ala Cys Thr Gly Tyr; 
Val Tyr Ile Gln Tyr Glu Gly Phe Gln Val Asn Leu Asp Ser Val Lys Lys Leu Asp Lys Leu Leu
Glu Gln Leu Arg Gly;
 
Phe His His Gln Met Gly Asp Gln Arg Asp Pro Ser Ile Leu Cys Ser Asp Pro Ala Leu Pro Ser
Asp Leu Gln Pro Val; and
 
Cys Glu Asn Ser Gln Ala Val Asn Ile Phe Arg Ala Leu Arg Tyr Ile Asn Gln Glu Glu Cys Glu
Leu Cys Ile Asn Met Ala Cys Thr Gly Tyr
E95. The method of E1 wherein the disorder is associated with fluid retention.
E96. The method of E1 wherein the disorder is associated with salt retention.
E97. The method of any of E1-94 wherein the disorder is a cardiovascular disorder.
E98. The method of E97 wherein the cardiovascular disorder is cardiomyopathy.
E99. The method of E98 wherein the cardiomyopathy is associated with Chagas disease.
E100. The method of E97 wherein the cardiovascular disorder is hypertension.
E101. The method of E 100 wherein the hypertension is salt-sensitive hypertension.
E102. The method of E97 wherein the cardiovascular disorder is congestive heart failure.
E103. The method of E97 wherein the cardiovascular disorder is cardiac hypertrophy.
E104. The method of E97 wherein the cardiovascular disorder is a heart attack.
E105. The method of E97 wherein the cardiovascular disorder is stroke.
E106. The method of any of E1-94 wherein the patient is suffering from salt-sensitive hypertension.
E107. The method of any of E1-94 wherein the patient is suffering from congestive heart failure.
E108. The method of any of E1-94 wherein the patient is suffering from cardiac hypertrophy.
E109. The method of any of E1-94 wherein the patient has suffered a heart attack.
E110. The method of any of E1-94 wherein the patient has suffered a stroke.
E111. The method of any of E1-94 wherein the patient is salt sensitive.
E112. The method of any of E1-94 wherein the patient is suffering from hypertension.
E113. The method of any of E1-94 wherein the disorder is a renal disorder.
E114. The method of E113 wherein the renal disorder is selected from chronic renal failure or acute renal failure.
E115. The method of any of E1-94 wherein the disorder is associated with ascites.
E116. The method of any of E1-94 wherein the disorder is cirrhosis.
E117. The method of any of E1-94 wherein the patient is suffering from alcoholism.
E118. The method of any of E1-94 wherein the disorder hepatitis.
E119. The method of E118 wherein the hepatitis is chronic hepatitis.
E120. The method of E118 wherein the hepatitis is severe alcoholic hepatitis without cirrhosis.
E121. The method of any of E1-94 wherein the disorder is Budd-Chiari syndrome.
E122. The method of any of E1-94 wherein the disorder is constrictive pericarditis.
E123. A pharmaceutical composition comprising: a first agent that is an anti-hypertensive agent and a second agent selected from: a) an agent that reduces sodium absorption in the intestine; b) an agent that increases anion secretion in the intestine; or c) an agent that both reduces sodium absorption in the intestine and increases anion secretion in the intestine.
E124. The pharmaceutical composition of E123 wherein the second agent reduces sodium absorption in the intestine.
E125. The pharmaceutical composition of E123 wherein the second agent increases anion secretion in the intestine.
E126. The pharmaceutical composition of E123 wherein the second agent both reduces sodium absorption in the intestine and increases anion secretion in the intestine.
E127. The pharmaceutical composition of E123 wherein the second agent is selected from: a) a guanylate cyclase receptor C agonist, b) a soluble guanylate cyclase modulator, c) a prostanoid, d) a chloride channel activator, e) a 5HT4 agonist, f) a cyclic nucleotide, g) a sodium transport inhibitor, h) a laxative, i) a cystic fibrosis transmembrane conductance regulator (CTFR) modulator, j) an agent that affects cAMP levels, k) a phosphodiesterase inhibitor, 1) a renin inhibitor, m) an aldosterone antagonist, n) potassium, and o) a polymer resin.
E128. The pharmaceutical composition of E123 wherein the second agent is a guanylate cyclase receptor C agonist.
E129. The pharmaceutical composition of E123 wherein the second agent is a soluble guanylate cyclase modulator.
E130. The pharmaceutical composition of E123 wherein the second agent is a prostanoid.
E131. The pharmaceutical composition of E123 wherein the second agent is a chloride channel activator.
E132. The pharmaceutical composition of E123 wherein the second agent is a 5HT4 agonist.
E133. The pharmaceutical composition of E123 wherein the second agent is a cyclic nucleotide.
E134. The pharmaceutical composition of E123 wherein the second agent is a sodium transport inhibitor.
E135. The pharmaceutical composition of E123 wherein the second agent is a laxative.
E136. The pharmaceutical composition of E123 wherein the second agent is a cystic fibrosis transmembrane conductance regulator (CTFR) modulator.
E137. The pharmaceutical composition of E123 wherein the second agent is an agent that alters cAMP level.
E138. The pharmaceutical composition of E137 wherein the second agent increases cAMP level.
E139. The pharmaceutical composition of E123 wherein the second agent is a phosphodiesterase inhibitor.
E140. The pharmaceutical composition of E123 wherein the second agent is a renin inhibitor.
E141. The pharmaceutical composition of E123 wherein the second agent is an aldosterone antagonist.
E142. The pharmaceutical composition of E123 wherein the second agent is potassium.
E143. The pharmaceutical composition of E123 wherein the second agent is a polymer resin.
E144. A pharmaceutical composition comprising: a first agent that is an anti-diabetic agent and a second agent selected from: a) an agent that reduces sodium absorption in the intestine; b) an agent that increases anion secretion in the intestine; or c) an agent that both reduces sodium absorption in the intestine and increases anion secretion in the intestine.
E145. The pharmaceutical composition of E144 wherein the second agent reduces sodium absorption in the intestine.
E146. The pharmaceutical composition of E144 wherein the second agent increases anion secretion in the intestine.
E147. The pharmaceutical composition of E144 wherein the second agent both reduces sodium absorption in the intestine and increases anion secretion in the intestine.
E148. The pharmaceutical composition of E144 wherein the second agent is selected from: a) a guanylate cyclase receptor C agonist, b) a soluble guanylate cyclase modulator, c) a prostanoid, d) a chloride channel activator, e) a 5HT4 agonist, f) a cyclic nucleotide, g) a sodium transport inhibitor, h) a laxative, i) a cystic fibrosis transmembrane conductance regulator (CTFR) modulator, j) an agent that affects cAMP levels, k) a phosphodiesterase inhibitor, 1) a renin inhibitor, m) an aldosterone antagonist, n) potassium, and o) a polymer resin.
E149. The pharmaceutical composition of E148 wherein the second agent is a guanylate cyclase receptor C agonist.
E150. The pharmaceutical composition of E148 wherein the second agent is a soluble guanylate cyclase modulator.
E151. The pharmaceutical composition of E148 wherein the second agent is a prostanoid.
E152. The pharmaceutical composition of E148 wherein the second agent is a chloride channel activator.
E153. The pharmaceutical composition of E148 wherein the second agent is a 5HT4 agonist.
E154. The pharmaceutical composition of E148 wherein the second agent is a cyclic nucleotide.
E155. The pharmaceutical composition of E148 wherein the second agent is a sodium transport inhibitor.
E156. The pharmaceutical composition of E148 wherein the second agent is a laxative.
E157. The pharmaceutical composition of E148 wherein the second agent is a cystic fibrosis transmembrane conductance regulator (CTFR) modulator.
E158. The pharmaceutical composition of E148 wherein the second agent is an agent that affects cAMP level.
E159. The pharmaceutical composition of E158 wherein the second agent increases cAMP level.
E160. The pharmaceutical composition of E148 wherein the second agent is a phosphodiesterase inhibitor.
E161. The pharmaceutical composition of E148 wherein the second agent is a renin inhibitor.
E162. The pharmaceutical composition of E148 wherein the second agent is an aldosterone antagonist.
E163. The pharmaceutical composition of E148 wherein the second agent is potassium.
E164. The pharmaceutical composition of E148 wherein the second agent is a polymer resin.
E165. A pharmaceutical composition comprising: a first agent that is an anti-obesity agent and a second agent selected from: a) an agent that reduces sodium absorption in the intestine; b) an agent that increases anion secretion in the intestine; or c) an agent that both reduces sodium absorption in the intestine and increases anion secretion in the intestine.
E166. The pharmaceutical composition of E165 wherein the second agent reduces sodium absorption in the intestine.
E167. The pharmaceutical composition of E165 wherein the second agent increases anion secretion in the intestine.
E168. The pharmaceutical composition of E165 wherein the second agent both reduces sodium absorption in the intestine and increases anion secretion in the intestine.
E169. The pharmaceutical composition of E165 wherein the second agent is selected from: a) a guanylate cyclase receptor C agonist, b) a soluble guanylate cyclase modulator, c) a prostanoid, d) a chloride channel activator, e) a 5HT4 agonist, f) a cyclic nucleotide, g) a sodium transport inhibitor, h) a laxative, i) a cystic fibrosis transmembrane conductance regulator (CTFR) modulator, j) an agent that affects cAMP levels, k) a phosphodiesterase inhibitor, 1) a renin inhibitor, m) an aldosterone antagonist, n) potassium, and o) a polymer resin.
E170. The pharmaceutical composition of E169 wherein the second agent is a guanylate cyclase receptor C agonist.
E171. The pharmaceutical composition of E169 wherein the second agent is a soluble guanylate cyclase modulator.
E172. The pharmaceutical composition of E169 wherein the second agent is a prostanoid.
E173. The pharmaceutical composition of E169 wherein the second agent is a chloride channel activator.
E174. The pharmaceutical composition of E169 wherein the second agent is a 5HT4 agonist.
E175. The pharmaceutical composition of E169 wherein the second agent is a cyclic nucleotide.
E176. The pharmaceutical composition of E169 wherein the second agent is a sodium transport inhibitor.
E177. The pharmaceutical composition of E169 wherein the second agent is a laxative.
E178. The pharmaceutical composition of E169 wherein the second agent is a cystic fibrosis transmembrane conductance regulator (CTFR) modulator.
E179. The pharmaceutical composition of E169 wherein the second agent is an agent that affects cAMP level.
E180. The pharmaceutical composition of E179 wherein the second agent increases cAMP level.
E 181. The pharmaceutical composition of E 169 wherein the second agent is a phosphodiesterase inhibitor.
E182. The pharmaceutical composition of E169 wherein the second agent is a renin inhibitor.
E183. The pharmaceutical composition of E169 wherein the second agent is an aldosterone antagonist.
E184. The pharmaceutical composition of E169 wherein the second agent is potassium.
E185. The pharmaceutical composition of E169 wherein the second agent is a polymer resin.
E186. A pharmaceutical composition comprising: potassium or a salt thereof and a second agent selected from: a) an agent that reduces sodium absorption in the intestine; b) an agent that increases anion secretion in the intestine; or c) an agent that both reduces sodium absorption in the intestine and increases anion secretion in the intestine.
E187. The pharmaceutical composition of E186 wherein the second agent reduces sodium absorption in the intestine.
E188. The pharmaceutical composition of E186 wherein the second agent increases anion secretion in the intestine.
E189. The pharmaceutical composition of E186 wherein the second agent both reduces sodium absorption in the intestine and increases anion secretion in the intestine.
E190. The pharmaceutical composition of E186 wherein the second agent is selected from: a) a guanylate cyclase receptor C agonist, b) a soluble guanylate cyclase modulator, c) a prostanoid, d) a chloride channel activator, e) a 5HT4 agonist, f) a cyclic nucleotide, g) a sodium transport inhibitor, h) a laxative, i) a cystic fibrosis transmembrane conductance regulator (CTFR) modulator, j) an agent that affects cAMP levels, k) a phosphodiesterase inhibitor, 1) a renin inhibitor, m) an aldosterone antagonist, n) potassium, and o) a polymer resin.
E191. The pharmaceutical composition of E190 wherein the second agent is a guanylate cyclase receptor C agonist.
E192. The pharmaceutical composition of E190 wherein the second agent is a soluble guanylate cyclase modulator.
E193. The pharmaceutical composition of E190 wherein the second agent is a prostanoid.
E194. The pharmaceutical composition of E190 wherein the second agent is a chloride channel activator.
E195. The pharmaceutical composition of E190 wherein the second agent is a 5HT4 agonist.
E196. The pharmaceutical composition of E190 wherein the second agent is a cyclic nucleotide.
E197. The pharmaceutical composition of E190 wherein the second agent is a sodium transport inhibitor.
E198. The pharmaceutical composition of E190 wherein the second agent is a laxative.
E199. The pharmaceutical composition of E190 wherein the second agent is a cystic fibrosis transmembrane conductance regulator (CTFR) modulator.
E200. The pharmaceutical composition of E190 wherein the second agent is an agent that affects cAMP level.
E201. The pharmaceutical composition of E200 wherein the second agent increases cAMP level.
E202. The pharmaceutical composition of E190 wherein the second agent is a phosphodiesterase inhibitor.
E203. The pharmaceutical composition of E190 wherein the second agent is a renin inhibitor.
E204. The pharmaceutical composition of E190 wherein the second agent is an aldosterone antagonist.
E205. The pharmaceutical composition of E190 wherein the second agent is potassium.
E206. The pharmaceutical composition of E190 wherein the second agent is a polymer resin.
E207. A pharmaceutical composition comprising: a first agent that is a PDE inhibitor and a second agent selected from: a) an agent that reduces sodium absorption in the intestine; b) an agent that increases anion secretion in the intestine; or c) an agent that both reduces sodium absorption in the intestine and increases anion secretion in the intestine.
E208. The pharmaceutical composition of E207 wherein the second agent reduces sodium absorption in the intestine.
E209. The pharmaceutical composition of E207 wherein the second agent increases anion secretion in the intestine.
E210. The pharmaceutical composition of E207 wherein the second agent both reduces sodium absorption in the intestine and increases anion secretion in the intestine.
E211. The pharmaceutical composition of E207 wherein the second agent is selected from: a) a guanylate cyclase receptor C agonist, b) a soluble guanylate cyclase modulator, c) a prostanoid, d) a chloride channel activator, e) a 5HT4 agonist, f) a cyclic nucleotide, g) a sodium transport inhibitor, h) a laxative, i) a cystic fibrosis transmembrane conductance regulator (CTFR) modulator, j) an agent that affects cAMP levels, k) a phosphodiesterase inhibitor, 1) a renin inhibitor, m) an aldosterone antagonist, n) potassium, and o) a polymer resin.
E212. The pharmaceutical composition of E211 wherein the second agent is a guanylate cyclase receptor C agonist.
E213. The pharmaceutical composition of E211 wherein the second agent is a soluble guanylate cyclase modulator.
E214. The pharmaceutical composition of E211 wherein the second agent is a prostanoid.
E215. The pharmaceutical composition of E211 wherein the second agent is a chloride channel activator.
E216. The pharmaceutical composition of E211 wherein the second agent is a 5HT4 agonist.
E217. The pharmaceutical composition of E211 wherein the second agent is a cyclic nucleotide.
E218. The pharmaceutical composition of E211 wherein the second agent is a sodium transport inhibitor.
E219. The pharmaceutical composition of E211 wherein the second agent is a laxative.
E220. The pharmaceutical composition of E211 wherein the second agent is a cystic fibrosis transmembrane conductance regulator (CTFR) modulator.
E221. The pharmaceutical composition of E211 wherein the second agent is an agent that affects cAMP level.
E222. The pharmaceutical composition of E221 wherein the second agent increases cAMP level.
E223. The pharmaceutical composition of E211 wherein the second agent is a phosphodiesterase inhibitor.
E224. The pharmaceutical composition of E211 wherein the second agent is a renin inhibitor.
E225. The pharmaceutical composition of E211 wherein the second agent is an aldosterone antagonist.
E226. The pharmaceutical composition of E211 wherein the second agent is potassium.
E227. The pharmaceutical composition of E211 wherein the second agent is a polymer resin.
E228. The method of any of E207-227 wherein the PDE inhibitor is a PDE5-specific PDE inhibitor.
E229. The method of any of E207-227 wherein the PDE inhibitor is a cGMP-specific PDE inhibitor.
E230. The method of any of E207-227 wherein the PDE inhibitor is a cAMP-specific PDE inhibitor.
E231. A pharmaceutical composition comprising: a first agent that is a polymer resin and a second agent selected from: a) an agent that reduces sodium absorption in the intestine; b) an agent that increases anion secretion in the intestine; or c) an agent that both reduces sodium absorption in the intestine and increases anion secretion in the intestine.
E232. The pharmaceutical composition of E231 wherein the second agent reduces sodium absorption in the intestine.
E233. The pharmaceutical composition of E231 wherein the second agent increases anion secretion in the intestine.
E234. The pharmaceutical composition of E231 wherein the second agent both reduces sodium absorption in the intestine and increases anion secretion in the intestine.
E235. The pharmaceutical composition of E231 wherein the second agent is selected from: a) a guanylate cyclase receptor C agonist, b) a soluble guanylate cyclase modulator, c) a prostanoid, d) a chloride channel activator, e) a 5HT4 agonist, f) a cyclic nucleotide, g) a sodium transport inhibitor, h) a laxative, i) a cystic fibrosis transmembrane conductance regulator (CTFR) modulator, j) an agent that affects cAMP levels, k) a phosphodiesterase inhibitor, 1) a renin inhibitor, m) an aldosterone antagonist, n) potassium, and o) a polymer resin.
E236. The pharmaceutical composition of E235 wherein the second agent is a guanylate cyclase receptor C agonist.
E237. The pharmaceutical composition of E235 wherein the second agent is a soluble guanylate cyclase modulator.
E238. The pharmaceutical composition of E235 wherein the second agent is a prostanoid.
E239. The pharmaceutical composition of E235 wherein the second agent is a chloride channel activator.
E240. The pharmaceutical composition of E235 wherein the second agent is a 5HT4 agonist.
E241. The pharmaceutical composition of E235 wherein the second agent is a cyclic nucleotide.
E242. The pharmaceutical composition of E235 wherein the second agent is a sodium transport inhibitor.
E243. The pharmaceutical composition of E235 wherein the second agent is a laxative.
E244. The pharmaceutical composition of E235 wherein the second agent is a cystic fibrosis transmembrane conductance regulator (CTFR) modulator.
E245. The pharmaceutical composition of E235 wherein the second agent is an agent that affects cAMP level.
E246. The pharmaceutical composition of E235 wherein the second agent is a sodium transport inhibitor.
E247. The pharmaceutical composition of E235 wherein the second agent is a phosphodiesterase inhibitor.
E248. The pharmaceutical composition of E235 wherein the second agent is a renin inhibitor.
E249. The pharmaceutical composition of E235 wherein the second agent is an aldosterone antagonist.
E250. The pharmaceutical composition of E235 wherein the second agent is potassium.
E251. The pharmaceutical composition of any of E231-250 wherein the polymer resin is psyllium.
E252. The pharmaceutical composition of any of E235-250 wherein the polymer resin is a nonabsorbed polymer resin.
E253. The pharmaceutical composition of E252 wherein the nonabsorbed polymer resin is selected from hyaluronic acid, polycarbophil calcium, polyvinyl acetate, and polyvinyl pyrrolidine.
E254. The pharmaceutical composition of any of E231-250 wherein the polymer resin is a lipid lowering polymer.
E255. The pharmaceutical composition of E254 wherein the lipid lowering polymer is selected from: cholestyramine, colesevelam or sevalmer.
E256. A pharmaceutical composition comprising: a first agent that is an anti-hypertensive agent. and a second agent selected from: a) an agent that reduces sodium absorption in the intestine; b) an agent that increases anion secretion in the intestine; or c) an agent that both reduces sodium absorption in the intestine and increases anion secretion in the intestine.
E257. The pharmaceutical composition of E256 wherein the second agent reduces sodium absorption in the intestine.
E258. The pharmaceutical composition of E256 wherein the second agent increases anion secretion in the intestine.
E259. The pharmaceutical composition of E256 wherein the second agent both reduces sodium absorption in the intestine and increases anion secretion in the intestine.
E260. The pharmaceutical composition of E256 wherein the second agent is selected from: a) a guanylate cyclase receptor C agonist, b) a soluble guanylate cyclase modulator, c) a prostanoid, d) a chloride channel activator, e) a 5HT4 agonist, f) a cyclic nucleotide, g) a sodium transport inhibitor, h) a laxative, i) a cystic fibrosis transmembrane conductance regulator (CTFR) modulator, j) an agent that affects cAMP levels, k) a phosphodiesterase inhibitor, 1) a renin inhibitor, m) an aldosterone antagonist, n) potassium, and o) a polymer resin.
E261. The pharmaceutical composition of E260 wherein the second agent is a guanylate cyclase receptor C agonist.
E262. The pharmaceutical composition of E260 wherein the second agent is a soluble guanylate cyclase modulator.
E263. The pharmaceutical composition of E260 wherein the second agent is a prostanoid.
E264. The pharmaceutical composition of E260 wherein the second agent is a chloride channel activator.
E265. The pharmaceutical composition of E260 wherein the second agent is a 5HT4 agonist.
E266. The pharmaceutical composition of E260 wherein the second agent is a cyclic nucleotide.
E267. The pharmaceutical composition of E260 wherein the second agent is a sodium transport inhibitor.
E268. The pharmaceutical composition of E260 wherein the second agent is a laxative.
E269. The pharmaceutical composition of E260 wherein the second agent is a cystic fibrosis transmembrane conductance regulator (CTFR) modulator.
E270. The pharmaceutical composition of E260 wherein the second agent is an agent that affects cAMP level.
E271. The pharmaceutical composition of E270 wherein the second agent increases cAMP level.
E272. The pharmaceutical composition of E260 wherein the second agent is a phosphodiesterase inhibitor.
E273. The pharmaceutical composition of E260 wherein the second agent is a renin inhibitor.
E274. The pharmaceutical composition of E260 wherein the second agent is an aldosterone antagonist.
E275. The pharmaceutical composition of E260 wherein the second agent is potassium.
E276. The pharmaceutical composition of E260 wherein the second agent is a polymer resin.
E277. The pharmaceutical composition of any of E256-276 wherein the antihypertensive agent is selected from: a diuretic, an inhibitor of angiotensin converting enzyme, an angiotensin II receptor antagonist, a calcium channel blocker, a beta-adrenergic antagonist, alpha-adrenergic antagonist, a renin inhibitor, and an aldosterone antagonist.
E278. The pharmaceutical composition of any of E256-276 wherein the composition comprises two or more anti-hypertensive agents wherein the two or more antihypertensive agents are independently selected from: a diuretic, an inhibitor of angiotensin converting enzyme, an angiotensin II receptor antagonist, a calcium channel blocker, a beta-adrenergic antagonist, alpha-adrenergic antagonist, a renin inhibitor, and an aldosterone antagonist.
E279. The pharmaceutical composition of E277 wherein the anti-hypertensive agent is a diuretic.
E280. The pharmaceutical composition of E279 wherein the diuretic is selected from the group consisting of: a loop diuretic, a thiazide, a potassium sparing agent, and an osmotic diuretic.
E281. The pharmaceutical composition of E279 wherein the diuretic is a loop diuretic.
E282. The pharmaceutical composition of E279 wherein the diuretic is furosemide, bumetanide, ethacrynic or torsemide.
E283. The pharmaceutical composition of E279 wherein the diuretic is a thiazide.
E284. The pharmaceutical composition of E283 wherein the thiazide is bendroflumethiazide, hydrochlorothiazide, indapamide, chlortalidone or metolazone.
E285. The pharmaceutical composition of E279 wherein the diuretic is a potassium sparing agent.
E286. The pharmaceutical composition of E285 wherein the potassium sparing agents is amiloride or triamterene.
E287. The pharmaceutical composition of E279 wherein the diuretic is an osmotic diuretic.
E288. The pharmaceutical composition of E287 wherein the osmotic diuretic is glucose or mannitol.
E289. The pharmaceutical composition of E277 wherein the antihypertensive agent is an angiotensin converting enzyme inhibitor.
E290. The pharmaceutical composition of E289 wherein the angiotensin converting enzyme inhibitor is selected from: Benazepril (Lotensin), Captopril (Capoten), Enalapril/Enalaprilat (Vasotec), Fosinopril (Monopril), Lisinopril (Zestril and Prinivil), Moexipril (Univasc), Perindopril (Aceon), Quinapril (Accupril), Ramipril (Altace), and Trandolapril (Mavik).
E291. The method of E277 wherein the antihypertensive agent is an angiotensin II receptor antagonist.
E292. The pharmaceutical composition of E291 wherein the angiotensin II receptor antagonist is selected from: Candesartan, Eprosartan, Irbesartan, Losartan, Olmesartan, Telmisartan, Valsartan.
E293. The pharmaceutical composition of E277 wherein the antihypertensive agent is a calcium channel blocker.
E294. The pharmaceutical composition of E293 wherein the calcium channel blocker is selected from: Amlodipine (Norvasc), Felodipine (Plendil), Nicardipine (Cardene), Nifedipine (Procardia, Adalat), Nimodipine (Nimotop), Nisoldipine (Sular), Nitrendipine (Cardif, Nitrepin), and Lacidipine (Motens), Lercanidipine (Zanidip), Verapamil (Calan, Isoptin), Gallopamil (D600), Diltiazem (Cardizem), and Menthol (mint oil).
E295. The pharmaceutical composition of E294 wherein the calcium channel blocker is Amlodipine.
E296. The pharmaceutical composition of E277 wherein the antihypertensive agent is a beta-adrenergic antagonist.
E297. The pharmaceutical composition of E296 wherein the beta-adrenergic antagonist is selected from: Dichloroisoprenaline, Practolol, Pronethaolol, Alprenolol, Carteolol, Levobunolol, Mepindolol, Metipranolol, Nadolol, Oxprenolol, Penbutolol, Pindolol, Propranolol, Sotalol, Timolol, Acebutolol, Atenolol, Betaxolol, Bisoprolol, Esmolol Metoprolol, Nebivolol, Carvedilol, Celiprolol, Labetalol, and Butoxamine.
E298. The pharmaceutical composition of E277 wherein the antihypertensive agent is an alpha-adrenergic antagonist.
E299. The pharmaceutical composition of E298 wherein the alpha-adrenergic antagonist is selected from: Doxazosin (Cardura), Prazosin (Minipress), Phenoxybenzamine, Phentolamine (Regitine), Tamsulosin (Flomaxtra/Flomax), Alfuzosin (Uroxatral), and Terazosin (Hytrin).
E300. The pharmaceutical composition of E277 wherein the antihypertensive agent is a renin inhibitor.
E301. The pharmaceutical composition of E300 wherein the renin inhibitor is selected from: Tekturna® (Rasilez and Aliskiren) and SPP635.
E302. The pharmaceutical composition of E277 wherein the antihypertensive agent is an aldosterone antagonist.
E303. The pharmaceutical composition of E302 wherein the aldosterone antagonist is Spironolactone, Canrenone, or Eplerenone.
E304. A pharmaceutical composition comprising: a first agent that is a lipid altering agent. and a second agent selected from: a) an agent that reduces sodium absorption in the intestine; b) an agent that increases anion secretion in the intestine; or c) an agent that both reduces sodium absorption in the intestine and increases anion secretion in the intestine.
E305. The pharmaceutical composition of E304 wherein the second agent reduces sodium absorption in the intestine.
E306. The pharmaceutical composition of E304 wherein the second agent increases anion secretion in the intestine.
E307. The pharmaceutical composition of E304 wherein the second agent both reduces sodium absorption in the intestine and increases anion secretion in the intestine.
E308. The pharmaceutical composition of E304 wherein the second agent is selected from: a) a guanylate cyclase receptor C agonist, b) a soluble guanylate cyclase modulator, c) a prostanoid, d) a chloride channel activator, e) a 5HT4 agonist, f) a cyclic nucleotide, g) a sodium transport inhibitor, h) a laxative, i) a cystic fibrosis transmembrane conductance regulator (CTFR) modulator, j) an agent that affects cAMP levels, k) a phosphodiesterase inhibitor, 1) a renin inhibitor, m) an aldosterone antagonist, n) potassium, and o) a polymer resin.
E309. The pharmaceutical composition of E308 wherein the second agent is a guanylate cyclase receptor C agonist.
E310. The pharmaceutical composition of E308 wherein the second agent is a soluble guanylate cyclase modulator.
E311. The pharmaceutical composition of E308 wherein the second agent is a prostanoid.
E312. The pharmaceutical composition of E308 wherein the second agent is a chloride channel activator.
E313. The pharmaceutical composition of E308 wherein the second agent is a 5HT4 agonist.
E314. The pharmaceutical composition of E308 wherein the second agent is a cyclic nucleotide.
E315. The pharmaceutical composition of E308 wherein the second agent is a sodium transport inhibitor.
E316. The pharmaceutical composition of E308 wherein the second agent is a laxative.
E317. The pharmaceutical composition of E308 wherein the second agent is a cystic fibrosis transmembrane conductance regulator (CTFR) modulator.
E318. The pharmaceutical composition of E308 wherein the second agent is an agent that affects cAMP level.
E319. The pharmaceutical composition of E318 wherein the second agent increases cAMP level.
E320. The pharmaceutical composition of E308 wherein the second agent is a phosphodiesterase inhibitor.
E321. The pharmaceutical composition of E308 wherein the second agent is a renin inhibitor.
E322. The pharmaceutical composition of E308 wherein the second agent is an aldosterone antagonist.
E323. The pharmaceutical composition of E308 wherein the second agent is potassium.
E324. The pharmaceutical composition of E308 wherein the second agent is a polymer resin.
E325. The pharmaceutical composition of any one of E304-324 wherein the lipid altering agent is a cholesterol lowering agent.
E326. The pharmaceutical composition of E325 wherein the cholesterol lowering agent lowers low density cholesterol.
E327. The pharmaceutical composition of any one of E304-324 wherein the lipid altering agent is selected from the group consisting of: a statin; a fibrate; niacin; a CETP inhibitor; a MTP inhibitor; a cholesterol absorption inhibitor; a squalene synthesis inhibitor; and a bile acid sequestrant.
E328. The pharmaceutical composition of E327 wherein the lipid altering agent is a statin.
E329. The pharmaceutical composition of E328 wherein the statin is chosen from simvastatin, rosuvastatin and atorvastatin.
E330. The pharmaceutical composition of E327 wherein the lipid altering agent is a cholesterol absorption inhibitor.
E331. The method of E330 wherein the cholesterol absorption inhibitor is ezetimibe.
E332. The pharmaceutical composition of E327 wherein the lipid altering agent is a bile acid sequestrant.
E333. The pharmaceutical composition of E332 wherein the bile acid sequestrant is chosen from cholestyramine, colesevelam and colestipol.
E334. The pharmaceutical composition of E327 wherein the lipid altering agent is a fibrate.
E335. The pharmaceutical composition of E334 wherein the fibrate is fenofibrate.
E336. The pharmaceutical composition of any of E132, 153, 174, 195, 216, 240, 265, and 313 wherein the 5HT4 agonist is Zelnorm.
E337. The pharmaceutical composition of any of E130, 151, 172, 193,214,238,263 and 311 wherein the prostanoid is selected from: the compound represented by CAS Registry No. 333963-40-9, the compound represented by CAS Registry No. 136790-76-6, (-)-7-[(2R,4aR,5R,7aR)-2-(1,1-difluoropentyl)-2-hydroxy-6-oxooctahydrocyclopenta[b]pyran-5-yl]heptanoic acid; and the 13, 14-dihydro-15-keto prostaglandins Edisclosed in US5284858 including 13,14-dihydro-15-keto-PGE₂ alkyl ester, 13,14-dihydro-15-keto-PGE₂ cycloalkyl ester; 13,14-dihydro-15-keto-PGE₂ hydroxy alkyl ester, 13,14-dihydro-15-keto-PGE₂ benzyl ester, 13,14-dihydro-15-keto-PGE₁ alkyl ester, 13,14-dihydro-6,15-diketo-PGE₁ alkyl ester, 13,14-dihydro-15-keto-18-methoxy-19, 20-dinor-PGE₂ or an alkyl ester thereof, 13,14-dihydro-15-keto-18-methoxy-PGE₂ or an alkyl ester thereof, 13,14-dihydro-15-keto-Δ²-PGE₂ or an alkyl ester thereof, 13,14-dihydro-15-keto-20-methoxy-Δ²-PGE₂ or an alkyl ester thereof, 13,14-dihydro-15-keto-3R,S-methyl-PGE₂ or an alkyl ester thereof, 13,14-dihydro-15-keto-3R,S-methyl-20-methoxy-PGE₂ or an alkyl ester thereof, 13, 14-dihydro-15-keto-11-dehydroxy-11R-methyl-PGE₂ or an alkyl ester thereof, 13,14-dihydro-15-keto-16R,S-fluoro-11-dehydroxy-11R-methyl-PGE₂ or an alkyl ester thereof, 13,14-dihydro-15-keto-16R,S-hydroxy-PGE₂ or an alkyl ester thereof, 13,14-dihydro-15-keto-16R,S-fluoro-PGE₂ or an alkyl ester thereof, 13,14-dihydro-15-keto-16R,S-methyl-PGE₂ or an alkyl ester thereof, 13,14-dihydro-15-keto-16,16-dimethyl-PGE₂ or an alkyl ester thereof, 13,14-dihydro-15-keto-16,16-dimethyl-20-methoxy-PGE₂ or an alkyl ester thereof, 13,14-dihydro-15-keto-17S-methyl-PGE₂ or an alkyl ester thereof, 13,14-dihydro-15-keto-19-methy-PGE₂ or an alkyl ester thereof, 13,14-dihydro-15-keto-20-isopropropylidene PGE₂ or an alkyl ester thereof, 13,14-dihydro-15-keto-20-ethyl-PGE₂ or an alkyl ester thereof, 13,14-dihydro-15-keto-20-ethyl-11-dehydroxy-11R-methyl-PGE₂ or an alkyl ester thereof, 13,14-dihydro-15-keto-20-n-propyl-PGE₂ or an alkyl ester thereof, 13,14-dihydro-15-keto-20-ethyl-PGE₁ or an alkyl ester thereof, 13,14-dihydro-6,15-diketo-16R,S-fluoro-PGE₁ or an alkyl ester thereof, 13,14-dihydro-6,15-diketo-16R,S-fluoro-11-dehydroxy-11R-methyl-PGE₁ or an alkyl ester thereof, 13,14-dihydro-6,15-diketo-16R,S-methyl-PGE₁ or an alkyl ester thereof, 13,14-dihydro-6,15-diketo-16,16-dimethyl-PGE₁ or an alkyl ester thereof, 13,14-dihydro-6,15-diketo-19-methyl-PGE₁ or an alkyl ester thereof, 13,14-dihydro-6,15-diketo-20-methyl-PGE₁ or an alkyl ester thereof, 13,14-dihydro-6,15-diketo-11-dehydroxy-11R-methyl-PGE₁ or an alkyl ester thereof, 13, 14-dihydro-6,15-diketo-11-dehydroxy-11R-hydroxymethyl PGE₁ alkyl ester, 13,14-dihydro-15-keto-20-methyl-PGE₁ or an alkyl ester thereof, 13,14-dihydro-15-keto-Δ²-PGE₁ or an alkyl ester thereof, 13,14-dihydro-15-keto-16R,S-fluoro-20-methyl-PGE₂ or an alkyl ester thereof, 13,14-dihydro-15-keto-16,16-difluoro-PGE₂ or an alkyl ester thereof, 13,14-dihydro-15-keto-5,6-dehydro-20-methoxy-PGE₂ or an alkyl ester thereof, amd 13,14-dihydro-6,15-diketo-16R,S-fluoro-PGE₁ or an alkyl ester thereof.
E338. The pharmaceutical composition of any of E130, 151, 172, 193,214,238,263 and 311 wherein the prostanoid is the free acid of the compound associated with CAS registry NO. 59122-49-5
E339. The pharmaceutical composition of any of E130, 151, 172, 193,214,238,263 and 311 wherein the prostanoid comprises a mixture of steroisomers.
E340. The pharmaceutical composition of any of E130, 151, 172, 193, 214, 238, 263 and 311 wherein only a single isomer of a prostanoid is present.
E341. The pharmaceutical composition of any of E135, 156, 177, 198, 219, 243, 268, and 316 wherein the laxative is selected from: a stimulant, a bulk-producing agent and a stool softener.
E342. The pharmaceutical composition of any of E135, 156, 177, 198, 219, 243, 268, and 316 wherein the laxative is selected from dexloxiglumide, psyllium husk, docusate sodium, bisacodyl, and phenolphthalein.
E343. The pharmaceutical composition of any of E143, 164, 185, 206, 227, 276, and 324 wherein the polymer resin is selected from psyllium, lipid lowering polymers, nonabsorbed polymer resins, and sodium binding polymers.
E344. The pharmaceutical composition of E343 wherein the polymer resin is psyllium.
E345. The pharmaceutical composition of E343 wherein the polymer resin is a lipid lowering polymer.
E346. The pharmaceutical composition of E343 wherein the polymer resin is a nonabsorbed polymer resin.
E347. The pharmaceutical composition of E343 wherein the polymer resin is a sodium binding polymer.
E348. The pharmaceutical composition of E345 wherein the lipid lowering polymer is selected from: Colesevelam, Sevalmer, Cholestyramine.
E349. The pharmaceutical composition of E346 wherein nonabsorbed polymer resin is selected from: hyaluronic acid, polycarbophil calcium, polyvinyl acetate, polyvinyl pyrrolidone, polystyrene sulfate.
E350. The pharmaceutical composition of E347 wherein the sodium-binding polymer is selected from: crosslinked polyvinylsulfamate polymer, N-(bis-phosphonic-ethyl) polyvinylamine polymer, poly-α-acrylic acid polymer, poly-α-fluoroacrylic acid polymer, polyvinylphosphoramidic polymer, polyvinylsulfamate polymer, polyvinylsulfamate/vinylsulfate copolymer, polyvinylsulfate polymer, polyvinylsulfonate polymer, polyvinylsulfonate polymer, vinylphosphonate/α-fluoroacrylic acid copolymer, vinylphosphonate/α-fluoroacrylic acid copolymer, or vinylphosphonate/acrylic acid copolymer.
E351. The pharmaceutical composition of E350 wherein the sodium-binding polymer is administered as a core-shell composition which further comprises a semi-permeable shell.
E352. The pharmaceutical composition of E351 wherein the semi-permeable shell comprises at least one of a poly-11 trimethylammonioundecylmethacrylate polymer, a styrene-vinylpyridine polymer, 11-dimethyl-aminodecylmethacrylate/laurylmethacrylate copolymer, or a polyallylamine/polystyrene sulfonate polymer.
E353. A pharmaceutical composition comprising misoprostol and psyllium.
E354. A pharmaceutical composition comprising psyllium and a peptide that activates the guanylate cyclase C receptor.
E355. The pharmaceutical composition of E354 wherein the peptide comprises an amino acid sequence selected from:
   Cys Cys Glu Tyr Cys Cys Asn Pro Ala Cys Thr Gly Cys Tyr;
   Cys Cys Glu Leu Cys Cys Asn Pro Ala Cys Thr Gly Cys Tyr;
   Cys Cys Glu Phe Cys Cys Asn Pro Ala Cys Thr Gly Cys Tyr;
   Cys Cys Glu Trp Cys Cys Asn Pro Ala Cys Thr Gly Cys Tyr;
   Cys Cys Glu Tyr Cys Cys Asn Pro Ala Cys Thr Gly Cys;
   Cys Cys Glu Leu Cys Cys Asn Pro Ala Cys Thr Gly Cys;
   Cys Cys Glu Phe Cys Cys Asn Pro Ala Cys Thr Gly Cys;
   Cys Cys Glu Trp Cys Cys Asn Pro Ala Cys Thr Gly Cys;
   d-Cys Cys Glu Tyr Cys Cys Asn Pro Ala Cys Thr Gly Cys Tyr;
   d-Cys Cys Glu Leu Cys Cys Asn Pro Ala Cys Thr Gly Cys Tyr;
   d-Cys Cys Glu Phe Cys Cys Asn Pro Ala Cys Thr Gly Cys Tyr;
   d-Cys Cys Glu Trp Cys Cys Asn Pro Ala Cys Thr Gly Cys Tyr;
   d-Cys Cys Glu Tyr Cys Cys Asn Pro Ala Cys Thr Gly Cys;
   d-Cys Cys Glu Leu Cys Cys Asn Pro Ala Cys Thr Gly Cys;
   d-Cys Cys Glu Phe Cys Cys Asn Pro Ala Cys Thr Gly Cys;
   d-Cys Cys Glu Trp Cys Cys Asn Pro Ala Cys Thr Gly Cys;
   Asn Asp Asp Cys Glu Leu Cys Val Asn Val Ala Cys Thr Gly Cys Leu;
   Asn Asp Glu Cys Glu Leu Cys Val Asn Val Ala Cys Thr Gly Cys Leu;

Phe Lys Thr Leu Arg Thr Ile Ala Asn Asp Asp Cys Glu Leu Cys Val Asn Val Ala Cys Thr Gly
Cys Leu;

Phe Lys Thr Leu Arg Thr Ile Ala Asn Asp Glu Cys Glu Leu Cys Val Asn Val Ala Cys Thr Gly
Cys Leu;

Val Tyr Ile Gln Tyr Gln Gly Phe Arg Val Gln Leu Glu Ser Met Lys Lys Leu Ser Asp Leu Glu
Ala Gln Trp Ala Pro; Ser Pro Arg Leu Gln Ala Gln Ser Leu Leu Pro Ala Val Cys His His Pro
Ala Leu Pro Gln Asp Leu Gln Pro Val Cys;

Ala Ser Gln Glu Ala Ser Ser Ile Phe Lys Thr Leu Arg Thr Ile Ala Asn Asp Asp Cys Glu Leu Cys
Val Asn Val Ala Cys Thr Gly Cys Leu;

Val Tyr Ile Gln Tyr Gln Gly Phe Arg Val Gln Leu Glu Ser Met Lys Lys Leu Ser Asp Leu Glu
Ala Gln Trp Ala Pro; Ser Pro Arg Leu Gln Ala Gln Ser Leu Leu Pro Ala Val Cys His His Pro
Ala Leu Pro Gln Asp Leu Gln Pro Val Cys; Ala Ser Gln Glu Ala Ser Ser Ile Phe Lys Thr Leu
Arg Thr Ile Ala Asn Asp Glu Cys Glu Leu Cys Val Asn Val Ala Cys Thr Gly Cys Leu;

   Pro Gly Thr Cys Glu Ile Cys Ala Tyr Ala Ala Cys Thr Gly Cys;

Val Thr Val Gln Asp Gly Asn Phe Ser Phe Ser Leu Glu Ser Val Lys Lys Leu Lys Asp Leu Gln
Glu Pro Gln Glu Pro;

Arg Val Gly Lys Leu Arg Asn Phe Ala Pro Ile Pro Gly Glu Pro Val Val Pro Ile Leu Cys Ser
Asn Pro Asn Phe Pro; Glu Glu Leu Lys Pro Leu Cys Lys Glu Pro Asn Ala Gln Glu Ile Leu Gln
Arg Leu Glu Glu Ile Ala Glu Asp Pro Gly Thr Cys Glu Ile Cys Ala Tyr Ala Ala Cys Thr Gly
Cys;

Met Pro Ser Thr Gln Tyr Ile Arg Arg Pro Ala Ser Ser Tyr Ala Ser Cys Ile Trp Cys Thr Thr Ala
Cys Ala Ser Cys His Gly Arg Thr Thr Lys Pro Ser Leu Ala Thr;

   Ala Asp Leu Cys Glu Ile Cys Ala Phe Ala Ala Cys Thr Gly Cys Leu;

Val Gln Val Glu Glu Gly Gly Phe Ser Phe Pro Leu Asp Ala Val Lys Lys Leu Glu Glu Leu Met
Gly Val Asp Met; Thr Val Lys Gln Ser Pro Arg Leu Ala Lys Thr Ser Thr Thr Ala Val Cys Thr
Asn Pro Asp Leu Pro Ala Val Phe Leu; Pro Leu Cys Lys Ser Lys Gly Ala Ala Asn Ser Phe Phe

Arg Leu Gly Phe Val Ala Ala Arg Ala Asp Leu Cys Glu Ile Cys Ala Phe Ala Ala Cys Thr Gly
Cys Leu;

   Gln Glu Glu Cys Glu Leu Cys Ile Asn Met Ala Cys Thr Gly Tyr;

Val Tyr Ile Gln Tyr Glu Gly Phe Gln Val Asn Leu Asp Ser Val Lys Lys Leu Asp Lys Leu Leu
Glu Gln Leu Arg Gly;

Phe His His Gln Met Gly Asp Gln Arg Asp Pro Ser Ile Leu Cys Ser Asp Pro Ala Leu Pro Ser
Asp Leu Gln Pro Val; and

Cys Glu Asn Ser Gln Ala Val Asn Ile Phe Arg Ala Leu Arg Tyr Ile Asn Gln Glu Glu Cys Glu
Leu Cys Ile Asn Met Ala Cys Thr Gly Tyr.

## Claims

1. Use of an agent for the preparation of a medicament for reducing the risk of or treating a disorder associated with fluid and/or salt retention in a patient, wherein the agent is selected from:
a) an agent that reduces sodium absorption in the intestine;
b) an agent that increases anion secretion in the intestine; and
c) an agent that both reduces sodium absorption in the intestine and increases anion secretion in the intestine.

2. The use of claim 1, wherein the agent is selected from: a) a guanylate cyclase receptor C agonist, b) a soluble guanylate cyclase modulator, c) a prostanoid, d) a chloride channel activator, e) a 5HT4 agonist, f) a cyclic nucleotide, g) a sodium transport inhibitor, h) a laxative, i) a cystic fibrosis transmembrane conductance regulator (CTFR) modulator, j) an agent that affects cAMP levels, k) a renin inhibitor, and 1) an aldosterone antagonist.

3. The use of claim 1 or claim 2, wherein the agent is a guanylate cyclase receptor C agonist or a soluble guanylate cyclase modulator.

4. The use of any one of claims 1 to 3, wherein the preparation of the medicament further comprises the use of an agent selected from an anti-diabetic agent, an anti-obesity agent, an anti-hypertensive agent, potassium or salt thereof, a PDE inhibitor, a polymer resin, or a lipid altering agent.

5. The use of any one of claims 1 to 4, wherein the agent comprises a peptide that activates the guanylate cyclase C receptor.

6. The use of claim 5, wherein the preparation of the medicament further comprises the use of psyllium.

7. The use of claim 5 or claim 6, wherein the peptide is selected from:
Cys Cys Glu Tyr Cys Cys Asn Pro Ala Cys Thr Gly Cys Tyr;
Cys Cys Glu Leu Cys Cys Asn Pro Ala Cys Thr Gly Cys Tyr;
Cys Cys Glu Phe Cys Cys Asn Pro Ala Cys Thr Gly Cys Tyr;
Cys Cys Glu Trp Cys Cys Asn Pro Ala Cys Thr Gly Cys Tyr;
Cys Cys Glu Tyr Cys Cys Asn Pro Ala Cys Thr Gly Cys;
Cys Cys Glu Leu Cys Cys Asn Pro Ala Cys Thr Gly Cys;
Cys Cys Glu Phe Cys Cys Asn Pro Ala Cys Thr Gly Cys;
Cys Cys Glu Trp Cys Cys Asn Pro Ala Cys Thr Gly Cys;
d-Cys Cys Glu Tyr Cys Cys Asn Pro Ala Cys Thr Gly Cys Tyr;
d-Cys Cys Glu Leu Cys Cys Asn Pro Ala Cys Thr Gly Cys Tyr;
d-Cys Cys Glu Phe Cys Cys Asn Pro Ala Cys Thr Gly Cys Tyr;
d-Cys Cys Glu Trp Cys Cys Asn Pro Ala Cys Thr Gly Cys Tyr;
d-Cys Cys Glu Tyr Cys Cys Asn Pro Ala Cys Thr Gly Cys;
d-Cys Cys Glu Leu Cys Cys Asn Pro Ala Cys Thr Gly Cys;
d-Cys Cys Glu Phe Cys Cys Asn Pro Ala Cys Thr Gly Cys;
d-Cys Cys Glu Trp Cys Cys Asn Pro Ala Cys Thr Gly Cys;
Asn Asp Asp Cys Glu Leu Cys Val Asn Val Ala Cys Thr Gly Cys Leu;
Asn Asp Glu Cys Glu Leu Cys Val Asn Val Ala Cys Thr Gly Cys Leu; 
Phe Lys Thr Leu Arg Thr Ile Ala Asn Asp Asp Cys Glu Leu Cys Val Asn Val Ala Cys
Thr Gly Cys Leu;
 
Phe Lys Thr Leu Arg Thr Ile Ala Asn Asp Glu Cys Glu Leu Cys Val Asn Val Ala Cys
Thr Gly Cys Leu;
 
Val Tyr Ile Gln Tyr Gln Gly Phe Arg Val Gln Leu Glu Ser Met Lys Lys Leu Ser Asp Leu
Glu Ala Gln Trp Ala Pro;
 
Ser Pro Arg Leu Gln Ala Gln Ser Leu Leu Pro Ala Val Cys His His Pro Ala Leu Pro Gln
Asp Leu Gln Pro Val Cys;
 
Ala Ser Gln Glu Ala Ser Ser Ile Phe Lys Thr Leu Arg Thr Ile Ala Asn Asp Asp Cys Glu
Leu Cys Val Asn Val Ala Cys Thr Gly Cys Leu;
 
Val Tyr Ile Gln Tyr Gln Gly Phe Arg Val Gln Leu Glu Ser Met Lys Lys Leu Ser Asp Leu
Glu Ala Gln Trp Ala Pro;
 
Ser Pro Arg Leu Gln Ala Gln Ser Leu Leu Pro Ala Val Cys His His Pro Ala Leu Pro Gln
Asp Leu Gln Pro Val Cys;
 
Ala Ser Gln Glu Ala Ser Ser Ile Phe Lys Thr Leu Arg Thr Ile Ala Asn Asp Glu Cys Glu
Leu Cys Val Asn Val Ala Cys Thr Gly Cys Leu;
Pro Gly Thr Cys Glu Ile Cys Ala Tyr Ala Ala Cys Thr Gly Cys; 
Val Thr Val Gln Asp Gly Asn Phe Ser Phe Ser Leu Glu Ser Val Lys Lys Leu Lys Asp
Leu Gln Glu Pro Gln Glu Pro;
 
Arg Val Gly Lys Leu Arg Asn Phe Ala Pro Ile Pro Gly Glu Pro Val Val Pro Ile Leu Cys
Ser Asn Pro Asn Phe Pro;
 
Glu Glu Leu Lys Pro Leu Cys Lys Glu Pro Asn Ala Gln Glu Ile Leu Gln Arg Leu Glu
Glu Ile Ala Glu Asp Pro Gly Thr Cys Glu Ile Cys Ala Tyr Ala Ala Cys Thr Gly Cys;
 
Met Pro Ser Thr Gln Tyr Ile Arg Arg Pro Ala Ser Ser Tyr Ala Ser Cys Ile Trp Cys Thr
Thr Ala Cys Ala Ser Cys His Gly Arg Thr Thr Lys Pro Ser Leu Ala Thr;
Ala Asp Leu Cys Glu Ile Cys Ala Phe Ala Ala Cys Thr Gly Cys Leu; 
Val Gln Val Glu Glu Gly Gly Phe Ser Phe Pro Leu Asp Ala Val Lys Lys Leu Glu Glu
Leu Met Gly Val Asp Met;
 
Thr Val Lys Gln Ser Pro Arg Leu Ala Lys Thr Ser Thr Thr Ala Val Cys Thr Asn Pro Asp
Leu Pro Ala Val Phe Leu;
 
Pro Leu Cys Lys Ser Lys Gly Ala Ala Asn Ser Phe Phe Arg Leu Gly Phe Val Ala Ala
Arg Ala Asp Leu Cys Glu Ile Cys Ala Phe Ala Ala Cys Thr Gly Cys Leu;
Gln Glu Glu Cys Glu Leu Cys Ile Asn Met Ala Cys Thr Gly Tyr;

Val Tyr Ile Gln Tyr Glu Gly Phe Gln Val Asn Leu Asp Ser Val Lys Lys Leu Asp Lys
Leu Leu Glu Gln Leu Arg Gly;

Phe His His Gln Met Gly Asp Gln Arg Asp Pro Ser Ile Leu Cys Ser Asp Pro Ala Leu Pro
Ser Asp Leu Gln Pro Val; and

Cys Glu Asn Ser Gln Ala Val Asn Ile Phe Arg Ala Leu Arg Tyr Ile Asn Gln Glu Glu Cys
Glu Leu Cys Ile Asn Met Ala Cys Thr Gly Tyr.

8. The use of any one of claims 5 to 7, wherein the peptide comprises the amino acid sequence Cys Cys Glu Tyr Cys Cys Asn Pro Ala Cys Thr Gly Cys Tyr.

9. The use of any one of claims 5 to 8, wherein the peptide consists of the amino acid sequence Cys Cys Glu Tyr Cys Cys Asn Pro Ala Cys Thr Gly Cys Tyr.

10. The use of any one of claims 1 to 9, wherein the disorder is a cardiovascular disorder.

11. The use of claim 10, wherein the cardiovascular disorder is selected from cardiomyopathy, hypertension, congestive heart failure, cardiac hypertrophy, heart attack, and stroke.

12. A pharmaceutical composition comprising:
a first agent selected from: an anti-hypertensive agent; an anti-diabetic agent; an anti-obesity agent; potassium or a salt thereof; a PDE inhibitor; a polymer resin; and a lipid altering agent; and
a second agent selected from: a) an agent that reduces sodium absorption in the intestine; b) an agent that increases anion secretion in the intestine; and c) an agent that both reduces sodium absorption in the intestine and increases anion secretion in the intestine.

13. The pharmaceutical composition of claim 12, wherein the second agent is selected from: a) a guanylate cyclase receptor C agonist, b) a soluble guanylate cyclase modulator, c) a prostanoid, d) a chloride channel activator, e) a 5HT4 agonist, f) a cyclic nucleotide, g) a sodium transport inhibitor, h) a laxative, i) a cystic fibrosis transmembrane conductance regulator (CTFR) modulator, j) an agent that affects cAMP levels, k) a phosphodiesterase inhibitor, 1) a renin inhibitor, m) an aldosterone antagonist, n) potassium, and o) a polymer resin.

14. The pharmaceutical composition of claim 12 or claim 13, wherein the second agent is a guanylate cyclase receptor C agonist or a soluble guanylate cyclase modulator.

15. The pharmaceutical composition of any one of claims 12 to 14, wherein the second agent comprises a peptide that activates the guanylate cyclase C receptor.

16. The pharmaceutical composition of claim 15, further comprising psyllium.

17. The pharmaceutical composition of claim 15 or claim 16, wherein the peptide comprises an amino acid sequence selected from:
Cys Cys Glu Tyr Cys Cys Asn Pro Ala Cys Thr Gly Cys Tyr;
Cys Cys Glu Leu Cys Cys Asn Pro Ala Cys Thr Gly Cys Tyr;
Cys Cys Glu Phe Cys Cys Asn Pro Ala Cys Thr Gly Cys Tyr;
Cys Cys Glu Trp Cys Cys Asn Pro Ala Cys Thr Gly Cys Tyr;
Cys Cys Glu Tyr Cys Cys Asn Pro Ala Cys Thr Gly Cys;
Cys Cys Glu Leu Cys Cys Asn Pro Ala Cys Thr Gly Cys;
Cys Cys Glu Phe Cys Cys Asn Pro Ala Cys Thr Gly Cys;
Cys Cys Glu Trp Cys Cys Asn Pro Ala Cys Thr Gly Cys;
d-Cys Cys Glu Tyr Cys Cys Asn Pro Ala Cys Thr Gly Cys Tyr;
d-Cys Cys Glu Leu Cys Cys Asn Pro Ala Cys Thr Gly Cys Tyr;
d-Cys Cys Glu Phe Cys Cys Asn Pro Ala Cys Thr Gly Cys Tyr;
d-Cys Cys Glu Trp Cys Cys Asn Pro Ala Cys Thr Gly Cys Tyr;
d-Cys Cys Glu Tyr Cys Cys Asn Pro Ala Cys Thr Gly Cys;
d-Cys Cys Glu Leu Cys Cys Asn Pro Ala Cys Thr Gly Cys;
d-Cys Cys Glu Phe Cys Cys Asn Pro Ala Cys Thr Gly Cys;
d-Cys Cys Glu Trp Cys Cys Asn Pro Ala Cys Thr Gly Cys;
Asn Asp Asp Cys Glu Leu Cys Val Asn Val Ala Cys Thr Gly Cys Leu;
Asn Asp Glu Cys Glu Leu Cys Val Asn Val Ala Cys Thr Gly Cys Leu; 
Phe Lys Thr Leu Arg Thr Ile Ala Asn Asp Asp Cys Glu Leu Cys Val Asn Val Ala Cys
Thr Gly Cys Leu;
 
Phe Lys Thr Leu Arg Thr Ile Ala Asn Asp Glu Cys Glu Leu Cys Val Asn Val Ala Cys
Thr Gly Cys Leu;
 
Val Tyr Ile Gln Tyr Gln Gly Phe Arg Val Gln Leu Glu Ser Met Lys Lys Leu Ser Asp Leu
Glu Ala Gln Trp Ala Pro;
 
Ser Pro Arg Leu Gln Ala Gln Ser Leu Leu Pro Ala Val Cys His His Pro Ala Leu Pro Gln
Asp Leu Gln Pro Val Cys;
 
Ala Ser Gln Glu Ala Ser Ser Ile Phe Lys Thr Leu Arg Thr Ile Ala Asn Asp Asp Cys Glu
Leu Cys Val Asn Val Ala Cys Thr Gly Cys Leu;
 
Val Tyr Ile Gln Tyr Gln Gly Phe Arg Val Gln Leu Glu Ser Met Lys Lys Leu Ser Asp Leu
Glu Ala Gln Trp Ala Pro;
 
Ser Pro Arg Leu Gln Ala Gln Ser Leu Leu Pro Ala Val Cys His His Pro Ala Leu Pro Gln
Asp Leu Gln Pro Val Cys;
 
Ala Ser Gln Glu Ala Ser Ser Ile Phe Lys Thr Leu Arg Thr Ile Ala Asn Asp Glu Cys Glu
Leu Cys Val Asn Val Ala Cys Thr Gly Cys Leu;
Pro Gly Thr Cys Glu Ile Cys Ala Tyr Ala Ala Cys Thr Gly Cys; 
Val Thr Val Gln Asp Gly Asn Phe Ser Phe Ser Leu Glu Ser Val Lys Lys Leu Lys Asp
Leu Gln Glu Pro Gln Glu Pro;
 
Arg Val Gly Lys Leu Arg Asn Phe Ala Pro Ile Pro Gly Glu Pro Val Val Pro Ile Leu Cys
Ser Asn Pro Asn Phe Pro;
 
Glu Glu Leu Lys Pro Leu Cys Lys Glu Pro Asn Ala Gln Glu Ile Leu Gln Arg Leu Glu
Glu Ile Ala Glu Asp Pro Gly Thr Cys Glu Ile Cys Ala Tyr Ala Ala Cys Thr Gly Cys;
 
Met Pro Ser Thr Gln Tyr Ile Arg Arg Pro Ala Ser Ser Tyr Ala Ser Cys Ile Trp Cys Thr
Thr Ala Cys Ala Ser Cys His Gly Arg Thr Thr Lys Pro Ser Leu Ala Thr;
Ala Asp Leu Cys Glu Ile Cys Ala Phe Ala Ala Cys Thr Gly Cys Leu; 
Val Gln Val Glu Glu Gly Gly Phe Ser Phe Pro Leu Asp Ala Val Lys Lys Leu Glu Glu
Leu Met Gly Val Asp Met;
 
Thr Val Lys Gln Ser Pro Arg Leu Ala Lys Thr Ser Thr Thr Ala Val Cys Thr Asn Pro Asp
Leu Pro Ala Val Phe Leu;
 
Pro Leu Cys Lys Ser Lys Gly Ala Ala Asn Ser Phe Phe Arg Leu Gly Phe Val Ala Ala
Arg Ala Asp Leu Cys Glu Ile Cys Ala Phe Ala Ala Cys Thr Gly Cys Leu;
Gln Glu Glu Cys Glu Leu Cys Ile Asn Met Ala Cys Thr Gly Tyr;

Val Tyr Ile Gln Tyr Glu Gly Phe Gln Val Asn Leu Asp Ser Val Lys Lys Leu Asp Lys
Leu Leu Glu Gln Leu Arg Gly;

Phe His His Gln Met Gly Asp Gln Arg Asp Pro Ser Ile Leu Cys Ser Asp Pro Ala Leu Pro
Ser Asp Leu Gln Pro Val; and

Cys Glu Asn Ser Gln Ala Val Asn Ile Phe Arg Ala Leu Arg Tyr Ile Asn Gln Glu Glu Cys
Glu Leu Cys Ile Asn Met Ala Cys Thr Gly Tyr.

18. The pharmaceutical composition of any one of claims 15 to 17, wherein the peptide comprises the amino acid sequence Cys Cys Glu Tyr Cys Cys Asn Pro Ala Cys Thr Gly Cys Tyr.

19. The pharmaceutical composition of any one of claims 15 to 18, wherein the peptide consists of the amino acid sequence Cys Cys Glu Tyr Cys Cys Asn Pro Ala Cys Thr Gly Cys Tyr.
